# EUROPEAN PATENT APPLICATION

(11) **EP 3 858 993 A1**
(43) Date of publication of application: **04.08.2021**
(21) Application number: 21151113.4
(22) Date of filing: 07.10.2016
(51) Int. Cl.: C12N 15/113, A61K 31/7125, C07H 21/04

(54) **COMPOSITIONS AND METHODS FOR TREATING DUCHENNE MUSCULAR DYSTROPHY AND RELATED DISORDERS**

(30) Priority: 09.10.2015 US 201562239812 P
(62) Divisional of application: 16854468.2
(71) Applicant: Sarepta Therapeutics, Inc., Cambridge, MA 02142 (US)
(72) Inventor: DICKSON, George, London, NW7 4NR (GB)
(74) Representative: D Young & Co LLP

(57) **Abstract**

The present disclosure relates to compositions and methods for the treatment of Duchenne muscular dystrophy and related disorders. Modified antisense oligomers are disclosed for the treatment of Duchenne muscular dystrophy and related disorders.

## Description

### BACKGROUND

### Field of the Disclosure

The present invention relates to compositions and methods for the treatment of Duchenne muscular dystrophy and related disorders.

### Description of the Related Art

Duchenne muscular dystrophy (DMD) is caused by a defect in the expression of the protein dystrophin. The gene encoding the protein contains 79 exons spread out over more than 2 million nucleotides of DNA. Any exonic mutation that changes the reading frame of the exon, or introduces a stop codon, or is characterized by removal of an entire out of frame exon or exons, or duplications of one or more exons, has the potential to disrupt production of functional dystrophin, resulting in DMD.

Disease onset can be documented at birth with elevated creatine kinase levels, and significant motor deficits may be present in the first year of life. By the age of seven or eight, most patients with DMD have an increasingly labored gait and are losing the ability to rise from the floor and climb stairs; by ages 10 to 14, most are wheelchair-dependent. DMD is uniformly fatal; affected individuals typically die of respiratory and/or cardiac failure in their late teens or early 20s. The continuous progression of DMD allows for therapeutic intervention at all stages of the disease; however, treatment is currently limited to glucocorticoids, which are associated with numerous side effects including weight gain, behavioral changes, pubertal changes, osteoporosis, Cushingoid facies, growth inhibition, and cataracts.

A less severe form of muscular dystrophy, Becker muscular dystrophy (BMD), a related disorder as described herein, has been found to arise where a mutation, typically a deletion of one or more exons, results in a correct reading frame along the entire dystrophin transcript, such that translation of mRNA into protein is not prematurely terminated. If the joining of the upstream and downstream exons in the processing of a mutated dystrophin pre-mRNA maintains the correct reading frame of the gene, the result is an mRNA coding for a protein with a short internal deletion that retains some activity, resulting in a Becker phenotype.

For many years it has been known that deletions of an exon or exons which do not alter the reading frame of a dystrophin protein would give rise to a BMD phenotype, whereas an exon deletion that causes a frame-shift will give rise to DMD (Monaco, Bertelson *et al.* 1988). In general, dystrophin mutations including point mutations and exon deletions that change the reading frame and thus interrupt proper protein translation result in DMD. It should also be noted that some BMD and DMD patients have exon deletions covering multiple exons.

Recent clinical trials testing the safety and efficacy of splice switching oligonucleotides (SSOs) for the treatment of DMD are based on SSO technology to induce alternative splicing of pre-mRNAs by steric blockade of the spliceosome (Cirak *et al.,* 2011; Goemans *et al.,* 2011; Kinali *et al.,* 2009; van Deutekom *et al.,* 2007). However, despite these successes, the pharmacological options available for treating DMD are limited.

Thus, a strong need remains for improved therapeutic approaches for the treatment of DMD.

### SUMMARY

The present disclosure is based, at least in part, on the surprising findings that systemic treatment of mdx mice (a murine model of Duchenne muscular dystrophy) with a dystrophin therapeutic in conjunction with a myostatin therapeutic increased, among other things, muscle grip strength in the mice. In addition to increased muscle grip strength, this combined therapeutic approach also increased exon skipping efficiency and protein expression as well as other in vivo and in vitro endpoints over the solo therapy alone. These include improvements in body weight, muscle mass, certain muscle fiber hypertrophy and muscle regeneration, among others.

Further surprising findings relate to the age of receptive populations for treatment according to the methods and combinations, among others, described herein. It is generally known that young mdx mice experience greater defined periods of muscle growth and regeneration, and thus tend to have a milder pathology. See, e.g., McGreevy et al. Disease Models & Mechanisms 8:195-213 (2015) . By contrast, aged mdx mice exhibit a much more consistent loss of muscle integrity and function, and are characterized by a more severe pathology that is difficult to treat. However, surprisingly, the in vivo and in vitro outcomes noted above were found to occur not only in young mdx mice but also in aged mice as well. This indicates that the compositions and methods described herein would be useful for treating older patients (e.g., pediatric patients seven years of age and older), with more severe pathology and poorer prognosis.

Further surprising findings relate to greater longevity and/or survivability in the treatment populations tested. It is known that despite being dystrophin deficient, young mdx mice have minimal clinical symptoms (McGreevy *et al.* 2015). Severe dystrophic phenotypes that better represent the clinical phenotype, such as muscle wasting, scoliosis and heart failure, do not occur until mice are 15 months or older. However, premature loss of life frequently occurs at this point, as the lifespan of mdx mice is approximately 25% shorter than wild-type mice. Here, however, the inventors have surprisingly found that treatment according to methods and combinations herein provided prolongation of survival in the mdx mice, from at least about 18 to 24 months, well-surpassing typical longevity/survivability in this model. These increased therapeutic benefits and lifespan observed in the aged mdx mice in accordance with the various aspects and embodiments herein is surprising.

Accordingly, various aspects presented herein include methods of treating Duchenne muscular dystrophy in a subject by administering a combination of a dystrophin therapeutic agent and a myostatin therapeutic agent.

Various aspects include methods of treating a subject with Duchenne muscular dystrophy having a mutation in the dystrophin gene that is amenable to treatment by an antisense oligomer capable of inducing exon skipping during processing of dystrophin pre-mRNA. The method comprises administering to the subject an effective amount of an antisense oligomer comprising 17 to 40 subunits, and further comprising a targeting sequence complementary to 12 or more contiguous nucleotides in a target region comprising an exon of human dystrophin pre-mRNA, where the antisense oligomer induces skipping of the exon; where, said oligomer comprises at least one subunit that is a nucleotide analog having (i) a modified internucleoside linkage, (ii) a modified sugar moiety, or (iii) a combination of the foregoing; and where, said subject has been administered a myostatin therapeutic that inhibits one or both of myostatin activity and myostatin expression in the subject to thereby treat Duchenne muscular dystrophy.

In various embodiments, said exon is selected from exon 7, exon 8, exon 9, exon 19, exon 23, exon 44, exon 45, exon 50, exon 51, exon 52, exon 53, or exon 55. In some embodiments, said exon comprises exon 23. In some embodiments, said exon comprises exon 45. In some embodiments, said exon comprises exon 51. In some embodiments, said exon comprises exon 53. In further embodiments, said exon comprises exon 8, exon 44, exon 50, exon 52 or exon 55.

In various embodiments, the antisense oligomer comprises 20 to 30 subunits. In some embodiments, said antisense oligomer is selected from SEQ ID NOS: 76-SEQ ID NO: 3485. In further embodiments, said antisense oligomer is SEQ ID NO: 76.

In various embodiments, said targeting sequence is complementary to at least 15 contiguous nucleotides in the target region. In some embodiments, said targeting sequence is complementary to at least 17 contiguous nucleotides in the target region. In further embodiments, wherein the targeting sequence is 100% complementary to the target region.

In various embodiments, said myostatin therapeutic is a protein or nucleic acid. In some embodiments, said protein is an anti-myostatin antibody. In some embodiments, said protein is a soluble receptor. In further embodiments, said soluble receptor is ACVR2. In some embodiments, said nucleic acid is at least one of an antisense oligomer or an siRNA.

In various embodiments, said antisense oligomer comprises 12 to 40 subunits, and further comprises a targeting sequence complementary to 12 or more contiguous nucleotides in a target region of myostatin pre-mRNA; and where, said oligomer comprises at least one subunit that is a nucleotide analog having (i) a modified internucleoside linkage, (ii) a modified sugar moiety, or (iii) a combination of the foregoing. In embodiments, the antisense oligomer comprises 20 to 30 subunits. In some embodiments, said targeting sequence is complementary to at least 15 contiguous nucleotides in the target region. In further embodiments, said targeting sequence is complementary to at least 17 contiguous nucleotides in the target region. In embodiments, said targeting sequence is 100% complementary to the target region. In embodiments, the target region comprises SEQ ID NO: 1. In embodiments, said exon comprises exon 2.

In various embodiments, said target region is selected from (i) a nucleotide sequence where at least one nucleotide spans a splice junction associated with intron 1/exon 2 and exon 2/intron 2; or (ii) a nucleotide sequence where no nucleotide spans a splice junction associated with intron 1/exon 2 and exon 2/intron 2. In embodiments, the splice junction is selected from a sequence comprising a splice acceptor site or a splice donor site. In embodiments, the splice junction is selected from a sequence comprising a splice acceptor site or a splice donor site. In some embodiments, the splice acceptor site is provided within SEQ ID NO: 2 and the splice donor site is provided within SEQ ID NO: 3.

In various embodiments, said nucleotide of (i) is selected from SEQ ID NOS: 16-43. In embodiments, said nucleotide of (ii) is selected from SEQ ID NOS: 44-70.

In various embodiments, the subject is a pediatric patient of age 7 or greater.

Various aspects include, methods of treating Duchenne muscular dystrophy, the method comprising: administering to a subject an effective amount of an antisense oligomer of 12 to 40 subunits, and further comprising a targeting sequence complementary to 12 or more contiguous nucleotides comprising an exon of human myostatin pre-mRNA; and wherein, said oligomer comprises at least one subunit that is a nucleotide analog having (i) a modified internucleoside linkage, (ii) a modified sugar moiety, or (iii) a combination of the foregoing; and where said subject has been administered a dystrophin therapeutic that increases dystrophin expression in the subject to thereby treat Duchenne muscular dystrophy.

In various embodiments, wherein the subject has a mutation in the dystrophin gene that is amenable to treatment by an antisense oligomer capable of inducing exon skipping during processing of human myostatin pre-mRNA.

In various embodiments, the antisense oligomer comprises 20 to 30 subunits. In embodiments, said targeting sequence is complementary to at least 15 contiguous nucleotides in the target region. In embodiments, said targeting sequence is complementary to at least 17 contiguous nucleotides in the target region. In embodiments, the antisense oligomer is 100% complementary to the target region. In embodiments, the target region comprises SEQ ID NO: 1. In embodiments, said exon comprises exon 2.

In various embodiments, said target region is selected from (i) a nucleotide sequence wherein at least one nucleotide spans a splice junction associated with intron 1/exon 2 and exon 2/intron 2; or (ii) a nucleotide sequence wherein no nucleotide spans a splice junction associated with intron 1/exon 2 and exon 2/intron 2. In embodiments, the splice junction is selected from a sequence comprising a splice acceptor site or a splice donor site. In embodiments, the splice acceptor site is provided within SEQ ID NO: 2 and the splice donor site is provided within SEQ ID NO: 3.

In various embodiments, said dystrophin therapeutic is selected from one or more of a protein or nucleic acid. In embodiments, said nucleic acid is an antisense oligomer. In some embodiments, said antisense oligomer comprising 20 to 50 subunits, and further comprising a targeting sequence complementary to 10 or more contiguous nucleotides in a target region comprising an exon of human dystrophin pre-mRNA; and where, said oligomer comprises at least one subunit that is a nucleotide analog having (i) a modified internucleoside linkage, (ii) a modified sugar moiety, or (iii) a combination of the foregoing.

Various aspects and embodiments include methods of treating Duchenne muscular dystrophy and related disorders in a subject having a mutation in the dystrophin gene that is amenable to treatment by an antisense oligomer capable of inducing exon skipping during processing of dystrophin pre-mRNA. In various embodiments, the method comprises administering to a subject a targeting sequence comprising formula (I) 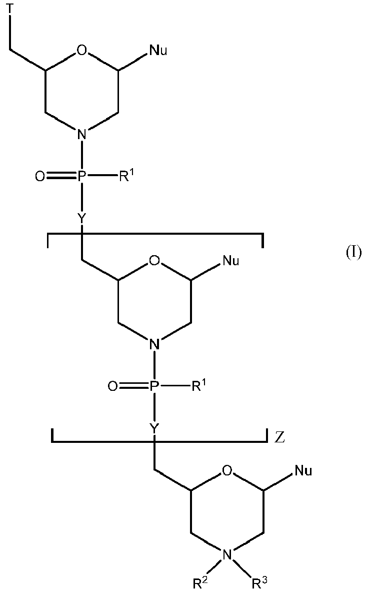 or a pharmaceutically acceptable salt thereof, where:
each Nu is a nucleobase which taken together form a targeting sequence;
Z is an integer from 8 to 48;
each Y is independently selected from O and -NR⁴, wherein each R⁴ is independently selected from H, C₁-C₆ alkyl, aralkyl, C(=NH)NH₂, C(O)(CH₂)ₙNR⁵C(=NH)NH₂, C(O)(CH₂)₂NHC(O)(CH₂)₅NR⁵C(=NH)NH₂, and G, wherein R⁵ is selected from H and C₁ C₆ alkyl and n is an integer from 1 to 5;
T is selected from OH and a moiety of the formula: 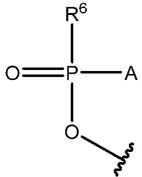 where:
   A is selected from -OH, -N(R⁷)₂R⁸, where:
   each R⁷ is independently selected from H and C₁-C₆ alkyl, and
   R⁸ is selected from an electron pair and H, and
R⁶ is selected from OH, -N(R⁹)CH₂C(O)NH₂, and a moiety of the formula: 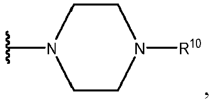 where:
   R⁹ is selected from H and C₁-C₆ alkyl; and
   R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, where:
m is an integer from 1 to 5,
R¹¹ is of the formula -(O-alkyl)y- where y is an integer from 3 to 10 and each of the y alkyl groups is independently selected from C₂-C₆ alkyl; and
R¹² is selected from H and C₁-C₆ alkyl; each instance of R¹ is independently selected from :
   -N(R¹³)₂R¹⁴, where each R¹³ is independently selected from H and C₁-C₆ alkyl, and R¹⁴ is selected from an electron pair and H;
   a moiety of formula (II): 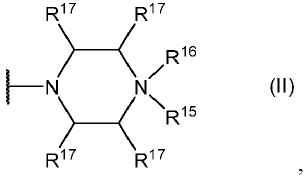 where:
   R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, where:
      R¹⁸ is selected from H and C₁-C₆ alkyl; and
      q is an integer from 1 to 5,
   R¹⁶ is selected from an electron pair and H; and
   each R¹⁷ is independently selected from H and methyl; and
   a moiety of formula(III): 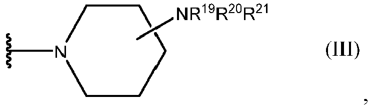 where:
   R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, where:
      R²² is selected from H and C₁-C₆ alkyl; and
      r is an integer from 1 to 5,
   R²⁰ is selected from H and C₁-C₆ alkyl; and
   R²¹ is selected from an electron pair and H;
   R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂,
   -C(O)(CH₂)₂NHC(O)(CH₂)₅NR²⁴C(=NH)NH₂, -C(O)CH(NH₂)(CH₂)₃NHC(=NH)NH₂, and a moiety of the formula: 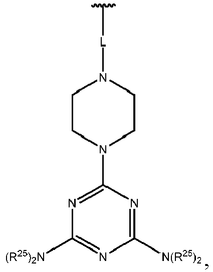 where,
R²³ is of the formula -(O-alkyl)ᵥ-OH where v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and
   each R²⁵ is of the formula -(CH₂)₂OC(O)N(R²⁶)₂ where each R²⁶ is of the formula
-(CH₂)₆NHC(=NH)NH₂; and
   R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP,
   and -C(O)CH₂NH-CPP, or G is of the formula: 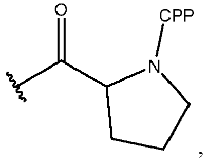
where the CPP is attached to the linker moiety by an amide bond at the CPP carboxy terminus, with the proviso that up to one instance of G is present, and
where the targeting sequence is complementary to 10 or more contiguous nucleotides in a target region comprising an exon of human dystrophin pre-mRNA; and
where, said subject has been administered a myostatin therapeutic to thereby suppress one or both of myostatin activity or expression in the subject.

In various embodiments, each Nu is independently adenine, guanine, thymine, uracil, cytosine, inosine, hypoxanthine, 2,6-diaminopurine, 5-methyl cytosine, C5-propynyl-modified pyrimidines, or 10-(9-(aminoethoxy)phenoxazinyl).

In various embodiments, the target region is selected from (i) a nucleotide sequence wherein at least one nucleotide spans a splice junction associated with said exon; or (ii) a nucleotide sequence wherein no nucleotide spans a splice junction associated with said exon junction. In embodiments, the targeting sequence comprises a sequence selected from SEQ ID NOS: 76-3485, is a fragment of at least 10 contiguous nucleotides of a targeting sequence selected from SEQ ID NOS: 76-3485, or is a variant having at least 90% sequence identity to a targeting sequence selected from SEQ ID NOS: 76-3485.

In various embodiments,
i) Y is O, R² is selected from H or G, R³ is selected from an electron pair or H;
ii) R² is G wherein the CPP is of a sequence selected from SEQ ID NOS: 3486-3501;
iii) each R¹ is -N(CH₃)₂;
iv) at least one R¹ is selected from: 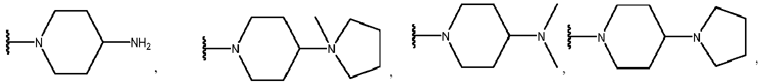 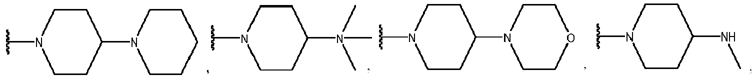 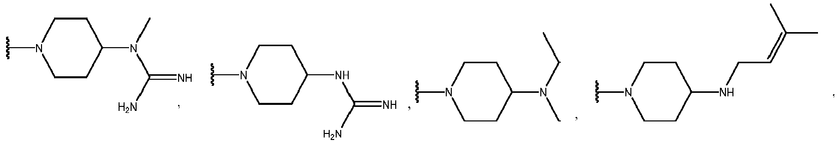 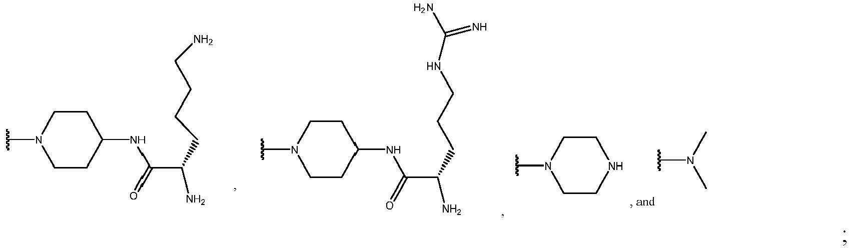 or
v) 50-90% of the R¹ groups are -N(CH₃)₂.

In various embodiments, T is of the formula: 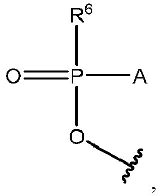 where A is -N(CH₃)₂, and R⁶ is of the formula: 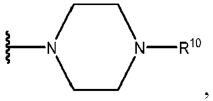 where R¹⁰ is -C(O)R¹¹OH.

In various embodiments, each Y is O, and T is selected from:

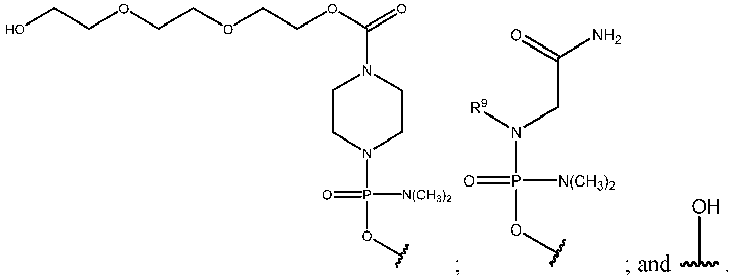

In various embodiments, T is of the formula:

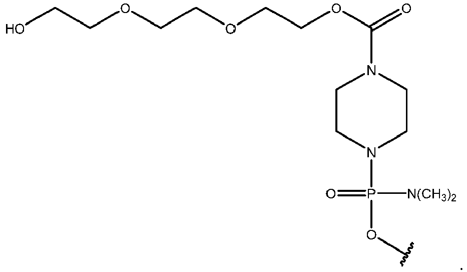

Various aspects include methods of treating Duchenne muscular dystrophy and related disorders in a subject having a mutation in the dystrophin gene that is amenable to treatment by an antisense oligomer capable of inducing exon skipping during processing of dystrophin pre-mRNA. In various embodiments, the method comprises administering to a subject a compound comprising formula (VI): 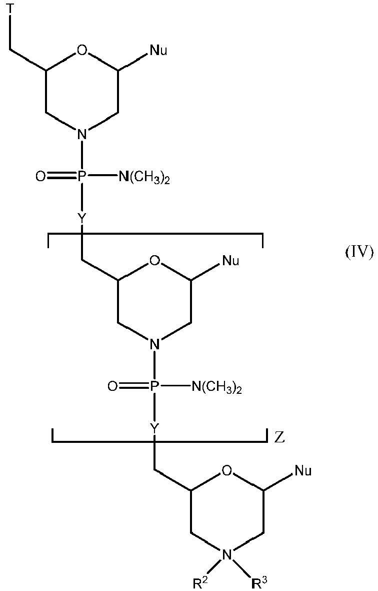 or a pharmaceutically acceptable salt thereof, where:
each Nu is a nucleobase which taken together form a targeting sequence;
Z is an integer from 8 to 48;
each Y is independently selected from O and -NR⁴, where each R⁴ is independently selected from H, C₁-C₆ alkyl,
   aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, where R⁵ is selected from H and C₁-C₆ alkyl and n is an integer from 1 to 5;
T is selected from OH and a moiety of the formula: 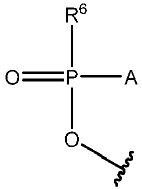 where:
   A is selected from -OH and -N(R⁷)₂R⁸, where:
      each R⁷ is independently selected from H and C₁-C₆ alkyl, and
      R⁸ is selected from an electron pair and H, and
   R⁶ is selected from OH, -N(R⁹)CH₂C(O)NH₂, and a moiety of the formula: 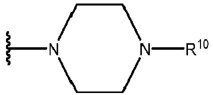 where:
      R⁹ is selected from H and C₁-C₆ alkyl; and
      R¹⁰ is selected from G, -C(O)-R¹¹OH, acyl, trityl, 4-methoxytrityl, -C(=NH)NH₂, -C(O)(CH₂)ₘNR¹²C(=NH)NH₂, and -C(O)(CH₂)₂NHC(O)(CH₂)₅NR¹²C(=NH)NH₂, where:
         m is an integer from 1 to 5,
         R¹¹ is of the formula -(O-alkyl)_{y}- where y is an integer from 3 to 10 and
            each of the y alkyl groups is independently selected from C₂-C₆ alkyl; and
R¹² is selected from H and C₁-C₆ alkyl;
R² is selected from H, G, acyl, trityl, 4-methoxytrityl, C₁-C₆ alkyl, -C(=NH)NH₂, and -C(O)-R²³; and
   R³ is selected from an electron pair, H, and C₁-C₆ alkyl, and wherein the targeting sequence comprises a sequence selected from SEQ ID NOS: 76-3485, is selected from SEQ ID NOS: 76-3485, is a fragment of at least 10 contiguous nucleotides of a sequence selected from SEQ ID NOS: 76-3485, or is a variant having at least 90% sequence identity to a sequence selected from SEQ ID NOS: 76-3485.

Various aspects include methods of treating Duchenne muscular dystrophy and related disorders in a subject having a mutation in the dystrophin gene that is amenable to treatment by an antisense oligomer capable of inducing exon skipping during processing of human myostatin pre-mRNA. In embodiments, the method comprises administering to a subject a compound comprising formula (I): 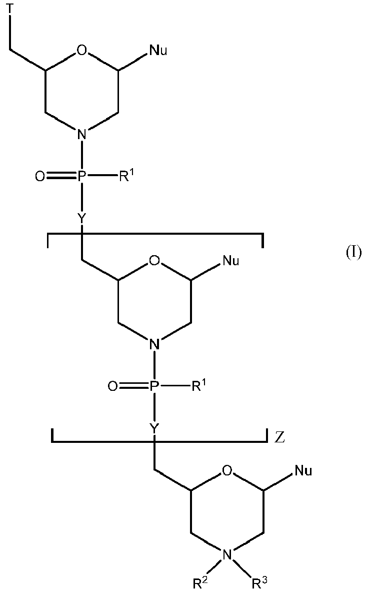 or a pharmaceutically acceptable salt thereof, where:
each Nu is a nucleobase which taken together form a targeting sequence;
Z is an integer from 8 to 48;
each Y is independently selected from O and -NR4, where each R4 is independently selected from H, C₁-C₆ alkyl, aralkyl, C(=NH)NH2, C(O)(CH2)nNR5C(=NH)NH2, C(O)(CH2)2NHC(O)(CH2)5NR5C(=NH)NH2, and G, wherein R5 is selected from H and C1 C6 alkyl and n is an integer from 1 to 5;
T is selected from OH and a moiety of the formula: 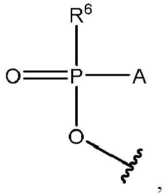 where:
   A is selected from -OH, -N(R⁷)₂R⁸, where:
      each R⁷ is independently selected from H and C₁-C₆ alkyl, and
      R⁸ is selected from an electron pair and H, and
   R⁶ is selected from OH, -N(R⁹)CH₂C(O)NH₂, and a moiety of the formula: 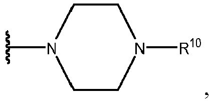 where:
      R⁹ is selected from H and C₁-C₆ alkyl; and
      R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
   m is an integer from 1 to 5,
   R¹¹ is of the formula -(O-alkyl)y- where y is an integer from 3 to 10 and each of the y alkyl groups is independently selected from C₂-C₆ alkyl; and
   R¹² is selected from H and C₁-C₆ alkyl; each instance of R¹ is independently selected from :
   -N(R¹³)₂R¹⁴, where each R¹³ is independently selected from H and C₁-C₆ alkyl, and R¹⁴ is selected from an electron pair and H;
   a moiety of formula (II): 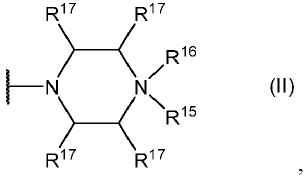 where:
   R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
   R¹⁸ is selected from H and C₁-C₆ alkyl; and
   q is an integer from 1 to 5,
R¹⁶ is selected from an electron pair and H; and
each R¹⁷ is independently selected from H and methyl; and a moiety of formula(III): 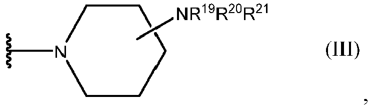 where:
   R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, where:
   R²² is selected from H and C₁-C₆ alkyl; and
   r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂, -C(O)(CH₂)₂NHC(O)(CH₂)₅NR²⁴C(=NH)NH₂, -C(O)CH(NH₂)(CH₂)₃NHC(=NH)NH₂, and a moiety of the formula: 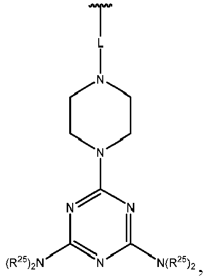 where,
   R²³ is of the formula -(O-alkyl)ᵥ-OH wherein v is an integer from 3 to 10 and each of the v alkyl groups is independently selected from C₂-C₆ alkyl; and
   R²⁴ is selected from H and C₁-C₆ alkyl;
   s is an integer from 1 to 5;
   L is selected from -C(O)(CH₂)₆C(O)- and -C(O)(CH₂)₂S₂(CH₂)₂C(O)-; and
each R²⁵ is of the formula -(CH₂)₂OC(O)N(R²⁶)₂ where each R²⁶ is of the formula -(CH₂)₆NHC(=NH)NH₂; and
R³ is selected from an electron pair, H, and C₁-C₆ alkyl,
   where G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP,
   and -C(O)CH₂NH-CPP, or G is of the formula: 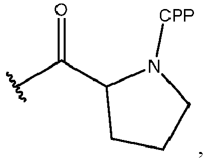
   where the CPP is attached to the linker moiety by an amide bond at the CPP carboxy terminus, with the proviso that up to one instance of G is present, and
   where the targeting sequence is complementary to 10 or more contiguous nucleotides in a target region comprising an exon of human dystrophin pre-mRNA; and
   where, said subject has been administered a myostatin therapeutic to thereby suppress
   one or both of myostatin activity or expression in the subject.

In various embodiments, each Nu is independently adenine, guanine, thymine, uracil, cytosine, inosine, hypoxanthine, 2,6-diaminopurine, 5-methyl cytosine, C5-propynyl-modified pyrimidines, or 10-(9-(aminoethoxy)phenoxazinyl).

In various embodiments, the targeting sequence comprises a sequence selected from SEQ ID NOS: 16-75, is a fragment of at least 10 contiguous nucleotides of a targeting sequence selected from SEQ ID NOS: 16-75, or is a variant having at least 90% sequence identity to a targeting sequence selected from SEQ ID NOS: 16-75.

In various embodiments,
i) Y is O, R² is selected from H or G, R³ is selected from an electron pair or H;
ii) R² is G where the CPP is of a sequence selected from SEQ ID NOS: 3486-3501;
iii) each R¹ is -N(CH₃)₂;
iv) at least one R¹ is selected from: 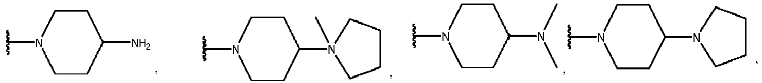 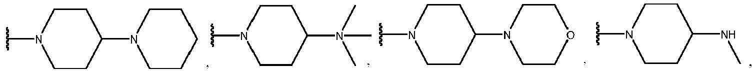 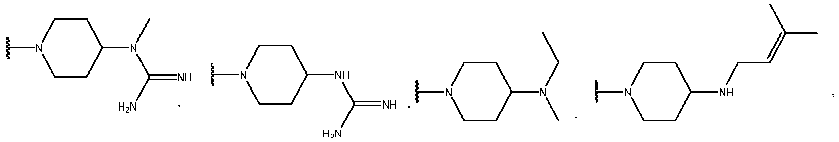 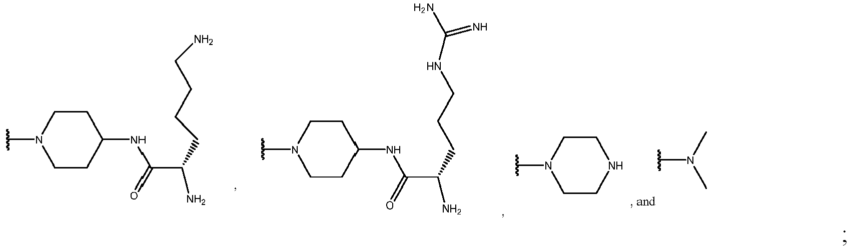 or
v) 50-90% of the R¹ groups are -N(CH₃)₂.

In various embodiments, T is of the formula: 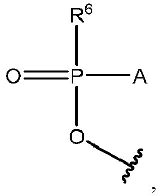 where A is -N(CH₃)₂, and R⁶ is of the formula: 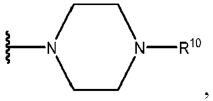 where R¹⁰ is -C(O)R¹¹OH.

In various embodiments, each Y is O, and T is selected from:

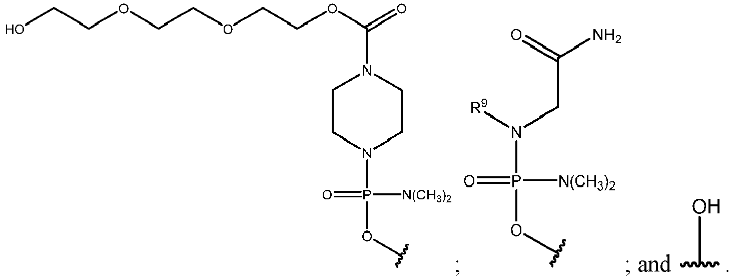

In various embodiments, T is of the formula:

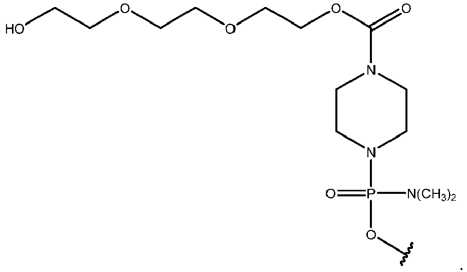

Various aspects include, methods of treating Duchenne muscular dystrophy and related disorders in a subject having a mutation in the dystrophin gene that is amenable to treatment by an antisense oligomer capable of inducing exon skipping during processing of myostatin pre-mRNA. In various embodiments, the method comprises administering to a subject a compound comprising formula (VI): 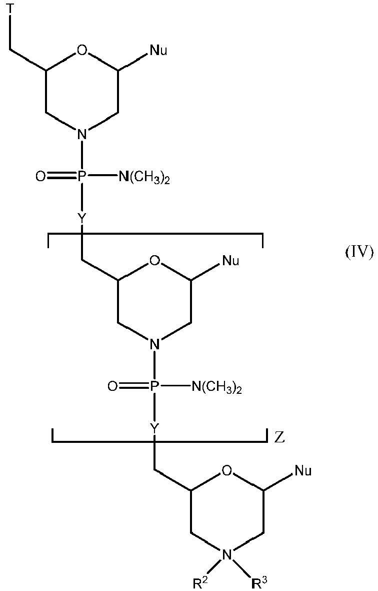 or a pharmaceutically acceptable salt thereof, where:
each Nu is a nucleobase which taken together form a targeting sequence;
Z is an integer from 8 to 48;
each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, where R⁵ is selected from H and C₁-C₆ alkyl and n is an integer from 1 to 5;
T is selected from OH and a moiety of the formula: 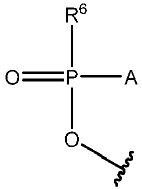 where:
   A is selected from -OH and -N(R⁷)₂R⁸, where:
      each R⁷ is independently selected from H and C₁-C₆ alkyl, and
      R⁸ is selected from an electron pair and H, and
   R⁶ is selected from OH, -N(R⁹)CH₂C(O)NH₂, and a moiety of the formula: 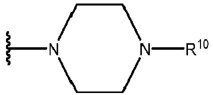 where:
      R⁹ is selected from H and C₁-C₆ alkyl; and
      R¹⁰ is selected from G, -C(O)-R¹¹OH, acyl, trityl, 4-methoxytrityl, -C(=NH)NH₂, -C(O)(CH₂)ₘNR¹²C(=NH)NH₂, and -C(O)(CH₂)₂NHC(O)(CH₂)₅NR¹²C(=NH)NH₂, where:
         m is an integer from 1 to 5,
         R¹¹ is of the formula -(O-alkyl)_{y}- where y is an integer from 3 to 10 and
            each of the y alkyl groups is independently selected from C₂-C₆ alkyl; and
   R¹² is selected from H and C₁-C₆ alkyl;
   R² is selected from H, G, acyl, trityl, 4-methoxytrityl, C₁-C₆ alkyl, -C(=NH)NH₂, and -C(O)-R²³; and
      R³ is selected from an electron pair, H, and C₁-C₆ alkyl, and where the targeting sequence comprises a sequence selected from SEQ ID NOS: 16-75, is selected from SEQ ID NOS: 16-75, is a fragment of at least 10 contiguous nucleotides of a sequence selected from SEQ ID NOS: 16-75, or is a variant having at least 90% sequence identity to a sequence selected from SEQ ID NOS: 16-75.

Various aspects include a dystrophin-related pharmaceutical composition, comprising an antisense oligomer compound of 20 to 50 subunits and a pharmaceutically acceptable carrier, the compound comprising: at least one subunit that is a nucleotide analog having (i) a modified internucleoside linkage, (ii) a modified sugar moiety, or (iii) a combination of the foregoing; and a targeting sequence complementary to 10 or more contiguous nucleotides in a target region comprising an exon of human dystrophin pre-mRNA;
together with a myostatin-related pharmaceutical composition, comprising an antisense oligomer compound of 12 to 40 subunits and a pharmaceutically acceptable carrier, the compound comprising: at least one subunit that is a nucleotide analog having (i) a modified internucleoside linkage, (ii) a modified sugar moiety, or (iii) a combination of the foregoing; and
a targeting sequence complementary to 12 or more contiguous nucleotides in a target region comprising an exon of human myostatin pre-mRNA. In various embodiments, the dystrophin-related composition and the myostatin-related composition are provided in the same pharmaceutical composition.

Various aspects include, methods for modulating myostatin expression in a subject having a genetic mutation amenable to treatment by an antisense oligomer capable of inducing exon skipping during processing of human myostatin pre-mRNA, the methodcomprising: administering to the subject an effective amount of an antisense oligomer comprising 12 to 40 subunits, and further comprising a targeting sequence complementary to 12 or more contiguous nucleotides comprising an exon of human myostatin pre-mRNA;and where, said oligomer comprises at least one subunit that is a nucleotide analog having (i) a modified internucleoside linkage, (ii) a modified sugar moiety, or (iii) a combination of the foregoing; binding the antisense oligomer to the target region in the myostatin pre-mRNA transcript; and, inhibiting transcription of the target region into a human myostatin mRNA transcript, where said subject has been administered a dystrophin therapeutic that increases dystrophin expression in the subj ect.

Various aspects include, methods for decreasing expression of exon 2 in a subject having a genetic mutation amenable to treatment by an antisense oligomer capable of inducing exon skipping during processing of human myostatin pre-mRNA, the method comprising: administering to the subject an effective amount of an antisense oligomer comprising 12 to 40 subunits, and further comprising a targeting sequence complementary to 12 or more contiguous nucleotides comprising an exon of human myostatin pre-mRNAand inhibiting transcription of exon 2 in a myostatin mRNA transcript, where said subject has been administered a dystrophin therapeutic that increases dystrophin expression in the subject. In various embodiments, exon 2 expression is decreased by about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% in a myostatin mRNA transcript.

Various aspects include, methods for decreasing the accumulation of functional myostatin protein in a muscle cell or tissue in a subject having a mutation in the dystrophin gene that is amenable to treatment by an antisense oligomer capable of inducing exon skipping during processing of human myostatin pre-mRNA, the method comprising: administering to the subject an effective amount of an antisense oligomer comprising 12 to 40 subunits, and further comprising a targeting sequence complementary to 12 or more contiguous nucleotides comprising an exon of human myostatin pre-mRNA, and inhibiting transcription of exon 2 in a myostatin mRNA transcript, where said subject has been administered a dystrophin therapeutic that increases dystrophin expression in the subject.

Various aspects include, a medicament for the treatment of Duchenne muscular dystrophy and related disorders comprising: an antisense oligomer compound comprising 12 to 40 subunits, comprising at least one subunit that is a nucleotide analog having (i) a modified internucleoside linkage, (ii) a modified sugar moiety, or (iii) a combination of the foregoing; and further comprising a targeting sequence complementary to 12 or more contiguous nucleotides comprising an exon of human myostatin pre mRNA; and a dystrophin therapeutic that increases dystrophin expression.

Various aspects include, methods for inhibiting the progression of Duchenne muscular dystrophy and related disorders in a subject having a mutation in the dystrophin gene that is amenable to treatment by an antisense oligomer capable of inducing exon skipping during processing of human myostatin pre-mRNA, the method comprising: administering to the subject an effective amount of an antisense oligomer comprising 12 to 40 subunits, and further comprising a targeting sequence complementary to 12 or more contiguous nucleotides comprising an exon of human myostatin pre-mRNA; and, inhibiting transcription of exon 2 in a myostatin mRNA transcript, where said subject has been administered a dystrophin therapeutic that increases dystrophin expression in the subject to thereby inhibit the progression of Duchenne muscular dystrophy.

Various aspects include, methods of decreasing the accumulation of a functional myostatin protein in a subject with Duchenne muscular dystrophy and related disorders, said method comprising: administering to the subject an effective amount of an antisense oligomer comprising 12 to 40 subunits, and further comprising a targeting sequence complementary to 12 or more contiguous nucleotides comprising an exon of human myostatin pre-mRNA; inhibiting transcription of exon 2 in a myostatin mRNA transcript, where the accumulation of functional myostatin protein in the subject is decreased, and where said subject has been administered a dystrophin therapeutic that increases dystrophin expression in the subject.

Various aspects include, methods for treating Duchenne muscular dystrophy and related disorders in a subject in need of such treatment, comprising: administering an antisense oligomer in an effective amount to result in a peak blood concentration of at least about 200-400 nM of antisense oligomer in the subject.

Various aspects include, a method of treating skeletal muscle mass deficiency in a subject having a mutation in the dystrophin gene that is amenable to treatment by an antisense oligomer capable of inducing exon skipping during processing of human myostatin pre-mRNA, the method comprising: (a) measuring blood or tissue levels of myostatin protein in the subject; (b) administering to the subject, an effective amount of an antisense oligomer comprising 12 to 40 subunits, and further comprising a targeting sequence complementary to 12 or more contiguous nucleotides comprising an exon of human myostatin pre-mRNA; (c) inhibiting transcription of exon 2 in a myostatin mRNA transcript; (d) measuring myostatin protein levels in the subject after a select time; and, (e) repeating said administering using the levels measured in (d) to adjust the dose or dosing schedule of the amount of antisense oligomer administered, wherein the level of myostatin protein is decreased in the subject after administering the antisense oligomer, and where said subject has been administered a dystrophin therapeutic that increases dystrophin expression in the subject.

Various aspects include, methods of inhibiting the progression of Duchenne muscular dystrophy and related disorders in a subject having a mutation in the dystrophin gene that is amenable to treatment by an antisense oligomer capable of inducing exon skipping during processing of dystrophin pre-mRNA, the method comprising: administering to the subject an effective amount of an antisense oligomer comprising 17 to 40 subunits, and further comprising a targeting sequence complementary to 12 or more contiguous nucleotides in a target region comprising an exon of human dystrophin pre-mRNA, wherein the antisense oligomer induces skipping of the exon; where, said oligomer comprises at least one subunit that is a nucleotide analog having (i) a modified internucleoside linkage, (ii) a modified sugar moiety, or (iii) a combination of the foregoing; and where, said subject has been administered a myostatin therapeutic that inhibits one or both of myostatin activity and expression in the subject to thereby inhibit the progression of Duchenne muscular dystrophy.

Various aspects include, methods of inhibiting the progression of Duchenne muscular dystrophy, the method comprising: administering to the subject an effective amount of an antisense oligomer comprising 12 to 40 subunits, and further comprising a targeting sequence complementary to 12 or more contiguous nucleotides comprising an exon of human myostatin pre-mRNA; and where, said oligomer comprises at least one subunit that is a nucleotide analog having (i) a modified internucleoside linkage, (ii) a modified sugar moiety, or (iii) a combination of the foregoing; and where said subject has been administered a dystrophin therapeutic that increases dystrophin expression in the subject to thereby inhibit the progression of Duchenne muscular dystrophy.

Various aspects and embodiments include antisense oligomers further comprising an arginine-rich peptide sequence conjugated to the 3' terminal end or the 5' terminal end of the antisense oligomer, where the arginine-rich peptide sequence comprises a sequence selected from SEQ ID NOS: 3486-3501.

Various aspects include, a composition comprising: an antisense oligomer comprising 17 to 40 subunits, and further comprising a targeting sequence complementary to 12 or more contiguous nucleotides in a target region comprising an exon of human dystrophin pre-mRNA; where said dystrophin-targeted oligomer comprises at least one subunit that is a nucleotide analog having (i) a modified internucleoside linkage, (ii) a modified sugar moiety, or (iii) a combination of the foregoing; and an antisense oligomer comprising 12 to 40 subunits, and further comprising a targeting sequence complementary to 12 or more contiguous nucleotides comprising an exon of human myostatin pre-mRNA; where said myostatin-targeted oligomer comprises at least one subunit that is a nucleotide analog having (i) a modified internucleoside linkage, (ii) a modified sugar moiety, or (iii) a combination of the foregoing.

Various aspects and embodiments include administering a dystrophin therapeutic agent and a myostatin therapeutic agent to a subject where said subject is a pediatric patient of age 7 or greater.

Various aspects include methods for modulating dystrophin expression in a subject having a genetic mutation amenable to treatment by an antisense oligomer capable of inducing exon skipping during processing of human myostatin pre-mRNA, the methodcomprising: administering to the subject an effective amount of an antisense oligomer comprising 17 to 40 subunits, and further comprising a targeting sequence complementary to 12 or more contiguous nucleotides comprising an exon of human dystrophin pre-mRNA;and where, said oligomer comprises at least one subunit that is a nucleotide analog having (i) a modified internucleoside linkage, (ii) a modified sugar moiety, or (iii) a combination of the foregoing; wherein said subject has been administered a myostatin therapeutic that inhibits one or both of myostatin activity and myostatin expression in the subject.

Various apsects and embodiments further includ methods of modulating muscle mass in subjects with DMD and related disorders are provided.

In another aspect, the disclosure provides a method for treating a patient with DMD, the method comprising administering to the subject one or both of any dystrophin therapeutic described herein and any myostatin therapeutic described herein to thereby treat DMD. The patient can be one having a mutation in the DMD gene that is amenable to exon skipping, e.g., using an oligonucleotide capable of inducing exon skipping. In some embodiments, the patient has a mutation in the DMD gene that is amenable to exon 51 skipping. In some embodiments, the patient has a mutation in the DMD gene that is amenable to exon 53 skipping. In some embodiments, the patient has a mutation in the DMD gene that is amenable to exon 45 skipping. In some embodiments, the patient has a mutation in the DMD gene that is amenable to exon 44 skipping. In some embodiments, the patient has a mutation in the DMD gene that is amenable to exon 52 skipping. In some embodiments, the patient has a mutation in the DMD gene that is amenable to exon 50 skipping. In some embodiments, the patient has a mutation in the DMD gene that is amenable to exon 8 skipping. In some embodiments, the patient has a mutation in the DMD gene that is amenable to exon 55 skipping.

In another aspect, the disclosure provides a composition (e.g., a pharmaceutical composition) comprising any one or more of the dystrophin therapeutics described herein and one or more of the myostatin therapeutics described herein.

In some embodiments of the methods or compositions described herein, the dystrophin therapeutic is eteplirsen.

In some embodiments of the methods or compositions described herein, the dystrophin therapeutic does not comprise, and does not consist of, the sequence set forth in SEQ ID NO: 927.

In some embodiments of the methods or compositions described herein, dystrophin is human dystrophin. In some embodiments of the methods or compositions described herein, myostatin is human myostatin. In some embodiments of the methods or compositions described herein, the subject is human.

In some embodiments of any of the methods or compositions described herein, the subject is a human (e.g., a human patient). In some embodiments of any of the methods or compositions described herein, the subject is a male subject. In some embodiments of any of the methods or compositions described herein, the subject is a pediatric patient. In some embodiments of any of the methods or compositions described herein, the patient is seven years of age or older. In some embodiments of any of the methods or compositions described herein, the patient is at least seven years of age, but less than about 21 years of age.

In some embodiments of any of the methods or compositions described herein, one or both of the dystrophin therapeutic and the myostatin therapeutic are systemically delivered to the subject, e.g., by intravenous administration. In some embodiments of any of the methods or compositions described herein, the dystrophin therapeutic is systemically delivered to the subject. In some embodiments of any of the methods or compositions described herein, the myostatin therapeutic is systemically delivered to the subject.

In some embodiments of any of the methods or compositions described herein, one or both of the dystrophin therapeutic and the myostatin therapeutic are chronically administered to the subject. For example, in some embodiments of any of the methods or compositions described herein, one or both of the therapeutic agents can each, independently, be administered daily, weekly, monthly, bi weekly, or bi monthly. In some embodiments of any of the methods or compositions described herein, a therapeutically effective amount of one or both of the therapeutic agents can each, independently, can be delivered to the subject as a single dose (e.g., a single weekly dose) or as multiple doses (e.g., two or more, e.g., three, four, five, six, or seven doses) within a treatment period, e.g., once per week (weekly) or twice per week.

In some embodiments of any of the methods described herein, the dystrophin therapeutic is administered first in time and the myostatin therapeutic is administered second in time. For example, a dystrophin therapeutic (e.g., eteplirsen) can be administered first in time in an amount for a duration sufficient to increase dystrophin production in muscle cells of the subject, prior to administering the myostatin therapeuitic to the subject. Thus, in some embodiments, a dystrophin therapeutic (e.g., eteplirsen) is administered to a subject at about 30 mg per kg body weight of the subject once weekly for a period of time (e.g., 6 months, 1 year, 18 months, 2 years or more) to increase dystrophin expression in the muscle cells of the subject, prior to administering the myostatin therapeutic. In some embodiments, a dystrophin therapeutic (e.g., eteplirsen) is administered to a subject at about 30 to about 50 mg per kg body weight of the subject once weekly for a period of time (e.g., 6 months, 1 year, 18 months, 2 years or more) to increase dystrophin expression in the muscle cells of the subject, prior to administering the myostatin therapeutic. In some embodiments of any of the methods described herein, the myostatin therapeutic is administered first in time and the dystrophin therapeutic is administered second in time.

In some embodiments, the antisense oligonucleotide compounds for use in the compositions and methods described herein do not include a cell-penetrating peptide.

### BRIEF DESCRIPTION OF THE DRAWINGS

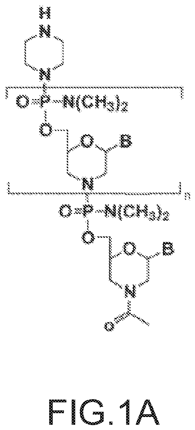

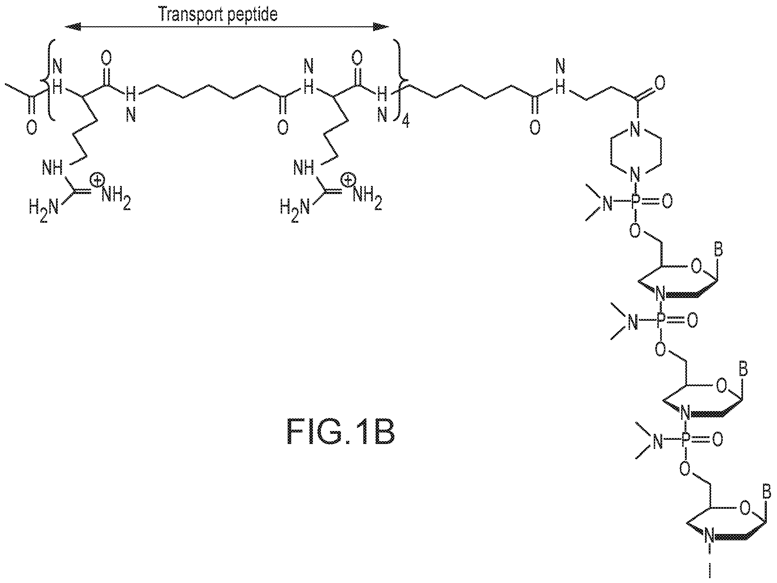

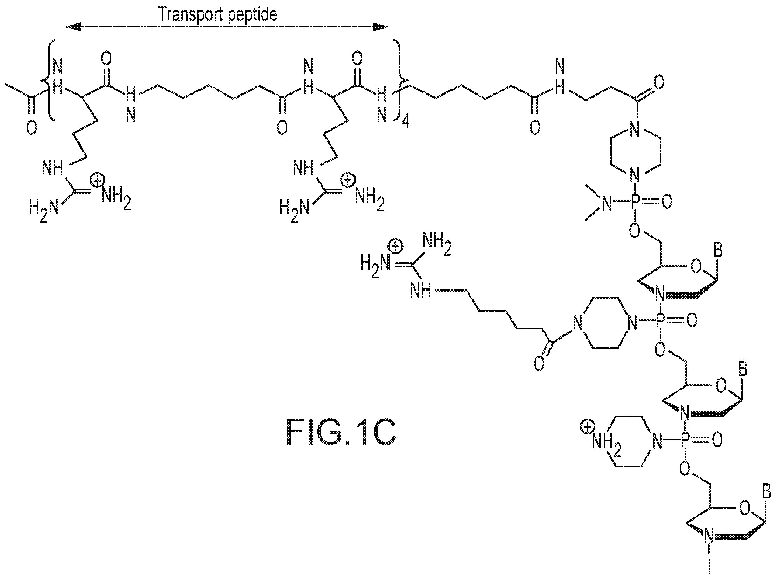

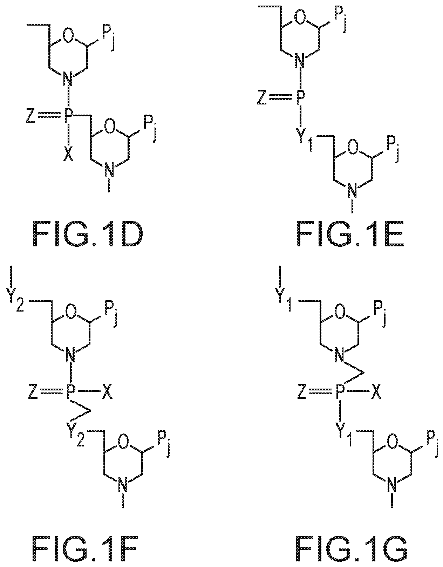

**FIG. 1A** illustrates a modified oligomer at the 5' end to add a linker. **FIG. 1B** **and** **1C** illustrates an antisense oligonucleotide conjugated to a cell penetrating peptide (CPP). **FIGS. 1D, 1**E, **1F and 1G** illustrate a repeating subunit segment of exemplary morpholino oligonucleotides.

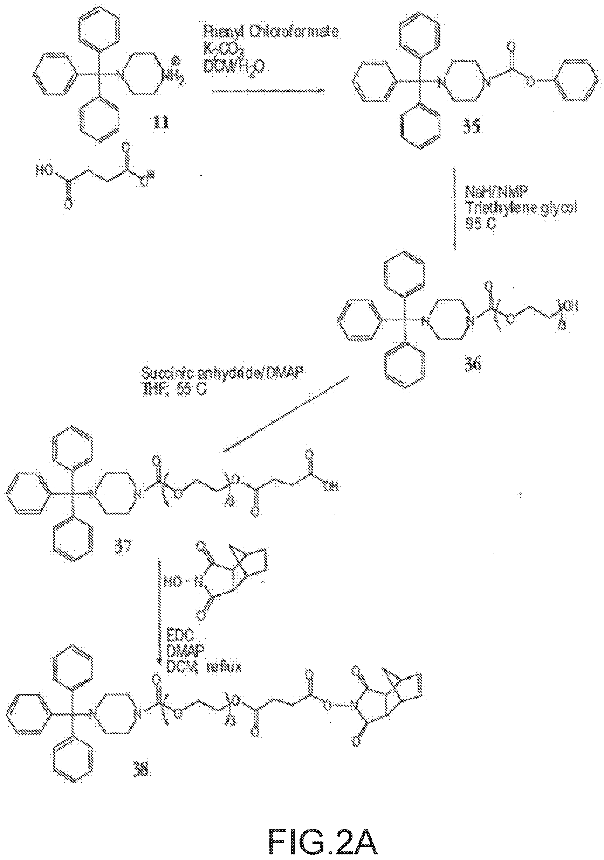

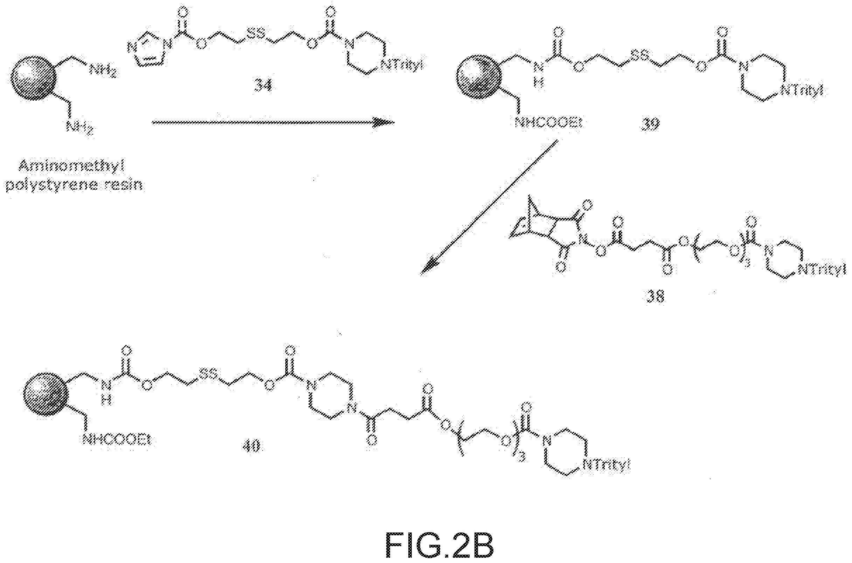

**FIG. 2A** illustrates preparation of trityl piperazine phenyl carbamate. **FIG. 2B** illustrates preparation of a resin/reagent mixture.

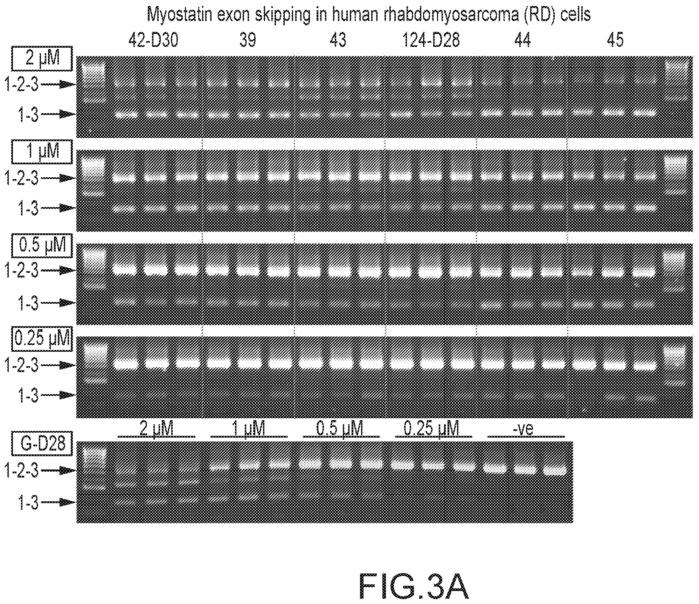

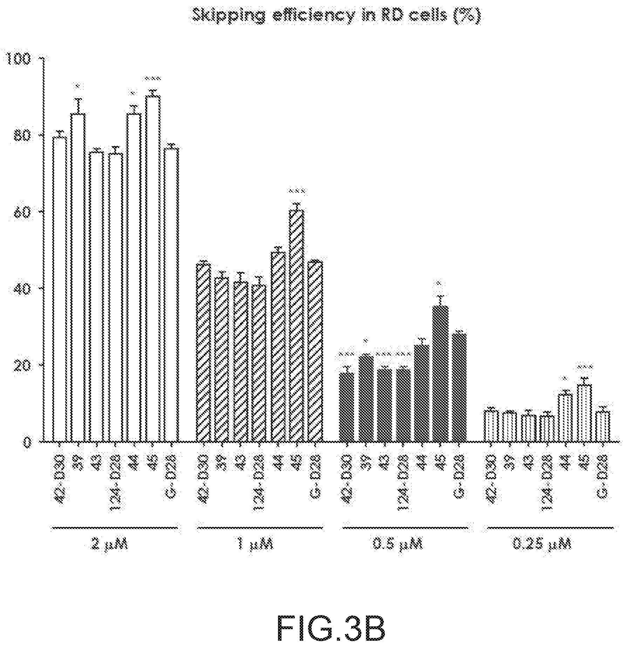

**FIG. 3A** illustrates a gel electrophoresis of RT-PCR products of myostatin exon 2 skipping in human Rhabdomyosarcoma (RD) cells. **FIG. 3B** illustrates skipping efficiency of myostatin exon 2 in RD cells (%).

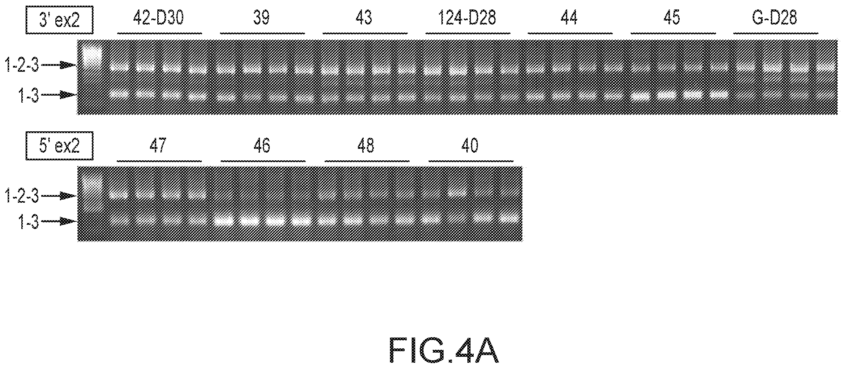

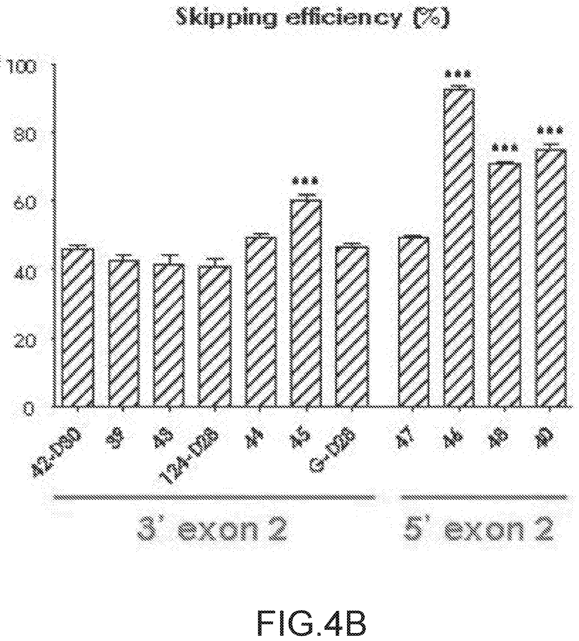

**FIG. 4A** illustrates a gel electrophoresis of RT-PCR products of myostatin exon 2 skipping in RD cells. **FIG. 4B** illustrates relative densitometric analysis of myostatin exon 2 skipping.

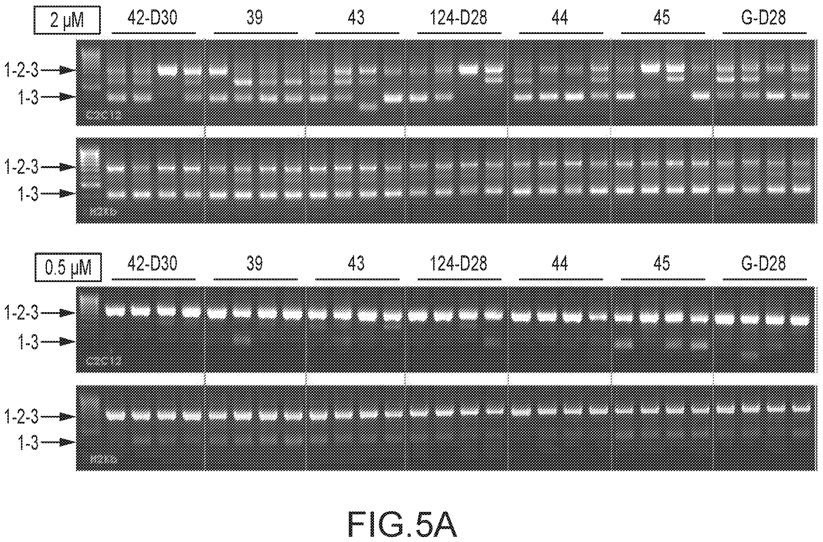

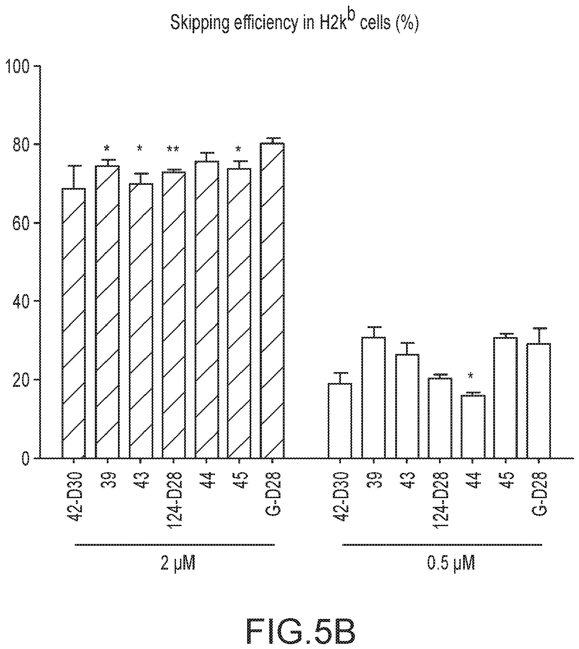

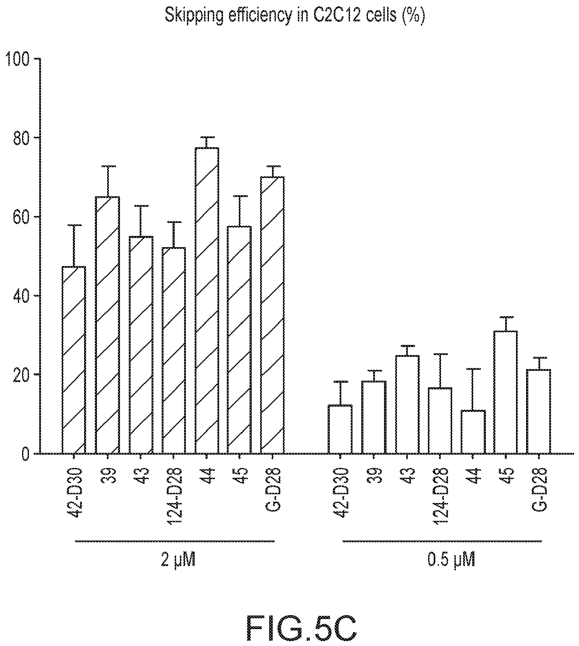

**FIG. 5A** illustrates myostatin exon 2 skipping in C2C12 and H2Kb^{mdx} cells. **FIG. 5B** illustrates densitometric analysis of RT-PCR products myostatin exon 2 skipping efficiency in C2C12 cells (%). **FIG. 5C** illustrates densitometric analysis of RT-PCR products myostatin exon 2 skipping efficiency in H2Kb^{mdx} cells (%).

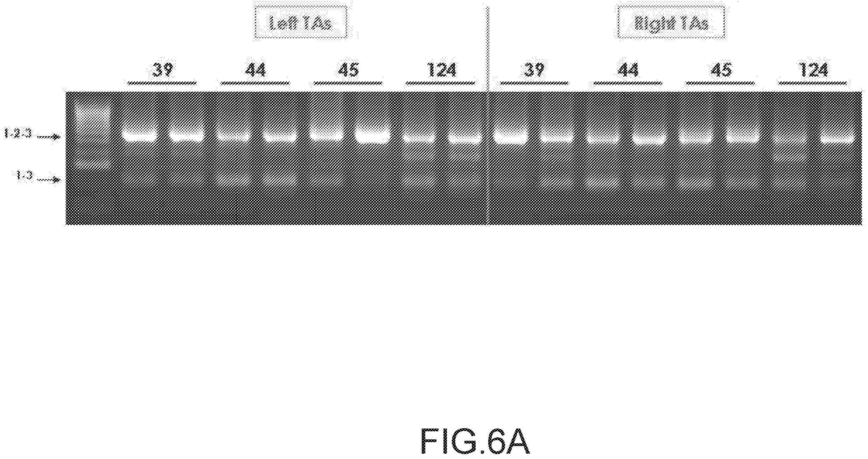

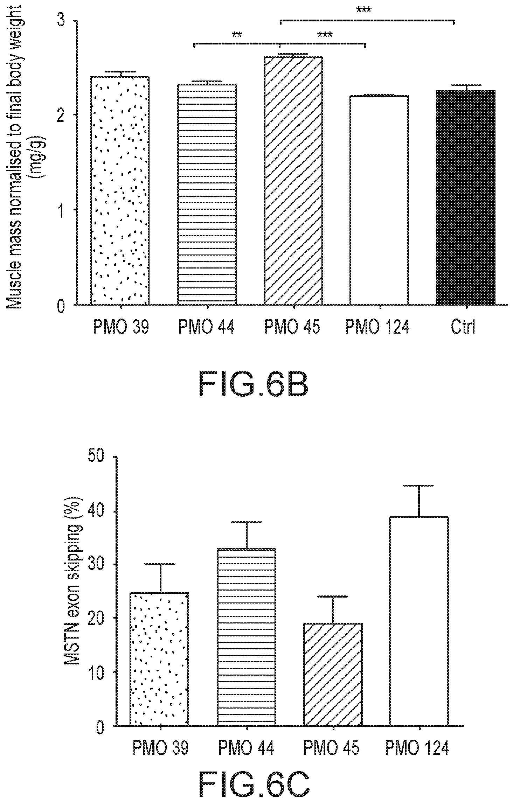

**FIG. 6A** illustrates gel electrophoresis products of myostatin exon 2 skipping in tibialis anterior (TA) muscle. **FIG. 6B** illustrates muscle mass normalized to body weight. **FIG. 6C** illustrates densitometric analysis of RT-PCR products of myostatin exon 2 skipping.

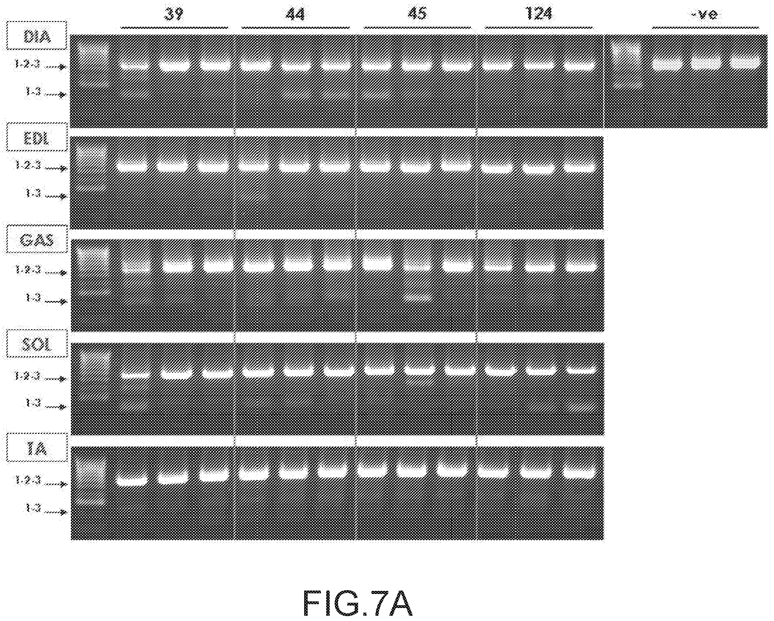

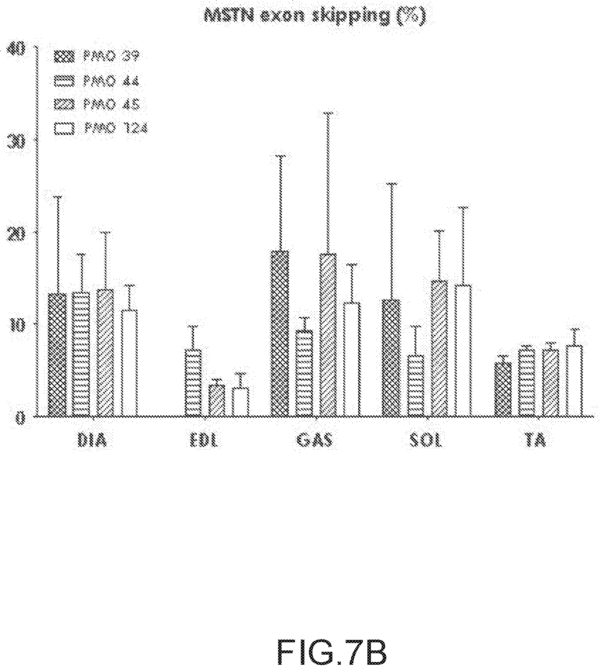

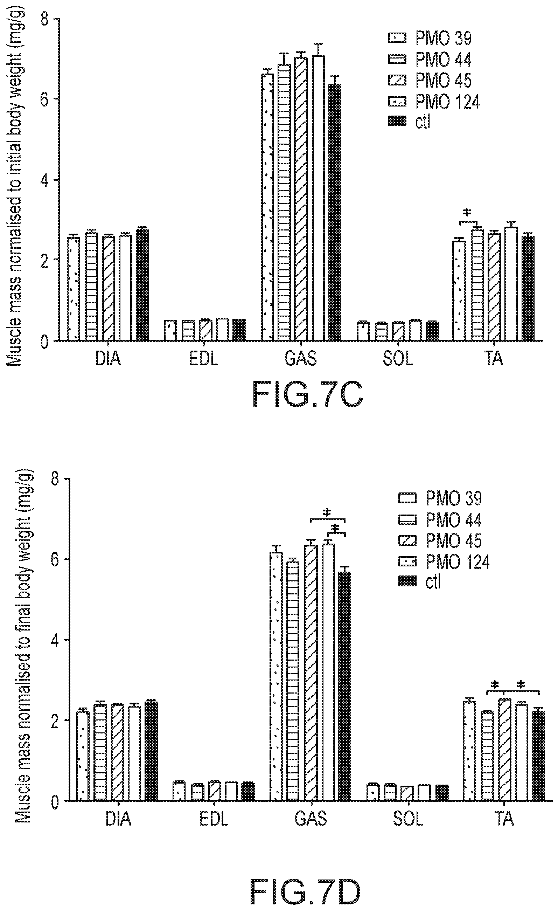

**FIG. 7A** illustrates a gel electrophoresis of myostatin exon 2 skipping in mdx mice muscles. **FIG. 7B** illustrates densitometric analysis of RT-PCR products of myostatin exon 2 skipping in mdx mice. **FIG. 7C** illustrates muscle weight normalized to initial body weight in mdx mice. **FIG. 7D** illustrates muscle weight normalized to final body weight in mdx mice.

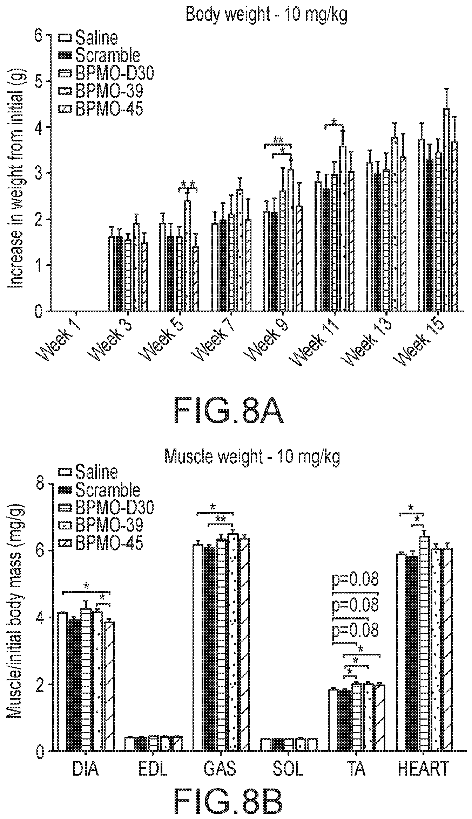

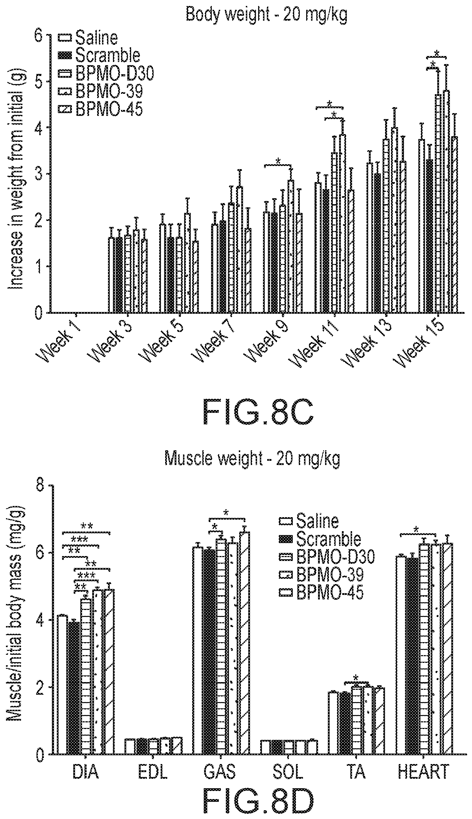

**FIG. 8A** illustrates increase in body weight in mdx mice administered 10 mg/kg BPMO. **FIG. 8B** illustrtates increase in muscle mass in mdx mice administered 10 mg/kg BPMO. **FIG. 8C** illustrates increase in body weight in mdx mice administered 20 mg/kg BPMO. **FIG. 8D** illustrtates increase in muscle mass in mdx mice administered 20 mg/kg BPMO.

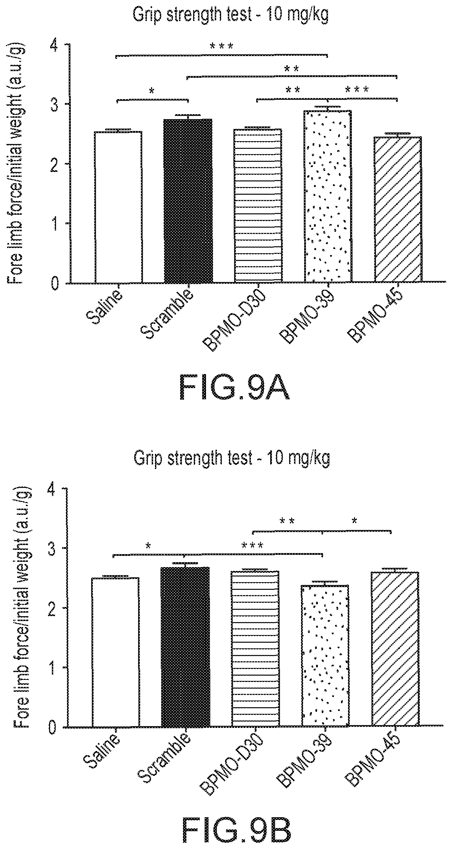

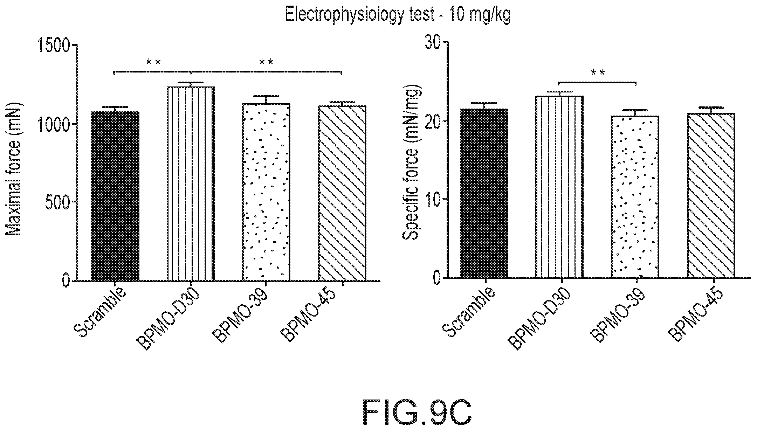

**FIG. 9A** illustrates grip strength test of mdx mice administered 10 mg/kg BPMO. **FIG. 9B** illustrates grip strength test of mdx mice administered 20 mg/kg BPMO. **FIG. 9C** illustrates electrophysiology test in TA muscles in mdx mice administered 10 mg/kg BPMO.

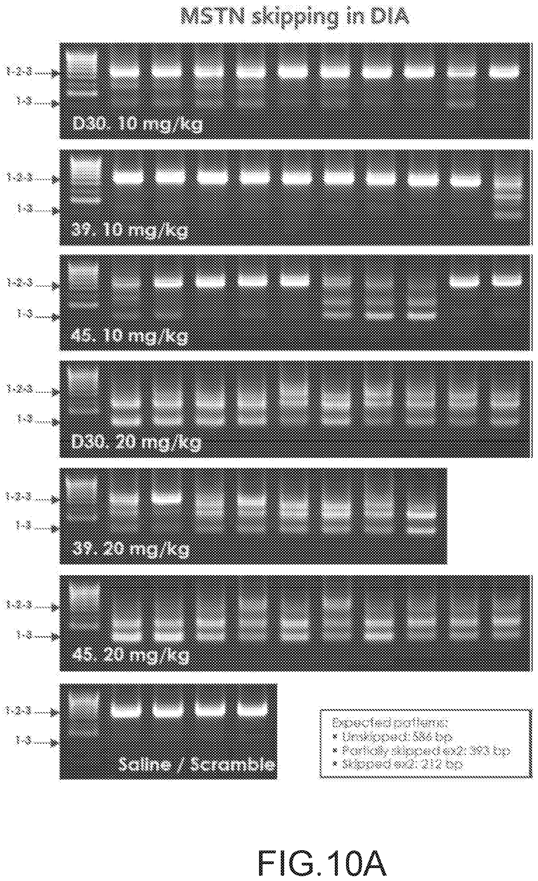

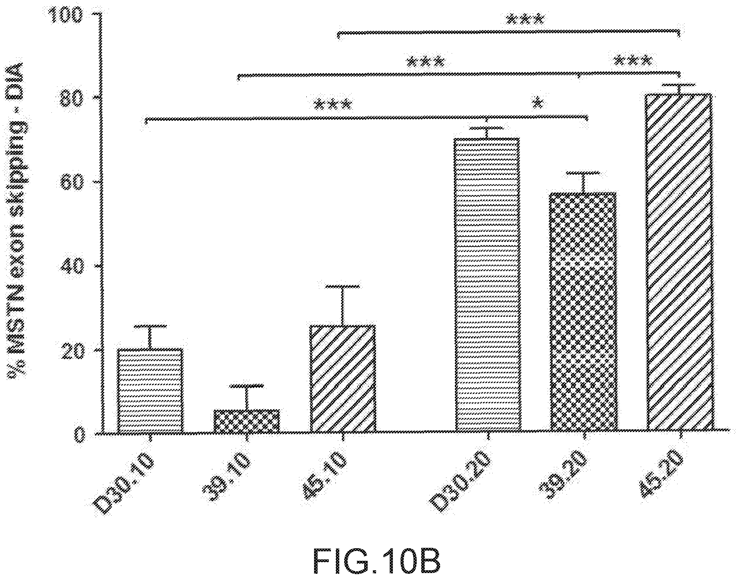

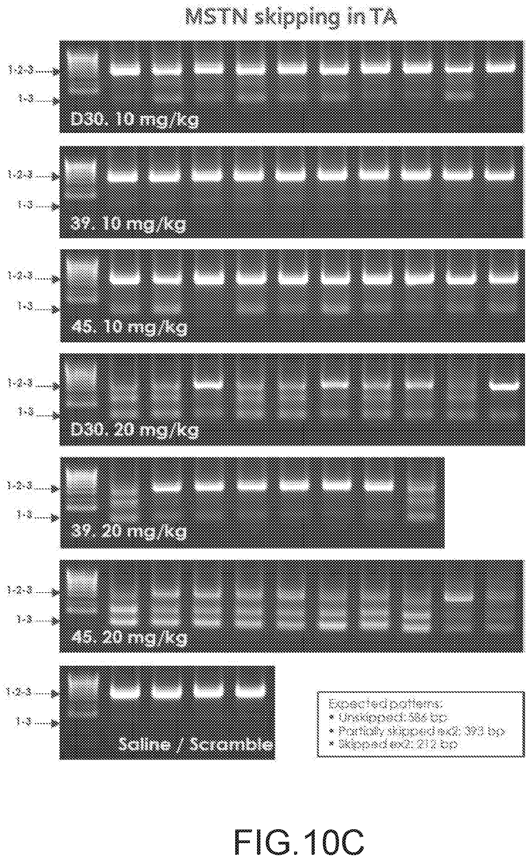

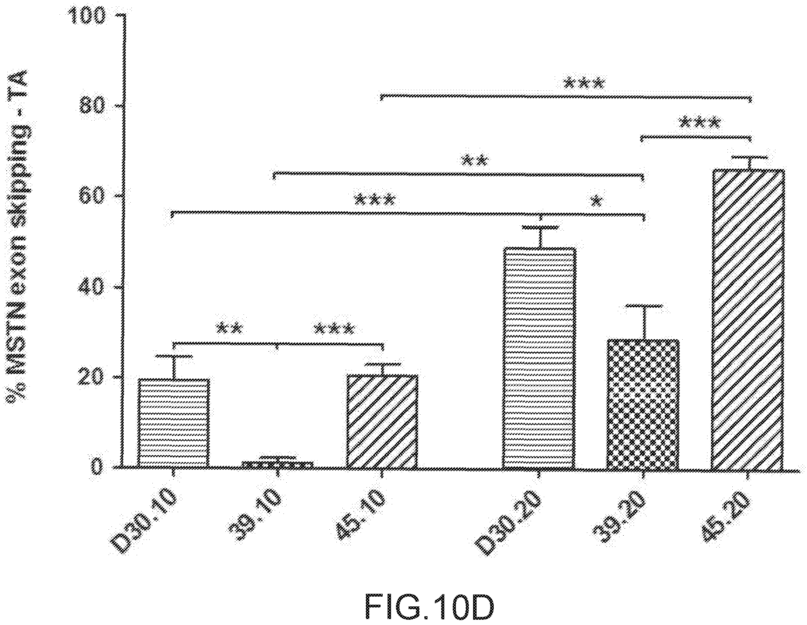

**FIG. 10A** illustrates gel electrophoresis of RT-PCR products of myostatin exon 2 skipping in the diaphragm (DIA). **FIG. 10B** illustrates densitometric analysis of RT-PCR products of myostatin exon 2 skipping in the DIA. **FIG. 10C** illustrates gel electrophoresis of RT-PCR products of myostatin exon 2 skipping in the TA. **FIG. 10D** illustrates densitometric analysis of RT-PCR products of myostatin exon 2 skipping in the TA.

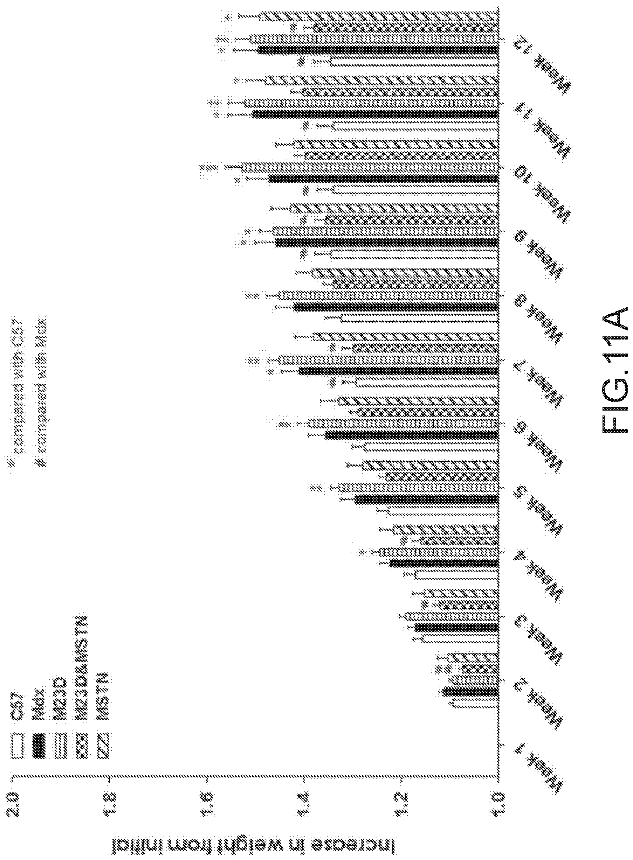

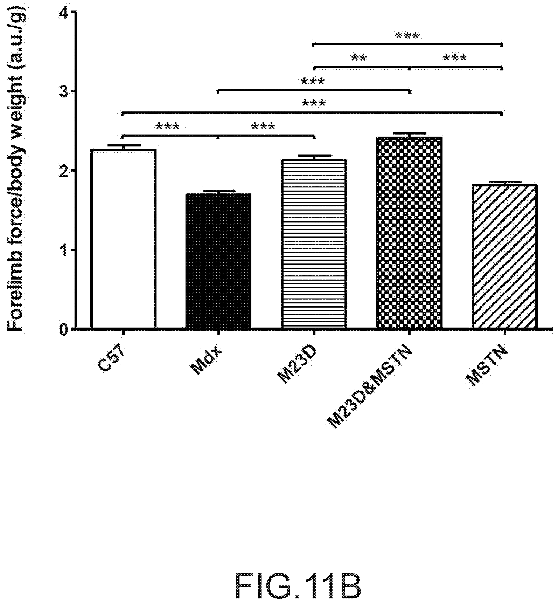

**FIG. 11A** illustrates body weight normalized to initial weight of young dystrophic miceC57BL10 administered saline (positive control), mdx mice administered saline (negative control), mdx mice administered BPMO-M23D (10 mg/kg), mdx mice administered BPMO-M23D (10 mg/kg) & BPMO-MSTN (10 mg/kg), or mdx mice administered BPMO-MSTN (10 mg/kg). Statistical analysis was by one-way ANOVA & Bonferroni *post-hoc* test comparing all groups at each week; error bars represent the S.E.M. **FIG. 11B** illustrates grip strength analysis of mouse forelimbs force in young dystrophic mice C57BL10 administered saline (positive control), mdx mice administered saline (negative control), mdx mice administered BPMO-M23D (10 mg/kg), mdx mice administered BPMO-M23D (10 mg/kg) & BPMO-MSTN (10 mg/kg), or mdx mice administered BPMO-MSTN (10 mg/kg).

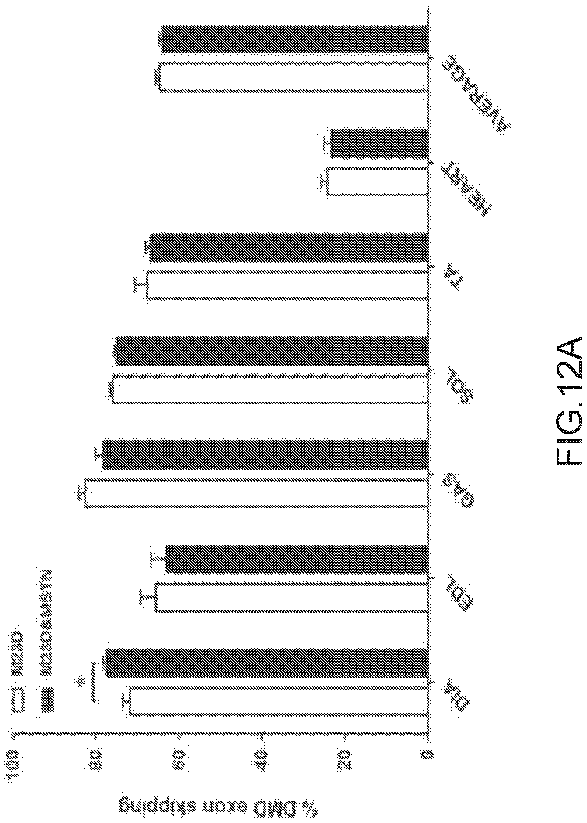

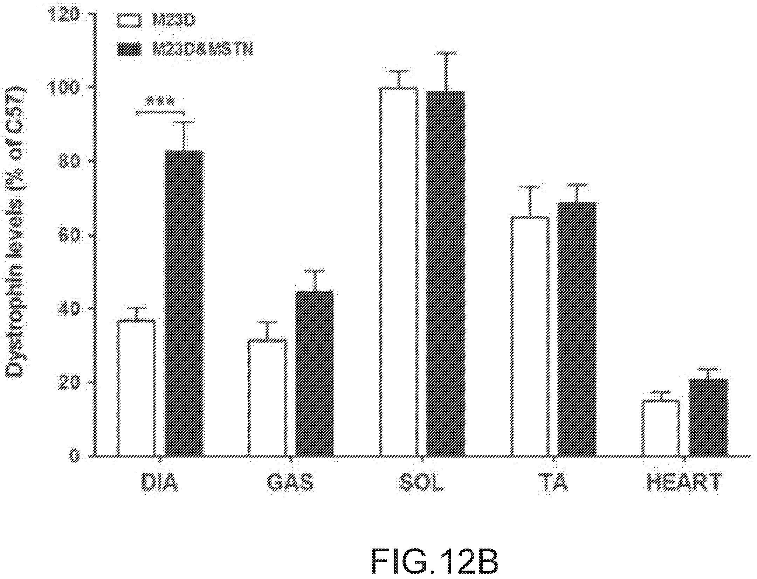

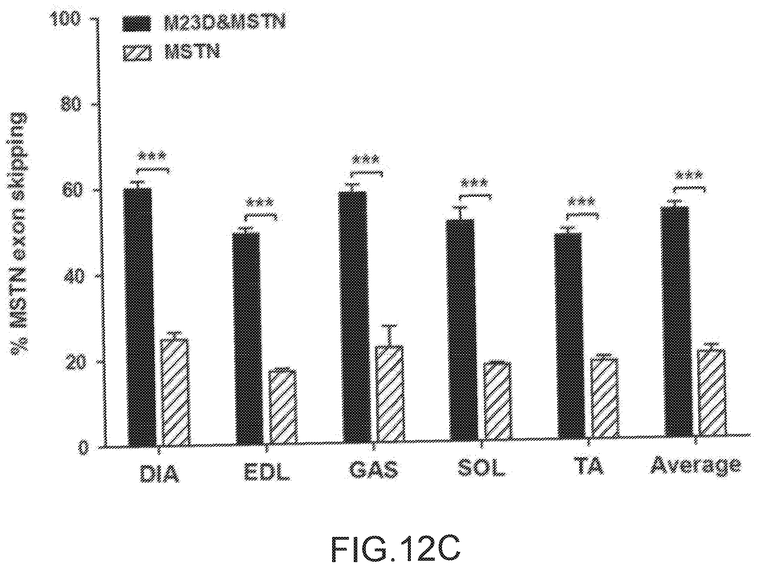

**FIG. 12A** illustrates quantification of dystrophin RNA reframing by exon skipping. **FIG. 12B** illustrates quantification of dystrophin protein expression by immunoblot. **FIG. 12C** illustrates quantification of myostatin exon 2 skipping.

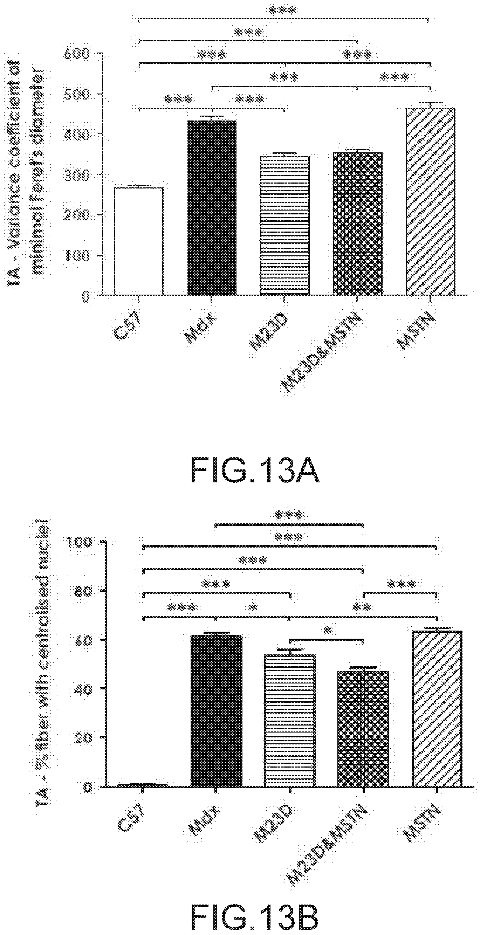

**FIG. 13A** illustrates variance coefficient of the minimal Feret's diameter in the TA of young dystrophin mice. **FIG. 13B** illustrates percentage of centrally nucleated fibers in TA muscles of young dystrophin mice.

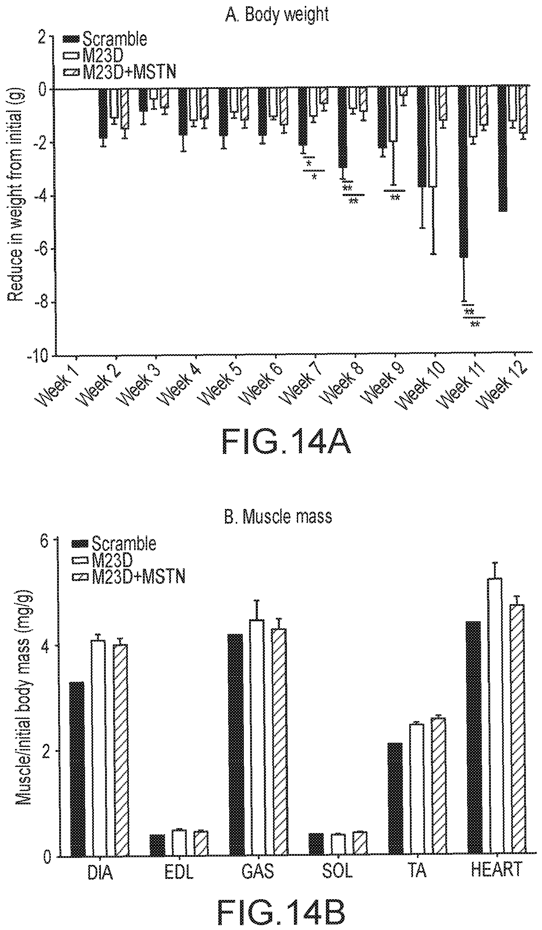

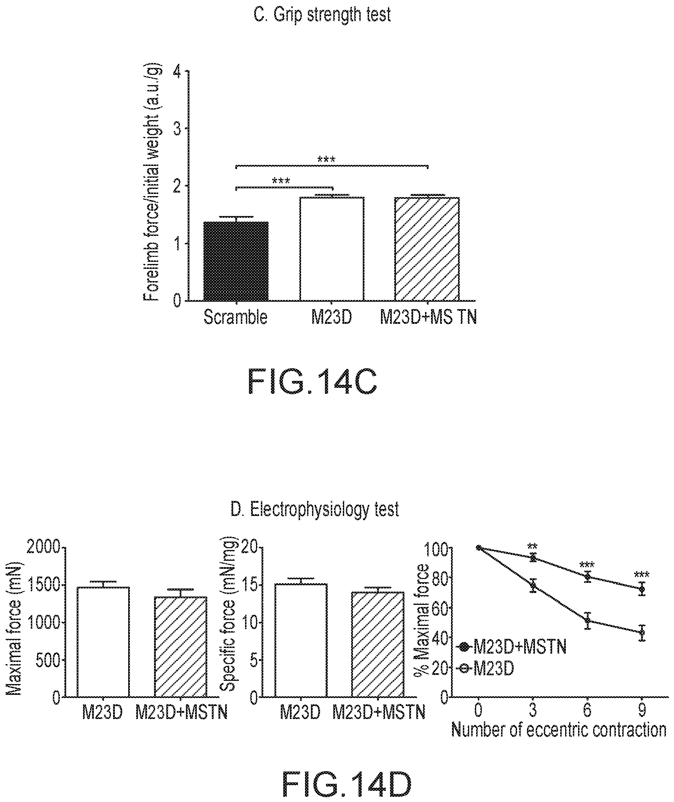

**FIG. 14A** illustrates increase in body weight in mdx mice. **FIG. 14B** illustrates increase in muscle mass in mdx mice administered BPMO-M23D or BPMO-M23D + BPMO-MSTN. **FIG. 14C** illustrates grip strength analysis of forelimb force in mdx mice administered BPMO-M23D or BPMO-M23D + BPMO-MSTN. **FIG. 14D** illustrates electrophysiology measurements *in situ* of mdx mice administered BPMO-M23D or BPMO-M23D + BPMO-MSTN.

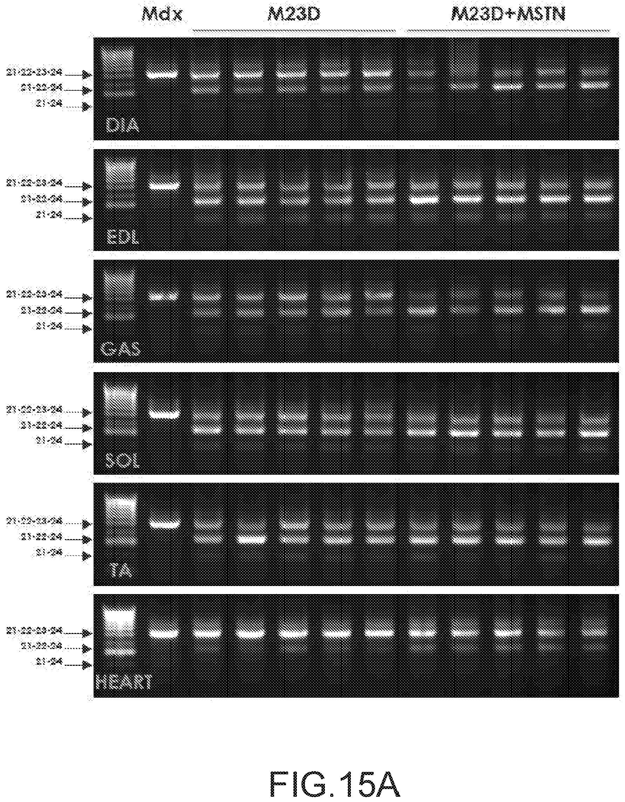

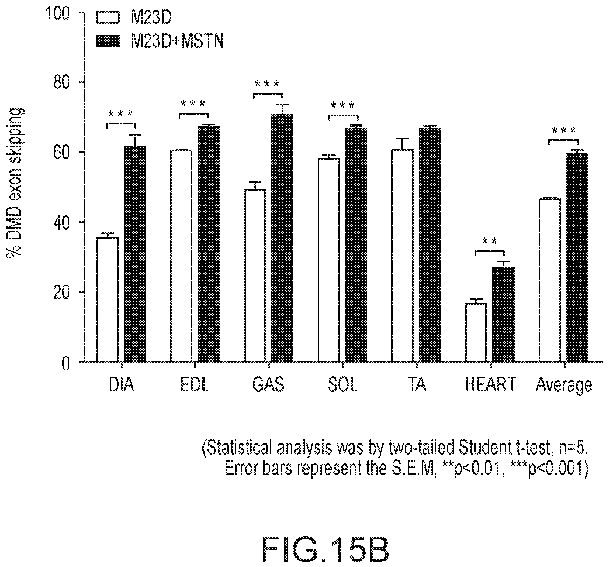

**FIG. 15A** illustrates a gel electrophoresis showing dystrophin RNA reframing by exon skipping in muscles of mdx mice administered BPMO-M23D or BPMO-M23D + BPMO-MSTN. **FIG. 15B** illustrates relative densitometric analysis of RT-PCR products in mdx mice administered BPMO-M23D or BPMO-M23D + BPMO-MSTN.

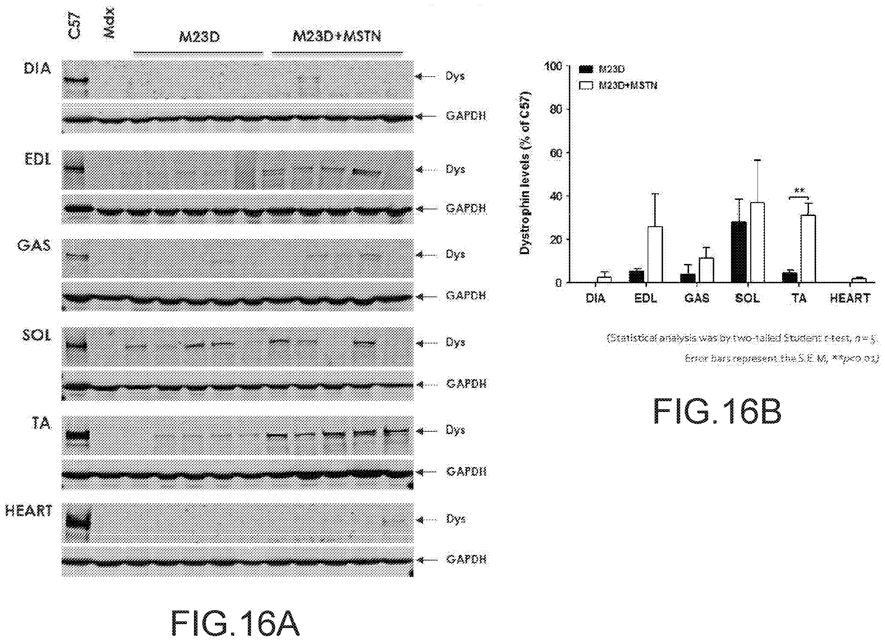

**FIG. 16A** illustrates dystrophin protein expression in muscles harvested from mdx mice administered BPMO-M23D or BPMO-M23D + BPMO-MSTN. **FIG. 16B** illustrates relative densitometric quantification of dystrophin protein expression in muscles harvested from mdx mice administered BPMO-M23D or BPMO-M23D + BPMO-MSTN.

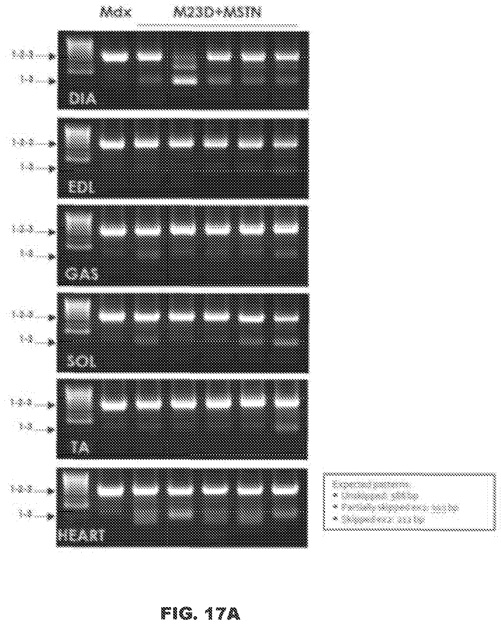

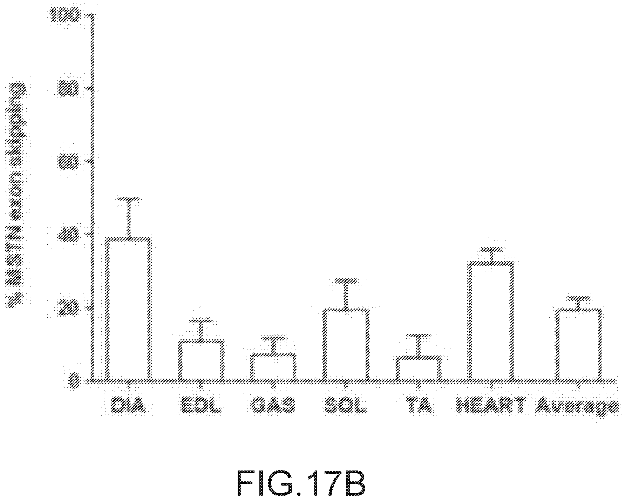

**FIG. 17A** illustrates a gel electrophoresis showing variable myostatin skipping in muscles of mdx mice administered BPMO-M23D or BPMO-M23D + BPMO-MSTN. **FIG. 17B** illustrates relative densitometric analysis of RT-PCR products of myostatin skipping in mdx mice administered BPMO-M23D or BPMO-M23D + BPMO-MSTN.

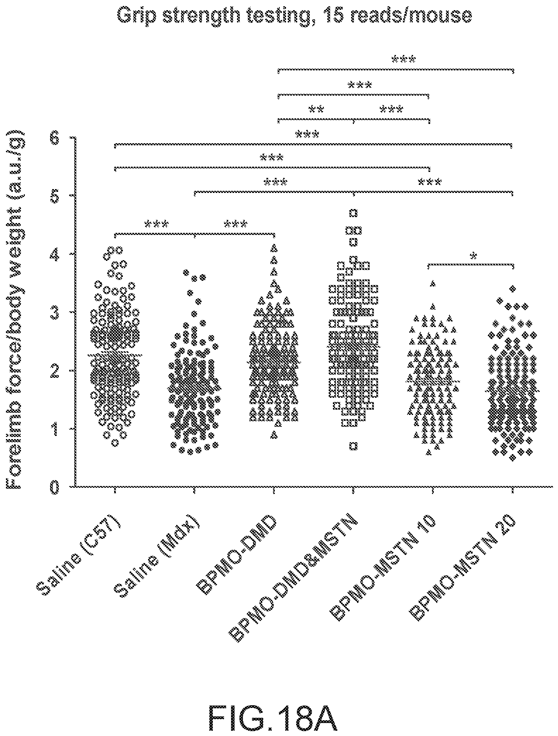

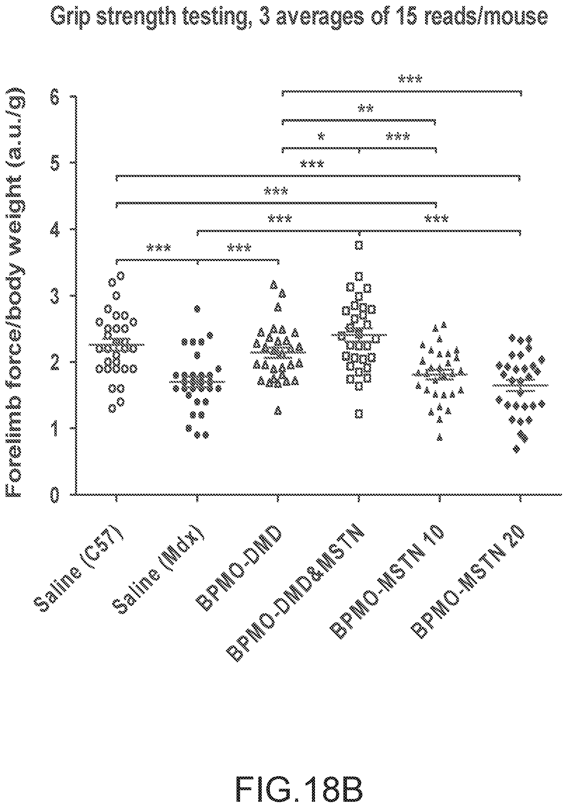

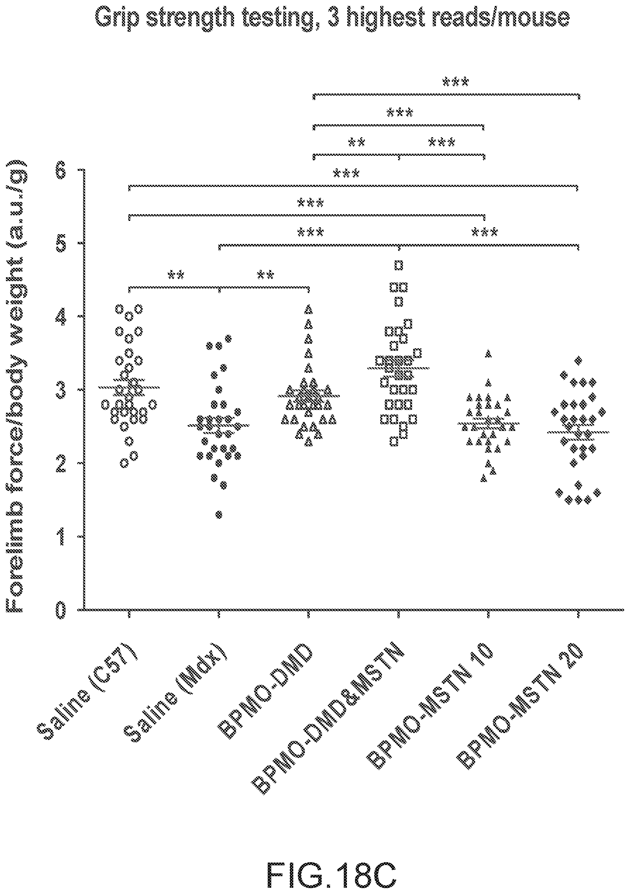

**FIG. 18A** illustrates gripstrength testing in mice 15 reads per mouse. **FIG. 18B** illustrates gripstength testing in mice 3 averages of 15 reads per mouse. **FIG. 18C** illustrates gripstrength testing in mice 3 highest reads per mouse.

### DETAILED DESCRIPTION

The following description is merely exemplary in nature and is not intended to limit the present invention, its applications, or its uses. It should be understood that throughout the drawings, corresponding reference numerals indicate like or corresponding parts and features. The description of specific examples indicated in various embodiments of the present invention are intended for purposes of illustration only and are not intended to limit the scope of the invention disclosed herein. Moreover, recitation of multiple embodiments having stated features is not intended to exclude other embodiments having additional features or other embodiments incorporating different combinations of the stated features.

Furthermore, the detailed description of various embodiments herein makes reference to the accompanying drawing FIGS, which show various embodiments by way of illustration. While the embodiments are described in sufficient detail to enable those skilled in the art to practice the invention, it should be understood that other embodiments may be realized and that logical and mechanical changes may be made without departing from the spirit and scope of the present invention. Thus, the detailed description herein is presented for purposes of illustration only and not of limitation. For example, steps or functions recited in descriptions, any method, system, or process, may be executed in any order and are not limited to the order presented. Moreover, any of the step or functions thereof may be outsourced to or performed by one or more third parties. Furthermore, any reference to singular includes plural embodiments, and any reference to more than one component may include a singular embodiment.

### I. Definitions

Unless defined otherwise, all technical and scientific terms used herein have the same meaning as commonly understood by those of ordinary skill in the art to which the disclosure belongs. Although any methods and materials similar or equivalent to those described herein can be used in the practice or testing of the subject matter of the present disclosure, preferred methods and materials are described. For the purposes of the present disclosure, the following terms are defined below.

The articles "a" and "an" are used herein to refer to one or to more than one (i.e., to at least one) of the grammatical object of the article. By way of example, "an element" means one element or more than one element.

The term "about" means a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length. When the term "about" is used in conjunction with a numerical range, it modifies that range by extending the boundaries above and below the numerical values set forth. In general, the term "about" is intended to modify a numerical value above and below the stated value by a variance of ≦ 10%.

Throughout this disclosure, unless the context requires otherwise, the words "comprise," "comprises," and "comprising" will be understood to imply the inclusion of a stated step or element or group of steps or elements but not the exclusion of any other step or element or group of steps or elements.

The term "consisting of' means including, and limited to, whatever follows the phrase "consisting of." Thus, the phrase "consisting of' indicates that the listed elements are required or mandatory, and that no other elements may be present. The term "consisting essentially of' means including any elements listed after the phrase, and limited to other elements that do not interfere with or contribute to the activity or action specified in the disclosure for the listed elements. Thus, the phrase "consisting essentially of' indicates that the listed elements are required or mandatory, but that other elements are optional and may or may not be present depending upon whether or not they materially affect the activity or action of the listed elements.

The terms "administering," or "administer" include delivery of the therapeutic agent including modified antisense oligomers of the disclosure to a subject either by local or systemic administration. Administration may be topical (including ophthalmic and to mucous membranes including vaginal and rectal delivery), pulmonary, e.g., by inhalation or insufflation of powders or aerosols, including by nebulizer, intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

"Co-administration" or "co-administering" or "combination therapy" as used herein, generally refers to the administration of a DMD exon-skipping antisense oligonucleotide in combination with one or more myostatin therapeutic compounds disclosed herein. In other words, the terms "co-administering" or "co-administration" or "combination therapy" mean the administration of the DMD exon-skipping antisense oligonucleotide, such as eteplirsen, concomitantly in a pharmaceutically acceptable dosage form with one or more myostatin therapeutic compounds and optionally one or more glucocorticoids disclosed herein: (i) in the same dosage form, e.g., the same tablet or pharmaceutical composition, meaning a pharmaceutical composition comprising a DMD exon-skipping antisense oligonucleotide, such as eteplirsen, one or more myostatin therapeutic compounds disclosed herein, and optionally one or more glucocorticoids and a pharmaceutically acceptable carrier; (ii) in a separate dosage form having the same mode of administration, e.g., a kit comprising a first pharmaceutical composition suitable for parenteral administration comprising a DMD exon-skipping antisense oligonucleotide, such as eteplirsen and a pharmaceutically acceptable carrier, a second pharmaceutical composition suitable for parenteral administration comprising one or more myostatin therapeutic compounds disclosed herein and a pharmaceutically acceptable carrier, and optionally a third pharmaceutical composition suitable for parenteral administration comprising one or more glucocorticoids disclosed herein and a pharmaceutically acceptable carrier; and (iii) in a separate dosage form having different modes of administration, e.g., a kit comprising a first pharmaceutical composition suitable for parenteral administration comprising a DMD exon-skipping antisense oligonucleotide, such as eteplirsen and a pharmaceutically acceptable carrier, a second pharmaceutical composition suitable for oral administration comprising one or more myostatin therapeutic compounds disclosed herein and a pharmaceutically acceptable carrier, and optionally a third pharmaceutical composition suitable for oral administration comprising one or more glucocorticoids disclosed herein and a pharmaceutically acceptable carrier.

Further, those of skill in the art given the benefit of the present disclosure will appreciate that when more than one myostatin therapeutic compound disclosed herein is being administered, the agents need not share the same mode of administration, e.g., a kit comprising a first pharmaceutical composition suitable for parenteral administration comprising a DMD exon-skipping antisense oligonucleotide, such as eteplirsen and a pharmaceutically acceptable carrier, a second pharmaceutical composition suitable for oral administration comprising a first myostatin therapeutic compound disclosed herein and a pharmaceutically acceptable carrier, and a third pharmaceutical composition suitable for parenteral administration comprising a second non-steroidal anti-inflammatory compound disclosed herein and a pharmaceutically acceptable carrier. Those of skill in the art will appreciate that the concomitant administration referred to above in the context of "co-administering" or "co-administration" means that the pharmaceutical composition comprising the DMD exon-skipping antisense oligonucleotide and a pharmaceutical composition(s) comprising the myostatin therapeutic compound can be administered on the same schedule, i.e., at the same time and day, or on a different schedule, i.e., on different, although not necessarily distinct, schedules.

In that regard, when the pharmaceutical composition comprising a DMD exon-skipping antisense oligonucleotide and a pharmaceutical composition(s) comprising the myostatin therapeutic compound is administered on a different schedule, such a different schedule may also be referred to herein as "background" or "background administration." For example, the pharmaceutical composition comprising a DMD exon-skipping antisense oligonucleotide may be administered in a certain dosage form twice a day, and the pharmaceutical composition(s) comprising the myostatin therapeutic compound may be administered once a day, such that the pharmaceutical composition comprising the DMD exon-skipping antisense oligonucleotide may but not necessarily be administered at the same time as the pharmaceutical composition(s) comprising the myostatin therapeutic compound during one of the daily administrations. Of course, other suitable variations to "co-administering", "co-administration" or "combination therapy" will be readily apparent to those of skill in the art given the benefit of the present disclosure and are part of the meaning of this term.

"Chronic administration," as used herein, refers to continuous, regular, long-term therapeutic administration, *i.e.,* periodic administration without substantial interruption. For example, daily, for a period of time of at least several weeks or months or years, for the purpose of treating muscular dystrophy in a patient. For example, weekly, for a period of time of at least several months or years, for the purpose of treating muscular dystrophy in a patient (e.g., weekly for at least six weeks, weekly for at least 12 weeks, weekly for at least 24 weeks, weekly for at least 48 weeks, weekly for at least 72 weeks, weekly for at least 96 weeks, weekly for at least 120 weeks, weekly for at least 144 weeks, weekly for at least 168 weeks, weekly for at least 180 weeks, weekly for at least 192 weeks, weekly for at least 216 weeks, or weekly for at least 240 weeks). In certain embodiments, the DMD exon skipping compound, such as eteplirsen, is chronically administered 30 mg/kg once weekly via an intravenous infusion in combination with a myostatin therapeutic compound disclosed herein.

The terms "contacting a cell," "introducing" or "delivering" include delivery of the therapeutic agents of the disclosure into a cell by methods routine in the art, including, transfection (e.g., liposome, calcium-phosphate, polyethyleneimine), electroporation (e.g., nucleofection), microinjection).

The term "alkyl" refers to a linear (i.e., unbranched or acyclic), branched, cyclic, or polycyclic non aromatic hydrocarbon groups, which are optionally substituted with one or more functional groups. Unless otherwise specified, "alkyl" groups contain one to eight, and preferably one to six carbon atoms. C₁-C₆ alkyl, is intended to include at least C₁, C₂, C₃, C₄, C₅, and C₆ alkyl groups. Lower alkyl refers to alkyl groups containing 1 to 6 carbon atoms. Examples of alkyl include, but are not limited to, methyl, ethyl, n-propyl, isopropyl, cyclopropyl, butyl, isobutyl, sec-butyl, tert-butyl, cyclobutyl, pentyl, isopentyl tert-pentyl, cyclopentyl, hexyl, isohexyl, cyclohexyl, etc. Alkyl may be substituted or unsubstituted. Illustrative substituted alkyl groups include, but are not limited to, fluoromethyl, difluoromethyl, trifluoromethyl, 2-fluoroethyl, 3-fluoropropyl, hydroxymethyl, 2-hydroxyethyl, 3-hydroxypropyl, benzyl, substituted benzyl, phenethyl, substituted phenethyl, etc.

The term "alkoxy" refers to a subset of alkyl in which an alkyl group as defined above with the indicated number of carbons attached through an oxygen bridge. For example, "alkoxy" refers to groups -O-alkyl, where the alkyl group contains 1 to 8 carbons atoms of a linear, branched, cyclic configuration. Examples of "alkoxy" include, but are not limited to, methoxy, ethoxy, n-propoxy, i-propoxy, t-butoxy, n-butoxy, s-pentoxy and the like.

The term "aryl" used alone or as part of a larger moiety as in "aralkyl,", "aralkoxy," or "aryloxy-alkyl," refers to aromatic ring groups having six to fourteen ring atoms, such as phenyl, 1-naphthyl, 2-naphthyl, 1-anthracyl and 2-anthracyl. An "aryl" ring may contain one or more substituents. The term "aryl" may be used interchangeably with the term "aryl ring." "Aryl" also includes fused polycyclic aromatic ring systems in which an aromatic ring is fused to one or more rings. Non-limiting examples of useful aryl ring groups include phenyl, hydroxyphenyl, halophenyl, alkoxyphenyl, dialkoxyphenyl, trialkoxyphenyl, alkylenedioxyphenyl, naphthyl, phenanthryl, anthryl, phenanthro and the like, as well as 1-naphthyl, 2-naphthyl, 1-anthracyl and 2-anthracyl. Also included within the scope of the term "aryl," as it is used herein, is a group in which an aromatic ring is fused to one or more nonaromatic rings, such as in an indanyl, phenanthridinyl, or tetrahydronaphthyl, where the radical or point of attachment is on the aromatic ring.

The term "acyl" refers to a C(O)R group (in which R signifies H, alkyl or aryl as defined above). Examples of acyl groups include formyl, acetyl, benzoyl, phenylacetyl and similar groups.

The term "homolog" refers to compounds differing regularly by the successive addition of the same chemical group. For example, a homolog of a compound may differ by the addition of one or more -CH2- groups, amino acid residues, nucleotides, or nucleotide analogs.

The terms "cell penetrating peptide" (CPP) or "a peptide moiety which enhances cellular uptake" are used interchangeably and refer to cationic cell penetrating peptides, also called "transport peptides," "carrier peptides," or "peptide transduction domains." For example, a peptide-conjugated phosphoramidate or phosphorodiamidate morpholino (PPMO) may include a cell penetrating peptide or peptide moiety which enhances cellular uptake as described herein. In various embodiments, a peptide may be covalently bonded to the modified antisense oligomer. In further embodiments, a peptide may be conjugated to the 3' end or the 5' end of the modified antisense oligomer. In further embodiments, a peptide may be linked to a piperazinyl moiety or to a nitrogen atom of the 3' terminal morpholino ring. In some embodiments, a cell penetrating peptide or peptide moiety which enhances cellular uptake may include an arginine-rich peptide as described herein. In a non-limiting example, modified antisense oligomers as disclosed herein can be coupled to an arginine-rich peptide such as (Arg)₆Gly (6 arginine and 1 glycine linked to an oligonucleotide).

The peptides, as shown herein, have the capability of inducing cell penetration within about or at least about 30%, 40%, 50%, 60%, 70%, 80%, 90%, or 100% of cells of a given cell culture population and allow macromolecular translocation within multiple tissues in vivo upon systemic administration. In some embodiments, the CPPs are of the formula [(C(O)CHR'NH)ₘ]R" where R' is a side chain of a naturally occurring amino acid or a one- or two-carbon homolog thereof, R" is selected from Hydrogen or acyl, and m is an integer up to 50. Additional CPPs are well-known in the art and are disclosed, for example, in U.S. Published Application No. 20100016215, which is hereby incorporated by reference in its entirety. In other embodiments, m is an integer selected from 1 to 50 where, when m is 1, the moiety is a single amino acid or derivative thereof.

The term "amino acid" refers to a compound comprising a carbon atom to which are attached a primary amino group, a carboxylic acid group, a side chain, and a hydrogen atom. For example, the term "amino acid" includes, but is not limited to, Glycine, Alanine, Valine, Leucine, Isoleucine, Asparagine, Glutamine, Lysine, Aspartic Acid, Histidine, Methionine, Proline, Phenylalanine, Threonine, Tryptophan, Cysteine, Glutamic Acid, Serine, Tyrosine, Pyrolysine, Selenocystenine and Arginine. Additionally, as used herein, "amino acid" also includes derivatives of amino acids such as esters, and amides, and salts, as well as other derivatives, including derivatives having pharmaco properties upon metabolism to an active form. Accordingly, the term "amino acid" is understood to include naturally occurring and non-naturally occurring amino acids.

The term "an electron pair" refers to a valence pair of electrons that are not bonded or shared with other atoms.

The term "homology" refers to the amount or degree of similarity between two or more amino acid sequences or two or more nucleotide sequences. In some examples, sequence homology may include one or more conservative substitutions such that one or more substitutions would not affect the basic structure or function of a subject protein A conservative nucleotide substitution may include a substitution of one nucleic acid for another such that the substitution does not alter the amino acid encoded by the codon. A conservative amino acid substitution may include a substitution of one amino acid for another such that the substituted amino acid is of the same or similar class as the substituting amino acid, for example substitution of an aliphatic amino acid with another aliphatic amino acid. Homology may be determined using sequence comparison programs such as GAP (Deveraux et al., 1984, Nucleic Acids Research 12, 387-395). In this way sequences of a similar or substantially different length to those cited herein could be compared by insertion of gaps into the alignment, such gaps being determined, for example, by the comparison algorithm used by GAP.

The term "isolated" refers to a material that is substantially or essentially free from components that normally accompany it in its native state. For example, an "isolated oligonucleotide," or "isolated oligomer" as used herein, may refer to an oligomer that has been purified or removed from the sequences that flank it in a naturally-occurring state, e.g., a DNA fragment that is removed from the sequences that are adjacent to the fragment in the genome. The term "isolating" as it relates to cells may refer to the purification of cells (e.g., fibroblasts, lymphoblasts) from a source subject (e.g., a subject with an oligonucleotide repeat disease). In the context of mRNA or protein, "isolating" may refer to the recovery of mRNA or protein from a source, e.g., cells.

The term "modulate" includes to "increase" or "decrease" one or more quantifiable parameters, optionally by a defined and/or statistically significant amount. By "increase" or "increasing," "enhance" or "enhancing," or "stimulate" or "stimulating," refers generally to the ability of one or more modified antisense oligomer compounds or compositions, and/or one or more therapeutic agents to produce or cause a greater physiological response (e.g., downstream effects) in a cell or a subject relative to the response caused by either no antisense oligomer compound and/or therapeutic agent, or a control compound. Relevant physiological or cellular responses (*in vivo* or *in vitro*) will be apparent to persons skilled in the art, and may include a decrease in the inclusion of exon 2 (or an increase in the exclusion of exon 2) in myostatin mRNA, and/or a decrease in the expression of functional myostatin protein in a cell, or tissue, such as in a subject in need thereof. Other relevant physiological or cellular responses (*in vivo* or *in vitro*) may include a decrease in the inclusion of (or an increase in the exclusion of) one or more exons having a genetic mutation in dystrophin mRNA, and/or an increase in the expression of functional or semi-functional dystrophin protein in a cell, or tissue. A "decreased" or "reduced" amount is typically a "statistically significant" amount, and may include a decrease that is 1, 2, 3, 4, 5, 6, 7, 8, 9, 10, 15, 20, 30, 40, 50 or more times less (e.g., 100, 500, 1000 times), including all integers and decimal points in between and above 1 (e.g., 1.1, 1.2, 1.3, 1.4, 1.5, 1.6, 1.7, 1.8, 1.9), in comparison to the amount produced by a subject in need thereof in the absence of administration of a modified antisense oligomer compound and/or therapeutic (e.g. the "native" or "natural" rate of expression of a specific subject or cohort) or a control compound. The terms "reduce" or "inhibit" may relate generally to the ability of one or more antisense oligomer compounds or compositions, and/or one or more therapeutic to "decrease" a relevant physiological or cellular response, such as a symptom of a disease or condition described herein, as measured according to routine techniques in the diagnostic art. An "increased" or "enhanced" amount is typically a "statistically significant" amount, and may include an increase that is 1, 2, 3, 4, 5, 6, 7, 8, 9, 10, 15, 20, 30, 40, 50 or more times greater than (e.g., 100, 500, 1000 times), including all integers and decimal points in between and above 1 (e.g., 1.1, 1.2, 1.3, 1.4, 1.5, 1.6, 1.7, 1.8, 1.9), in comparison to the amount produced by a subject in need thereof in the absence of administration of a modified antisense oligomer compound and/or therapeutic (e.g. the "native" or "natural" rate of expression of a specific subject or cohort) or a control compound. The term "enhance" may relate generally to the ability of one or more modified antisense oligomer compounds or compositions, and/or one or more therapeutic to "increase" a relevant physiological or cellular response, such as a symptom of a disease or condition described herein, as measured according to routine techniques in the diagnostic art.

Relevant physiological or cellular responses (*in vivo* or *in vitro*) will be apparent to persons skilled in the art, and may include reductions in the symptoms or pathology of Duchenne muscular dystrophy (DMD) and related disorders, such as Becker muscular dystrophy (BMD), limb-girdle muscular dystrophy, congenital muscular dystrophy, facioscapulohumeral muscular dystrophy, myotonic muscular dystrophy, oculopharyngeal muscular dystrophy, distal muscular dystrophy, Emery-Dreifuss muscular dystrophy, muscle wasting conditions or disorders, such as AIDS, cancer or chemotherapy related muscle wasting, and fibrosis or fibrosis-related disorders (for example, skeletal muscle fibrosis). In other embodiments, methods of treating Duchenne muscular dystrophy and related disorders are provided, for example, where a reduction in symptoms or pathology may accompany or relate to an increase in the expression of functional dystrophin protein and/or a decrease in the expression of functional myostatin protein. An "increase" in a response may be "statistically significant" as compared to the response produced by a subject in need thereof in the absence of administration of a modified antisense oligomer compound and/or therapeutic (e.g. when compared to the "native" or "natural" rate of expression of a specific subject or cohort) or when compared to a control compound, and may include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% increase, including all integers in between.

The term "therapeutic" or "therapeutic agent" as used herein means an agent capable of producing a therapeutic effect. In some embodiments, a therapeutic is, or comprises a polypeptide, a polypeptide analog, a nucleic acid, a nucleic acid analog, an aptamer, or a small molecule.. "Polypeptide," "peptide," and "protein" are used interchangeably and mean any peptide-linked chain of amino acids, regardless of length or post-translational modification. A polypeptide can be wildtype proteins, functional fragments of a wildtype protein, or variants of a wildtype protein or fragment. Variants can comprise one or more amino acid substitutions, deletions, or insertions. The substitutions can be conservative or non-conservative. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine. In some embodiments, a protein includes an antibody or a soluble receptor. In embodiments, a soluble receptor is ACVR2 (e.g., ACVR2B). In some embodiments, the nucleic acid is one that encodes a protein, such as dystrophin, microdystrophin, or minidystrophin. In embodiments, a nucleic acid is an antisense oligomer or a siRNA. In some embodiments, an antisense oligomer is a modified antisense oligomer as described herein.

As used herein, the term "antibody" refers to a whole antibody comprising two light chain polypeptides and two heavy chain polypeptides. Whole antibodies include different antibody isotypes including IgM, IgG, IgA, IgD, and IgE antibodies. The term "antibody" includes a polyclonal antibody, a monoclonal antibody, a chimerized or chimeric antibody, a humanized antibody, a primatized antibody, a deimmunized antibody, and a fully human antibody. The antibody can be made in or derived from any of a variety of species, e.g., mammals such as humans, non-human primates (e.g., orangutan, baboons, or chimpanzees), horses, cattle, pigs, sheep, goats, dogs, cats, rabbits, guinea pigs, gerbils, hamsters, rats, and mice. The antibody can be a purified or a recombinant antibody.

As used herein, the term "antibody fragment," "antigen-binding fragment," or similar terms refer to a fragment of an antibody that retains the ability to bind to a target antigen and inhibit the activity of the target antigen. Such fragments include, e.g., a single chain antibody, a single chain Fv fragment (scFv), an Fd fragment, an Fab fragment, an Fab' fragment, or an F(ab')2 fragment. A scFv fragment is a single polypeptide chain that includes both the heavy and light chain variable regions of the antibody from which the scFv is derived. In addition, intrabodies, minibodies, triabodies, and diabodies are also included in the definition of antibody and are compatible for use in the methods described herein. See, e.g., Todorovska et al. (2001) J Immunol Methods 248(1):47-66; Hudson and Kortt (1999) J Immunol Methods 231(1):177-189; Poljak (1994) Structure 2(12):1121-1123; Rondon and Marasco (1997) Annual Review of Microbiology 51:257-283, each of which are incorporated herein by reference in their entirety.

As used herein, the term "antibody fragment" also includes, e.g., single domain antibodies such as camelized single domain antibodies. See, e.g., Muyldermans et al. (2001) Trends Biochem Sci 26:230-235; Nuttall et al. (2000) Curr Pharm Biotech 1:253-263; Reichmann et al. (1999) J Immunol Meth 231:25-38; PCT application publication nos. WO 94/04678 and WO 94/25591; and U.S. Pat. No. 6,005,079, each of which are incorporated herein by reference in their entirety. In some embodiments, the disclosure provides single domain antibodies comprising two VH domains with modifications such that single domain antibodies are formed.

In some embodiments, an antigen-binding fragment includes the variable region of a heavy chain polypeptide and the variable region of a light chain polypeptide. In some embodiments, an antigen-binding fragment described herein comprises the CDRs of the light chain and heavy chain polypeptide of an antibody.

Myostatin, also referred to as growth differentiation factor 8 (GDF-8), belongs to the transforming growth factor-beta (TGF-β) superfamily. Myostatin is a protein encoded by the MSTN gene. The myostatin amino acid sequence is MQKLQLCVYIYLFMLIV AGPVDLNENSEQKENVEKEGLCNACTWRQNTKSSRIEAIKIQ ILSKLRLETAPNISKDVIRQLLPKAPPLRELIDQYDVQRDDSSDGSLEDDDYHATTETIIT MPTESDFLMQVDGKPKCCFFKFSSKIQYNKVVKAQLWIYLRPVETPTTVFVQILRLIKP MKDGTRYTGIRSLKLDMNPGTGIWQSIDVKTVLQNWLKQPESNLGIEIKALDENGHDL AVTFPGPGEDGLNPFLEVKVTDTPKRSRRDFGLDCDEHSTESRCCRYPLTVDFEAFGWD WIIAPKRYKANYCSGECEFVFLQKYPHTHL VHQANPRGSAGPCCTPTKMSPINML YFNG KEQIIYGKIPAMVVDRCGCS (SEQ ID NO: 3502). The MSTN gene is largely expressed in human skeletal muscle and acts as a negative regulator of muscle growth. For example, in mice engineered to lack the myostatin gene demonstrate the development of twice the muscle mass of normal mice (McPherron et al., (1997), Nature 387:83-90).

In embodiments, a myostatin therapeutic is capable of suppressing one or both of myostatin activity and myostatin expression in a subject. A myostatin therapeutic may be a therapeutic that targets myostatin pre-mRNA and interferes with transcription of the myostatin pre-mRNA to mature mRNA. In embodiments, a myostatin therapeutic is capable of inducing exon skipping during the processing of human myostatin pre-mRNA. In embodiments, a myostatin therapeutic induces skipping of exon 2 in myostatin pre-mRNA and inhibits the expression of exon 2 containing myostatin pre-mRNA. A myostatin therapeutic may be a therapeutic that targets myostatin protein and interferes with the myostatin protein binding with the myostatin receptor. A myostatin therapeutic protein may be an anti-myostatin antibody, for example anti-GDF8 (Abcam, Cambridge MA), Domagrozumab (PF-06252616; Pfizer Inc.); Stamulumab (Cambridge Antibody Technology); PF-3446879 (Pfizer Inc.); Landogrozumab (LY-2495655; Eli Lilly); or Trevogrumab (REGN-103; Regeneron). In embodiments, a myostatin therapeutic may be a soluble receptor where the soluble receptor is ACVR2 (e.g., ACVR2B;
MTAPWVALALLWGSLCAGSGRGEAETRECIYYNANWELERTNQSGLERCEGEQDKRL HCYASWRNSSGTIELVKKGCWLDDFNCYDRQECVATEENPQVYFCCCEGNFCNERFTH LPEAGGPEVTYEPPPTAPTLLTVLAYSLLPIGGLSLIVLLAFWMYRHRKPPYGHVDIHED PGPPPPSPLVGLKPLQLLEIKARGRFGCVWKAQLMNDFVAVKIFPLQDKQSWQSEREIFS TPGMKHENLLQFIAAEKRGSNLEVELWLITAFHDKGSLTDYLKGNIITWNELCHVAETM SRGLSYLHEDVPWCRGEGHKPSIAHRDFKSKNVLLKSDLTAVLADFGLAVRFEPGKPPG DTHGQVGTRRYMAPEVLEGAINFQRDAFLRIDMYAMGLVLWELVSRCKAADGPVDEY MLPFEEEIGQHPSLEELQEVVVHKKMRPTIKDHWLKHPGLAQLCVTIEECWDHDAEAR LSAGCVEERVSLIRRSVNGTTSDCLVSLVTSVTNVDLPPKESSI; SEQ ID NO: 3503). In some embodiments, the soluble receptor (e.g., ACVR2B) is conjugated to a heterologous moiety, e.g., a moiety that increases the circulatory half-life of the therapeutic in a subject. In some embodiments, the moiety is the Fc portion of an immunoglobulin (e.g., a human IgG Fc). In some embodiments, the moiety is a polyethylene glycol moiety. In some embodiments, the moiety comprises all or a portion of an albumin polypeptide (e.g., human albumin). In some embodiments, the myostatin therapeutic is a human ACVR2-Fc fusion, e.g., ramatercept (Acceleron). A myostatin therapeutic includes a nucleic acid where the nucleic acid is selected from an antisense oligomer and a siRNA. An antisense oligomer may be a modified myostatin antisense oligomer as described herein. In some embodiments, the myostatin therapeutic is a small molecule, such as OSX-200 (Ossianix Inc) or SRK-015 (Scholar Rock Inc.).

Antagonists of myostatin useful in the methods and compositions described herein include, e.g., agents that bind to directly to myostatin (GDF-8), such as anti-myostatin antibodies. Such antibodies are known in the art and described in, e.g., International Patent Application Publication No. WO2006116269 (Pfizer), U.S. Patent No. 8,066,996 (Eli Lilly), U.S. Patent No. 7,807,159 (Amgen), U.S. Patent No. 6,096,506, and U.S. Patent No. 6,468,535, the disclosures of each of which are incorporated herein by reference in their entirety. Myostatin antagonists also include soluble Activin receptor proteins, or fusion protein comprising soluble Activin proteins (e.g., ACVR2-Fc fusion proteins). Soluble Activin receptor proteins are described in, e.g., International Patent Application Publication No. WO 2010129406 (Johns Hopkins University), U.S. Patent Application Publication No. 20090005308 (Acceleron), International Patent Application Publication No. WO 2008/097541 (Acceleron), and International Patent Application Publication No. WO 2010019261 (Acceleron), the disclosures of each of which are incorporated herein by reference in their entirety. In some embodiments, the myostatin antagonist is a nucleic acid that inhibits expression of myostatin, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against myostatin. Such molecules are described in, e.g., U.S Patent Application Publication No. 20050124566 and U.S. Patent No. 7,887,793, the disclosures of each of which are incorporated herein by reference in their entirety. In some embodiments, the nucleic acids inhibit the promoter of myostatin to thereby inhibit myostatin expression, as described in, e.g., U.S. Patent No. 6,284,882 to Abbott Laboratories. Inhibitors of myostatin also include agents that inhibit myostatin signaling via its receptor, such as anti-ACVR2B antibodies (see, e.g., U.S. Patent Application Publication No. 20100272734 (Novartis) and International Patent Application Publication No. WO2014172448 (Anaptysbio)). Yet additional exemplary inhibitors of myostatin are described in International Patent Application Publication No. WO 2006/083183.

In some embodiments, a dystrophin therapeutic is administered first in time and the myostatin therapeutic is administered second in time. For example, the dystrophin therapeutic is administered for a time sufficient to promote, restore, and/or increase expression of functional dystrophin protein in muscle of the subject to which the therapeutic is administered. Subsequently, the myostatin therapeutic is administered to the subject for a time sufficient to, e.g., enhance muscle mass, strength, and/or elasticity in the subject.In embodiments, a dystrophin therapeutic is capable of increasing expression of dystrophin in a subject. A dystrophin therapeutic may increase the expression of dystrophin or a truncated form of dystrophin that is functional or semi-functional. A truncated form of dystrophin includes, but is not limited to, micro-dystrophin and mini-dystrophin (disclosed in EP Patent no. 2125006, which is hereby incorporated by reference in its entirety). A dystrophin therapeutic may be a therapeutic that targets dystrophin pre-mRNA and modulates the transcription of the dystrophin pre-mRNA to mature mRNA, for example, a modified antisense oligomer as described herein. In embodiments, a dystrophin therapeutic is capable of inducing exon skipping during processing of human dystrophin pre-mRNA. In embodiments, a targeted dystrophin pre-mRNA may have one or more genetic mutations. A dystrophin therapeutic induces exon skipping such that one or more exons containing one or more genetic mutations are removed from the dystrophin pre-mRNA during processing to mature mRNA. The resulting truncated mRNA may be translated into a functional or semi-functional dystrophin protein.

In some embodiments, the dystrophin therapeutic is or comprises a nucleic acid encoding a functional dystrophin protein, e.g., a microdystrophin or minidystrophin protein. In some embodiments, the nucleic acid is introduced into muscle cells of the subject by means of viral delivery. In some embodiments, expression of the functional dystrophin protein from the nucleic acid is driven by a muscle-specific promoter, such as the promoter for muscle creatine kinase (MCK). The use of viral vectors comprising a functional dystrophin protein for DMD gene therapy has been described in, e.g., Shin et al. (2013) Mol Ther 21(4):750-757; Rodino-Klapac et al. (2011) Methods Mol Biol 709:287-298; Okada et al. (2013) Pharmaceuticals 6(7):813-836; Rodino-Klapac et al. (2010) Mol Ther 18(1):109-117; Vincent et al. (1993) Nature Genetics 5:130-134; Xu et al. (2007) Neuromusc Disorders 17: 209-220; Martin et al. (2009) Am J Physiol Cell Physiol 296: 476-488; International Patent Application Publication No. WO 2009/088895, and U.S. Patent Application Publication Nos. 2010003218 and 20140323956, the disclosures of each of which are incorporated herein by reference in their entirety. One of skill in the art is also well aware of other vector systems that can be used to deliver a transgene to cells of interest, e.g., U.S. Patent No. 5,707,618; Verhaart et al. (2012) Curr Opin Neurol 25(5):588-596; Odom et al. (2011) Mol Ther 19(1):36-45; and Koppanati et al. (2010) Gene Ther 17(11):1355-1362. Ongoing clinical studies evaluating transgenic delivery of functional dystrophin protein include, e.g., the studies having U.S. ClinicalTrials.gov identifiers: NCT02376816 (Nationwide Children's Hospital) and NCT00428935 (Nationwide Children's Hospital) as well as the trial described by Bowles et al. (2012) Mol Ther 20(2):443-455.

One of skill in the art is well aware that various mutations in the dystrophin gene are amenable to therapeutic exon skipping. For example, non-limiting examples of mutations in the following exons are amenable to exon 51 skipping include, e.g.: 45-50, 47-50, 48-50, 49-50, 50, 52, 52-63 (Leiden Duchenne muscular dystrophy mutation database, Leiden University Medical Center, The Netherlands). Determining whether a patient has a mutation in the DMD gene that is amenable to exon skipping is also well within the purview of one of skill in the art (see, e.g., Aartsma-Rus et al. (2009) Hum Mut 30:293-299 and Abbs et al. (2010) Neuromusc Disorders 20:422-427, the disclosures of each of which are incorporated herein by reference in their entirety).

Eteplirsen (see e.g., U.S. Patent No. 7,807,816, incorporated herein by reference in its entirety) has been the subject of clinical studies to test its safety and efficacy, and clinical development is ongoing. Eteplirsen is a phosphorodiamidate mopholino (PMO) antisense oligonucleotide. In some embodiments, the dystrophin therapeutic is eteplirsen. "Eteplirsen", also known as "AVN-4658" is a PMO having the base sequence 5'-CTCCAACATCAAGGAAGATGGCATTTCTAG-3' (SEQ ID NO: 76). Eteplirsen is registered under CAS Registry Number 1173755-55-9. Chemical names include: RNA, [*P*-deoxy-*P-*(dimethylamino)](2',3'-dideoxy-2',3'-imino-2',3'-seco)(2'a→5')(C-m5U-C-C-A-A-C-A-m5U-C-A-A-G-G-A-A-G-A-m5U-G-G-C-A-m5U-m5U-m5U-C-m5U-A-G) (SEQ ID NO: 263), 5'-[*P*-[4-[[2-[2-(2-hydroxyethoxy)ethoxy]ethoxy]carbonyl]-1-piperazinyl]-*N*,*N*-dimethylphosphonamidate] and P,2',3'-trideoxy-P-(dimethylamino)-5'-O-{P-[4-(10-hydroxy-2,5,8-trioxadecanoyl)piperazin-1-yl]-N,N-dimethylphosphonamidoyl}-2',3'-imino-2',3'-secocytidylyl-(2'a→5')-P,3'-dideoxy-P-(dimethylamino)-2',3'-imino-2',3'-secothymidylyl- (2'a→5')-P,2',3'-trideoxy-P-(dimethylamino)-2',3'-imino-2',3'-secocytidylyl-(2'a→5')-P,2',3'-trideoxy-P-(dimethylamino)-2',3'-imino-2',3'-secocytidylyl-(2'a→5')-P,2',3'- trideoxy-P-(dimethylamino)-2',3'-imino-2',3'-secoadenylyl-(2'a→5')-P,2',3'-trideoxy-P- (dimethylamino)-2',3'-imino-2',3'-secoadenylyl-(2'a→5')-P,2',3'-trideoxy-P- (dimethylamino)-2',3'-imino-2',3'-secocytidylyl-(2'a→5')-P,2',3'-trideoxy-P- (dimethylamino)-2',3'-imino-2',3'-secoadenylyl-(2'a→5')-P,3'-dideoxy-P- (dimethylamino)-2',3'-imino-2',3'-secothymidylyl-(2'a→5')-P,2',3'-trideoxy-P-(dimethylamino)-2',3'-imino-2',3'-secocytidylyl-(2'a→5')-P,2',3'-trideoxy-P- (dimethylamino)-2',3'-imino-2',3'-secoadenylyl-(2'a→5')-P,2',3'-trideoxy-P- (dimethylamino)-2',3'-imino-2',3'-secoadenylyl-(2'a→5')-P,2',3'-trideoxy-P- (dimethylamino)-2',3'-imino-2',3'-secoguanylyl-(2'a→5')-P,2',3'-trideoxy-P- (dimethylamino)-2',3'-imino-2',3'-secoguanylyl-(2'a→5')-P,2',3'-trideoxy-P- (dimethylamino)-2',3'-imino-2',3'-secoadenylyl-(2'a→5')-P,2',3'-trideoxy-P-(dimethylamino)-2',3'-imino-2',3'-secoadenylyl-(2'a→5')-P,2',3'-trideoxy-P- (dimethylamino)-2',3'-imino-2',3'-secoguanylyl-(2'a→5')-P,2',3'-trideoxy-P- (dimethylamino)-2',3'-imino-2',3'-secoadenylyl-(2'a→5')-P,3'-dideoxy-P- (dimethylamino)-2',3'-imino-2',3'-secothymidylyl-(2'a→5')-P,2',3'-trideoxy-P- (dimethylamino)-2',3'-imino-2',3'-secoguanylyl-(2'a→5')-P,2',3'-trideoxy-P- (dimethylamino)-2',3'-imino-2',3'-secoguanylyl-(2'a→5')-P,2',3'-trideoxy-P-(dimethylamino)-2',3'-imino-2',3'-secocytidylyl-(2'a→5')-P,2',3'-trideoxy-P- (dimethylamino)-2',3'-imino-2',3'-secoadenylyl-(2'a→5')-P,3'-dideoxy-P- (dimethylamino)-2',3'-imino-2',3'-secothymidylyl-(2'a→5')-P,3'-dideoxy-P- (dimethylamino)-2',3'-imino-2',3'-secothymidylyl-(2'a→5')-P,3'-dideoxy-P- (dimethylamino)-2',3'-imino-2',3'-secothymidylyl-(2'a→5')-P,2',3'-trideoxy-P- (dimethylamino)-2',3'-imino-2',3'-secocytidylyl-(2'a→5')-P,3'-dideoxy-P-(dimethylamino)- 2',3'-imino-2',3'-secothymidylyl-(2'a→5')-P,2',3'-trideoxy-P-(dimethylamino)-2',3'-imino- 2',3'-secoadenylyl-(2'a→5')-2',3'-dideoxy-2',3'-imino-2',3'-secoguanosine.

Eteplirsen has the following structure:

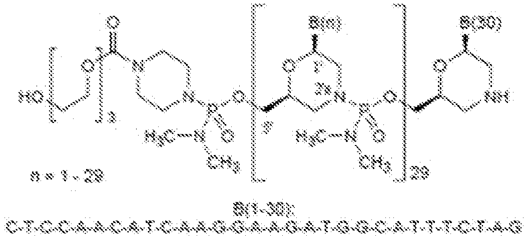

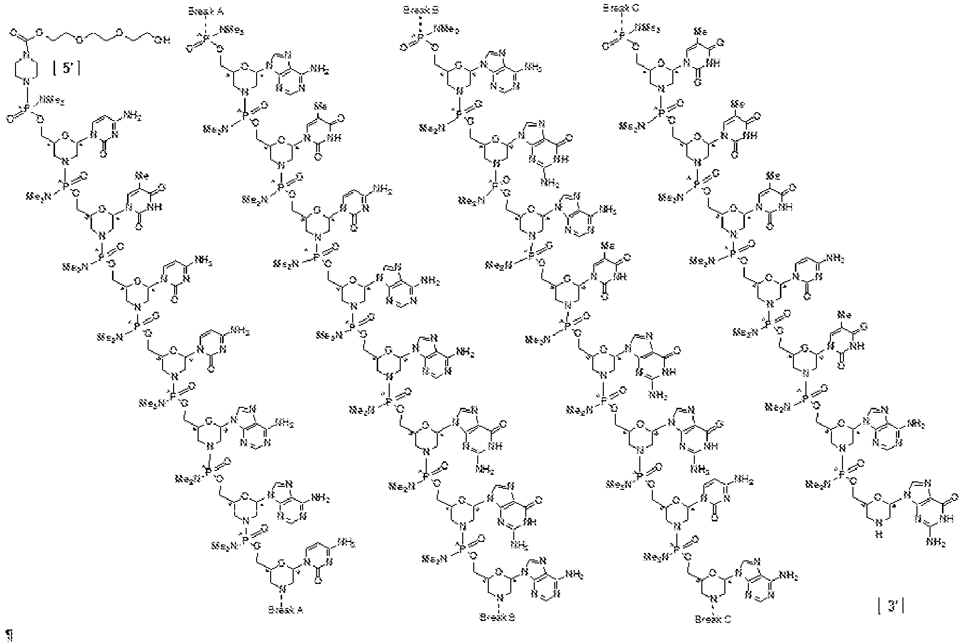

"Dystrophin" is a rod-shaped cytoplasmic protein, and a vital part of the protein complex that connects the cytoskeleton of a muscle fiber to the surrounding extracellular matrix through the cell membrane, encoded by the dystrophin (i.e., *DMD*) gene. Dystrophin contains multiple functional domains. For instance, dystrophin contains an actin binding domain at about amino acids 14-240 and a central rod domain at about amino acids 253-3040. This large central domain is formed by 24 spectrin-like triple-helical elements of about 109 amino acids, which have homology to alpha-actinin and spectrin. The repeats are typically interrupted by four proline-rich non-repeat segments, also referred to as hinge regions. Repeats 15 and 16 are separated by an 18 amino acid stretch that appears to provide a major site for proteolytic cleavage of dystrophin. The sequence identity between most repeats ranges from 10-25%. One repeat contains three alpha-helices: 1, 2 and 3. Alpha-helices 1 and 3 are each formed by 7 helix turns, probably interacting as a coiled-coil through a hydrophobic interface. Alpha-helix 2 has a more complex structure and is formed by segments of four and three helix turns, separated by a Glycine or Proline residue. Each repeat is encoded by two exons, typically interrupted by an intron between amino acids 47 and 48 in the first part of alpha-helix 2. The other intron is found at different positions in the repeat, usually scattered over helix-3. Dystrophin also contains a cysteine-rich domain at about amino acids 3080-3360), including a cysteine-rich segment (i.e., 15 Cysteines in 280 amino acids) showing homology to the C-terminal domain of the slime mold (Dictyostelium discoideum) alpha-actinin. The carboxy-terminal domain is at about amino acids 3361-3685.

The amino-terminus of dystrophin binds to F-actin and the carboxy-terminus binds to the dystrophin-associated protein complex (DAPC) at the sarcolemma. The DAPC includes the dystroglycans, sarcoglycans, integrins and caveolin, and mutations in any of these components cause autosomally inherited muscular dystrophies. The DAPC is destabilized when dystrophin is absent, which results in diminished levels of the member proteins, and in turn leads to progressive fibre damage and membrane leakage. In various forms of muscular dystrophy, such as Duchenne's muscular dystrophy (DMD) and Becker's muscular dystrophy (BMD), muscle cells produce an altered and functionally defective form of dystrophin, or no dystrophin at all, mainly due to mutations in the gene sequence that lead to incorrect splicing. The predominant expression of the defective dystrophin protein, or the complete lack of dystrophin or a dystrophin-like protein, leads to rapid progression of muscle degeneration, as noted above. In this regard, a "defective" dystrophin protein may be characterized by the forms of dystrophin that are produced in certain subjects with DMD or BMD, as known in the art, or by the absence of detectable dystrophin.

The term "functional" in reference to a dystrophin protein includes those proteins derived from an mRNA transcript containing sequences corresponding to all of exons 1 to 79 of a dystrophin gene, also referred to as a wildtype protein. A functional dystrophin protein refers generally to a dystrophin protein having sufficient biological activity to reduce the progressive degradation of muscle tissue that is otherwise characteristic of Duchenne muscular dystrophy, typically as compared to the altered or "defective" form of dystrophin protein that is present in certain subjects with DMD or related disorders. A functional dystrophin protein may have about 10%, 20%, 30%, 40%, 50%, 60%, 70%, 80%, 90%, or 100% (including all integers in between) of the *in vitro* or *in vivo* biological activity of wildtype dystrophin, as measured according to routine techniques in the art. As one example, dystrophin-related activity in muscle cultures *in vitro* can be measured according to myotube size, myofibril organization (or disorganization), contractile activity, and spontaneous clustering of acetylcholine receptors (see, e.g., Brown et al., Journal of Cell Science. 112:209-216, 1999). Animal models are also valuable resources for studying the pathogenesis of disease, and provide a means to test dystrophin-related activity. Two of the most widely used animal models for DMD research are the mdx mouse and the golden retriever muscular dystrophy (GRMD) dog, both of which are dystrophin negative (see, e.g., Collins & Morgan, Int J Exp Pathol 84: 165-172, 2003). These and other animal models can be used to measure the functional activity of various dystrophin proteins. Included are truncated forms of dystrophin, such as those forms that are produced by certain of the antisense oligomer compounds of the present invention.

The term "functional" or "semi-functional" dystrophin protein includes those proteins derived from an mRNA transcript containing sequences corresponding to a truncated form of the transcript, for example, a dystrophin mRNA transcript having less than all of exons 1 to 79 of a dystrophin gene. In other words, a truncated form of a dystrophin mRNA may exclude one or more exons of a corresponding dystrophin gene. A truncated form of a dystrophin mRNA may express a truncated or shortened form of a dystrophin protein, also referred to as a microdystrophin protein.

The term "functional" in reference to a myostatin protein includes those proteins derived from an mRNA transcript containing all sequences corresponding to exon 1, exon 2 and exon 3 of a myostatin gene, also referred to as a wildtype protein.

A non-functional, dysfunctional or inactive myostatin protein includes a protein derived from a myostatin mRNA transcript missing all or any portion of the full gene corresponding to the sequence of exon 1, exon 2 and exon 3, or that contains all or a portion of the sequences corresponding to intron 1, intron 2, or other intron sequences, or where the non-functional state relates to missing functional elements as derived from a respective exon, or as otherwise derived from the inclusion of a respective intron, including partial or full sequences thereof. A non-functional, dysfunctional or inactive myostatin protein includes a protein derived from a myostatin mRNA transcript which excludes exon 2, for example, and/or having reduced functionality relative to the wildtype myostatin protein.

Thus, in various embodiments, the presence of, expression of, or increased expression of functional or semi-functional dystrophin protein may be determined, for example, by western blot analysis and dystrophin gene expression of, for example, DMD patient derived muscle cells treated with a modified antisense oligomer and/or a therapeutic of the present disclosure. In various embodiments, treatment of DMD muscle cells or a subject in need of treatment of DMD with a modified antisense oligomer and/or therapeutic of the disclosure may result in expression of functional dystrophin protein in an amount that is, for example, about 5%, 10%, 15%, 20%, 25%, 30%, 35%, 40%, 45%, 50%, 55%, 60%, 65%, 70%, 75%, 80%, 85%, 90%, 95%, or 100%, of the normal amount of dystrophin protein expressed in normal cells or a normal subject.

In various embodiments, the functionality of dystrophin or truncated dystrophin protein expressed by a tissue or a subject in need of treatment of DMD may be determined by immunohistochemical analysis of, for example, the number of muscle fibers, the increase in muscle mass, the percent of muscle fiber with centralized nuclei, and the amount of functional dystrophin protein as compared to untreated equivalents. The functionality of dystrophin or truncated dystrophin protein of a subject in need of treatment of DMD may be further analyzed by physical and physiological tests such as motor function tests including measurements of muscle mass and grip strength.

In some embodiments, the dystrophin therapeutic restores dystrophin expression in cells of interest. The term "restoration" of dystrophin synthesis or production refers generally to the production of a dystrophin protein including truncated forms of dystrophin in a patient with muscular dystrophy following treatment with eteplirsen as described herein. In some embodiments, treatment results in an increase in novel dystrophin production in a patient by 1%, 5%, 10%, 20%, 30%, 40%, 50%, 60%, 70%, 80%, 90% or 100% (including all integers in between). In some embodiments, treatment increases the number of dystrophin-positive fibers to at least 20%, about 30%, about 40%, about 50%, about 60%, about 70%, about 80%, about 90 % or about 95% to 100% of normal in the subject. In other embodiments, treatment increases the number of dystrophin-positive fibers to about 20% to about 60%, or about 30% to about 50% of normal in the subject. The percent of dystrophin-positive fibers in a patient following treatment can be determined by a muscle biopsy using known techniques. For example, a muscle biopsy may be taken from a suitable muscle, such as the biceps brachii muscle in a patient.

Analysis of the percentage of positive dystrophin fibers may be performed pretreatment and/or post-treatment or at time points throughout the course of treatment. In some embodiments, a post-treatment biopsy is taken from the contralateral muscle from the pretreatment biopsy. Pre- and post-treatment dystrophin expression studies may be performed using any suitable assay for dystrophin. In one embodiment, immunohistochemical detection is performed on tissue sections from the muscle biopsy using an antibody that is a marker for dystrophin, such as a monoclonal or a polyclonal antibody. For example, the MANDYS106 antibody can be used which is a highly sensitive marker for dystrophin. Any suitable secondary antibody may be used.

In some embodiments, the percent dystrophin-positive fibers are calculated by dividing the number of positive fibers by the total fibers counted. Normal muscle samples have 100% dystrophin-positive fibers. Therefore, the percent dystrophin-positive fibers can be expressed as a percentage of normal. To control for the presence of trace levels of dystrophin in the pretreatment muscle as well as revertant fibers a baseline can be set using sections of pretreatment muscles from each patient when counting dystrophin-positive fibers in post-treatment muscles. This may be used as a threshold for counting dystrophin-positive fibers in sections of post-treatment muscle in that patient. In other embodiments, antibody-stained tissue sections can also be used for dystrophin quantification using Bioquant image analysis software (Bioquant Image Analysis Corporation, Nashville, TN). The total dystrophin fluorescence signal intensity can be reported as a percentage of normal. In addition, Western blot analysis with monoclonal or polyclonal anti-dystrophin antibodies can be used to determine the percentage of dystrophin positive fibers. For example, the anti dystrophin antibody NCL-Dys1 from Novacastra may be used. Dystrophin production can also be measured by reverse-transcription polymerase chain reaction (RT-PCR). Primers can be designed to measure dystrophin genes that will produce a functional dystrophin protein. The percentage of dystrophin-positive fibers can also be analyzed by determining the expression of the components of the sarcoglycan complex (□□□) and/or neuronal NOS.

In some embodiments, treatment slows or reduces the progressive respiratory muscle dysfunction and/or failure in patients with DMD that would be expected without treatment. In some embodiments, treatment stabilizes respiratory muscle function in patients with DMD. In one embodiment, treatment with eteplirsen may reduce or eliminate the need for ventilation assistance that would be expected without treatment. In one embodiment, measurements of respiratory function for tracking the course of the disease, as well as the evaluation of potential therapeutic interventions include Maximum inspiratory pressure (MIP), maximum expiratory pressure (MEP) and forced vital capacity (FVC). MIP and MEP measure the level of pressure a person can generate during inhalation and exhalation, respectively, and are sensitive measures of respiratory muscle strength. MIP is a measure of diaphragm muscle weakness.

In some embodiments, treatment may stabilize, maintain, improve or increase walking ability (e.g., stabilization of ambulation) in the subject. In some embodiments, treatment maintains, increases, or reduces loss of a stable walking distance in a patient, as measured by, for example, the 6 Minute Walk Test (6MWT), described by McDonald, et al. (Muscle Nerve, 2010; 42:966-74; Muscle Nerve, 2010; 41:500-10, the contents of which are herein incorporated by reference in its entirety). The 6MWT is a clinically meaningful endpoint focused on ambulation that characterizes changes in walking function over time as an expression of changes in disease state.

A change in the 6 Minute Walk Distance (6MWD) may be expressed as an absolute value, a percentage change or a change in the %-predicted value. The performance of a DMD patient in the 6MWT relative to the typical performance of a healthy peer can be determined by calculating a %-predicted value. For example, the %-predicted 6MWD may be calculated using the following equation for males: 196.72 + (39.81 x age) - (1.36 x age2) + (132.28 x height in meters). For females, the %-predicted 6MWD may be calculated using the following equation: 188.61 + (51.50 x age) - (1.86 x age2) + (86.10 x height in meters) (Henricson et al. PLoS Curr., 2012, version 2, the contents of which are herein incorporated by reference in its entirety).

Ambulation can be measured through various methods, including the North Star Ambulatory Assessment (NSAA). The NSAA was developed by the Physiotherapy Assessment and Evaluation Group of the North Start Clinical Network to assess ambulant boys with DMD, and provides a list of activities that are scored from 2-0, with 2 being normal and 0 being "unable to achieve independently" (2006-2011 MDC/North Star Clinical Network). These activities range from standing for a minimum of 3 seconds to climbing up and down a box to running. In certain embodiments, treatment with eteplirsen may maintain, stabilize, increase, or improve ambulation, for example, as determined by the NSAA.

In the present case, therapeutic agents, including modified antisense oligomers are used to induce a decrease in myostatin mRNA containing exon 2, resulting in an amelioration of Duchenne muscular dystrophy symptoms (e.g. reduction of functional myostatin protein) in the range of about 30% to about 100% or the percentages disclosed above with regard to functionality, as compared to non-treatment. Such amelioration of symptoms may be observed on a micro level (e.g. reduction of myostatin protein expression measured by, for example, immunohistochemistry, immunofluorescence, western-blot analyses; increase of muscle growth; restoration of muscle function) and physiological level (e.g. improvement of motor function assessed by physical examination).

Modified antisense oligomers are used to induce exon skipping during the processing of dystrophin pre-mRNA where the dystrophin pre-mRNA includes exons having one or more genetic mutations, or in which one or more regions of the dystrophin gene have been deleted, resulting in an amelioration of symptoms related to Duchenne muscular dystrophy and related disorders (e.g. restoration of functional or semi-functional dystrophin protein). Functional or semi-functional dystrophin protein may be increased in the range of about 30% to about 100% or the percentages disclosed above with regard to functionality, as compared to non-treatment. Such amelioration of symptoms may be observed on a micro level (e.g. increase of dystrophin protein expression measured by, for example, immunohistochemistry, immunofluorescence, western-blot analyses; increase of muscle growth; restoration of muscle function) and physiological level (e.g. improvement of motor function assessed by physical examination).

The term "nucleotide" refers to a naturally occurring nucleotide comprising a nucleobase, a sugar and at least one phosphate group (e.g., a phosphodiester linking group).

The term "nucleotide analog" refers to a derivative of, or modification to, a naturally occurring nucleotide, for example, a nucleotide comprising at least one modification. Such modifications may include at least one of (i) a modified internucleoside linkage, (ii) a modified sugar moiety, or (iii) a combination of the foregoing. The skilled practitioner will appreciate that where a modification is specified with respect to any one component of a nucleotide subunit (e.g., a modified sugar), the unspecified portion(s) of the nucleotide subunit may remain unmodified (e.g., an unmodified internucleoside linkage, an unmodified nucleobase).

The terms "oligonucleotide," "oligomer," "oligo," "antisense oligonucleotide," "antisense oligomer," "modified antisense oligomer" and "antisense oligo," and other appropriate combinations and derivations thereof, refer to linear sequences of nucleotides, or nucleotide analogs, where one or more nucleobases may hybridize to a portion of a target RNA against which the oligomer is directed, referred to as a target sequence, by Watson-Crick base pairing, to form an oligomer:RNA heteroduplex within the target sequence. Specifically, the terms "antisense," "oligonucleotide," "oligomer," "oligo" and "compound" may be used in various combinations and interchangeably to refer to such an oligomer. Cyclic subunits comprising portions of the nucleotides may be based on ribose or another pentose sugar, sugar analog or, in certain embodiments may be a modified sugar, for example, a morpholino group (see description of morpholino-based oligomers below).

The term "modified," "non-naturally-occurring," or "analogs," and other appropriate combinations and derivatives thereof, when referring to oligomers, refer to oligomers having one or more nucleotide subunits having at least one modification selected from (i) a modified internucleoside linkage, e.g., an internucleoside linkage other than the standard phosphodiester linkage found in naturally-occurring oligonucleotides, (ii) modified sugar moieties, e.g., moieties other than ribose or deoxyribose moieties found in naturally occurring oligonucleotides, or (iii) a combination of the foregoing. In various embodiments, a modified internucleoside linkage is selected from a phosphorothioate internucleoside linkage, a phosphoramidate internucleoside linkage, a phosphorodiamidate internucleoside linkage, and a phosphorotriamidate internucleoside linkage. In further embodiments, the phosphorodiamidate internucleoside linkage comprises a phosphorous atom that is covalently bonded to a (1,4-piperazin)-1-yl moiety, a substituted (1,4-piperazin)-1-yl moiety, a 4-aminopiperidin-1-yl moiety, or a substituted 4-aminopiperidin-1-yl moiety. In various embodiments, the modified sugar moiety is selected from a peptide nucleic acid (PNA) subunit, a locked nucleic acid (LNA) subunit, a 2'O,4'C-ethylene-bridged nucleic acid (ENA) subunit, a tricyclo-DNA (tc-DNA) subunit, a 2' O-methyl subunit, a 2' O-methoxyethyl subunit, a 2'-fluoro subunit, a 2'-O-[2-(N-methylcarbamoyl)ethyl] subunit, and a morpholino subunit.

A modification to the internucleoside linkage may be between at least two sugar and/or modified sugar moieties of an oligomer. Nucleotide analogs support bases capable of hydrogen bonding by Watson-Crick base pairing to naturally occurring oligonucleotide bases, where the analog presents the bases in a manner to permit such hydrogen bonding in a sequence-specific fashion between the oligomer analog molecule and bases in the naturally occurring oligonucleotide (e.g., single-stranded RNA or single-stranded DNA). Exemplary analogs are those having a substantially uncharged, phosphorus containing internucleoside linkages.

A "nuclease-resistant" oligomer refers to one whose internucleoside linkage is substantially resistant to nuclease cleavage, in non-hybridized or hybridized form; by common extracellular and intracellular nucleases in the body (for example, by exonucleases such as 3'-exonucleases, endonucleases, RNase H); that is, the oligomer shows little or no nuclease cleavage under normal nuclease conditions in the body to which the oligomer is exposed. A "nuclease-resistant heteroduplex" refers to a heteroduplex formed by the binding of a modified antisense oligomer to its complementary target, such that the heteroduplex is substantially resistant to *in vivo* degradation by intracellular and extracellular nucleases, which are capable of cutting double-stranded RNA/RNA or RNA/DNA complexes. A "heteroduplex" refers to a duplex between a modified antisense oligomer and the complementary portion of a target RNA. For example, a nuclease-resistant oligomer may be a modified antisense oligomer as described herein.

The terms "nucleobase" (Nu), "base pairing moiety" or "base" are used interchangeably to refer to a purine or pyrimidine base found in naturally occurring, or "native" DNA or RNA (e.g., uracil, thymine, adenine, cytosine, and guanine), as well as analogs of these naturally occurring purines and pyrimidines, that may confer improved properties, such as binding affinity to the oligomer. Exemplary analogs include hypoxanthine (the base component of the nucleoside inosine); 2, 6-diaminopurine; 5-methyl cytosine; C5-propynyl-modified pyrimidines; 10-(9-(aminoethoxy)phenoxazinyl) (G-clamp) and the like.

Further examples of base pairing moieties include, but are not limited to, uracil, thymine, adenine, cytosine, guanine and hypoxanthine (inosine) having their respective amino groups protected by acyl protecting groups, 2-fluorouracil, 2-fluorocytosine, 5-bromouracil, 5-iodouracil, 2,6-diaminopurine, azacytosine, pyrimidine analogs such as pseudoisocytosine and pseudouracil and other modified nucleobases such as 8-substituted purines, xanthine, or hypoxanthine (the latter two being the natural degradation products). The modified nucleobases disclosed in Chiu and Rana, RNA, 2003, 9, 1034-1048, Limbach et al. Nucleic Acids Research, 1994, 22, 2183-2196 and Revankar and Rao, Comprehensive Natural Products Chemistry, 1999, vol. 7, 313, are also contemplated, the contents of which are incorporated herein by reference.

Further examples of base pairing moieties include, but are not limited to, expanded-size nucleobases in which one or more benzene rings has been added. Nucleic base replacements are described in the following examples: the Glen Research catalog (www.glenresearch.com); Krueger AT et al., Acc. Chem. Res., 2007, 40, 141-150; Kool, ET, Acc. Chem. Res., 2002, 35, 936-943; Benner S.A., et al., Nat. Rev. Genet., 2005, 6, 553-543; Romesberg, F.E., et al., Curr. Opin. Chem. Biol., 2003, 7, 723-733; Hirao, I., Curr. Opin. Chem. Biol., 2006, 10, 622-627, the contents of each example are incorporated herein by reference. These are contemplated as useful for the synthesis of various oligomers described herein. Examples of expanded-size nucleobases are shown below:

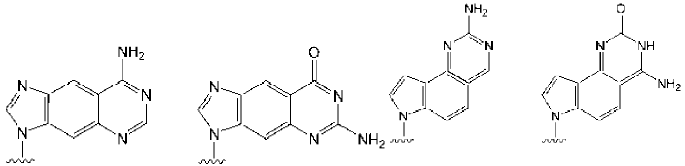

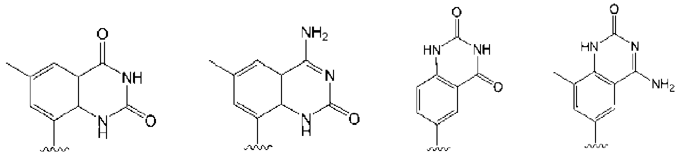

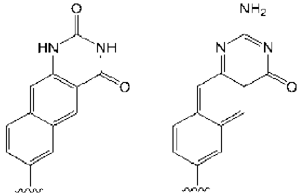

A nucleobase covalently linked to a ribose, sugar analog, modified sugar or morpholino comprises a nucleoside. "Nucleotides" comprise a nucleoside together with at least one linking phosphate group. The phosphate groups comprise covalent linkages to adjacent nucleosides form an oligomer. Thus, the phosphate group of the nucleotide is commonly referred to as forming an "internucleoside linkage." Accordingly, a nucleotide comprises a nucleoside as further described herein and an internucleoside linkage. In some embodiments, a modified antisense oligomer of the disclosure comprises subunits wherein a "subunit" includes naturally occurring nucleotides, nucleotide analogs as described herein, and combinations thereof. In certain embodiments, a modified antisense oligomer of the disclosure comprises subunits wherein at least one subunit is a nucleotide analog.

The terms "sequence identity," "sequence homology," and "complementarity" (e.g. a "sequence 50% identical to," a "sequence 50% homologous to," and "a sequence 50% complementary to") in the context of nucleic acids refer to the extent that a sequence is identical on a nucleotide-by-nucleotide basis over a window of comparison. A "percentage identity," "percentage homology," and "percentage complementary to" may be calculated by comparing two optimally aligned sequences over the window of comparison, determining the number of positions at which the identical nucleic acid base (e.g., A, T, C, G, I) occurs in both sequences to yield the number of matched positions, dividing the number of matched positions by the total number of positions in the window of comparison (i.e., the window size), and multiplying the result by 100 to yield the percentage of sequence identity. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, Wis., USA) or by inspection and the best alignment (i.e., resulting in the highest percentage homology over the comparison window) generated by any of the various methods selected. Reference also may be made to the BLAST family of programs as for example disclosed by Altschul et al., Nucl. Acids Res. 25:3389, 1997. In various embodiments, a modified antisense oligomer of the disclosure may have at least 70%, 75%, 80%, 85%, 90%, 95%, 99% or 100% sequence identity with a targeting sequence in Table 1 (SEQ ID NOS: 1 to 3) and Table 2 (SEQ ID NOS: 4-15).

As used herein, a targeting sequence of an oligomer "specifically hybridizes" to a target region of an oligonucleotide if the oligomer hybridizes to the target region under physiological conditions, with a melting point (Tm) substantially greater than 40 °C, 45 °C, 50 °C, and in various embodiments, 60 °C-80 °C or higher. Such hybridization preferably corresponds to stringent hybridization conditions. At a given ionic strength and pH, the Tm is the temperature at which 50% of a targeting sequence hybridizes to a complementary sequence in a target region. Such hybridization may occur with "near" or "substantial" complementarity of the modified antisense oligomer to the target region, as well as with exact complementarity. In some embodiments, an oligomer may hybridize to a target region at about 50%, 55%, 60%, 65%, 70%, 75%, 80%, 85%, 90%, 95%, 99% or 100%.

As used herein, the term "subunit" refers to a naturally occurring nucleotide or a naturally occurring nucleotide comprising at least one modification. A modification may comprise at least one of (i) a modified internucleoside linkage, (ii) a modified sugar moiety, or (iii) a combination of the foregoing. In further embodiments, a modification may include a modified nucleobase.

As used herein, the term "sufficient length" refers to a modified antisense oligomer that is complementary to at least 20 to 50 contiguous nucleobases in a target region within a pre-mRNA, where such complementarity may be completely internal to a target region within an exon or may span a splice junction across an intron/exon or exon/intron region. In embodiments, sufficient length may refer to a modified antisense oligomer that is complementary to at least 12, contiguous nucleobases in a target region within a pre-mRNA, where such complementarity may be completely internal to a target region within an exon or may span a splice junction across an intron/exon or exon/intron region.

A modified myostatin antisense oligomer may, for example, be complementary to intron 1/exon 2, exon 2 or exon 2/intron 2 of myostatin pre-mRNA. In various embodiments, the modified myostatin antisense oligomer comprises at least a number of nucleotides to be capable of specifically hybridizing to a target region of a myostatin pre-mRNA sequence. Preferably an oligomer of sufficient length is from 12 to 40 nucleotides, 12 to 30 nucleotides, 12 to 15 nucleotides, 12 to 20 nucleotides, 15 to 20 nucleotides, 15 to 22 nucleotides, 12 to 22 nucleotides in length, including all integers in between these ranges. In some embodiments, the myostatin antisense oligomer is about 12 to about 40 or about 12 to about 30 bases in length. In some embodiments, the antisense oligomer is about 12 to about 25, about 15 to about 25, or about 15 to about 20 bases in length. In some embodiments, a myostatin antisense oligomer sequence comprises at least about 12, 13, 14, 15, 16, 17, 18, 19, 20, 21, 22, 23, 24, 25, 26, 27, 28, 29, 30, 31, 32, 33, 34, 35, 36, 37, 38, 39, or 40 contiguous or non-contiguous bases that are complementary to the target sequences of Table 1 (e.g., SEQ ID NO: 1, SEQ ID NO: 2 or SEQ ID NO: 3, or sequences that span at least a portion of SEQ ID NO: X, SEQ ID NO: Y or SEQ ID NO: Z).

A modified dystrophin antisense oligomer may be complementary to a target region completely internal to exon 7, exon 8, exon 9, exon 19, exon 23, exon 44, exon 45, exon 50, exon 51, exon 52, exon 53 or exon 55. In various embodiments, the modified dystrophin antisense oligomer comprises at least a number of nucleotides to be capable of specifically hybridizing to a target region of a dystrophin pre-mRNA sequence. Preferably an oligomer of sufficient length is from 17 to 50 nucleotides, 17 to 40 nucleotides, 14 to 25 nucleotides, 15 to 30 nucleotides, 17 to 30 nucleotides, 17 to 27 nucleotides, 10 to 27 nucleotides, 10 to 25 nucleotides, or 10 to 20 nucleotides in length, including all integers in between these ranges. In some embodiments, the antisense oligomer is about 17 to about 40 or about 10 to about 30 bases in length. In some embodiments, the antisense oligomer is about 14 to about 25 or about 17 to about 27 bases in length. In some embodiments, an antisense oligomer sequence comprises at least about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 contiguous or non-contiguous bases that are complementary to the target sequences of Table 2 (e.g., SEQ ID NOS: 4-15).

As used herein, the term a "subject" or a "subject in need thereof' includes a mammalian subject such as a human subject. Exemplary mammalian subjects have or are at risk for having Duchenne muscular dystrophy and related disorders. As used herein, the term "muscular dystrophy," "Duchenne muscular dystrophy" and "related disorders" refers to a human autosomal recessive disease that is often characterized by over expression of myostatin protein or by genetic mutations in the dystrophin gene in affected individuals. In some embodiments, Duchenne muscular dystrophy and related disorders include, but are not limited to, Becker muscular dystrophy, limb-girdle muscular dystrophy, congenital muscular dystrophy, facioscapulohumeral muscular dystrophy, myotonic muscular dystrophy, oculopharyngeal muscular dystrophy, distal muscular dystrophy, Emery-Dreifuss muscular dystrophy, muscle wasting conditions or disorders, such as AIDS, cancer or chemotherapy related muscle wasting, and fibrosis or fibrosis-related disorders (for example, skeletal muscle fibrosis).

A "patient," as used herein, includes any person that exhibits a symptom, or is at risk for exhibiting a symptom, which can be treated as described herein, such as a subject that has or is at risk for having DMD or BMD, or any of the symptoms associated with these conditions (e.g., muscle fibre loss).

A "pediatric patient" as used herein is a patient from age 1 to 21, inclusive. In some embodiments, the pediatric patient is a patient from age 7 to 21 (e.g., age 7, 8, 9, 10, 11, 12, 13, 14, 15, 16, 17, 18, 19, 20 or 21). In some embodiments, the pediatric patient is a patient of less than seven years of age. In some embodiments, the pediatric patient is a patient of seven years of age or older.

As used herein, the term "target" or "target region" refers to a region within a pre-mRNA transcript such as myostatin or dystrophin pre-mRNA. In various embodiments, a myostatin target region is a region comprising intron 1/exon 2, exon 2, or exon 2/intron 2 of the myostatin pre-mRNA. In various embodiments, a dystrophin target region is a region comprising one or more of exon 7, exon 8, exon 9, exon 19, exon 23, exon 44, exon 45, exon 50, exon 51, exon 52, exon 53 or exon 55.

In various embodiments, the term "targeting sequence" refers to the sequence in the modified antisense oligomer or oligomer analog that is complementary to the target sequence in the pre-mRNA transcript. The entire sequence, or only a portion, of the modified antisense oligomer may be complementary to the target sequence. For example, in an oligomer having 12-50 bases, about 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 may contain sequences (e.g. "targeting sequences") that are complementary to the target region within the pre-mRNA transcript. Typically, the targeting sequence is formed of contiguous bases in the oligomer, but may alternatively be formed of non-contiguous sequences that when placed together, e.g., from opposite ends of the oligomer, constitute a sequence that spans the target sequence.

A "targeting sequence" may have "near" or "substantial" complementarity to the target sequence and still function for its intended purpose, for example, to increase the level of dystrophin mRNA expression which excludes one or more exons having a genetic mutation, or to increase expression of functional or semi-functional dystrophin protein. In the case of myostatin, a targeting sequence may function to reduce the level of expression of exon 2 containing myostatin mRNA, or decrease expression of functional myostatin protein. Preferably, modified antisense oligomer compounds in the present disclosure have at most one mismatch with the target sequence out of 10 nucleotides, or one mismatch out of 20. Alternatively, the modified antisense oligomers herein have at least 90% sequence homology, at least 95% sequence homology, at least 99% sequence homology, or 100% sequence homology, with the exemplary target sequences as designated herein.

In the case of dystrophin, a targeting sequence may comprise a sequence selected from SEQ ID NOS: 76 to 3485, is selected from SEQ ID NOS: 76 to 3485, is a fragment of at least 10 contiguous nucleotides of a sequence selected from SEQ ID NOS: 76 to 3485, or is a variant having at least 90% sequence identity to a sequence selected from SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In the case of myostatin, a targeting sequence may comprise a sequence selected from SEQ ID NOS: 16 to 75, is selected from SEQ ID NOS: 16 to 75, is a fragment of at least 10 contiguous nucleotides of a sequence selected from SEQ ID NOS: 16 to 75, or is a variant having at least 90% sequence identity to a sequence selected from SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, at least one X of the targeting sequence is T. In various embodiments, each X of the targeting sequence is T.

In various embodiments, at least one X of the targeting sequence is U. In various embodiments, each X of the targeting sequence is U.

In various embodiments, at least one Y of the targeting sequence is 5mC. In various embodiments, each Y of the targeting sequence is 5mC.

In various embodiments, at least one Y of the targeting sequence is C. In various embodiments, each Y of the targeting sequence is C.

In various embodiments, at least one X of SEQ ID NOS: 16 to 75 and SEQ ID NOS: 76 to 3485 is T. In various embodiments, each X of SEQ ID NOS: 16 to 75 and SEQ ID NOS: 76 to 3485 is T.

In various embodiments, at least one X of the targeting sequence is U. In various embodiments, each X of SEQ ID NOS: 16 to 75 and SEQ ID NOS: 76 to 3485 is U.

In various embodiments, at least one Y of SEQ ID NOS: 16 to 75 and SEQ ID NOS: 76 to 3485 is 5mC. In various embodiments, each Y of SEQ ID NOS: 16 to 75 and SEQ ID NOS: 76 to 3485 is 5mC.

In various embodiments, at least one Y of SEQ ID NOS: 16 to 75 and SEQ ID NOS: 76 to 3485 is C. In various embodiments, each Y of SEQ ID NOS: 16 to 75 and SEQ ID NOS: 76 to 3485 is C.

As used herein, the term "TEG," "triethylene glycol tail," or "EG3" refers to triethylene glycol moieties conjugated to the oligomer, e.g., at its 3'- or 5'-end. For example, in some embodiments, "TEG" includes wherein T of the compound of, for example, formulas (I), (IV), (V), (VI), (VII), and (VIII) is of the formula:

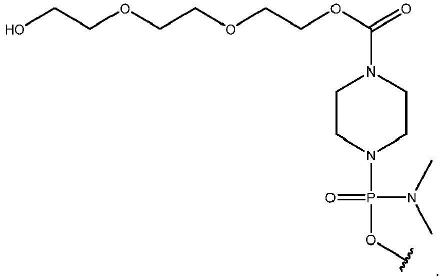

As used herein, the term a "therapeutically effective amount" or "effective amount" of a therapeutic agent or composition refers to an amount effective in the prevention or treatment of a disorder for the treatment of which the composition is effective. A "disorder" refers to any Duchenne muscular dystrophy or related disorder, including BMD, limb-girdle muscular dystrophy, congenital muscular dystrophy, facioscapulohumeral muscular dystrophy, myotonic muscular dystrophy, oculopharyngeal muscular dystrophy, distal muscular dystrophy, Emery-Dreifuss muscular dystrophy, muscle wasting conditions or disorders, such as AIDS, cancer or chemotherapy related muscle wasting, and fibrosis or fibrosis-related disorders (for example, skeletal muscle fibrosis).

As used herein, the terms "quantifying," "quantification" or other related words refer to determining the quantity, mass, or concentration in a unit volume, of a nucleic acid, oligonucleotide, oligomer, peptide, polypeptide, or protein.

In various embodiments, as used herein, the term "treatment" includes treatment of a subject (e.g. a mammal, such as a human) or a cell to alter the current course of the subject or cell. Treatment includes, but is not limited to, administration of a pharmaceutical composition, and may be performed either prophylactically or subsequent to the initiation of a pathologic event or contact with an etiologic agent. Also included are "prophylactic" treatments, which can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition, or associated symptoms thereof.

### II. Modulation of the Splicing of a Pre-mRNA Transcript

For illustration purposes, and without being bound by theory, where a therapeutic agent is a modified antisense oligomer, these are believed to facilitate blocking, inhibiting or modulating the processing of a pre-mRNA, such as by inhibiting the action of a spliceosome and production of a mature mRNA transcript, and may also induce degradation of targeted mRNAs. In some instances, a spliceosome may be inhibited from binding to an exon/intron splice junction such that an exon/intron splice junction is skipped and one or more exons are removed from an mRNA transcript. A mature mRNA transcript having one or more exons less than a wildtype mRNA transcript may result in an mRNA transcript that maintains the open reading frame such that the mRNA transcript may be translated to functional protein rather than degraded. A protein translated from an mRNA transcript having fewer exons than the wildtype mRNA may result in a transcribed protein comprising fewer amino acid residues than a protein transcribed from a wildtype mRNA transcript. A functional protein composed of fewer amino acid residues than a wildtype protein may have the same or similar activity/functionality as the wildtype protein. The modified antisense oligomer may be said to be "directed to" or "targeted against" a target sequence or target region with which it hybridizes. In certain embodiments, the target sequence includes a region including a 3' or 5' splice junction site of a pre-mRNA, a branch point, Exonic Splicing Enhancers (ESE) or Intronic Splicing Enhancers (ISE), or other sequence involved in the regulation of splicing. Within an intron, a donor site (5' end of the intron) and an acceptor site (3' end of the intron) are required for splicing. The splice donor site includes an almost invariant sequence GUat the 5' end of the intron, within a larger, less highly conserved region. The splice acceptor site at the 3' end of the intron terminates the intron with an almost invariant AG sequence. The target sequence may include sequences entirely within an exon where no part of the target sequence spans a splice junction, within an exon/intron splice junction site, or spanning an exon/intron splice junction. The target sequence may include an exon/intron donor splice site.

A modified antisense oligomer having a sufficient sequence complementarity to a target pre-mRNA sequence to modulate splicing of the target RNA includes where the modified antisense oligomer has a sequence sufficient to trigger the masking or hindrance of a binding site for a spliceosome complex that would otherwise affect such splicing and/or otherwise includes alterations in the three-dimensional structure of the targeted pre-mRNA.

### A. Modulation of the Splicing of Myostatin Pre-mRNA

Various aspects relate to methods for modulating the splicing of intron and exons of myostatin pre-mRNA. Further aspects relate to inhibiting splicing at the splice junction site of intron 1/exon 2 and exon 2/intron 2 of myostatin pre-mRNA. In further aspects, expression of myostatin exon 2 coding mRNA is inhibited, such as relative to exon-2 wildtype mRNA, in a given sample (e.g., serum, plasma, tissue, cellular etc.). Various methods include administering an antisense oligomer described herein containing a targeting sequence that is complementary to a target region within the myostatin pre-mRNA, where expression of myostatin exon 2 mRNA is inhibited relative to the expression of exon-2 wildtype (i.e. control) mRNA.

In various embodiments, the modified antisense oligomer targeting sequence has sufficient length and complementarity to a sequence within a target region of myostatin pre-mRNA. In various embodiments, targeting sequences within a modified antisense oligomer hybridize to a region of the target sequences entirely within exon 2 where no part of the targeting sequence spans a splice junction, or a region spanning an intron/exon or exon/intron splice junction of myostatin pre-mRNA, such as, for example, the +24/-01 or +18/-07 region of intron 2/exon 2 or the -01/+21, -01/+25, or -09/+15 region of exon 2/intron 2 of myostatin pre-mRNA. In some embodiments, the modified antisense oligomers may about 12 bases to about 40 bases, and include a small number of mismatches, as long as the targeting sequence is sufficiently complementary to effect splice modulation upon hybridization to the target sequence, and optionally forms with the pre-mRNA a heteroduplex having a Tm of 45 °C or greater.

In various embodiments, the degree of complementarity between the antisense targeting sequence and the target sequence is sufficient to form a stable duplex. The region of complementarity of the modified antisense oligomers with the target sequence may be as short as 12-15 bases but can be 12-20 bases or more, e.g., 12-40 bases, 12-30 bases, 12-25 bases, 12-22 bases, 15-25 bases, 15-22 bases, or 15-20 bases, including all integers in between these ranges. In certain embodiments, a minimum length of complementary bases may be required to achieve the requisite binding Tm, as discussed herein.

### B. Modulation of the Splicing of Dystrophin Pre-mRNA

Various aspects relate to methods for modulating the splicing of intron and exons of dystrophin pre-mRNA. Further aspects relate to inhibiting splicing at the splice junction site of an intron/exon and exon/intron splice junction of dystrophin pre-mRNA. In further aspects, expression of a truncated form of dystrophin coding mRNA is enhanced, such as relative to full length wildtype dystrophin mRNA, in a given sample (e.g., serum, plasma, tissue, cellular etc.). Various methods include administering an antisense oligomer described herein containing a targeting sequence that is complementary to a target region within the dystrophin pre-mRNA, where expression of a truncated form of dystrophin mRNA is enhanced relative to the expression of full length wildtype (i.e. control) mRNA.

In various embodiments, an antisense oligomer binds to a target region within an exon of dystrophin pre-mRNA. In embodiments, an antisense oligomer binds to an exon selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or is a region spanning an intron/exon or exon/intron splice junction. In embodiments, one or more exons of dystrophin pre-mRNA have one or more genetic mutations. In embodiments, an antisense oligomer targets an exon having one or more genetic mutations such that the exon is spliced out of the pre-mRNA transcript during processing to mature mRNA resulting in a shortened or truncated form of dystrophin mRNA.

In various embodiments, the modified antisense oligomer targeting sequence has sufficient length and complementarity to a sequence within a target region of dystrophin pre-mRNA. In various embodiments, targeting sequences within a modified antisense oligomer hybridize to a region of the target sequences entirely within one or more exons where no part of the targeting sequence spans a splice junction, or a region spanning an intron/exon or exon/intron splice junction of dystrophin pre-mRNA. In some embodiments, the modified antisense oligomers may about 8 bases to about 50 bases, and include a small number of mismatches, as long as the targeting sequence is sufficiently complementary to effect splice modulation upon hybridization to the target sequence, and optionally forms with the RNA a heteroduplex having a Tm of 45 °C or greater.

In various embodiments, the degree of complementarity between the antisense targeting sequence and the target sequence is sufficient to form a stable duplex. The region of complementarity of the modified antisense oligomers with the target sequence may be as short as 8-15 bases but can be 8-20 bases or more, e.g., 8-40 bases, 8-30 bases, 8-25 bases, 8-22 bases, 8-25 bases, 8-22 bases, 8-20 bases. 17 to 20 bases, 17 to 22 bases, 17 bases to 25 bases, 17 to 30 bases, 17 to 40 bases, or 20 to 30 bases, including all integers in between these ranges. In certain embodiments, a minimum length of complementary bases may be required to achieve the requisite binding Tm, as discussed herein.

In various aspects, the oligomers are configured for additional functionality, including but not limited to bio-availability, stability, cellular update, and resistance to nuclease degradation. Generally, oligomers comprising 50 bases may be suitable, where at least a minimum number of bases, e.g., 8 or 12 bases, are complementary to the target sequence. In various aspects, the oligomers are configured to enhance facilitated or active cellular uptake. In various aspects, the modified antisense oligomers comprise one or more phosphoramidate morpholino monomer or phosphorodiamidate morpholino monomer subunits. In various embodiments, the modified antisense oligomers, comprise about 8-50 phosphoramidate morpholino monomer or phosphorodiamidate morpholino monomer subunits. In various embodiments, the modified antisense oligomers, comprise about 8-30 phosphoramidate morpholino monomer or phosphorodiamidate morpholino monomer subunits. In various embodiments, the modified antisense oligomers, comprise about 17-40 phosphoramidate morpholino monomer or phosphorodiamidate morpholino monomer subunits. In various embodiments, the modified antisense oligomers, comprise about 12-25 phosphoramidate morpholino monomer or phosphorodiamidate morpholino monomer subunits. In various embodiments, the modified antisense oligomers, comprise about 15-25 phosphoramidate morpholino monomer or phosphorodiamidate morpholino monomer subunits. In various embodiments, the modified antisense oligomers, comprise about 15-22 phosphoramidate morpholino monomer or phosphorodiamidate morpholino monomer subunits.

In various aspects, the modified antisense oligomers comprise, consist of, or consist essentially of 8 to 50 subunits, optionally comprising at least one subunit that is a nucleotide analog having (i) a modified internucleoside linkage, (ii) a modified sugar moiety, or (iii) a combination of the foregoing; and a targeting sequence complementary to a target region of 10 or more contiguous nucleotides within a pre-mRNA. In various embodiments, the target region comprises 10, 12 or more contiguous nucleotides entirely within one or more exons where no part of the targeting sequence spans a splice junction, or within a region spanning an intron/exon or exon/intron splice junction of a myostatin or dystrophin gene.

In various embodiments, the target region of a myostatin pre-mRNA comprises a region within exon 2, intron 1/exon 2 or exon 2/intron 2 of myostatin pre-mRNA. In further embodiments, the target region comprises the +24/-01 or +18/-07 region of intron 2/exon 2 or the -01/+21, -01/+25, or -09/+15 region of exon 2/intron 2 of myostatin pre-mRNA.

In various embodiments, the target region of a dystrophin pre-mRNA comprises a region within one or more of an exon selected from exon 7, exon 8, exon 9, exon 19, exon 23, exon 44, exon 45, exon 50, exon 51, exon 52, exon 53, or exon 55 of dystrophin pre-mRNA.

In various aspects, the modified antisense oligomers comprise, consist of, or consist essentially of 10 to 50 subunits, optionally comprising at least one subunit that is a nucleotide analog having (i) a modified internucleoside linkage, (ii) a modified sugar moiety, or (iii) a combination of the foregoing; and a targeting sequence comprising, consisting of, or consisting essentially of, a sequence selected from SEQ IDS 16 to 75 and SEQ ID NOS: 76-3485. Preferably, in some aspects, the modified antisense oligomer comprises a sequence selected from SEQ IDS 71-75 and SEQ ID NO: 76.

Additional aspects include modified antisense oligomers of 8 to 50 subunits that specifically hybridize to a target region within myostatin or dystrophin pre mRNA.

In various embodiments, the target region within myostatin pre-mRNA comprises a region within exon 2, intron 1/exon 2 or exon 2/intron 2 (or a region which spans a splice junction) of the myostatin gene. In various embodiments, the target region comprises a region entirely within exon 2 of myostatin pre-mRNA. In various embodiments, the target region comprises a region within intron 1/exon2 or exon 2/intron 2. In various embodiments, the target region comprises a region spanning an intron 1/exon2 or exon 2/intron 2 splice junction. In further embodiments, the target region comprises the +24/-01 or +18/-07 region of intron 2/exon 2 or the -01/+21, -01/+25, or -09/+15 region of exon 2/intron 2 of myostatin pre-mRNA.

Additional aspects include modified antisense oligomers having a nucleotide analog subunit comprising a modified sugar moiety. In various embodiments, the modified sugar moiety is selected from a peptide nucleic acid (PNA) subunit, a locked nucleic acid (LNA) subunit, a 2'O,4'C-ethylene-bridged nucleic acid (ENA) subunit, a tricyclo-DNA (tc-DNA) subunit, a 2' O-methyl subunit, a 2' O-methoxyethyl subunit, a 2'-fluoro subunit, a 2'-O-[2-(N-methylcarbamoyl)ethyl] subunit, and a morpholino subunit.

Additional aspects include modified antisense oligomers having a nucleotide analog subunit comprising a modified internucleoside linkage. In various embodiments, the modified internucleoside linkage is selected from a phosphorothioate internucleoside linkage, a phosphoramidate internucleoside linkage, a phosphorodiamidate internucleoside linkage, and a phosphorotriamidate internucleoside linkage. In further embodiments, the phosphorodiamidate internucleoside linkage comprises a phosphorous atom that is covalently bonded to a (1,4-piperazin)-1-yl moiety, a substituted (1,4-piperazin)-1-yl moiety, a 4-aminopiperidin-1-yl moiety, or a substituted 4-aminopiperidin-1-yl moiety.

Additional aspects include modified antisense oligomers having a nucleotide analog subunit comprising at least one combination of a modified sugar moiety and a modified internucleoside linkage, wherein various embodiments, one or more subunits are selected from:
a morpholino subunit optionally substituted with a phosphoramidate internucleoside linkage, a phosphorodiamidate internucleoside linkage, phosphorotriamidate internucleoside linkage, or a phosphorothioate internucleoside linkage,
a 2' O-methyl subunit optionally substituted with a phosphoramidate internucleoside linkage, a phosphorodiamidate internucleoside linkage, or a phosphorothioate internucleoside linkage,
a 2'O-methoxyethyl subunit optionally substituted with a phosphoramidate internucleoside linkage, a phosphorodiamidate internucleoside linkage, or a phosphorothioate internucleoside linkage,
a 2'-fluoro subunit optionally substituted with a phosphoramidate internucleoside linkage, a phosphorodiamidate internucleoside linkage, or a phosphorothioate internucleoside linkages,
a 2'O,4'C-ethylene-bridged nucleic acid subunit optionally substituted with a phosphoramidate internucleoside linkage, a phosphorodiamidate internucleoside linkage, or a phosphorothioate internucleoside linkage,
a 2'-O-[2-(N-methylcarbamoyl)ethyl] subunit optionally substituted with a phosphoramidate internucleoside linkage, a phosphorodiamidate internucleoside linkage, or a phosphorothioate internucleoside linkage,
a tricyclo-DNA subunit optionally substituted with a phosphoramidate internucleoside linkage, a phosphorodiamidate internucleoside linkage, or a phosphorothioate internucleoside linkage,
a locked nucleic acid subunit optionally substituted with a phosphoramidate internucleoside linkage, a phosphorodiamidate internucleoside linkage, or a phosphorothioate internucleoside linkage,
a morpholino subunit further comprising a phosphorodiamidate internucleoside linkage where a phosphorous atom of the phosphorodiamidate is covalently bonded to the nitrogen atom of the morpholino ring, and is covalently bonded to a (1,4-piperazin)-1-yl moiety or to a substituted (1,4-piperazin)-1-yl moiety,
a morpholino subunit further comprising a phosphorodiamidate internucleoside linkage where a phosphorus atom of the phosphorodiamidate is covalently bonded to a 4-aminopiperdin-1-yl moiety or a substituted 4-aminopiperdin-1-yl moiety,
a morpholino subunit further comprising a phosphorodiamidate internucleoside linkage where a phosphorus atom of the phosphorodiamidate is covalently bonded to the nitrogen atom of the morpholino ring, and is covalently bonded to a dimethylamino moiety,
a ribose sugar subunit substituted with a phosphorothioate internucleoside or a phosphoramidate internucleoside linkage,
a deoxyribose sugar subunit substituted with a phosphorothioate internucleoside linkage or a phosphoramidate internucleoside linkage,
a peptide nucleic acid subunit optionally substituted,
   or any combination of the foregoing.

In various aspects and embodiments, modified antisense oligomers of the disclosure further comprise a peptide covalently bonded to the modified antisense oligomer. In various embodiments, an arginine-rich cell-penetrating peptide is conjugated to the 3' or the 5' end of the modified antisense oligomer.

In various embodiments, a modified antisense oligomer may consist of about 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 bases, or range from 8 to 50, 8 to 40, 8 to 30, 8 to 25, 8 to 20, 8 to 18, 12 to 30, 12 to 25, 10 to 20, 10 to 18, 15 to 30, 15 to 25, 15 to 20, 15 to 18, 17 to 20, 17 to 30, 17 to 40, 18 to 30, 18 to 25, or 18 to 20 bases, including all integers in between these ranges. In some embodiments, the modified antisense oligomer is about 8 to about 50, about 8 to about 40 or about 8 to about 30 bases in length. In some embodiments, the modified antisense oligomer is about 12 to about 25 bases in length. In some embodiments, a modified antisense oligomer sequence comprises at least about 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 contiguous or non-contiguous bases that are complementary to a target sequence within myostatin or dystrophin pre-mRNA, such as, exon 2, intron 1/exon 2 or exon 2/intron 2 of myostatin pre-mRNA, or one or more of exon 7, exon 8, exon 9, exon 19, exon 23, exon 44, exon 45, exon 50, exon 51, exon 52, exon 53, or exon 55 of dystrophin pre-mRNA, or sequences that span at least a portion of myostatin or dystrophin pre-mRNA.

A modified antisense oligomer may typically comprise a base sequence which is sufficiently complementary to a sequence or region within exon 2, intron 1/exon 2 or exon 2/intron 2 of the myostatin pre-mRNA sequence of the myostatin protein. Table 1 below recites sequences or regions within exon 2, intron 1/exon 2 and exon 2/intron 2.

**Table 1.** Exemplary Sequences of exon 2, intron 1/exon 2 and exon 2/intron 2 of the Myostatin Gene

| Region Within Myostatin Gene | Sequence |
|---|---|
| Intron 1/exon 2/intron 2 (SEQ ID NO: 1) | agcaacttttcttttcttattcatttatag/ctgattttctaatgcaagtggatggaaaaccca aatgttgcttctttaaatttagctctaaaatacaatacaataaagtagtaaaggcccaact atggatatatttgagacccgtcgagactcctacaacagtgtttgtgcaaatcctgagact catcaaacctatgaaagacggtacaaggtatactggaatccgatctctgaaacttgaca tgaacccaggcactggtatttggcagagcattgatgtgaagacagtgttgcaaaattg gctcaaacaacctgaatccaacttaggcattgaaataaaagctttagatgagaatggtc atgatcttgctgtaaccttcccaggaccaggagaagatgggctg/gtaagtgataactg aaaataacattataat |
| SA2 intron 1/exon 2 (SEQ ID NO: 2) | cttttcttttcttattcatttatag/ ctgattttctaatgcaagtggatgg |
| SD2 exon 2/intron 2 (SEQ ID NO: 3) | accttcccaggaccaggagaagatgggctg/gtaagtgataactgaaaataacattat aat |
| "/" indicates the splice site | |

A modified antisense oligomer may typically comprise a base sequence which is sufficiently complementary to a sequence or region within one or more of exons exon 7, exon 8, exon 9, exon 19, exon 23, exon 44, exon 45, exon 50, exon 51, exon 52, exon 53, or exon 55 of dystrophin pre-mRNA sequence of the dystrophin protein. Table 2 below recites sequences or regions within exons exon 7, exon 8, exon 9, exon 19, exon 23, exon 44, exon 45, exon 50, exon 51, exon 52, exon 53, and exon 55.

Table 2. Exemplary Sequences of Exon 7, Exon 8, Exon 9, Exon 19, Exon 23, Exon 44, Exon 45, Exon 50, Exon 51, Exon 52, Exon 53, or Exon 55 of the Dystrophin Gene.

| Region Within Dystrophin Gene | Sequence |
|---|---|
| Exon 7 (SEQ ID NO: 4) | gccagacctatttgactggaatagtgtggtttgccagcagtcagccacacaacgactg gaacatgcattcaacatcgccagatatcaattaggcatagagaaactactcgatcctg aag |
| Exon 8 (SEQ ID NO: 5) | atgttgataccacctatccagataagaagtccatcttaatgtacatcacatcactcttcca agttttgcctcaacaagtgagcattgaagccatccaggaagtggaaatgttgccaagg ccacctaaagtgactaaagaagaacattttcagttacatcatcaaatgcactattctcaa cag |
| Exon 9 (SEQ ID NO: 6) | atcacggtcagtctagcacagggatatgagagaacttcttcccctaagcctcgattcaa gagctatgcctacacacaggctgcttatgtcaccacctctgaccctacacggagccca tttccttcacag |
| Exon 19 (SEQ ID NO: 7) | gccatagagcgagaaaaagctgagaagttcagaaaactgcaagatgccagcagatc agctcaggccctggtggaacagatggtgaatg |
| Exon 23 (SEQ ID NO: 8) | gctttacaaagttctctgcaagagcaacaaagtggcctatactatctcagcaccactgt gaaagagatgtcgaagaaagcgccctctgaaattagccggaaatatcaatcagaattt gaagaaattgagggacgctggaagaagctctcctcccagctggttgagcattgtcaa aagctagaggagcaaatgaataaactccgaaaaattcag |
| Exon 44 (SEQ ID NO: 9) | gcgatttgacagatctgttgagaaatggcggcgttttcattatgatataaagatatttaat cagtggctaacagaagctgaacagtttctcagaaagacacaaattcctgagaattggg aacatgctaaatacaaatggtatcttaag |
| Exon 45 (SEQ ID NO: 10) | gaactccaggatggcattgggcagcggcaaactgttgtcagaacattgaatgcaact ggggaagaaataattcagcaatcctcaaaaacagatgccagtattctacaggaaaaat tgggaagcctgaatctgcggtggcaggaggtctgcaaacagctgtcagacagaaaa aagag |
| Exon 50 (SEQ ID NO: 11) | aggaagttagaagatctgagctctgagtggaaggcggtaaaccgtttacttcaagag ctgagggcaaagcagcctgacctagctcctggactgaccactattggagcct |
| Exon 51 (SEQ ID NO: | ctcctactcagactgttactctggtgacacaacctgtggttactaaggaaactgccatct |
| 12) | ccaaactagaaatgccatcttccttgatgttggaggtacctgctctggcagatttcaacc gggcttggacagaacttaccgactggctttctctgcttgatcaagttataaaatcacaga gggtgatggtgggtgaccttgaggatatcaacgagatgatcatcaagcagaag |
| Exon 52 (SEQ ID NO: 13) | gcaacaatgcaggatttggaacagaggcgtccccagttggaagaactcattaccgct gcccaaaatttgaaaaacaagaccagcaatcaagaggctagaacaatcattacggat cgaa |
| Exon 53 (SEQ ID NO: 14) | ttgaaagaattcagaatcagtgggatgaagtacaagaacaccttcagaaccggaggc aacagttgaatgaaatgttaaaggattcaacacaatggctggaagctaaggaagaag ctgagcaggtcttaggacaggccagagccaagcttgagtcatggaaggagggtccc tatacagtagatgcaatccaaaagaaaatcacagaaaccaag |
| Exon 55 (SEQ ID NO: 15) | ggtgagtgagcgagaggctgctttggaagaaactcatagattactgcaacagttcccc ctggacctggaaaagtttcttgcctggcttacagaagctgaaacaactgccaatgtcct acaggatgctacccgtaaggaaaggctcctagaagactccaagggagtaaaagag ctgatgaaacaatggcaa |

Preferably, a modified antisense oligomer effectively decreases expression of an exon, such as exon 2, thereby decreasing expression of a functional myostatin protein. Preferably, a modified dystrophin antisense oligomer effectively modulates abberant splicing of the dystrophin pre-mRNA, thereby increasing expression of a functional or semi-functional dystrophin protein. This requirement is optionally met when the oligomer compound has the ability to be actively taken up by mammalian cells, and once taken up, form a stable duplex (or heteroduplex) with the target mRNA, optionally with a Tm greater than about 40 °C or 45 °C.

"Complementary" or "complementary" as used herein, refers to a targeting sequence of a modified antisense oligomer having about 90% to about 100% of the nucleotide targeting sequence complementary to a target sequence. In embodiments, a complementary nucleotide targeting sequence specifically hybridizes to a target sequence to induce a desired effect, for example, a therapeutic effect as described herein. In certain embodiments, targeting sequences of modified antisense oligomers may be 100% complementary to the target sequence, or may include mismatches, e.g., to accommodate variants, as long as a heteroduplex formed between the oligomer targeting sequence and target sequence is sufficiently stable to withstand the action of cellular nucleases and other modes of degradation which may occur *in vivo.* Hence, certain oligomer targeting sequences may have substantial complementarity, meaning, about or at least about 90% sequence complementarity, e.g., 90%, 91%, 92%, 93%, 94%, 95%, 96%, 97%, 98%, 99% or 100% sequence complementarity, between the oligomer targeting sequence and the target sequence. Oligomer internucleoside linkages that are less susceptible to cleavage by nucleases are provided herein. Mismatches, if present, are typically less destabilizing toward the end regions of the hybrid duplex than in the middle. The number of mismatches allowed will depend on the length of the oligomer, the percentage of G:C base pairs in the duplex, and the position of the mismatch(es) in the duplex, according to well understood principles of duplex stability. Although such a modified antisense oligomer need not necessarily comprise 100% complementary to the target sequence, it should have sufficient complementarity to effectively, stably and specifically bind to the target sequence, such that splicing of the target pre-mRNA is sufficiently modulated, for example, to achieve a therapeutic effect, as described herein.

Without being bound by theory, the stability of the duplex formed between an oligomer and a target sequence is believed to be a function of the binding Tm and the susceptibility of the duplex to cellular enzymatic cleavage. The Tm of an oligomer with respect to a complementary-sequence RNA duplex may be measured by conventional methods, such as those described by Hames et al., Nucleic Acid Hybridization, IRL Press, 1985, pp. 107-108 or as described in Miyada C. G. and Wallace R. B., 1987, Oligomer Hybridization Techniques, Methods Enzymol. Vol. 154 pp. 94-107, the contents of which are incorporated herein by reference. In various embodiments, the modified antisense oligomers have a binding Tm, with respect to a complementary-sequence RNA duplex, of greater than body temperature, such as, for example, greater than about 45 °C or 50 °C. Tm's in the range 60-80 °C or greater are also included. According to well-known principles, the Tm of an oligomer, with respect to a complementary-based RNA hybrid duplex, can be increased by increasing the ratio of C:G paired bases in the duplex, and/or by increasing the length (in base pairs) of the heteroduplex.

**Table 3** below shows exemplary targeting sequences (in a 5'-to-3' orientation) that are complementary to the target regions within exon 2, intron 1/exon 2 or exon 2/intron 2 of myostatin pre-mRNA.

| Targeting Sequence | Sequence |
|---|---|
| MS TN-D3 0 (SEQ ID NO: 16) | cagcccatcttctcctggtcctgggaaggt |
| muhuMSTN-SD2(+24-01) (SEQ ID NO: 17) | ccagcccatcttctcctggtcctgg |
| muhuMSTN-SD2(+18-07) (SEQ ID NO: 18) | cacttaccagcccatcttctcctgg |
| huMSTN-SA2(-01+25) (SEQ ID NO: 19) | ccatccgcttgcattagaaagtcagc |
| huMSTN-SA2(-09+15) (SEQ ID NO: 20) | gcattagaaaatcagctataaatg |
| huMSTN-SA2(-01+21) (SEQ ID NO: 21) | ccacttgcattagaaaatcagc |
| huMSTN-SA2(-07+18) (SEQ ID NO: 22) | cttgcattagaaaatcagctataaa |
| huMSTN-SA2(-05+20) (SEQ ID NO: 23) | cacttgc attagaaaatcagctata |
| huMSTN-SA2(-04+21) (SEQ ID NO: 24) | ccacttgcattagaaaatcagctat |
| huMSTN-SA2(-03+22) (SEQ ID NO: 25) | tccacttgcattagaaaatcagcta |
| huMSTN-SA2(-02+23) (SEQ ID NO: 26) | atccacttgcattagaaaatcagct |
| huMSTN-SA2(-01+24) (SEQ ID NO: 27) | catccacttgcattagaaaatcagc |
| muhuMSTN-SD2(+04-21) (SEQ ID NO: 28) | ttattttcagttatcacttaccagc |
| muhuMSTN-SD2(+07-18) (SEQ ID NO: 29) | ttttcagttatcacttaccagccca |
| muhuMSTN-SD2(+10-15) (SEQ ID NO: 30) | tcagttatcacttaccagcccatct |
| muhuMSTN-SD2(+13-12) (SEQ ID NO: 31) | gttatcacttaccagcccatcttct |
| muhuMSTN-SD2(+16-09) (SEQ ID NO: 32) | atcacttaccagcccatcttctcct |
| muhuMSTN-SD2(+19-06) (SEQ ID NO: 33) | acttaccagcccatcttctcctggt |
| muhuMSTN-SD2(+22-03) (SEQ ID NO: 34) | taccagcccatcttctcctggtcct |
| muhuMSTN-SD2(+01-24) (SEQ ID NO: 35) | atgttattttcagttatcacttacc |
| muhuMSTN-SD2(+02-23) (SEQ ID NO: 36) | tgttattttcagttatcacttacca |
| muhuMSTN-SD2(+03-22) (SEQ ID NO: 37) | gttattttcagttatcacttaccag |
| muhuMSTN-SD2(+05-20) (SEQ ID NO: 38) | tattttcagttatcacttaccagcc |
| muhuMSTN-SD2(+06-19) (SEQ ID NO: 39) | attttcagttatcacttaccagccc |
| muhuMSTN-SD2(+08-17) (SEQ ID NO: 40) | tttcagttatcacttaccagcccat |
| muhuMSTN-SD2(+09-16) (SEQ ID NO: 41) | ttcagttatcacttaccagcccatc |
| muhuMSTN-SD2(+11-14) (SEQ ID NO: 42) | cagttatcacttaccagcccatctt |
| muhuMSTN-SD2(+12-13) (SEQ ID NO: 43) | agttatcacttaccagcccatcttc |
| Mstn D ('139 app) (SEQ ID NO: 44) | cagcccatcttctcctggtcctgggaaggt |
| GDF8/D3 ('139 app) (SEQ ID NO: 45) | cagcccatcttctcctggtc |
| GDF8/D2 ('139 app) (SEQ ID NO: 46) | tctcctggtcctgggaaggt |
| GDF8/D1 ('139 app) (SEQ ID NO: 47) | ctgggaaggttacagcaaga |
| huMSTN-SD2(+18+1) (SEQ ID NO: 48) | cagcccatcttctcctgg |
| muhuMSTN-SD2(+25+03) (SEQ ID NO: 49) | gcccatcttctcctggtcctggg |
| huMSTN-SA2(+26+50) (SEQ ID NO: 50) | tttaaagaagcaacatttgggtttt |
| huMSTN-SA2(+41+65) (SEQ ID NO: 51) | tattttagagctaaatttaaagaag |
| huMS TN-SA2(+56+80) (SEQ ID NO: 52) | tactttattgtattgtattttagag |
| huMSTN-SA2(+71+95) (SEQ ID NO: 53) | tagttgggcctttactactttattg |
| huMSTN-SA2(+86+110) (SEQ ID NO: 54) | tctcaaatatatccatagttgggcc |
| huMSTN-SA2(+91+115) (SEQ ID NO: 55) | ac gggtctcaaatatatccatagtt |
| huMSTN-SA2(+101+130) (SEQ ID NO: 56) | gttgtaggagtctc gac gggtctcaaatat |
| huMSTN-SA2(+111+135) (SEQ ID NO: 57) | acactgttgtaggagtctcgacggg |
| huMSTN-SA2(+141+165) (SEQ ID NO: 58) | taggtttgatgagtctcaggatttg |
| huMSTN-SA2(+151+175) (SEQ ID NO: 59) | ccgtctttcataggtttgatgagtc |
| huMSTN-S A2(+160+189) (SEQ ID NO: 60) | cagtataccttgtaccgtctttcataggtt |
| huMSTN-SA2(+196+220) (SEQ ID NO: 61) | gggttcatgtcaagtttcagagatc |
| huMSTN-SA2(+204+233) (SEQ ID NO: 62) | aataccagtgcctgggttcatgtcaagttt |
| huMSTN-SA2(+210+234) (SEQ ID NO: 63) | aaataccagtgcctgggttcatgtc |
| huMSTN-SA2(+216+240) (SEQ ID NO: 64) | tctgccaaataccagtgcctgggtt |
| huMSTN-SA2(+231+255) (SEQ ID NO: 65) | tcttcacatcaatgctctgccaaat |
| huMSTN-SA2(+261+285) (SEQ ID NO: 66) | caggttgtttgagccaattttgcaa |
| huMSTN-SA2(+276+291) (SEQ ID NO: 67) | tgcctaagttggattcaggttgttt |
| huMSTN-SA2(+291+305) (SEQ ID NO: 68) | aagcttttatttcaatgcctaagtt |
| huMSTN-SA2(+306+330) (SEQ ID NO: 69) | gaccattctcatctaaagcttttat |
| huMSTN-SA2(+321+345) (SEQ ID NO: 70) | ttacagcaagatcatgaccattctc |
| "/" indicates the splice site | |

Certain modified antisense oligomers thus comprise, consist, or consist essentially of a sequence in Table 3 (e.g., SEQ ID NOS: 16 to 75), is selected from SEQ ID NOS: 16 to 75, is a fragment of at least 12 contiguous nucleotides of a sequence selected from SEQ ID NOS: 16 to 75, or is a variant having at least 90% sequence identity to a sequence selected from SEQ ID NOS: 16 to 75, wherein each X is independently selected from uracil (U) or thymine (T), and wherein each Y is independently selected from cytosine (C) or 5-Methylcytosine (5mC). For instance, certain modified antisense oligomers comprise about or at least about 12, 13, 14, 15, 16, 17, 18, 19, 20, 21, 22, 23, 24, 25, 26, 27, 28, 29, 30, 31, 32, 33, 34, 35, 36, 37, 38, 39, or 40 contiguous or non-contiguous nucleotides of any of SEQ ID NOS: 16 to 75. For non-contiguous portions, intervening nucleotides can be deleted or substituted with a different nucleotide, or intervening nucleotides can be added. Additional examples of variants include oligomers having about or at least about 90% sequence identity or homology, e.g., 90%, 91%, 92%, 93%, 94%, 95%, 96%, 97%, 98%, 99% or 100% sequence identity or homology, over the entire length of any of SEQ ID NOS: 16 to 75. In certain embodiments, the targeting sequence is selected from SEQ ID NOS: 16 to 75.

Oligonucleotides that target the dystrophin gene are disclosed in WO 2006/000057, WO 2011/057350, WO 2010/048586, WO 2014/100714, WO 2014/153220, US Application No. US20140315862, US Application No. US20140323544, US Application No. US20120202752, US Application No. US20030235845, US Application No. US20110312086, US Application No. US20090312532, US Application No. US20090269755, US Application No. US20130211062, US Application No. US20140343266, US Application No. US20120059042, US Application No. US20110294753, US Application No. US20140113955, US Application No. US20150166996, US Application No. US20150203849, US Application No. US20150045413, and US Application No. US20140057964, which are hereby incorporated by reference in their entireties.

Certain modified antisense oligomers thus comprise, consist, or consist essentially of a sequence in Table 4 (e.g., SEQ ID NOS: 76 to 3485), is selected from SEQ ID NOS: 76 to 3485, is a fragment of at least 10 contiguous nucleotides of a sequence selected from SEQ ID NOS: 76 to 3485, or is a variant having at least 90% sequence identity to a sequence selected from SEQ ID NOS: 76 to 3485, wherein each X is independently selected from uracil (U) or thymine (T), and wherein each Y is independently selected from cytosine (C) or 5-Methylcytosine (5mC). For instance, certain modified antisense oligomers comprise about or at least about 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 contiguous or non-contiguous nucleotides of any of SEQ ID NOS: 76 to 3485. For non-contiguous portions, intervening nucleotides can be deleted or substituted with a different nucleotide, or intervening nucleotides can be added. Additional examples of variants include oligomers having about or at least about 90% sequence identity or homology, e.g., 90%, 91%, 92%, 93%, 94%, 95%, 96%, 97%, 98%, 99% or 100% sequence identity or homology, over the entire length of any of SEQ ID NOS: 76 to 3485. In certain embodiments, the targeting sequence is selected from SEQ ID NOS: 76 to 3485. In some embodiments, a targeting sequence may comprise SEQ ID NO: 76.

The activity/functionality of modified antisense oligomers and variants thereof can be assayed according to routine techniques in the art. For example, splice forms and expression levels of surveyed RNAs may be assessed by any of a wide variety of well-known methods for detecting splice forms and/or expression of a transcribed nucleic acid or protein. Non-limiting examples of such methods include RT-PCR of spliced forms of RNA followed by size separation of PCR products, nucleic acid hybridization methods e.g., Northern blots and/or use of nucleic acid arrays; nucleic acid amplification methods; immunological methods for detection of proteins; protein purification methods; and protein function or activity assays.

RNA expression levels can be assessed by preparing mRNA/cDNA (i.e., a transcribed oligonucleotide) from a cell, tissue or organism, and by hybridizing the mRNA/cDNA with a reference oligonucleotide that is a complement of the assayed nucleic acid, or a fragment thereof. cDNA can, optionally, be amplified using any of a variety of polymerase chain reaction or in vitro transcription methods prior to hybridization with the complementary oligonucleotide; preferably, it is not amplified. Expression of one or more transcripts can also be detected using quantitative PCR to assess the level of expression of the transcript(s).

### III. Modified Antisense Oligomer Chemistries

### A. General Characteristics

In various aspects and embodiments, the modified antisense oligomers specifically hybridize to target region within myostatin pre-mRNA. Exemplary modified antisense oligomers comprise a targeting sequence set forth in Table 3, a fragment of at least 12 contiguous nucleotides of a targeting sequence in Table 3, or a variant having at least 90% sequence identity to a targeting sequence in Table 3. Other exemplary modified antisense oligomers consist or consist essentially of a targeting sequence set forth in Table 3.

In various aspects and embodiments, the modified antisense oligomers specifically hybridize to target region within dystrophin pre-mRNA. Exemplary modified antisense oligomers comprise a targeting sequence set forth in Table 4, a fragment of at least 10 contiguous nucleotides of a targeting sequence in Table 4, or a variant having at least 90% sequence identity to a targeting sequence in Table 4. Other exemplary modified antisense oligomers consist or consist essentially of a targeting sequence set forth in Table 4.

Nuclease-resistant modified antisense oligomers are provided in a further aspect. In various embodiments, a modified antisense oligomer is provided comprising one or more internucleoside linkage modification(s). In other embodiments, a modified antisense oligomer is provided comprising one or more modified sugar moieties. In other embodiments, a modified antisense oligomer is provided comprising a combination of one or more modified internucleoside linkages and one or more modified sugar moieties. In other embodiments, a modified antisense oligomer is provided comprising a modified nucleobase, alone or in combination with any of a modified internucleoside linkage or a modified sugar moiety.

In various embodiments, a modified antisense oligomer may comprise an oligomer having completely modified internucleoside linkages, for example, 100% of the internucleoside linkages are modified (for example, a 25-mer modified antisense oligomer comprises 24 intemucleoside linkages modified with one or any combination of the modifications as described herein). In various embodiments, a modified antisense oligomer may comprise about 100% to 2.5% of its internucleoside linkages modified. In various embodiments, a modified antisense oligomer may comprise about 99%, 95%, 90%, 85%, 80%, 75%, 70%, 65%, 60%, 55%, 50%, 45%, 40%, 35%, 30%, 25%, 20%, 15%, 10%, 5%, or 2.5% of its internucleoside linkages modified, and iterations in between. In other embodiments, a modified antisense oligomer may comprise any combination of modifications as described herein.

In various embodiments, including embodiments in combination with embodiments of percent of modified intemucleoside linkages, a modified antisense oligomer may comprise an oligomer having completely modified sugar moieties, for example, 100% of the sugar moieties are modified (for example, a 25 mer modified antisense oligomer comprises 25 sugar moieties modified with one or any combination of the modifications as described herein). In various embodiments, a modified antisense oligomer may comprise about 100% to 2.5% of its sugar moieties modified. In various embodiments, a modified antisense oligomer may comprise about 99%, 95%, 90%, 85%, 80%, 75%, 70%, 65%, 60%, 55%, 50%, 45%, 40%, 35%, 30%, 25%, 20%, 15%, 10%, 5%, or 2.5% of its sugar moieties modified, and iterations in between. In other embodiments, a modified antisense oligomer may comprise any combination of modifications as described herein.

In various embodiments, the modified antisense oligomer is substantially uncharged, and is optionally suitable as a substrate for active or facilitated transport across the cell membrane. In some embodiments, all of the internucleoside linkages are uncharged. The ability of the oligomer to form a stable duplex with the target pre-mRNA may also relate to other features of the oligomer, including the length and degree of complementarity of the modified antisense oligomer with respect to the target, the ratio of G:C to A:T base matches, and the positions of any mismatched bases. The ability of the modified antisense oligomer to resist cellular nucleases may promote survival and ultimate delivery of the agent to the cell cytoplasm.

In various embodiments, the modified antisense oligomer has at least one intemucleoside linkage that is positively charged or cationic at physiological pH. In further embodiments, the modified antisense oligomer has at least one internucleoside linkage that exhibits a pKa between about 5.5 and about 12. In further embodiments, the modified antisense oligomer contains about, at least about, or no more than about 1, 2, 3, 4, 5, 6, 7, 8, 9, or 10 intemucleoside linkages that exhibits a pKa between about 4.5 and about 12. In some embodiments, the modified antisense oligomer contains about or at least about 10%, 15%, 20%, 25%, 30%, 35%, 40%, 45%, 50%, 55%, 60%, 65%, 70%, 75%, 80%, 85%, 90%, 95%, or 100% intemucleoside linkages that exhibit a pKa between about 4.5 and about 12. Optionally, the modified antisense oligomer has at least one internucleoside linkage with both a basic nitrogen and an alkyl, aryl, or aralkyl group. In particular embodiments, the cationic intemucleoside linkage or linkages comprise a 4-aminopiperdin-1-yl (APN) group, or a derivative thereof. In some embodiments, the modified antisense oligomer comprises a morpholino ring. While not being bound by theory, it is believed that the presence of a cationic linkage or linkages (e.g., APN group or APN derivative) in the oligomer facilitates binding to the negatively charged phosphates in the target nucleotide. Thus, the formation of a heteroduplex between mutant RNA and the cationic linkage-containing oligomer may be held together by both an ionic attractive force and Watson-Crick base pairing.

In various embodiments, the number of cationic linkages is at least 2 and no more than about half the total internucleoside linkages, e.g., about or no more than about 1, 2, 3, 4, 5, 6, 7, 8, 9, 10, 11, 12, 13, 14, 15, 16, 17, 18, 19, 20 cationic linkages. In some embodiments, however, up to all of the internucleoside linkages are cationic linkages, e.g., about or at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 of the total internucleoside linkages are cationic linkages. In further embodiments, an oligomer of about 19-20 monomer subunits may have 2-10, e.g., 4-8, cationic linkages, and the remainder uncharged linkages. In other specific embodiments, an oligomer of 14-15 subunits may have 2-7, e.g., 2, 3, 4, 5, 6, or 7 cationic linkages and the remainder uncharged linkages. The total number of cationic linkages in the oligomer can thus vary from about 1 to 10 to 18 to 20 to 30 or more (including all integers in between), and can be interspersed throughout the oligomer.

In some embodiments, a modified antisense oligomer may have about or up to about 1 cationic linkage per every 2-5 or 2, 3, 4, or 5 uncharged linkages, such as about 4-5 or 4 or 5 per every 10 uncharged linkages.

Certain embodiments include modified antisense oligomers that contain about 10%, 15%, 20%, 25%, 30%, 35%, 40%, 45%, 50%, 55%, 60%, 65%, 70%, 75%, 80%, 85%, 90%, 95%, or 100% cationic linkages. In certain embodiments, optimal improvement in antisense activity may be seen if about 25% of the internucleoside linkages are cationic. In certain embodiments, enhancement may be seen with a small number e.g., 10-20% cationic linkages, or where the number of cationic linkages is in the range 50-80%, such as about 60%.

In further embodiments, the cationic linkages are interspersed along the internucleoside linkage. Such oligomers optionally contain at least two consecutive uncharged linkages; that is, the oligomer optionally does not have a strictly alternating pattern along its entire length. In specific instances, each one or two cationic linkage(s) is/are separated along the internucleoside linkage by at least 1, 2, 3, 4, or 5 uncharged linkages.

Also included are oligomers having blocks of cationic linkages and blocks of uncharged linkages. For example, a central block of uncharged linkages may be flanked by blocks of cationic linkages, or vice versa. In some embodiments, the oligomer has approximately equal-length 5', 3' and center regions, and the percentage of cationic linkages in the center region is greater than about 50%, 60%, 70%, or 80% of the total number of cationic linkages.

In certain modified antisense oligomers, the bulk of the cationic linkages (e.g., 70, 75%, 80%, 90% of the cationic linkages) are distributed close to the "center-region" of the internucleoside linkages, e.g., the 6, 7, 8, 9, 10, 11, 12, 13, 14, or 15 centermost linkages. For example, a 16, 17, 18, 19, 20, 21, 22, 23, or 24-mer oligomer may have at least 50%, 60%, 70%, or 80% of the total cationic linkages localized to the 8, 9, 10, 11, or 12 centermost linkages.

### B. Chemistry Features

The modified antisense oligomers may contain a variety of nucleotide analog subunits. Further examples include:
phosphoramidate containing oligomers,
phosphorodiamidate containing oligomers,
phosphorotriamidate containing oligomers,
phosphorothioate containing oligomers,
morpholino containing oligomers optionally substituted with a phosphoramidate internucleoside linkage or a phosphorodiamidate internucleoside linkage,
2'O-methyl containing oligomers optionally substituted with a phosphorothioate internucleoside linkage,
   locked nucleic acid (LNA) containing oligomers optionally substituted with a phosphorothioate internucleoside linkage,
2' O-methoxyethyl (MOE) containing oligomers optionally substituted with a phosphorothioate internucleoside linkage,
2'-fluoro-containing oligomers optionally substituted with a phosphorothioate internucleoside linkage,
2'O,4'C-ethylene-bridged nucleic acids (ENAs) containing oligomers optionally substituted with a phosphorothioate internucleoside linkage,
tricyclo-DNA (tc-DNA) containing oligomers optionally substituted with a phosphorothioate internucleoside linkage,
2'-O-[2-(N-methylcarbamoyl)ethyl] containing oligomers optionally substituted with a phosphorothioate internucleoside linkage,
morpholino containing oligomers further comprising a phosphorodiamidate internucleoside linkage wherein the phosphorous atom of the phosphorodiamidate is covalently bonded to the nitrogen atom of a morpholino ring, and is covalently bonded to a (1,4-piperazin)-1-yl moiety or to a substituted (1,4-piperazin)-1-yl (PMOplus) moiety,
morpholino containing oligomers further comprising a phosphorodiamidate internucleoside linkage wherein the phosphorus atom of the phosphorodiamidate is covalently bonded to the nitrogen atom of a morpholino ring and is covalently bonded to a 4-aminopiperdin-1-yl moiety (i.e., APN) or a substituted 4-aminopiperdin-1-yl (PMO-X) moiety,
a morpholino subunit further comprising a phosphorodiamidate internucleoside linkage where a phosphorus atom of the phosphorodiamidate is covalently bonded to the nitrogen atom of the morpholino ring, and is covalently bonded to a dimethylamino moiety,
ribose sugar containing oligomers further comprising a phosphorothioate internucleoside linkage or a phosphoramidate internucleoside linkage,
deoxyribose sugar containing oligomers further comprising a phosphorothioate internucleoside linkage oligomer or a phosphoramidate internucleoside linkage,
peptide-conjugated phosphorodiamidate morpholino containing oligomers (PPMO) which are further optionally substituted,
peptide nucleic acid (PNA) oligomers which are further optionally substituted including further substitutions,
and combinations of any of the foregoing.

In certain embodiments, the phosphorous atom of a phosphorodiamidate linkage is further substituted with a (1,4-piperazin)-1-yl moiety, a substituted (1,4-piperazin)-1-yl moiety, a 4-aminopiperidin-1-yl moiety, or a substituted 4-aminopiperidin-1-yl moiety.

In general, PNA and LNA chemistries can utilize shorter targeting sequences because of their relatively high target binding strength relative to PMO and 2'O-Me oligomers. Phosphorothioate and 2'O-Me chemistries can be combined to generate a 2'O-Mephosphorothioate analog. See, e.g., PCT Publication Nos. WO/2013/112053 and WO/2009/008725, which are hereby incorporated by reference in their entireties.

In some instances, modified antisense oligomers, such as phosphorodiamidate morpholino oligomers (PMO), can be conjugated to cell penetrating peptides (CPPs) to facilitate intracellular delivery. Peptide-conjugated PMOs are called PPMOs and certain embodiments include those described in PCT Publication No. WO/2012/150960, which is hereby incorporated by reference in its entirety. In some embodiments, an arginine-rich peptide sequence conjugated or linked to, for example, the 3' terminal end of a modified antisense oligomer as described herein may be used.

### 1. Peptide Nucleic Acids (PNAs)

Peptide nucleic acids (PNAs) are analogs of DNA in which the backbone is structurally homomorphous with a deoxyribose backbone, consisting of N-(2-aminoethyl) glycine units to which pyrimidine or purine bases are attached. PNAs containing natural pyrimidine and purine bases hybridize to complementary oligomers obeying Watson-Crick base-pairing rules, and mimic DNA in terms of base pair recognition (Egholm, Buchardt *et al.* 1993). The internucleoside linkages of PNAs are formed by peptide bonds rather than phosphodiester bonds, making them well-suited for antisense applications (see structure below). The backbone is uncharged, resulting in PNA/DNA or PNA/RNA duplexes that exhibit greater than normal thermal stability. PNAs are not recognized by nucleases or proteases. A non-limiting example of a PNA oligomer comprising PNA subunits is depicted below:

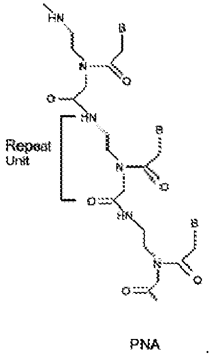

Despite a radical structural change to the natural structure, PNAs are capable of sequence-specific binding in a helix form to DNA or RNA. Characteristics of PNAs include a high binding affinity to complementary DNA or RNA, a destabilizing effect caused by single-base mismatch, resistance to nucleases and proteases, hybridization with DNA or RNA independent of salt concentration and triplex formation with homopurine DNA. PANAGENE (Daejeon, Korea) has developed Bts PNA monomers (Bts; benzothiazole-2-sulfonyl group) and oligomerization process. The PNA oligomerization using Bts PNA monomers is composed of repetitive cycles of deprotection, coupling and capping. PNAs can be produced synthetically using any technique known in the art. See, e.g., U.S. Patent Nos. 6,969,766, 7,211,668, 7,022,851, 7,125,994, 7,145,006 and 7,179,896. See also U.S. Patent Nos. 5,539,082; 5,714,331; and 5,719,262 for the preparation of PNAs. Further teaching of PNA compounds can be found in Nielsen et al., Science, 254:1497-1500, 1991. Each of the foregoing is hereby incorporated by reference in its entirety.

### 2. Locked Nucleic Acids (LNAs)

Modified antisense oligomer compounds may also contain "locked nucleic acid" subunits (LNAs). "LNAs" are a member of a class of modifications called bridged nucleic acid (BNA). BNA is characterized by a covalent linkage that locks the conformation of the ribose ring in a C30-endo (northern) sugar pucker. For LNA, the bridge is composed of a methylene between the 2'-O and the 4'-C positions. LNA enhances backbone preorganization and base stacking to increase hybridization and thermal stability.

The structures of LNAs can be found, for example, in Wengel, et al., Chemical Communications (1998) 455; Tetrahedron (1998) 54:3607, and Accounts of Chem. Research (1999) 32:301); Obika, et al., Tetrahedron Letters (1997) 38:8735; (1998) 39:5401, and Bioorganic Medicinal Chemistry (2008) 16:9230, which are hereby incorporated by reference in their entirety. A non-limiting example of an LNA oligomer comprising LNA subunits and phosphodiester internucleoside linkages is depicted below:

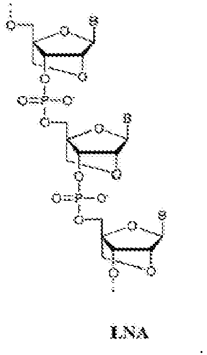

Compounds of the disclosure may incorporate one or more LNAs; in some cases, the compounds may be entirely composed of LNAs. Methods for the synthesis of individual LNA nucleoside subunits and their incorporation into oligomers are described, for example, in U.S. Patent Nos. 7,572,582, 7,569,575, 7,084,125, 7,060,809, 7,053,207, 7,034,133, 6,794,499, and 6,670,461, which are hereby incorporated by reference in their entirety. Typical internucleoside linkers include phosphodiester and phosphorothioate moieties; alternatively, non-phosphorous containing linkers may be employed. Further embodiments include an LNA containing compound where each LNA subunit is separated by a DNA subunit. Certain compounds are composed of alternating LNA and DNA subunits where the internucleoside linker is phosphorothioate.

2'O,4'C-ethylene-bridged nucleic acids (ENAs) are another member of the class of BNAs. A non-limiting example of an ENA subunit and phosphodiester internucleoside linkage is depicted below:

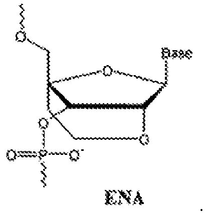

ENA oligomers and their preparation are described in Obika et al., Tetrahedron Ltt 38(50): 8735, which is hereby incorporated by reference in its entirety. Compounds of the disclosure may incorporate one or more ENA subunits.

### 3. Phosphorothioates

"Phosphorothioates" (or S-oligos) are a variant of native DNA or RNA in which one of the nonbridging oxygens of the phosphodiester internucleoside linkages is replaced by sulfur. A non-limiting example of a phosphorothioate DNA (left), comprising deoxyribose subunits and phosphorothioate internucleoside linkages, and phosphorothioate RNA (right), comprising ribose subunits and phosophorothioate internucleoside linkages, are depicted below:

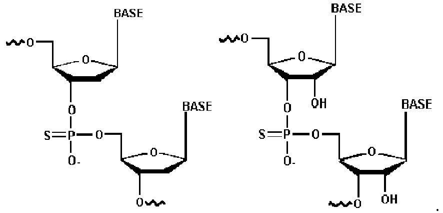

The sulfurization of the internucleoside bond reduces the action of endo-and exonucleases including 5' to 3' and 3' to 5' DNA POL 1 exonuclease, nucleases S1 and P1, RNases, serum nucleases and snake venom phosphodiesterase. Phosphorothioates may be made by two principal routes: by the action of a solution of elemental sulfur in carbon disulfide on a hydrogen phosphonate, or by the method of sulfurizing phosphite triesters with either tetraethylthiuram disulfide (TETD) or 3H-1, 2-bensodithiol-3-one 1, 1-dioxide (BDTD) (see, e.g., Iyer et al., J. Org. Chem. 55, 4693-4699, 1990, which are hereby incorporated by reference in their entirety). The latter methods avoid the problem of elemental sulfur's insolubility in most organic solvents and the toxicity of carbon disulfide. The TETD and BDTD methods also yield higher purity phosphorothioates.

### 4. Tricyclo-DNAs and Tricyclo-Phosphorothioate Nucleotides

Tricyclo-DNAs (tc-DNA) are a class of constrained DNA analogs in which each nucleotide is modified by the introduction of a cyclopropane ring to restrict conformational flexibility of the backbone and to optimize the backbone geometry of the torsion angle γ. Homobasic adenine- and thymine-containing tc-DNAs form extraordinarily stable A-T base pairs with complementary RNAs. Tricyclo-DNAs and their synthesis are described in PCT Publication No. WO 2010/115993, which is hereby incorporated by reference in its entirety. Compounds of the disclosure may incorporate one or more tricyclo-DNA subunits; in some cases, the compounds may be entirely composed of tricyclo-DNA subunits.

Tricyclo-phosphorothioate nucleotides are tricyclo-DNA subunits with phosphorothioate internucleoside linkages. Tricyclo-phosphorothioate nucleotides and their synthesis are described in PCT Publication No. WO 2013/053928, which is hereby incorporated by reference in its entirety. Compounds of the disclosure may incorporate one or more tricyclo-DNA subunits; in some cases, the compounds may be entirely composed of tricyclo-DNA nucleotides. A non-limiting example of a tricyclo-DNA/tricycle subunit and phosphodiester internucleoside linkage is depicted below:

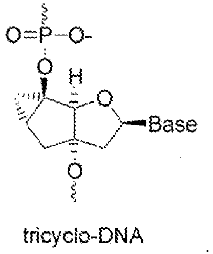

### 5. 2' O-Methyl, 2' O-MOE, and 2'-F Oligomers

"2'O-Me oligomer" molecules comprise subunits that carry a methyl group at the 2'-OH residue of the ribose molecule. 2'-O-Me-RNAs show the same (or similar) behavior as DNA, but are protected against nuclease degradation. 2'-O-Me-RNAs can also be combined with phosphorothioate oligomers (PTOs) for further stabilization. 2'O-Me oligomers (wherein the 2'-OMe subunits are connected by phosphodiester or phosphorothioate internucleoside linkages) can be synthesized according to routine techniques in the art (see, e.g., Yoo et al., Nucleic Acids Res. 32:2008-16, 2004, which is hereby incorporated by reference in its entirety). A non-limiting example of a 2' O-Me oligomer comprising 2'-OMe subunits and phosphodiester intersubunit linkages is depicted below:

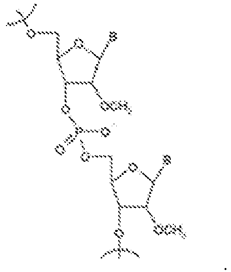

2' O-Me oligomers may also comprise a phosphorothioate linkage (2' O-Me phosphorothioate oligomers). 2' O-Methoxyethyl Oligomers (2'-O MOE), like 2' O-Me oligomers, comprise subunits that carry a methoxyethyl group at the 2'-OH residue of the ribose molecule and are discussed in Martin et al., Helv. Chim. Acta, 78, 486-504, 1995, which is hereby incorporated by reference in its entirety. A non-limiting example of a 2' O-MOE subunit is depicted below:

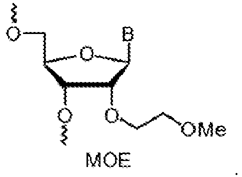

In contrast to the preceding alkylated 2'OH ribose derivatives, 2'-fluoro oligomers comprise subunits that have a fluoro radical in at the 2' position in place of the 2'OH. A non-limiting example of a 2'-F oligomer comprising 2'-F subunits and phosphodiester internucleoside linkages is depicted below:

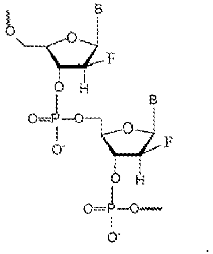

2'-fluoro oligomers are further described in WO 2004/043977, which is hereby incorporated by reference in its entirety. Compounds of the disclosure may incorporate one or more 2'O-Methyl, 2' O-MOE, and 2' F subunits and may utilize any of the internucleoside linkages described here. In some instances, a compound of the disclosure could be composed of entirely 2'O-Methyl, 2' O-MOE, or 2' F subunits. One embodiment of a compound of the disclosure is composed entirely of 2'O-methyl subunits.

### 6. 2'-O-[2-(N-methylcarbamoyl)ethyl] Oligomers (MCEs)

MCEs are another example of 2'O modified ribonucleotides useful in the compounds of the disclosure. Here, the 2'OH is derivatized to a 2-(N-methylcarbamoyl)ethyl moiety to increase nuclease resistance. A non-limiting example of an MCE oligomer comprising MCE subunits and phosphodiester internucleoside linkages is depicted below:

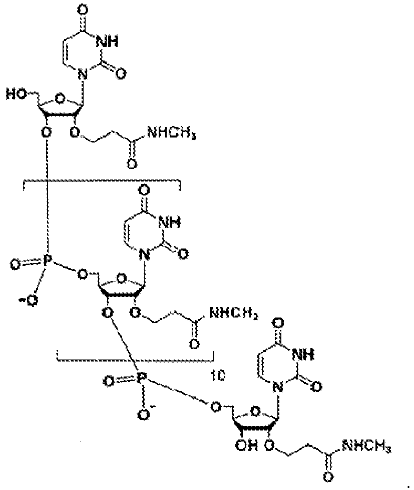

MCEs and their synthesis are described in Yamada et al., J. Org. Chem., 76(9):3042-53, which is hereby incorporated by reference in its entirety. Compounds of the disclosure may incorporate one or more MCE subunits.

### 7. Morpholino-based Oligomers

Morpholino-based oligomers refer to an oligomer comprising morpholino subunits supporting a nucleobase and, instead of a ribose, contains a morpholinyl ring. Exemplary internucleoside linkages include, for example, phosphoramidate or phosphorodiamidate internucleoside linkages joining the morpholinyl ring nitrogen of one morpholino subunit to the 4' exocyclic carbon of an adjacent morpholino subunit. Each morpholino subunit comprises a purine or pyrimidine nucleobase effective to bind, by base-specific hydrogen bonding, to a base in an oligonucleotide.

Morpholino-based oligomers (including modified antisense oligomers) are detailed, for example, in U.S. Patent Nos. 5,698,685; 5,217,866; 5,142,047; 5,034,506; 5,166,315; 5,185,444; 5,521,063; 5,506,337 and pending US Patent Application Nos. 12/271,036; 12/271,040; and PCT Publication No. WO/2009/064471 and WO/2012/043730 and Summerton et al. 1997, Antisense and Nucleic Acid Drug Development, 7, 187-195, which are hereby incorporated by reference in their entirety. The term "morpholino subunit," is used herein as described in Summerton *et al.*

Within the oligomer structure, the phosphate groups are commonly referred to as forming the "internucleoside linkages" of the oligomer. The naturally occurring internucleoside linkage of RNA and DNA is a 3' to 5' phosphodiester linkage. A "phosphoramidate" group comprises phosphorus having three attached oxygen atoms and one attached nitrogen atom, while a "phosphorodiamidate" group comprises phosphorus having two attached oxygen atoms and two attached nitrogen atoms. A "phosphorotriamidate" group (or a phosphoric acid triamide group) comprises phosphorus having one attached oxygen atom and three attached nitrogen atoms. In the uncharged or the cationic internucleoside linkages of the morpholino-based oligomers described herein, one nitrogen is always pendant to the linkage chain. The second nitrogen, in a phosphorodiamidate linkage, is typically the ring nitrogen in a morpholino ring structure.

"PMO" refers to phosphorodiamidate morpholino-based oligomers having a phosphorus atom with (i) a covalent bond to the nitrogen atom of a morpholino ring and (ii) a second covalent bond to the nitrogen of a dimethylamino. "PMO-X" refers to phosphorodiamidate morpholino-based oligomers having a phosphorus atom with (i) a covalent bond to the nitrogen atom of a morpholino ring and (ii) a second covalent bond to the ring nitrogen of, for example, a 4-aminopiperdin-1-yl (i.e., APN) or a derivative of 4-aminopiperdin-1-yl. Exemplary PMO-X oligomers are disclosed in PCT Application No. PCT/US2011/38459 and PCT Publication No. WO 2013/074834, which are hereby incorporated by reference in their entirety. PMO-X includes "PMO-apn," "PMO-APN" or "APN," which refers to a PMO-X oligomer which comprises at least one internucleoside linkage where a phosphorus atom is linked to a morpholino group and to the ring nitrogen of a 4-aminopiperdin-1-yl (i.e., APN). In specific embodiments, a modified antisense oligomer comprising a targeting sequence as set forth in Tables 3 and 4 comprises at least one APN-containing linkage or APN derivative-containing linkage. Various embodiments include morpholino-based oligomers that have about 10%, 15%, 20%, 25%, 30%, 35%, 40%, 45%, 50%, 55%, 60%, 65%, 70%, 75%, 80%, 85%, 90%, 95%, or 100% APN/APN derivative-containing linkages, where the remaining linkages (if less than 100%) are uncharged linkages, e.g., about or at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 of the total internucleoside linkages are APN/APN derivative-containing linkages.

In various embodiments, the modified antisense oligomer is a compound of formula (I): 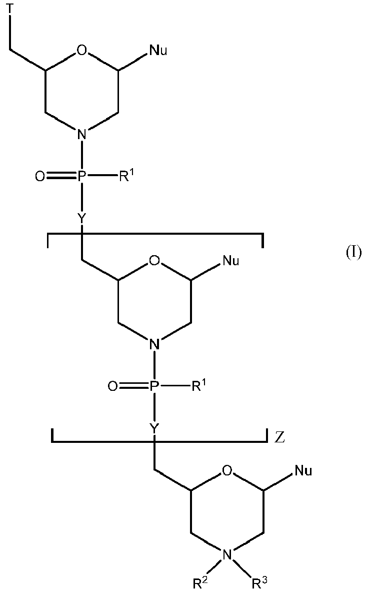 or a pharmaceutically acceptable salt thereof, wherein:
each Nu is a nucleobase which taken together forms a targeting sequence;
Z is an integer from 8 to 48;
each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, wherein R⁵ is selected from H and C₁-C₆ alkyl and n is an integer from 1 to 5;
T is selected from OH and a moiety of the formula: 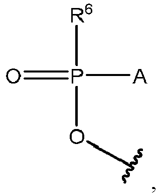 wherein:
   A is selected from -OH, -N(R⁷)₂R⁸, wherein:
      each R⁷ is independently selected from H and C₁-C₆ alkyl, and
      R⁸ is selected from an electron pair and H, and
      R⁶ is selected from OH, -N(R⁹)CH₂C(O)NH₂, and a moiety of the formula: 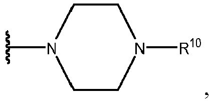 wherein:
      R⁹ is selected from H and C₁-C₆ alkyl; and
      R¹⁰ is selected from G, -C(O)-R¹¹OH, acyl, trityl, 4-methoxytrityl, -C(=NH)NH₂, -C(O)(CH₂)ₘNR¹²C(=NH)NH₂, and -C(O)(CH₂)₂NHC(O)(CH₂)₅NR¹²C(=NH)NH₂, wherein:
   m is an integer from 1 to 5,
   R¹¹ is of the formula -(O-alkyl)_{y}- wherein y is an integer from 3 to 10 and each of the y alkyl groups is independently selected from C₂-C₆ alkyl; and
   R¹² is selected from H and C₁-C₆ alkyl; each instance of R¹ is independently selected from :
   -N(R¹³)₂R¹⁴ wherein each R¹³ is independently selected from H and C₁-C₆ alkyl, and R¹⁴ is selected from an electron pair and H; a moiety of formula (II): 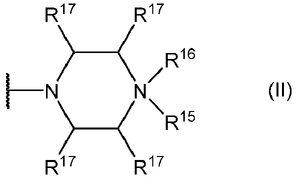 wherein:
   R¹⁵ is selected from H, G, C₁-C₆ alkyl, -C(=NH)NH₂, -C(O)(CH₂)_{q}NR¹⁸C(=NH)NH₂, and -C(O)(CH₂)₂NHC(O)(CH₂)₅NR¹⁸C(=NH)NH₂, wherein:
   R¹⁸ is selected from H and C₁-C₆ alkyl; and
   q is an integer from 1 to 5,
R¹⁶ is selected from an electron pair and H; and
each R¹⁷ is independently selected from H and methyl; and a moiety of formula(III): 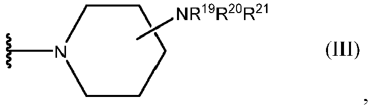 wherein:
   R¹⁹ is selected from H, C₁-C₆ alkyl, -C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂, and G wherein:
      R²² is selected from H and C₁-C₆ alkyl; and
      r is an integer from 1 to 5,
   R²⁰ is selected from H and C₁-C₆ alkyl; and
   R²¹ is selected from an electron pair and H; and
   R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂, -C(O)(CH₂)₂NHC(O)(CH₂)₅NR²⁴C(=NH)NH₂, -C(O)CH(NH₂)(CH₂)₃NHC(=NH)N H₂, and a moiety of the formula: 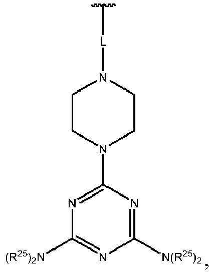 wherein,
   R²³ is of the formula -(O-alkyl)ᵥ-OH, wherein v is an integer from 3 to 10 and each of the v alkyl groups is independently selected from C₂-C₆ alkyl; and
   R²⁴ is selected from H and C₁-C₆ alkyl;
   s is an integer from 1 to 5;
   L is selected from -C(O)(CH₂)₆C(O)- and -C(O)(CH₂)₂S₂(CH₂)₂C(O)-; and each R²⁵ is of the formula -(CH₂)₂OC(O)N(R²⁶)₂ wherein each R²⁶ is of the formula -(CH₂)₆NHC(=NH)NH₂, and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH -CPP,
   and -C(O)CH₂NH-CPP, or G is of the formula: 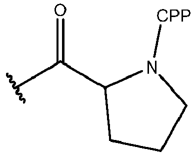 wherein the CPP is attached to the linker moiety by an amide bond at the CPP carboxy terminus, with the proviso that up to one instance of G is present.

In various embodiments, the targeting sequence is complementary to a target region within myostatin pre-mRNA. In some embodiments, the targeting sequence is complementary to 12 or more contiguous nucleotides in a target region entirely within exon 2 where no part of the targeting sequence spans a splice junction, or a region spanning an intron/exon or exon/intron splice junction (e.g., SEQ ID NOS: 2 to 3) of myostatin pre-mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction of dystrophin pre-mRNA.

In various embodiments, the myostatin targeting sequence comprises one of SEQ ID NOS: 16 to 75, is selected from one of SEQ ID NOS: 16 to 75, is a fragment of at least 12 contiguous nucleotides of a sequence selected from at least one of SEQ ID NOS: 16 to 75, or is a variant having at least 90% sequence identity to a sequence selected from at least one of SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence of formula (I) is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In various embodiments, at least one X of the targeting sequence is T. In various embodiments, each X of the targeting sequence is T.

In various embodiments, at least one X of the targeting sequence is U. In various embodiments, each X of the targeting sequence is U.

In various embodiments, at least one Y of the targeting sequence is 5mC. In various embodiments, each Y of the targeting sequence is 5mC.

In various embodiments, at least one Y of the targeting sequence is C. In various embodiments, each Y of the targeting sequence is C.

In various embodiments, at least one X of SEQ ID NOS: 16 to 75 and SEQ ID NO: 76 to 3485 is T. In various embodiments, each X of SEQ ID NOS: 16 to 75 and SEQ ID NO: 76 to 3485 is T.

In various embodiments, at least one X of the targeting sequence is U. In various embodiments, each X of SEQ ID NOS: 16 to 75 and SEQ ID NO: 76 to 3485 is U.

In various embodiments, at least one Y of SEQ ID NOS: 16 to 75 and SEQ ID NO: 76 to 3485 is 5mC. In various embodiments, each Y of SEQ ID NOS: 16 to 75 and SEQ ID NO: 76 to 3485 is 5mC.

In various embodiments, at least one Y of SEQ ID NOS: 16 to 75 and SEQ ID NO: 76 to 3485 is C. In various embodiments, each Y of SEQ ID NOS: 16 to 75 and SEQ ID NO: 76 to 3485 is C.

In some embodiments, R³ is a moiety of the formula: 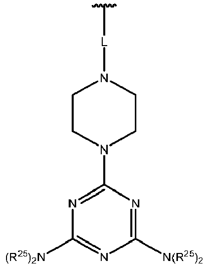 where L is selected from -C(O)(CH₂)₆C(O)- or -C(O)(CH₂)₂S₂(CH₂)₂C(O)- , and each R²⁵ is of the formula -(CH₂)₂OC(O)N(R²⁶)₂ wherein each R²⁶ is of the formula -(CH₂)₆NHC(=NH)NH₂. Such moieties are further described in U.S. Patent No. 7,935,816, which is hereby incorporated by reference in its entirety.

In certain embodiments, R³ may comprise either moiety depicted below:

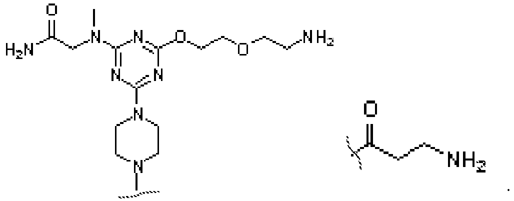

In various embodiments, each Y is O, R² is selected from H or G, R³ is selected from an electron pair or H. In some embodiments, R² is G wherein the CPP is of a sequence selected from SEQ ID NOS: 3486 to 3501. In certain embodiments, R² is H.

In certain embodiments, each R¹ is -N(CH₃)₂. In some embodiments, about 50-90% of the R¹ groups are dimethylamino (i.e. -N(CH₃)₂). In certain embodiments, about 70% to about 80% of the R¹ groups are diemethylamino. In certain embodiments about 75% of the R¹ groups are dimethylamino. In certain embodiments, about 66% of the R¹ groups are dimethylamino.

In some embodiments of the disclosure, R¹ may be selected from:

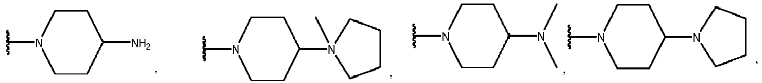

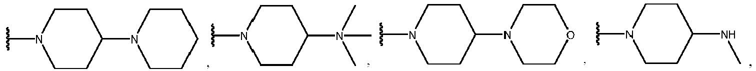

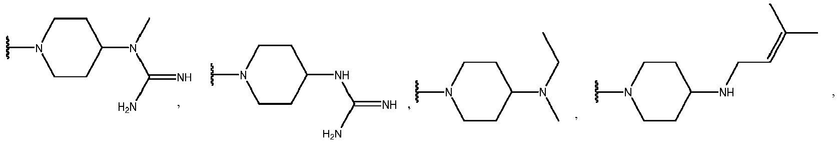

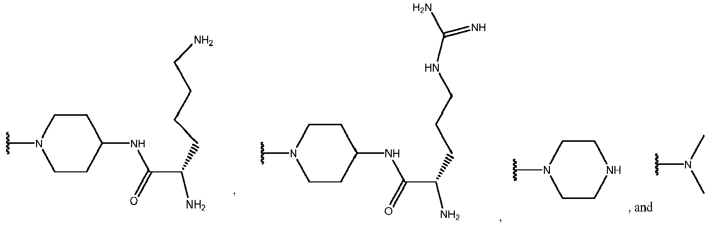

In certain embodiments, at least one R¹ is:

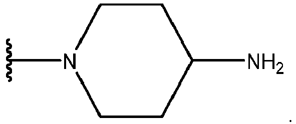

In certain embodiments, T is of the formula: 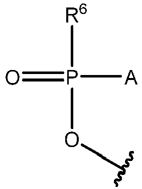 wherein A is -N(CH₃)₂, and R⁶ is of the formula: 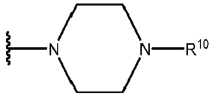 wherein R¹⁰ is -C(O)R¹¹OH.

In some embodiments, each Y is O, and T is selected from:

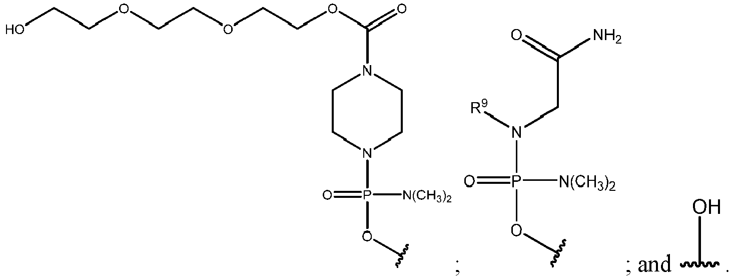

In certain embodiments, T is of the formula:

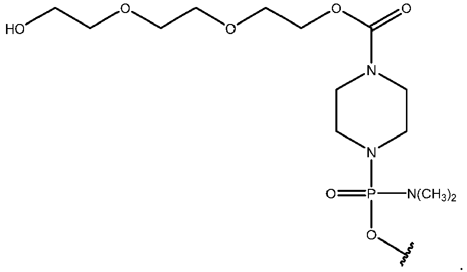

In various embodiments, each Y is O, and R² is selected from H or G, R³ is selected from an electron pair or H. In some embodiments, R² is G, wherein the CPP is of a sequence selected from SEQ ID NOS: 3486 to 3501 described below.

In other embodiments, the modified antisense oligomer is a compound of formula (IV): 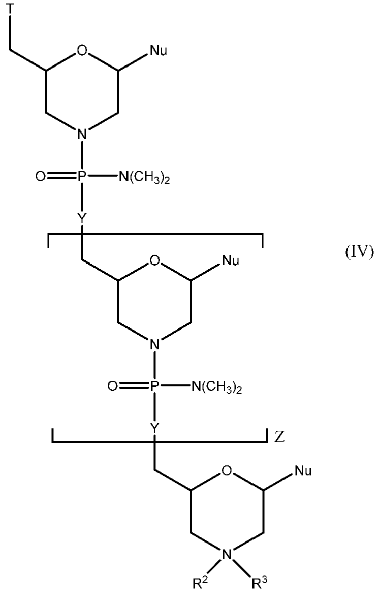 or a pharmaceutically acceptable salt thereof, where:
each Nu is a nucleobase which taken together forms a targeting sequence;
Z is an integer from 8 to 48;
each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, wherein R⁵ is selected from H and C₁-C₆ alkyl and n is an integer from 1 to 5;
T is selected from OH and a moiety of the formula: 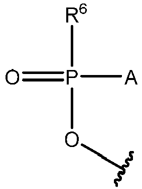 wherein:
   A is selected from -OH and -N(R⁷)₂R⁸, wherein:
      each R⁷ is independently selected from H and C₁-C₆ alkyl, and
      R⁸ is selected from an electron pair and H, and
   R⁶ is selected from OH, -N(R⁹)CH₂C(O)NH₂, and a moiety of the formula: 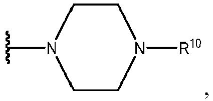 wherein:
      R⁹ is selected from H and C₁-C₆ alkyl; and
      R¹⁰ is selected from G, -C(O)-R¹¹OH, acyl, trityl, 4-methoxytrityl, -C(=NH)NH₂, -C(0)(CH₂)ₘNR¹²C(=NH)NH₂, and -C(O)(CH₂)₂NHC(O)(CH₂)₅NR¹²C(=NH)NH₂, wherein:
         m is an integer from 1 to 5,
         R¹¹ is of the formula -(O-alkyl)_{y}- wherein y is an integer from 3 to 10 and
            each of the y alkyl groups is independently selected from C₂-C₆ alkyl; and
R¹² is selected from H and C₁-C₆ alkyl;
R² is selected from H, G, acyl, trityl, 4-methoxytrityl, C₁-C₆ alkyl, -C(=NH)NH₂, and -C(O)-R²³; and
   R³ is selected from an electron pair, H, and C₁-C₆ alkyl.

In various embodiments, the targeting sequence is complementary to a target region within myostatin pre-mRNA. In some embodiments, the targeting sequence is complementary to 12 or more contiguous nucleotides in a target region entirely within exon 2 where no part of the targeting sequence spans a splice junction, or a region spanning an intron/exon or exon/intron splice junction (e.g., SEQ ID NOS: 1 to 3) of myostatin pre-mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction (e.g., SEQ ID NOS: 76 to 3485) of dystrophin pre-mRNA.

In various embodiments, the myostatin targeting sequence comprises one of SEQ ID NOS: 4 to 15, is selected from one of SEQ ID NOS: 4 to 15, is a fragment of at least 12 contiguous nucleotides of a sequence selected from at least one of SEQ ID NOS: 4 to 15, or is a variant having at least 90% sequence identity to a sequence selected from at least one of SEQ ID NOS: 4 to 15, wherein each X is independently selected from uracil (U) or thymine (T), and wherein each Y is independently selected from cytosine (C) or 5-Methylcytosine (5mC). In some embodiments, each X of SEQ ID NOS: 4 to 15 is thymine (T), and each Y of SEQ ID NOS: 4 to 15 is cytosine (C).

In some embodiments, the myostaton targeting sequence of formula (IV) is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In various embodiments, Y is O, R² is selected from H or G, R³ is selected from an electron pair or H. In some embodiments, R² is G, wherein the CPP is of a sequence selected from SEQ ID NOS: 9-24. In certain embodiments, R² is H.

In some embodiments, Y is O, and T is selected from: 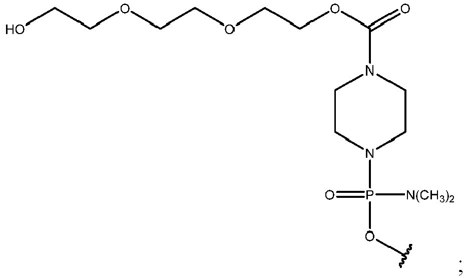 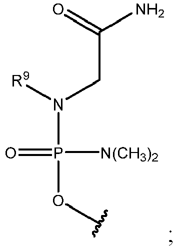 and

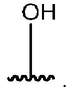

In some embodiments, T is of the formula: 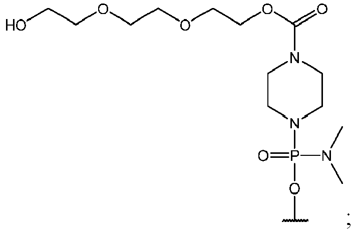 R² is hydrogen; and R³ is an electron pair.

In other embodiments, the modified antisense oligomer is a compound of formula (V): 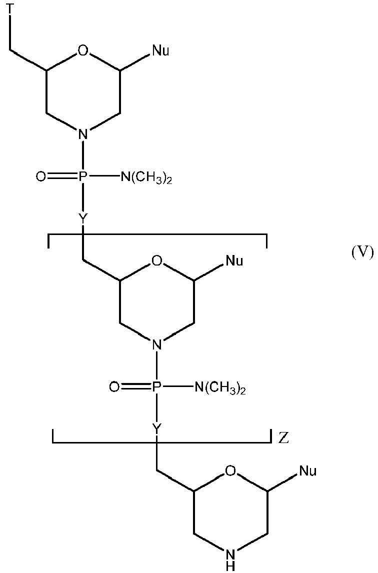 or a pharmaceutically acceptable salt thereof, wherein:
each Nu is a nucleobase which taken together forms a targeting sequence;
Z is an integer from 8 to 48;
each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, wherein R⁵ is selected from H and C₁-C₆ alkyl and n is an integer from 1 to 5;
T is selected from OH and a moiety of the formula: 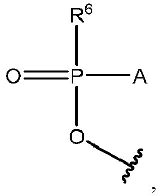 wherein:
   A is selected from -OH, -N(R⁷)₂R⁸, wherein:
      each R⁷ is independently selected from H and C₁-C₆ alkyl, and
      R⁸ is selected from an electron pair and H, and
      R⁶ is selected from OH, -N(R⁹)CH₂C(O)NH₂, and a moiety of the formula: 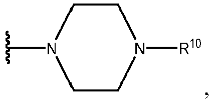 wherein:

   R⁹ is selected from H and C₁-C₆ alkyl; and
   R¹⁰ is selected from G, -C(O)-R¹¹OH, acyl, trityl, 4-methoxytrityl, -C(=NH)NH₂, -C(O)(CH₂)ₘNR¹²C(=NH)NH₂, and -C(O)(CH₂)₂NHC(O)(CH₂)₅NR¹²C(=NH)NH₂, wherein:
   m is an integer from 1 to 5,
   R¹¹ is of the formula -(O-alkyl)_{y}- wherein y is an integer from 3 to 10 and each of the y alkyl groups is independently selected from C₂-C₆ alkyl; and
   R¹² is selected from H and C₁-C₆ alkyl;
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH -CPP,
and -C(O)CH₂NH-CPP, or G is of the formula: 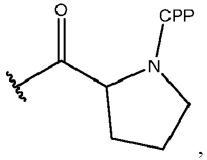
wherein the CPP is attached to the linker moiety by an amide bond at the CPP carboxy terminus, with the proviso that up to one instance of G is present.

In various embodiments, the targeting sequence is complementary to a target region within myostatin pre-mRNA. In some embodiments, the targeting sequence is complementary to 12 or more contiguous nucleotides in a target region entirely within exon 2 where no part of the targeting sequence spans a splice junction, or a region spanning an intron/exon or exon/intron splice junction (e.g., SEQ ID NOS: 1 to 3) of myostatin pre-mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction (e.g., SEQ ID NOS: 4 to 15) of dystrophin pre-mRNA.

In various embodiments, the myostatin targeting sequence comprises one of SEQ ID NOS: 16 to 75, is selected from one of SEQ ID NOS: 16 to 75, is a fragment of at least 12 contiguous nucleotides of a sequence selected from at least one of SEQ ID NOS: 16 to 75, or is a variant having at least 90% sequence identity to a sequence selected from at least one of SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence of formula (V) is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In various embodiments, each Y is O, and T is selected from: 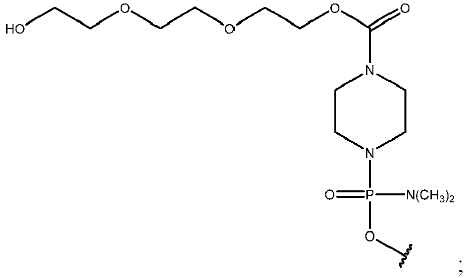 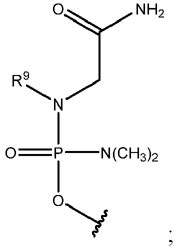 and

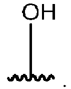

In some embodiments, T is of the formula:

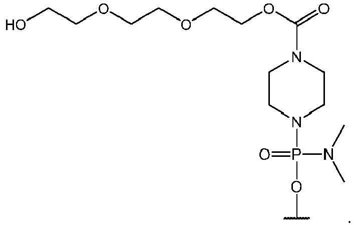

In certain embodiments, the antisense oligomer of the disclosure is a compound of formula (VI): 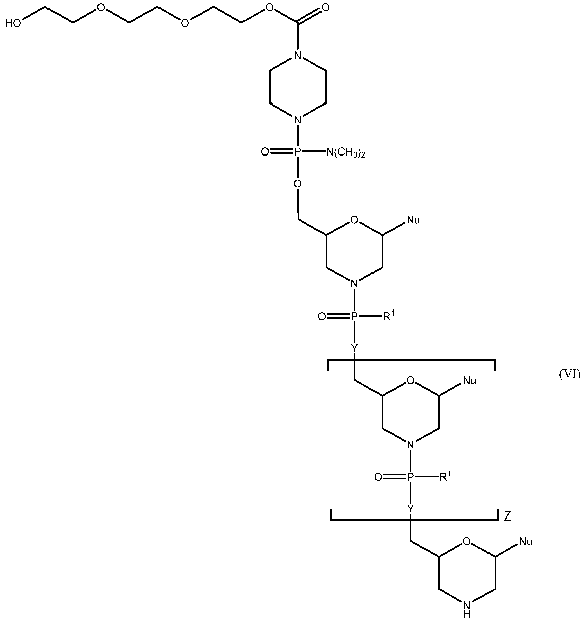 or a pharmaceutically acceptable salt thereof, where:
each Nu is a nucleobase which taken together form a targeting sequence;
Z is an integer from 15 to 25;
each Y is O;
each R¹ is independently selected from :

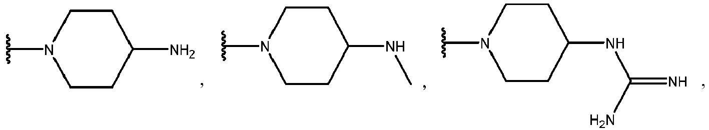

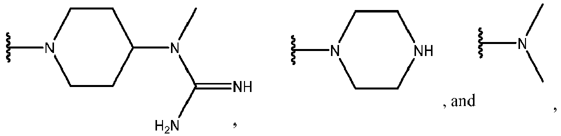

In various embodiments, at least one R¹ is -N(CH₃)₂. In some embodiments, each R¹ is -N(CH₃)₂.

In various embodiments, the targeting sequence is complementary to a target region within myostatin pre-mRNA. In some embodiments, the targeting sequence is complementary to 12 or more contiguous nucleotides in a target region entirely within exon 2 where no part of the targeting sequence spans a splice junction, or a region spanning an intron/exon or exon/intron splice junction (e.g., SEQ ID NOS: 1 to 3) of myostatin pre-mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction (e.g., SEQ ID NOS: 4 to 15) of dystrophin pre-mRNA.

In various embodiments, the myostatin targeting sequence comprises one of SEQ ID NOS: 16 to 75, is selected from one of SEQ ID NOS: 16 to 75, is a fragment of at least 12 contiguous nucleotides of a sequence selected from at least one of SEQ ID NOS: 16 to 75, or is a variant having at least 90% sequence identity to a sequence selected from at least one of SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence of formula (VI) is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In some embodiments, the antisense oligomer is a compound of formula (VII): 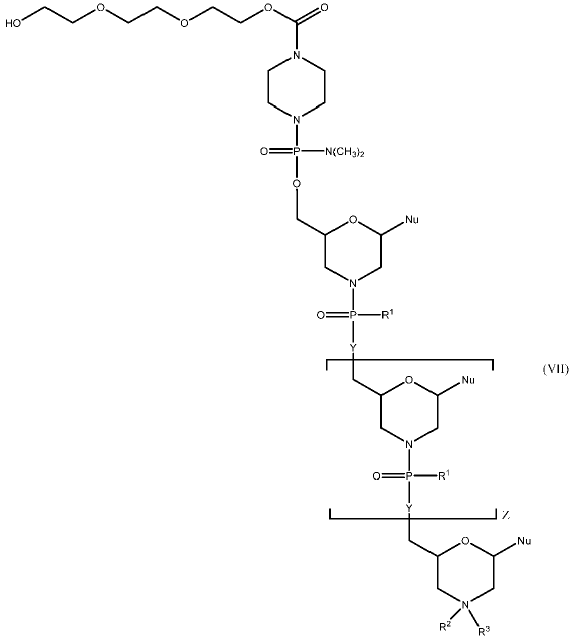 or a pharmaceutically acceptable salt thereof, where:
each Nu is a nucleobase which taken together form a targeting sequence; and
Z is an integer from 8 to 48;
each Y is O;
each R¹ is independently selected from: 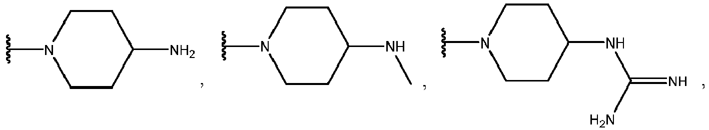 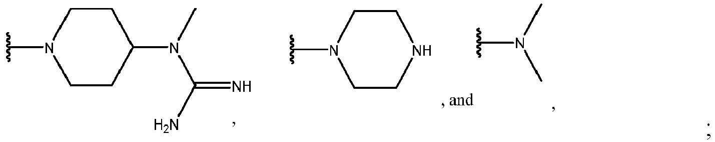
R² is selected from H, acyl, trityl, 4-methoxytrityl, C₁-C₆ alkyl, -C(=NH)NH₂, and -C(O)-R²³; and
R³ is selected from an electron pair, H, and C₁-C₆ alkyl.

In various embodiments, the targeting sequence is complementary to a target region within myostatin pre-mRNA. In some embodiments, the targeting sequence is complementary to 12 or more contiguous nucleotides in a target region entirely within an exon of myostatin pre-mRNA or a region spanning an intron/exon or exon/intron splice junction (e.g., SEQ ID NOS: 1 to 3) of myostatin pre mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction (e.g., SEQ ID NOS: 4 to 15) of dystrophin pre-mRNA.

In various embodiments, the myostatin targeting sequence comprises one of SEQ ID NOS: 16 to 75, is selected from one of SEQ ID NOS: 16 to 75, is a fragment of at least 12 contiguous nucleotides of a sequence selected from at least one of SEQ ID NOS: 16 to 75, or is a variant having at least 90% sequence identity to a sequence selected from at least one of SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence of formula (VII) is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In various embodiments, at least one X of the targeting sequence is T. In various embodiments, each X of the targeting sequence is T.

In various embodiments, at least one X of the targeting sequence is U. In various embodiments, each X of the targeting sequence is U.

In various embodiments, at least one Y of the targeting sequence is 5mC. In various embodiments, each Y of the targeting sequence is 5mC.

In various embodiments, at least one Y of the targeting sequence is C. In various embodiments, each Y of the targeting sequence is C.

In various embodiments, at least one X of SEQ ID NOS: 16 to 75 and SEQ ID NO: 76 to 3485 is T. In various embodiments, each X of SEQ ID NOS: 16 to 75 and SEQ ID NO: 76 to 3485 is T.

In various embodiments, at least one X of the targeting sequence is U. In various embodiments, each X of SEQ ID NOS: 16 to 75 and SEQ ID NO: 76 to 3485 is U.

In various embodiments, at least one Y of SEQ ID NOS: 16 to 75 and SEQ ID NO: 76 to 3485 is 5mC. In various embodiments, each Y of SEQ ID NOS: 16 to 75 and SEQ ID NO: 76 to 3485 is 5mC.

In various embodiments, at least one Y of SEQ ID NOS: 16 to 75 and SEQ ID NO: 76 to 3485 is C. In various embodiments, each Y of SEQ ID NOS: 16 to 75 and SEQ ID NO: 76 to 3485 is C.

In certain embodiments, the antisense oligomer is a compound of formula (VIII): 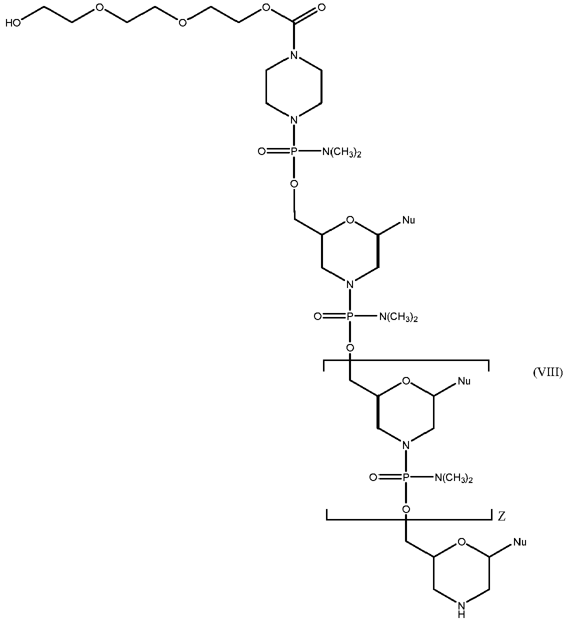 or a pharmaceutically acceptable salt thereof, where:
each Nu is a nucleobase which taken together form a targeting sequence; and
Z is an integer from 8 to 48.

In various embodiments, the targeting sequence is complementary to a target region within myostatin pre-mRNA. In some embodiments, the targeting sequence is complementary to 12 or more contiguous nucleotides in a target region within an exon/intron splice junction site, or a region spanning an intron/exon or exon/intron splice junction (e.g., SEQ ID NOS: 1 to 3) of myostatin pre mRNA.

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction,or a region spanning an intron/exon or exon/intron splice junction (e.g., SEQ ID NOS: 4 to 15) of dystrophin pre-mRNA.

In various embodiments, the myostatin targeting sequence comprises one of SEQ ID NOS: 16 to 75, is selected from one of SEQ ID NOS: 16 to 75, is a fragment of at least 12 contiguous nucleotides of a sequence selected from at least one of SEQ ID NOS: 16 to 75, or is a variant having at least 90% sequence identity to a sequence selected from at least one of SEQ ID NOS: 16 to 75, wherein each X is independently selected from uracil (U) or thymine (T), and wherein each Y is independently selected from cytosine (C) or 5-Methylcytosine (5mC). In some embodiments, each X of SEQ ID NOS: 16 to 75 is thymine (T), and each Y of SEQ ID NOS: 16 to 75 is cytosine (C).

In some embodiments, the myostatin targeting sequence is selected from:
a) SEQ ID NO: 71 (YYAGYYYAXYXXYXYYXGGXYYXGG) wherein Z is 25;
b) SEQ ID NO: 72 (YAYXXAYYAGYYYAXYXXYXYYXGG) wherein Z is 25;
c) SEQ ID NO: 73 (YYAYXXGYAXXAGAAAAXYAGY) wherein Z is 22;
d) SEQ ID NO: 74 (GYATTAGAAAATYAGYTATAAATG) wherein Z is 24; and
e) SEQ ID NO: 75 (YYATYYGYTTGYATTAGAAAGTYAGY) wherein Z is 26;
wherein each X is independently selected from uracil (U) or thymine (T), and wherein each Y is independently selected from cytosine (C) or 5-Methylcytosine (5mC). In some embodiments, each X of SEQ ID NOS: 71 to 75 is thymine (T), and each Y of SEQ ID NOS: 71 to 75 is cytosine (C).

In various embodiments, the dystrophin targeting sequence comprises one of SEQ ID NOS: 76 to 3485, is selected from one of SEQ ID NOS: 76 to 3485, is a fragment of at least 10 contiguous nucleotides of a sequence selected from at least one of SEQ ID NOS: 76 to 3485, or is a variant having at least 90% sequence identity to a sequence selected from at least one of SEQ ID NOS: 76 to 3485, wherein each X is independently selected from uracil (U) or thymine (T), and wherein each Y is independently selected from cytosine (C) or 5-Methylcytosine (5mC). In some embodiments, each X of SEQ ID NOS: 76 to 3485 is thymine (T), and each Y of SEQ ID NOS: 76 to 3485 is cytosine (C). In some embodiments, a targeting sequence may comprise SEQ ID NO: 76.

In some embodiments, each Nu of the antisense oligomers of the disclosure, including compounds of formula (I), (IV), (V), (VI), (VII) and (VIII), is independently selected from adenine, guanine, thymine, uracil, cytosine, hypoxanthine (inosine), 2,6-diaminopurine, 5-methyl cytosine, C5-propynyl-modified pyrimidines, and 10-(9-(aminoethoxy)phenoxazinyl). In some embodiments, the targeting sequence of the antisense oligomers of the disclosure, including compounds of formula (I), (IV), (V), (VI), (VII) and (VIII), comprises a sequence selected from SEQ ID NOS: 2, 3, 4 or 6, is selected from SEQ ID NOS: 2, 3, 4 or 6, is a fragment of at least 12 contiguous nucleotides of a sequence selected from SEQ ID NOS: 2, 3, 4 or 6, or is a variant having at least 90% sequence identity to a sequence selected from SEQ ID NOS: 2, 3, 4 or 6, where X is selected from uracil (U) or thymine (T), and wherein I is inosine.

Additional modified antisense oligomers/chemistries that can be used in accordance with the present disclosure include those described in the following patents and patent publications, which are hereby incorporated by reference in their entirety: PCT Publication Nos. WO 2007/002390; WO 2010/120820; and WO 2010/148249; U.S. Patent No. 7,838,657; and U.S. Patent Application No. 2011/0269820.

### C. The Preparation of Morpholino Subunits and Phosphoramidate Internucleoside Linkers

Morpholino monomer subunits, the modified internucleoside linkages, and oligomers comprising the same can be prepared as described, for example, in U.S. Patent Nos. 5,185,444, and 7,943,762, which are hereby incorporated by reference in their entirety. The morpholino subunits can be prepared according to the following general Reaction Scheme I.

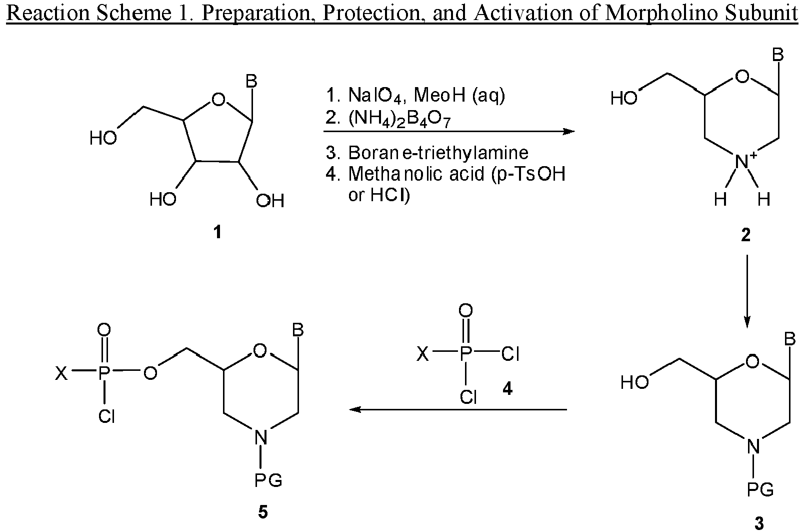

Referring to Reaction Scheme 1, where B represents a base pairing moiety and PG represents a protecting group, the morpholino subunits may be prepared from the corresponding ribonucleoside (1) as shown. The morpholino subunit (2) may be optionally protected by reaction with a suitable protecting group precursor, for example trityl chloride. The 3' protecting group is generally removed during solid-state oligomer synthesis as described in more detail below. The base pairing moiety may be suitably protected for sold phase oligomer synthesis. Suitable protecting groups include benzoyl for adenine and cytosine, phenylacetyl for guanine, and pivaloyloxymethyl for hypoxanthine (I). The pivaloyloxymethyl group can be introduced onto the N¹ position of the hypoxanthine heterocyclic base. Although an unprotected hypoxanthine subunit, may be employed, yields in activation reactions are far superior when the base is protected. Other suitable protecting groups include those disclosed in U.S. Patent No. 8,076,476, which is hereby incorporated by reference in its entirety.

Reaction of compound 3 with the activated phosphorous compound 4, results in morpholino subunits having the desired linkage moiety compound 5. Compounds of structure 4 can be prepared using any number of methods known to those of skill in the art. For example, such compounds may be prepared by reaction of the corresponding amine and phosphorous oxychloride. In this regard, the amine starting material can be prepared using any method known in the art, for example those methods described in the Examples and in U.S. Patent Nos. 5,185,444, 7,943,762, and 8,779,128, which are hereby incorporated by reference in its entirety.

Compounds of structure 5 can be used in solid-phase automated oligomer synthesis for preparation of oligomers comprising the internucleoside linkages. Such methods are well known in the art. Briefly, a compound of structure 5 may be modified at the 5' end to contain a linker to a solid support. For example, compound 5 may be linked to a solid support by a linker comprising L11 and L15. Once supported, the protecting group (e.g., trityl) is removed and the free amine is reacted with an activated phosphorous moiety of a second compound of structure 5. This sequence is repeated until the desired length of oligo is obtained. The protecting group in the terminal 5' end may either be removed or left on if a 5'-modification is desired. The oligo can be removed from the solid support using any number of methods, for example treatment with DTT followed by ammonium hydroxide.

The preparation of modified morpholino subunits and morpholino-based oligomers are described in more detail in the Examples. The morpholino-based oligomers containing any number of modified linkages may be prepared using methods described herein, methods known in the art and/or described by reference herein. Also described in the examples are global modifications of morpholino-based oligomers prepared as previously described (see e.g., PCT Publication No. WO 2008/036127, which is hereby incorporated by reference in its entirety).

The term "protecting group" refers to chemical moieties that block some or all reactive moieties of a compound and prevent such moieties from participating in chemical reactions until the protective group is removed, for example, those moieties listed and described in T.W. Greene, P.G.M. Wuts, Protective Groups in Organic Synthesis, 3rd ed. John Wiley & Sons (1999), which is hereby incorporated by reference in its entirety. It may be advantageous, where different protecting groups are employed, that each (different) protective group be removable by a different means. Protective groups that are cleaved under totally disparate reaction conditions allow differential removal of such protecting groups. For example, protective groups can be removed by acid, base, and hydrogenolysis. Groups such as trityl, dimethoxytrityl, acetal and tert-butyldimethylsilyl are acid labile and may be used to protect carboxy and hydroxy reactive moieties in the presence of amino groups protected with Cbz groups, which are removable by hydrogenolysis, and Fmoc groups, which are base labile. Carboxylic acid moieties may be blocked with base labile groups such as, without limitation, methyl, or ethyl, and hydroxy reactive moieties may be blocked with base labile groups such as acetyl in the presence of amines blocked with acid labile groups such as tert-butyl carba¬mate or with carbamates that are both acid and base stable but hydrolytically removable.

Carboxylic acid and hydroxyl reactive moieties may also be blocked with hydrolytically removable protective groups such as the benzyl group, while amine groups may be blocked with base labile groups such as Fmoc. A particularly useful amine protecting group for the synthesis of compounds of Formula (I) is the trifluoroacetamide. Carboxylic acid reactive moieties may be blocked with oxidatively-removable protective groups such as 2,4-dimethoxybenzyl, while co-existing amino groups may be blocked with fluoride labile silyl carbamates.

Allyl blocking groups are useful in the presence of acid- and base- protecting groups since the former are stable and can be subsequently removed by metal or pi-acid catalysts. For example, an allyl-blocked carboxylic acid can be deprotected with a palladium(0)-catalyzed reaction in the presence of acid labile t-butyl carbamate or base-labile acetate amine protecting groups. Yet another form of protecting group is a resin to which a compound or intermediate may be attached. As long as the residue is attached to the resin, that functional group is blocked and cannot react. Once released from the resin, the functional group is available to react.

Typical blocking/protecting groups are known in the art and include, but are not limited to the following moieties:

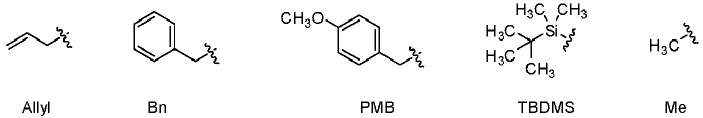

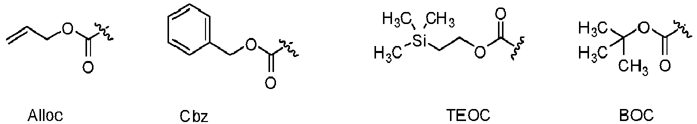

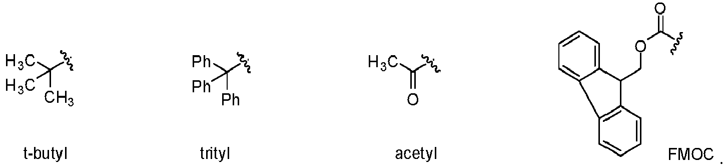

Unless otherwise noted, all chemicals were obtained from Sigma-Aldrich-Fluka (St. Louis, MO). Benzoyl adenosine, benzoyl cytidine, and phenylacetyl guanosine were obtained from Carbosynth Limited (Berkshire, UK).

Synthesis of PMO, PMOplus, PPMO, and PMO-X containing further linkage modifications as described herein was done using methods known in the art and described in pending U.S. Patent Application Nos. 12/271,036 and 12/271,040 and PCT Publication No. WO 2009/064471, which is hereby incorporated by reference in its entirety.

PMO with a 3' trityl modification are synthesized essentially as described in PCT Publication No. WO 2009/064471 with the exception that the detritylation step is omitted.

### D. Cell-Penetrating Peptides

The modified antisense oligomer compounds of the disclosure may be conjugated to a peptide, also referred to herein as a cell penetrating peptide (CPP). In certain preferred embodiments, the peptide is an arginine-rich peptide transport moiety effective to enhance transport of the compound into cells. The transport moiety is preferably attached to a terminus of the oligomer. The peptides have the capability of inducing cell penetration within 30%, 40%, 50%, 60%, 70%, 80%, 90% or 100% of cells of a given cell culture population, including all integers in between, and allow macromolecular translocation within multiple tissues in vivo upon systemic administration. In one embodiment, the cell-penetrating peptide may be an arginine-rich peptide transporter. In another embodiment, the cell-penetrating peptide may be Penetratin or the Tat peptide. These peptides are well known in the art and are disclosed, for example, in US Publication No. 2010-0016215 A1, which is hereby incorporated by reference in its entirety. One approach to conjugation of peptides to modified antisense oligomers of the disclosure can be found in PCT publication WO2012/150960, which is hereby incorporated by reference in its entirety. Some embodiments of a peptide conjugated oligomer of the present disclosure utilize glycine as the linker between the CPP and the modified antisense oligomer. For example, a peptide conjugated PMO of the disclosure consists of R₆-G-PMO.

The transport moieties as described above have been shown to greatly enhance cell entry of attached oligomers, relative to uptake of the oligomer in the absence of the attached transport moiety. Uptake is preferably enhanced at least ten fold, and more preferably twenty fold, relative to the unconjugated compound.

The use of arginine-rich peptide transporters (i.e., cell-penetrating peptides) are particularly useful in practicing the present disclosure. Certain peptide transporters have been shown to be highly effective at delivery of antisense compounds into primary cells including muscle cells (Marshall, Oda *et al.* 2007; Jearawiriyapaisarn, Moulton *et al.* 2008; Wu, Moulton *et al.* 2008, which are hereby incroporated by reference in their entirety). Furthermore, compared to other known peptide transporters such as Penetratin and the Tat peptide, the peptide transporters described herein, when conjugated to an antisense PMO, demonstrate an enhanced ability to alter splicing of several gene transcripts (Marshall, Oda *et al.* 2007, which is hereby incorporated by reference in its entirety).

Exemplary peptide transporters, excluding linkers are given below in Table 5.

**Table 5. Exemplary peptide transporters**

| **NAME (DESIGNATION)** | **SEQUENCE** | **CPP SEQ ID NO^{A}** |
|---|---|---|
| rTAT | rrrqrrkkr | 3486 |
| Tat | rkkrrqrrr | 3487 |
| R₉F₂ | rrrrrrrrrff | 3488 |
| R₅F₂R₄ | rrrrrffrrrr | 3489 |
| R₄ | rrrr | 3490 |
| R₅ | rrrrr | 3491 |
| R₆ | rrrrrr | 3492 |
| R₇ | rrrrrrr | 3493 |
| R₈ | rrrrrrrr | 3494 |
| R₉ | rrrrrrrrr | 3495 |
| (RX)₈ | rahxrahxrahxrahxrahxrahxrahxrahx | 3496 |
| (RAhxR)₄; (P007) | rahxrrahxrrahxrrahxr | 3497 |
| (RAhxR)₅; (CP04057) | rahxrrahxrrahxrrahxrrahxr | 3498 |
| (RAhxRRBR)₂; (CP06062) | rahxrrbrrahxrrbr | 3499 |
| (RAR)₄F₂ | rarrarrarrarff | 3500 |
| (RGR)₄F₂ | rgrrgrrgrrgrff | 3501 |

| | | |
|---|---|---|
| ^{A}Sequences assigned to CPP SEQ ID NOS do not include the linkage portion (*e.g.,* C (cys), G (gly), P (pro), Ahx, B, AhxB where Ahx and B refer to 6-aminohexanoic acid and beta-alanine, respectively). | | |

In various embodiments, G (as recited in formulas I, IV, and V) is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 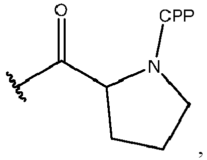 wherein the CPP is attached to the linker moiety by an amide bond at the CPP carboxy terminus. In some embodiments, the CPP is selected from SEQ ID NOS: 3486 to 3501.

In some embodiments, G (as recited in formulas I, IV, and V) is of the formula: 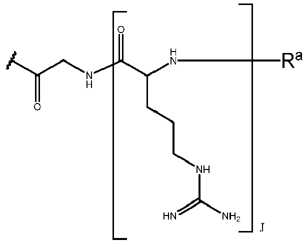 wherein R^{a} is selected from H, acetyl, benzoyl, and stearoyl, and J is an integer from 4 to 9. In certain embodiments J is 6.

In some embodiments, the CPP (as recited in formulas I, IV, and V) is of the formula: 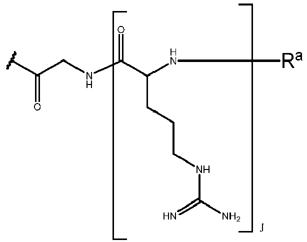 wherein R^{a} is selected from H, acetyl, benzoyl, and stearoyl, and J is an integer from 4 to 9. In certain embodiments, the CPP is SEQ ID NO: 15. In various embodiments, J is 6. In some embodiments Rₐ is selected from H and acetyl. For example, in some embodiments, Rₐ is H. In certain embodiments, Rₐ is acetyl.

### IV. Formulations

The compounds of the disclosure may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative United States patents that teach the preparation of such uptake, distribution and/or absorption-assisting formulations include, but are not limited to, U.S. Patent Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756, which are hereby incorporated by reference in their entirety.

The antisense compounds of the disclosure encompass any pharmaceutically acceptable salts, esters, or salts of such esters, or any other compound which, upon administration to an animal, including a human, is capable of providing (directly or indirectly) the biologically active metabolite or residue thereof. Accordingly, for example, the disclosure is also drawn to prodrugs and pharmaceutically acceptable salts of the compounds of the disclosure, pharmaceutically acceptable salts of such prodrugs, and other bioequivalents.

The term "prodrug" indicates a therapeutic agent that is prepared in an inactive form that is converted to an active form (i.e., drug) within the body or cells thereof by the action of endogenous enzymes or other chemicals and/or conditions. In particular, prodrug versions of the oligomers of the disclosure are prepared as SATE [(S-acetyl-2-thioethyl) phosphate] derivatives according to the methods disclosed in PCT Publication No. WO 1993/24510 to Gosselin et al., published Dec. 9, 1993 or in PCT Publication No. WO 1994/26764 and U.S. Patent No. 5,770,713 to Imbach et al.*,* which are hereby incorporated by reference in their entirety.

The term "pharmaceutically acceptable salts" refers to physiologically and pharmaceutically acceptable salts of the compounds of the disclosure: i.e., salts that retain the desired biological activity of the parent compound and do not impart undesired toxicological effects thereto. For oligomers, examples of pharmaceutically acceptable salts and their uses are further described in U.S. Patent No. 6,287,860, which is hereby incorporated by reference in its entirety.

The present disclosure also includes pharmaceutical compositions and formulations which include the antisense compounds of the disclosure. The pharmaceutical compositions of the present disclosure may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration. Oligomers with at least one 2'-O-methoxy ethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present disclosure may be formulated into any of many possible dosage forms such as, but not limited to, tablets, capsules, gel capsules, liquid syrups, soft gels, suppositories, and enemas. The compositions of the present disclosure may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

Pharmaceutical compositions of the present disclosure include, but are not limited to, solutions, emulsions, foams and liposome-containing formulations. The pharmaceutical compositions and formulations of the present disclosure may comprise one or more penetration enhancers, carriers, excipients or other active or inactive ingredients.

Emulsions are typically heterogeneous systems of one liquid dispersed in another in the form of droplets usually exceeding 0.1 µm in diameter. Emulsions may contain additional components in addition to the dispersed phases, and the active drug which may be present as a solution in either the aqueous phase, oily phase or itself as a separate phase. Microemulsions are included as an embodiment of the present disclosure. Emulsions and their uses are well known in the art and are further described in U.S. Patent No. 6,287,860, which is hereby incorporated by reference in its entirety.

Formulations of the present disclosure include liposomal formulations. As used in the present disclosure, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes which are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic oligomers, such as a polyethylene glycol (PEG) moiety. Liposomes and their uses are further described in U.S. Patent No. 6,287,860, which is hereby incorporated by reference in its entirety.

The pharmaceutical formulations and compositions of the present disclosure may also include surfactants. The use of surfactants in drug products, formulations and in emulsions is well known in the art. Surfactants and their uses are further described in U.S. Patent No. 6,287,860, which is hereby incorporated by reference in its entirety.

In some embodiments, the present disclosure employs various penetration enhancers to effect the efficient delivery of nucleic acids, particularly oligomers. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating non-surfactants. Penetration enhancers and their uses are further described in U.S. Patent No. 6,287,860, which is hereby incorporated by reference in its entirety.

One of skill in the art will recognize that formulations are routinely designed according to their intended use, i.e. route of administration.

Formulations for topical administration include those in which the oligomers of the disclosure are in admixture with a topical delivery agent such as lipids, liposomes, fatty acids, fatty acid esters, steroids, chelating agents and surfactants. Lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA).

For topical or other administration, therapeutics including oligomers of the disclosure may be encapsulated within liposomes or may form complexes thereto, in particular to cationic liposomes. Alternatively, therapeutics may be complexed to lipids, in particular to cationic lipids. Fatty acids and esters, pharmaceutically acceptable salts thereof, and their uses are further described in U.S. Patent No. 6,287,860, which is hereby incorporated by reference in its entirety. Topical formulations are described in detail in U.S. Patent Application No. 09/315,298 filed on May 20, 1999 and Mourich et al., 2009, J. Invest. Dermatol., 129(8):1945-53, which are hereby incorporated by reference in their entirety.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Oral formulations are those in which oligomers of the disclosure are administered in conjunction with one or more penetration enhancers surfactants and chelators. Surfactants include fatty acids and/or esters or salts thereof, bile acids and/or salts thereof. Bile acids/salts and fatty acids and their uses are further described in U.S. Patent No. 6,287,860, which is hereby incorporated by reference in its entirety. In some embodiments, the present disclosure provides combinations of penetration enhancers, for example, fatty acids/salts in combination with bile acids/salts. An exemplary combination is the sodium salt of lauric acid, capric acid and UDCA. Further penetration enhancers include polyoxyethylene-9-lauryl ether, polyoxyethylene-20-cetyl ether. Oligomers of the disclosure may be delivered orally, in granular form including sprayed dried particles, or complexed to form micro or nanoparticles. Oligomer complexing agents and their uses are further described in U.S. Patent No. 6,287,860, which is hereby incorporated by reference in its entirety. Oral formulations for oligomers and their preparation are described in detail in U.S. Patent Application Nos. 09/108,673 (filed Jul. 1, 1998), 09/315,298 (filed May 20, 1999) and 10/071,822 (filed Feb. 8, 2002), which are hereby incorporated by reference in their entirety.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

In another related embodiment, compositions of the disclosure may contain one or more antisense compounds, particularly oligomers, targeted to a first nucleic acid and one or more additional antisense compounds targeted to a second nucleic acid target. Alternatively, compositions of the disclosure may contain two or more antisense compounds targeted to different regions of the same nucleic acid target. Numerous examples of antisense compounds are known in the art. Two or more combined compounds may be used together or sequentially.

### V. Methods of Use

Certain aspects relate to methods of treating a subject having Duchenne muscular dystrophy or a related disorder comprising administering a dustrophin therapeutic to a subject also receiving a myostatin therapeutic.

In aspects, a therapeutic is administered to a subject having DMD or a related disorder. In embodiments, one or more therapeutic may be administered to the subject prior to treatment with a modified antisense oligomer as described herein. In embodiments, one or more therapeutic may be administered to the subject prior to, simultaneously or after administration of a modified antisense oligomer. In embodiments, a therapeutic is a protein or nucleic acid. In some embodiments, a protein is an antibody or a soluble receptor. In embodiments, a soluble receptor is ACVR2. In embodiments, a nucleic acid is an antisense oligomer or a siRNA. In some embodiments, an antisense oligomer is a modified antisense oligomer as described herein.

In embodiments, a therapeutic is a myostatin therapeutic capable of suppressing one or both of myostatin activity or myostatin expression in a subject. A myostatin therapeutic may be a therapeutic that targets myostatin pre-mRNA and interferes with transcription of the myostatin pre-mRNA to mature mRNA. In embodiments, a myostatin therapeutic is capable of inducing exon skipping during the processing of human myostatin pre-mRNA. In embodiments, a myostatin therapeutic induces skipping of exon 2 in myostatin pre-mRNA and inhibits the expression of exon 2 containing myostatin pre-mRNA. A myostatin therapeutic may be a therapeutic that targets myostatin protein and interferes with the myostatin protein binding with the myostatin receptor.

A myostatin therapeutic is selected from a protein and a nucleic acid. A protein may be an anti-myostatin antibody, for example anti-GDF8 (Abcam, Cambridge MA) or a soluble receptor. In embodiments, a soluble receptor is ACVR2. A nucleic acid is selected from an antisense oligomer and a siRNA. An antisense oligomer may be a modified myostatin antisense oligomer as described herein.

In embodiments, a therapeutic is a dystrophin therapeutic capable of increasing dystrophin in a subject. A dystrophin therapeutic may increase the expression of dystrophin or a truncated form of dystrophin that is functional or semi-functional. A truncated form of dystrophin includes, but is not limited to, micro-dystrophin and mini-dystrophin (disclosed in EP Patent no. 2125006, which is hereby incorporated by reference in its entirety). A dystrophin therapeutic may be a therapeutic that targets dystrophin pre-mRNA and modulates the transcription of the dystrophin pre-mRNA to mature mRNA. In embodiments, a dystrophin therapeutic is capable of inducing exon skipping during processing of human dystrophin pre-mRNA. In embodiments, a targeted dystrophin pre-mRNA has one or more genetic mutations. A dystrophin therapeutic induces exon skipping such that one or more exons containing one or more genetic mutations are removed from the dystrophin pre-mRNA during processing to mature mRNA. The resulting truncated mRNA is capable of translation into a functional or semi-functional dystrophin protein.

In some aspects, a modified dystrophin antisense oligomer comprises a nucleotide sequence of sufficient length and complementarity to specifically hybridize to a region within the pre-mRNA of the dystrophin gene, wherein binding of the modified antisense oligomer to the region induces exon skipping during processing of dystrophin pre-mRNA. In embodiments, exon skipping during processing of dystrophin pre-mRNA results in the removal of one or more exons having a genetic mutation from the pre-mRNA. In embodiments, the removal of one or more exons having a genetic mutation from the dystrophin pre-mRNA increases the level of non-mutated dystrophin pre-mRNA in a cell and/or tissue of the subject. The increase in the level of non-mutated dystrophin pre-mRNA in the subject may further translate to increased expression of functional or semi-functional dystrophin protein. Thus, the present disclosure relates to methods of increasing functional or semi-functional dystrophin protein by increasing the level of non-mutated dystrophin mRNA using the modified dystrophin antisense oligomers as described herein.

In some aspects, a modified myostatin antisense oligomer comprises a nucleotide sequence of sufficient length and complementarity to specifically hybridize to a region within the pre-mRNA of the myostatin gene, wherein binding of the modified antisense oligomer to the region induces exon skipping during processing of myostatin pre-mRNA. In embodiments, binding of the modified myostatin oligomer to the region decreases the level of exon 2-containing myostatin mRNA in a cell and/or tissue of the subject. The decrease in the level of exon 2-containing myostatin mRNA in the subject may further translate to decreased expression of functional myostatin protein.

Methods also include treating an individual afflicted with or at risk for developing Duchenne muscular dystrophy (DMD) or a related disorder, comprising administering an effective amount of a modified antisense oligomer of the disclosure to the subject in combination with a therapeutic agent. The modified antisense oligomer may or may not be in the same composition and may or may not be co-administered to a subject. In various embodiments, the modified antisense oligomer is administered at or near the same time as the therapeutic agent. In further embodiments, the modified antisense oligomer is administered at a substantially different time as the therapeutic agent. Exemplary sequences targeted by the modified antisense oligomers as described herein are shown in Tables 1 and 2.

Also included are therapeutics and modified antisense oligomers for treating DMD or related disorders or for use in the preparation of a medicament for the treatment of DMD or related disorders, the treatment or the medicament comprising a therapeutic. In embodiments, a medicament includes a modified antisense oligomer as described herein, e.g., where the modified antisense oligomer comprises 10 to 50 subunits, optionally having at least one subunit that is a nucleotide analog having (i) a modified internucleoside linkage, (ii) a modified sugar moiety, or (iii) a combination of the foregoing; and a targeting sequence complementary to 10 or more contiguous nucleotides in a target region within dystrophin or myostatin pre-mRNA.

In various embodiments, the targeting sequence is complementary to a target region within myostatin pre-mRNA. In some embodiments, the targeting sequence is complementary to 12 or more contiguous nucleotides in a target region entirely within exon 2 where no part of the targeting sequence spans a splice junction, or a region spanning an intron/exon or exon/intron splice junction (e.g., SEQ ID NOS: 1 to 3) of myostatin pre-mRNA. In some embodiments, the targeting sequence of the modified antisense oligomers (a) comprises a sequence selected from SEQ ID NOS: 16-75, (b) is selected from SEQ ID NOS: 16-75, (c) is a fragment of at least 12 contiguous nucleotides of a sequence selected from SEQ ID NOS: 16-75, or (d) is a variant having at least 90% sequence identity to a sequence selected from SEQ ID NOS: 16-75, where X is selected from uracil (U) or thymine (T), and C is selected from cytosine (C) or 5-methylcytosine (5mC).

In various embodiments, the targeting sequence is complementary to a target region within dystrophin pre-mRNA. In some embodiments, the targeting sequence is complementary to 10 or more contiguous nucleotides in a target region within an exon of dystrophin pre-mRNA selected from exon 7, exon 8, exon 9, exon 19, exon 23, exon 44, exon 45, exon 50, exon 51, exon 52, exon 53, or exon 55. In embodiments, the target region is entirely within an exon of dystrophin pre-mRNA where no part of the targeting sequence spans a splice junction, or a region spanning an intron/exon or exon/intron splice junction (e.g., SEQ ID NOS: 4 to 15) of dystrophin pre-mRNA. In some embodiments, the targeting sequence of the modified antisense oligomers (a) comprises a sequence selected from SEQ ID NOS: 76-3485, (b) is selected from SEQ ID NOS: 76-3485, (c) is a fragment of at least 10 contiguous nucleotides of a sequence selected from SEQ ID NOS: 76-3485, or (d) is a variant having at least 90% sequence identity to a sequence selected from SEQ ID NOS: 76-3485, where X is selected from uracil (U) or thymine (T), and C is selected from cytosine (C) or 5-methylcytosine (5mC).

In some embodiments, the methods of treating DMD or related disorders or the medicaments for the treatment of DMD or related disorders include modified antisense oligomers having a nucleotide analog subunit comprising a modified sugar moiety. The modified sugar moiety may be selected from a peptide nucleic acid (PNA) subunit, a locked nucleic acid (LNA) subunit, a 2'O,4'C-ethylene-bridged nucleic acid (ENA) subunit, a tricyclo-DNA (tc-DNA) subunit, a 2' O-methyl subunit, a 2' O-methoxyethyl subunit, a 2'-fluoro subunit, a 2'-O-[2-(N-methylcarbamoyl)ethyl] subunit, and a morpholino subunit.

These additional aspects and embodiments include modified antisense oligomers having a nucleotide analog subunit comprising a modified internucleoside linkage. In various embodiments, the modified internucleoside linkage is selected from a phosphorothioate internucleoside linkage, a phosphoramidate internucleoside linkage, a phosphorodiamidate internucleoside linkage. In further embodiments, the phosphorodiamidate internucleoside linkage comprises a phosphorous atom that is covalently bonded to a (1,4-piperazin)-1-yl moiety, a substituted (1,4-piperazin)-1-yl moiety, a 4-aminopiperidin-1-yl moiety, or a substituted 4-aminopiperidin-1-yl moiety.

These additional aspects and embodiments include modified antisense oligomers having a nucleotide analog subunit comprising at least one combination of a modified sugar moiety and a modified internucleoside linkage.

In some embodiments, the modified antisense oligomer is actively taken up by mammalian cells. In further embodiments, the modified antisense oligomer may be conjugated to a transport moiety (e.g., transport peptide or CPP) as described herein to facilitate such uptake. Various aspects relate to methods of decreasing the expression of exon 2-containing myostatin mRNA transcript and/or functional myostatin protein in a cell, tissue, and/or subject, using the modified antisense oligomers as described herein. In some instances, exon 2-containing myostatin mRNA transcript and/or functional myostatin protein is decreased or reduced by about or at least about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% relative to a control, for example, a control cell/subject (for example, a subject not having Duchenne muscular dystrophy or a related disorder), a control composition without the modified antisense oligomer, the absence of treatment, and/or an earlier time-point. Also included are methods of decreasing the expression of exon 2-containing mRNA transcript or functional myostatin protein relative to the levels of a healthy control, for example, a subject not having Duchenne muscular dystrophy or a related disorder. As used herein, an "effective amount" or "therapeutic amount" refers to the dose(s) of the modified antisense oligomers that is capable to bind to the target region of myostatin pre-mRNA transcript and to decrease the expression of exon 2-containing myostatin mRNA transcript and functional myostatin protein in the range of the percentages disclosed with regard to the increase when administered to a subject, as compared to a control cell/subject.

Various aspects relate to methods for modulating the splicing of intron and exons of dystrophin pre-mRNA and increasing the expression of dystrophin or truncated dystrophin pre-mRNA in a cell, tissue, and/or subject, using the modified antisense oligomers as described herein. In further aspects, expression of a truncated form of dystrophin pre-mRNA is enhanced, such as relative to full length wildtype dystrophin pre-mRNA. In some instances, dystrophin mRNA transcript and/or functional or semi-functional dystrophin protein is increased or enhanced by about or at least about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% relative to a control, for example, a control cell/subject (for example, a subject not having Duchenne muscular dystrophy or a related disorder), a control composition without the modified antisense oligomer, the absence of treatment, and/or an earlier time-point. The methods also include increasing the expression of dystrophin mRNA transcript or functional or semi-functional dystrophin protein relative to the levels of a healthy control, for example, a subject not having Duchenne muscular dystrophy or a related disorder. As used herein, an "effective amount" or "therapeutic amount" refers to the dose(s) of the modified antisense oligomers that is capable to bind to a target region of dystrophin pre-mRNA transcript and to increase the expression of dystrophin or truncated dystrophin mRNA transcript and functional dystrophin protein in the range of the percentages disclosed with regard to the increase when administered to a subject, as compared to a control cell/subject.

The methods also include decreasing expression of a functional/active myostatin protein in a cell, tissue, and/or subject, as described herein. In certain instances, the level of functional/active myostatin protein is decreased by about or at least about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% relative to a control, for example, a control cell/subject (for example, a subject having Duchenne muscular dystrophy or a related disorder), a control composition without the therapeutic, the absence of treatment, and/or an earlier time-point. The methods also include decreasing the expression of functional/active myostatin protein relative to the levels of an affected control, for example, a subject having Duchenne muscular dystrophy or a related disorder.

The methods also include increasing expression of a functional or semi-functional/active dystrophin protein in a cell, tissue, and/or subject, as described herein. In certain instances, the level of functional or semi-functional/active dystrophin protein is increased by about or at least about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% relative to a control, for example, a control cell/subject (for example, a subject not having Duchenne muscular dystrophy or a related disorder), a control composition without the therapeutic, the absence of treatment, and/or an earlier time-point. The methods also include increasing the expression of functional or semi-functional/active dystrophin protein relative to the levels of an affected control, for example, a subject having Duchenne muscular dystrophy or a related disorder.

The methods also include inhibiting the progression of Duchenne muscular dystrophy and related disorders in a subject using the therapeutics in combination with an antisense oligomer as described herein.

In some embodiments, the therapeutic and modified antisense oligomer are administered to a subject exhibiting one or more symptoms of DMD or a related disorder, in one or more suitable pharmaceutical carriers. As used herein, the term "treat" refers to an amelioration of DMD or a related disorder, or at least one discernible symptom related to DMD or a related disorder. In some embodiments, "treat" refers to an amelioration of at least one measurable physical and/or biological parameter that is not necessarily discernible by the subject. The subject may experience, for example, physical improvement of muscle strength and coordination. Those parameters may be assessed by e.g., self-evalulation tests, physician's examinations, lab tests for physical and physiological measurments, and biological tests of samples from the subject. In another embodiment, "treat" refers to slowing the progression or reversing the progression of DMD or a related disorder. As used herein, "prevent" or "inhibit" refers to delaying the onset or reducing the risk of developing DMD or a related disorder.

The methods include reducing, or improving, as appropriate, one or more symptoms of DMD and related disorders in a subject in need thereof. Particular examples include symptoms of progressive muscle weakness such as frequent falls, difficulty getting up from a lying or sitting position, trouble running and jumping, waddling gait, walking on the toes, large calf muscles, muscle pain and stiffness and learning disabilities.

The methods also include increasing skeletal muscle mass in a subject. The methods also include treating or preventing the decrease of muscle mass in a subject, in a healthy subject or a subject afflicted with a disease, disorder or condition. The methods also include treating skeletal muscle mass deficiency in a subject afflicted with a disease, disorder, or condition. In various embodiments, blood or tissue levels of one or both of myostatin and dystrophin protein are measured in a patient prior to administration of one or both of a therapeutic agent and an antisense oligomer described herein. An effective amount of one or both of a therapeutic agent and an antisense oligomer herein is administered to the subject. Blood or tissue levels of one or both of myostatin and dystrophin protein are measured in the subject after a select time and administration of the antisense oligomer. Optionally, the dosage and/or dosing schedule of one or both of a therapeutic agent and an antisense oligomer is adjusted according to the measurement, for example, to increase the dosage to ensure a therapeutic amount of one or both is present in the subject. A select time may include an amount of time after administration of one or both of a therapeutic agent and an antisense oligomer described herein, to allow time for absorption into the bloodstream and/or metabolization by the liver and other metabolic processes. In some embodiments, a select time may be about 1, 2, 3, 4, 5, 6, 7, 8, 9, 10, 11, 12, 15, 18, 20, 22, or 24 hours after administration. In some embodiments, a select time may be about 12, 18 or 24 hours after administration. In other embodiments, a select time may be about 1, 2, 3, 4, 5, 6 or 7 days after administration.

In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a therapeutic agent as well as tailoring the dosage and/or therapeutic regimen of treatment with a therapeutic agent.

Effective administration and delivery of the therapeutic agent including a modified antisense oligomer to the target nucleic acid is a further aspect. Routes of therapeutic agent delivery include, but are not limited to, various systemic routes, including oral and parenteral routes, e.g., intravenous, subcutaneous, intraperitoneal, and intramuscular, as well as inhalation, transdermal and topical delivery. The appropriate route may be determined by one of skill in the art, as appropriate to the condition of the subject under treatment. Vascular or extravascular circulation, the blood or lymph system, and the cerebrospinal fluid are some non-limiting sites where the RNA may be introduced.

In particular embodiments, the therapeutic agent(s) are administered to the subject by intravenous (IV) or subcutaneous (SC), i.e., they are administered or delivered intravenously into a vein or subcutaneously into the fat layer between the skin and muscle. Non-limiting examples of intravenous injection sites include a vein of the arm, hand, leg, or foot. Non-limiting examples of subcutaneous injections sites include the abdomen, thigh, lower back or upper arm. In exemplary embodiments, a PMO, PMO-X, or PPMO forms of the modified antisense oligomer is administered by IV or SC. In other embodiments, the modified antisense oligomer(s) are administered to the subject by intramuscular (IM), e.g., they are administered or delivered intramuscularly into the deltoid muscle of the arm, the vastus lateralis muscle of the leg, the ventrogluteal muscles of the hips, the dorsogluteal muscles of the buttocks, the diaphragm and the intercostal muscles of the rib cage.

In certain embodiments, the therapeutic agents of the disclosure can be delivered by transdermal methods (e.g., via incorporation of the modified antisense oligomers into, e.g., emulsions, with such modified antisense oligomers optionally packaged into liposomes). Such transdermal and emulsion/liposome-mediated methods of delivery are described for delivery of modified antisense oligomers in the art, e.g., in U.S. Patent No. 6,965,025, which are hereby incorporated by reference in their entirety.

The therapeutic agents described herein may also be delivered via an implantable device. Design of such a device is an art-recognized process, with, e.g., synthetic implant design described in, e.g., U.S. Patent No. 6,969,400, which are hereby incorporated by reference in their entirety.

Therapeutic agents can be introduced into cells using art-recognized techniques (e.g., transfection, electroporation, fusion, liposomes, colloidal polymeric particles and viral and non-viral vectors as well as other means known in the art). The method of delivery selected will depend, for example, on the oligomer chemistry, the cells to be treated and the location of the cells and will be apparent to the skilled artisan. For instance, localization can be achieved by liposomes with specific markers on the surface to direct the liposome, direct injection into tissue containing target cells, specific receptor-mediated uptake, or the like.

As known in the art, therapeutic agents may be delivered using, e.g., methods involving liposome-mediated uptake, lipid conjugates, polylysine-mediated uptake, nanoparticle-mediated uptake, and receptor-mediated endocytosis, as well as additional non-endocytic modes of delivery, such as microinjection, permeabilization (e.g., streptolysin-O permeabilization, anionic peptide permeabilization), electroporation, and various non-invasive non-endocytic methods of delivery that are known in the art (refer to Dokka and Rojanasakul, Advanced Drug Delivery Reviews 44, 35-49 (2000), which is hereby incorporated by reference in its entirety).

The therapeutic agents may be administered in any convenient vehicle or carrier which is physiologically and/or pharmaceutically acceptable. Such a composition may include any of a variety of standard pharmaceutically acceptable carriers employed by those of ordinary skill in the art. Examples include, but are not limited to, saline, phosphate buffered saline (PBS), water, aqueous ethanol, emulsions, such as oil/water emulsions or triglyceride emulsions, tablets and capsules. The choice of suitable physiologically acceptable carrier will vary dependent upon the chosen mode of administration. "Pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The modified antisense oligomers of the present disclosure may generally be utilized as the free acid or free base. Alternatively, the compounds of this disclosure may be used in the form of acid or base addition salts. Acid addition salts of the free amino compounds of the present disclosure may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids.

Suitable inorganic acids include hydrochloric, hydrobromic, sulfuric, phosphoric, and nitric acids. Base addition salts included those salts that form with the carboxylate anion and include salts formed with organic and inorganic cations such as those chosen from the alkali and alkaline earth metals (for example, lithium, sodium, potassium, magnesium, barium and calcium), as well as the ammonium ion and substituted derivatives thereof (for example, dibenzylammonium, benzylammonium, 2-hydroxyethylammonium, and the like). Thus, the term "pharmaceutically acceptable salt" is intended to encompass any and all acceptable salt forms.

In addition, prodrugs are also included within the context of this disclosure. Prodrugs are any covalently bonded carriers that release a compound in vivo when such prodrug is administered to a patient. Prodrugs are generally prepared by modifying functional groups in a way such that the modification is cleaved, either by routine manipulation or in vivo, yielding the parent compound. Prodrugs include, for example, compounds of this disclosure where hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol and amine functional groups of the modified antisense oligomers of the disclosure. Further, in the case of a carboxylic acid (-COOH), esters may be employed, such as methyl esters, ethyl esters, and the like.

In some instances, liposomes may be employed to facilitate uptake of the modified antisense oligomer into cells (see, e.g., Williams, S.A., Leukemia 10(12):1980-1989, 1996; Lappalainen et al., Antiviral Res. 23:119, 1994; Uhlmann et al., modified antisense oligomers: a new therapeutic principle, Chemical Reviews, Volume 90, No. 4, 25 pages 544-584, 1990; Gregoriadis, G., Chapter 14, Liposomes, Drug Carriers in Biology and Medicine, pp. 287-341, Academic Press, 1979). Hydrogels may also be used as vehicles for modified antisense oligomer administration, for example, as described in PCT Publication No. WO 1993/01286. Alternatively, the oligomers may be administered in microspheres or microparticles. (See, e.g., Wu, G.Y. and Wu, C.H., J. Biol. Chem. 262:4429-4432, 30 1987). Alternatively, the use of gas-filled microbubbles complexed with the modified antisense oligomers can enhance delivery to target tissues, as described in U.S. Patent No. 6,245,747. Sustained release compositions may also be used. These may include semipermeable polymeric matrices in the form of shaped articles such as films or microcapsules. Each such reference is hereby incorporated by reference in their entirety.

In some embodiments, the therapeutic agent is administered in an amount and manner effective to result in a peak blood concentration of at least 200-400 nM of therapeutic agent. Typically, one or more doses of therapeutic agent are administered, generally at regular intervals, for a period of about one to two weeks. Preferred doses for oral administration are from about 1-1000 mg oligomer per 70 kg. In some cases, doses of greater than 1000 mg oligomer/patient may be necessary. For i.v. administration, preferred doses are from about 0.5 mg to 1000 mg oligomer per 70 kg. The therapeutic agent may be administered at regular intervals for a short time period, e.g., daily for two weeks or less. However, in some cases the therapeutic agent is administered intermittently over a longer period of time. Administration may be followed by, or concurrent with, administration of an antibiotic or other therapeutic treatment. The treatment regimen may be adjusted (dose, frequency, route, etc.) as indicated, based on the results of immunoassays, other biochemical tests and physiological examination of the subject under treatment.

An effective *in vivo* treatment regimen using the therapeutic agents of the disclosure may vary according to the duration, dose, frequency and route of administration, as well as the condition of the subject under treatment (i.e., prophylactic administration versus administration in response to localized or systemic infection). Accordingly, such in vivo therapy will often require monitoring by tests appropriate to the particular type of disorder under treatment, and corresponding adjustments in the dose or treatment regimen, in order to achieve an optimal therapeutic outcome.

Treatment may be monitored, e.g., by general indicators of disease known in the art. The efficacy of an *in vivo* administered therapeutic agent may be determined from biological samples (tissue, blood, urine etc.) taken from a subject prior to, during and subsequent to administration of the therapeutic agent. Assays of such samples, wherein the therapeutic agent is a modified antisense oligomer, include (1) monitoring the presence or absence of heteroduplex formation with target and non-target sequences, using procedures known to those skilled in the art, e.g., an electrophoretic gel mobility assay; (2) monitoring the amount of an mRNA which does not comprise myostatin exon 2 in relation to a reference exon 2-containing myostatin mRNA; or (3) monitoring the amount of an mRNA which does not comprise dystrophin mRNA containing one or more exons having one or more genetic mutations in relation to a reference dystrophin mRNA containing one or more genetic mutations, as determined by standard techniques such as RT-PCR, northern blotting, ELISA or western blotting. In some embodiments, treatment is monitored by symptomatic assessments. Those assessments include, but not limited to, self-evalulation, physician's examinations, motor function tests (e.g., grip strength tests) including measurements of muscle size, muscle mass, strength, reflex, involuntary muscle movements, electrophysiology test, number of muscle fibers and fibers with centralized nuclei, and cardiovascular function tests including electrocardiogram (EKG or EGG).

In some embodiments, the methods described herein also include administration in combination with another therapeutic. The additional therapeutic may be administered prior, concurrently or non-concurrently, for example subsequently, to the administration of the therapeutic(s) of the present invention. For example, the therapeutic may be administered in combination with a steroid and/or an antibiotic. In another example, the patient has been treated with a corticosteroid (e.g., a stable dose of a corticosteroid for four to six, seven, eight, nine, 10, 11, 12, 13, 14, 15, 16, 17, 18, 19, 20, 21, 22, 23, or 24 or more weeks) prior to administration of eteplirsen. The steroid may be a glucocorticoid or prednisone. Glucocorticoids such as cortisol control carbohydrate, fat and protein metabolism, and are anti-inflammatory by preventing phospholipid release, decreasing eosinophil action and a number of other mechanisms. Mineralocorticoids such as aldosterone control electrolyte and water levels, mainly by promoting sodium retention in the kidney. Corticosteroids are a class of chemicals that includes steroid hormones naturally produced in the adrenal cortex of vertebrates and analogues of these hormones that are synthesized in laboratories. Corticosteroids are involved in a wide range of physiological processes, including stress response, immune response, and regulation of inflammation, carbohydrate metabolism, protein catabolism, blood electrolyte levels, and behavior. Corticosteroids include, but are not limited to, Betamethasone, Budesonide, Cortisone, Dexamethasone, Hydrocortisone, Methylprednisolone, Prednisolone, and Prednisone. One particular steroid of interest that may be administered prior, concurrently or subsequently to the administration of the composition of the present invention is deflazacort and formulations thereof (e.g., MP-104, Marathon Pharmaceuticals LLC).

In some embodiments, the dosage of a therapeutic (e.g., a therapeutic oligonucleotide, such as eteplirsen) is about 30 mg/kg over a period of time sufficient to treat DMD. In some embodiments, the therapeutic is administered to the patient at a dose of between about 25 mg/kg and about 50 mg/kg (e.g., about 25, 26, 27, 28, 29, 30, 31, 32. 33, 34, 35, 36, 37, 38, 39, 40, 41, 42, 43, 44, 45, 46, 47, 48, 49, or 50 mg/kg), e.g., once per week. In some embodiments, the therapeutic is administered to the patient at a dose of between about 25 mg/kg and about 50 mg/kg (e.g., about 30 mg/kg to about 50 mg/kg, about 25 mg/kg to about 40 mg/kg, about 28 mg/kg to about 32 mg/kg, or about 30 mg/kg to about 40 mg/kg), e.g., once per week.

In some embodiments, the therapeutic is administered intravenously once a week. In certain embodiments, the time of infusion is from about 15 minutes to about 4 hours. In some embodiments, the time of infusion is from about 30 minutes to about 3 hours. In some embodiments, the time of infusion is from about 30 minutes to about 2 hours. In some embodiments, the time of infusion is from about 1 hour to about 2 hours. In some embodiments the time of infusion is from about 30 minutes to about 1 hour. In some embodiments, the time of infusion is about 60 minutes. In some embodiments, the time of infusion is 35 to 60 minutes.

### VI. Dosing

The formulation of therapeutic compositions and their subsequent administration (dosing) is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligomers, and can generally be estimated based on EC50s found to be effective in in vitro and in vivo animal models. In general, dosage is from 0.01 µg to 100 g per kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, where the oligomer is administered in maintenance doses, ranging from 1-1000 mg oligomer per 70 kg of body weight for oral administration, or 0.5 mg to 1000 mg oligomer per 70 kg of body weight for i.v. administration, once or more daily, to once every 20 years.

While the present disclosure has been described with specificity in accordance with certain of its embodiments, the following examples serve only to illustrate the disclosure and are not intended to limit the same. Each of the references, patents, patent applications, GenBank accession numbers, and the like recited in the present application are hereby incorporated by reference in its entirety.

### VI. Examples

The following Examples may be used for illustrative purposes and should not be deemed to narrow the scope of the invention.

Modified antisense oligomers (illustrated in FIGS. 1A to 1G) of the disclosure were designed to bind to a target region within a dystrophin or myostatin pre-mRNA transcript and prepared using the following protocol:

### Procedure A for the preparation of active subunits:

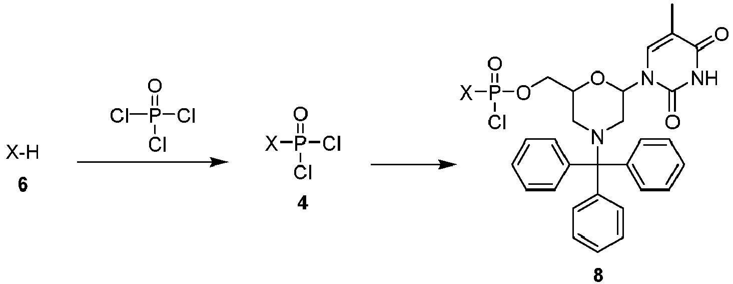

To a stirred solution of 6 (1 eq) in dichloromethane was added POCl3 (1.1 eq), followed by diisopropylethylamine (3 eq) at 0°C, cooled by an ice-bath. After 15 minutes, the ice-bath was removed and the solution was allowed to warm to room temperature for one hour. Upon reaction completion, the reaction solution was diluted with dichloromethane, washed with 10% aqueous citric acid three times. After drying over MgSO4, the organic layer was passed through a plug of silica gel and concentrated in vacuo. The resulting phosphoroamidodichloride (4) was used directly for the next step without further purification.

To a solution of the phosphoroamidodichloride (4) (1 eq), 2,6-lutidine (1 eq) in dichloromethane was added Mo(Tr)T (7) (0.5eq)/ dichloromethane solution, followed by N-methylimidazole (0.2 eq). The reaction stirred at room temperature overnight. Upon reaction completion, the reaction solution was diluted with dichloromethane, and washed with 10% aqueous citric acid three times. After drying over MgSO₄, the organic layer was filtered, then concentrated. The product (8) was purified by silica gel chromatography (eluting with a gradient of ethyl acetate/hexanes), and then stored at -20°C. The structure was confirmed by LCMS analysis.

### Procedure B for the preparation of activated subunits:

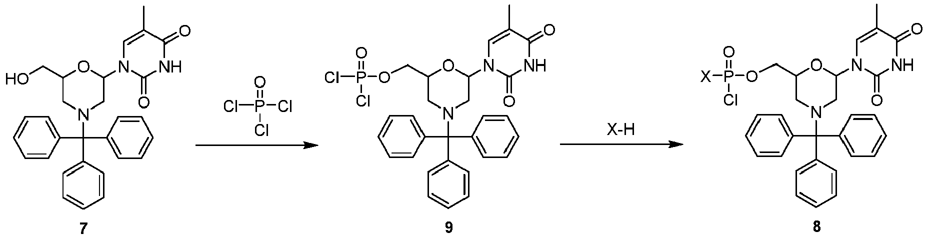

To a solution of POCl₃ (1.1eq) in dichloromethane was added 2,6-lutidine (2eq), followed by dropwise addition of Mo(Tr)T (7) (1eq)/ dichloromethane solution at 0°C. After 1 hour, the reaction solution was diluted with dichloromethane, and quickly washed three times with 10% aqueous citric acid. The desired phosphodichloridate (9) was obtained after drying over MgSO₄ and evaporation of solvent.

To a solution of the phosphodichloridate (leq) in dichloromethane was added amine (1eq)/ dichloromethane dropwise to the solution at 0°C. After 15 minutes, the reaction mixture was allowed to warm to room temperature for about an hour. Upon reaction completion, the product (**8**) as a white solid was collected by precipitation with the addition of hexanes, followed by filtration. The product was stored at -20°C after drying under vacuum. The structure was confirmed by LCMS analysis.

### Example 1: ((2S,6R)-6-(5-methyl-2,4-dioxo-3,4-dihydropyrimidin-1(2H)-yl)-4-tritylmorpholin-2-yl)methyl phosphorodichloridate

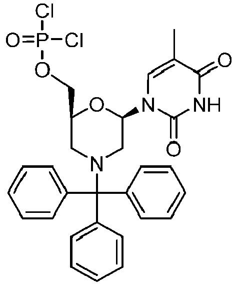

To a cooled (ice/water bath) DCM solution (20 mL) of phosphorus oxychloride (2.12 mL, 22.7 mmol) was added dropwise 2,6-lutidine (4.82 mL, 41.4 mmol) then a DCM solution (20 mL) Mo(Tr)T (2) (10.0 g, 20.7 mmol) was added dropwise over 15 min (int. temp. 0-10 °C) then bath was removed a stirring continued at ambient temperature for 20 min. The reaction was washed with citric acid solution (40 mL x 3, 10 % w/v aq), dried (MgSO4), filtered and concentrated to a white foam (9.79 g) then used directly for the following procedure.

### EXAMPLE 2: (6-(5-methyl-2,4-dioxo-3,4-dihydropyrimidin-1(2H)-yl)-4-tritylmorpholin-2-yl)methyl(4-(dimethylamino)piperidin-1-yl)phosphonochloridate

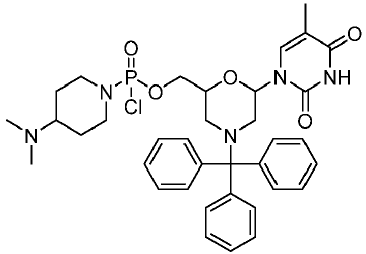

To a cooled (ice/water bath) DCM solution (5 mL) of the dichlorophosphate from example 1 (5.00 g, 5.00 mmol) was added a DCM solution (5 mL) of the piperidine (0.61 g, 4.76 mmol) dropwise then the bath was removed and stirring continued at ambient temperature for 30 min. The reaction was loaded directly onto a column. Chromatography with [SiO2 column (40 g), DCM/EtOH eluant (gradient 1:0 to 1:1)] afforded the title compound (2.5 g) as a white foam. ESI/MS calcd. for 1(4 nitrophenyl)piperazine derivative C46H55N8O7P 862.4, found m/z = 863.6 (M+1).

### EXAMPLE 3: 1-(1-(chloro((6-(5-methyl-2,4-dioxo-3,4-dihydropyrimidin-1(2H)-yl)-4-tritylmorpholin-2-yl)methoxy)phosphoryl)piperidin-4-yl)-1-methylpyrrolidin-1-ium chloride

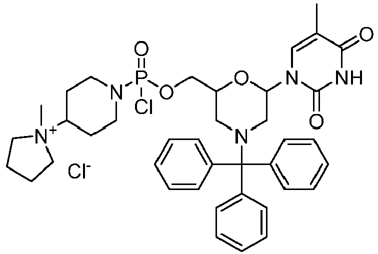

The title compound was synthesized in a manner analogous to that described in Example 2 to afford the title compound (0.6 g) as a white solid. ESI/MS calcd. for 1-(4-nitrophenyl)piperazine derivative C₄₉H₆₀N₈O₇P 903.4, found *m*/*z* = 903.7 (M+).

### EXAMPLE 4: ((2S,6R)-6-(5-methyl-2,4-dioxo-3,4-dihydropyrimidin-1(2H)-yl)-4-tritylmorpholin-2-yl)methyl (4-methylpiperazin-1-yl)phosphonochloridate

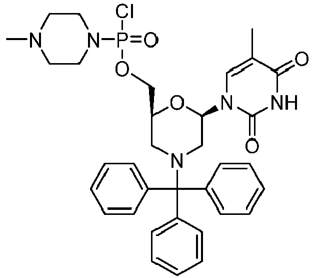

To a cooled (ice/water bath) DCM solution(10 mL) of phosphorus oxychloride (1.02 mL, 11.0 mmol) was added dropwise 2,6-lutidine (3.49 mL, 29.9 mmol) then a DCM solution (10 mL) of methyl piperazine (1.00 g, 10.0 mmol) was added dropwise and stirring continued for 1 h. A DCM solution (10 mL) of Mo(Tr)T (2) (4.82, 10.0 mmol) and NMI (79 µL, 1.0 mmol) was added and stirred 4 h then loaded directly onto a column.

Chromatography with [SiO2 column (80 g), DCM/Acetone with 2% TEA eluant (gradient 1:0 to 0: 1)] afforded the title compound (0.8 g) as a white foam. ESI/MS calcd. for 1-(4-nitrophenyl)piperazine derivative C43H48N7O8P 834.4, found m/z = 835.5 (M+1).

### EXAMPLE 5: ((2S,6R)-6-(5-methyl-2,4-dioxo-3,4-dihydropyrimidin-1(2H)-yl)-4-tritylmorpholin-2-yl)methyl(4-ethylpiperazin-1-yl)phosphonochloridate

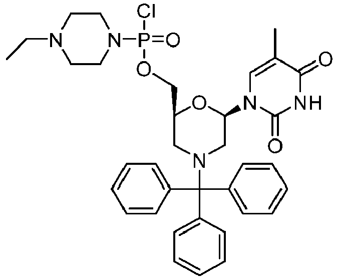

The title compound was synthesized in a manner analogous to that described in Example 4 to afford the title compound (11.5 g) as a white foam. ESI/MS calcd. for 1-(4-nitrophenyl)piperazine derivative C45H53N8O7P 848.4, found m/z = 849.7 (M+1).

### EXAMPLE 6: ((2S,6R)-6-(6-benzamido-9H-purin-9-yl)-4-tritylmorpholin-2-yl)methyl(4-ethylpiperazin-1-yl)phosphonochloridate

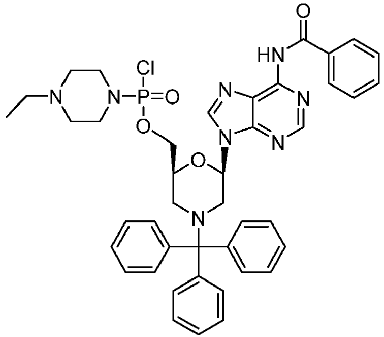

The title compound was synthesized in a manner analogous to that described in Example 4 to afford the title compound (4.5 g) as a white foam. ESI/MS calcd. for 1-(4-nitrophenyl)piperazine derivative C52H56N11O6P 961.4, found m/z = 962.8 (M+1).

### EXAMPLE 7: ((2S,6R)-6-(5-methyl-2,4-dioxo-3,4-dihydropyrimidin-1(2H)-yl)-4-tritylmorpholin-2-yl)methyl (4-isopropylpiperazin-1-yl)phosphonochloridate

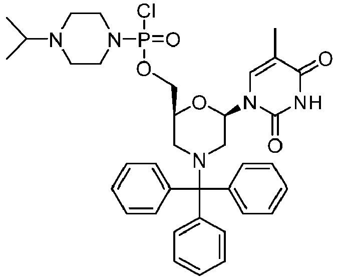

The title compound was synthesized in a manner analogous to that described in Example 4 to afford the title compound (3.5 g) as a white foam. ESI/MS calcd. for 1-(4-nitrophenyl)piperazine derivative C₄₆H₅₅N₈O₇P 862.4, found *m*/*z* = 863.7 (M+1).

### EXAMPLE 8: ((2S,6R)-6-(5-methyl-2,4-dioxo-3,4-dihydropyrimidin-1(2H)-yl)-4-tritylmorpholin-2-yl)methylmethyl(2-(2,2,2-trifluoroacetamido)ethyl)phosphoramidochloridate

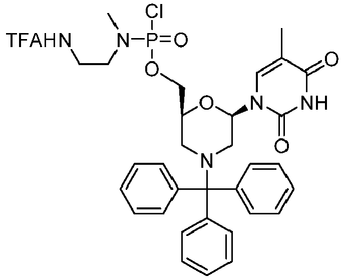

The title compound was synthesized in a manner analogous to that described in Example 4 to afford the title compound (1.0 g) as a white foam. ESI/MS calcd. for 1-(4-nitrophenyl)piperazine derivative C₄₄H₄₈F₃N₈O₈P 904.3, found *m*/*z* = 903.7 (M-1).

### EXAMPLE 9: ((2S,6R)-6-(5-methyl-2,4-dioxo-3,4-dihydropyrimidin-1(2H)-yl)-4-tritylmorpholin-2-yl)methylmethyl(2-(2,2,2-trifluoro-N-methylacetamido)ethyl)phosphoramidochloridate

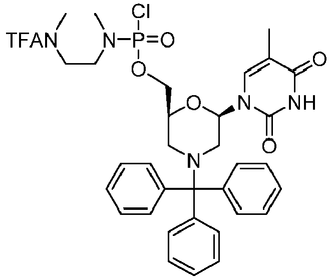

The title compound was synthesized in a manner analogous to that described in Example 4 to afford the title compound (1.8 g) as a white foam. ESI/MS calcd. for 1-(4-nitrophenyl)piperazine derivative C₄₅H₅₀F₃N₈O₈P 918.3, found m/z = 1836.6 (2M+).

### EXAMPLE 10: ((2S,6R)-6-(5-methyl-2,4-dioxo-3,4-dihydropyrimidin-1(2H)-yl)-4-tritylmorpholin-2-yl)methyl(4-(2,2,2-trifluoroacetamido)piperidin-1-yl)phosphonochloridate

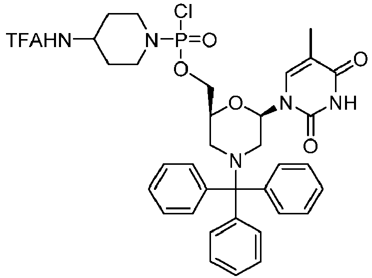

To a cooled solution (ice/water bath) of phosphorus oxychloride (17.7 mL, 190 mmol) in DCM (190 mL) was added dropwise 2,6-lutidine (101 mL, 864 mmol) then Mo(Tr)T (2) (83.5 g, 173 mmol) portionwise over 15 min (int. temp. 0-10 °C) and stirred. After 30 min, the 4-aminopiperidine monotrifluoroacetamide (48.9 g, ∼190 mmol) was added dropwise over 15 min (int. temp. 0-8 °C) and stirred. After lh, DIPEA (50 mL) was added dropwise (int. temp. 0-10 °C) and stirred lh. The reaction was washed with citric acid solution (500 mL x 3, 10 % w/v aq), dried (MgSO4), filtered and concentrated to a viscous oil which was loaded directly onto a column. Chromatography with [SiO2 column (330 g), hexanes/EtOAc eluant (gradient 1:0 to 0: 1)] afforded the title compound (91.3 g, 70% yield) as a white foam. ESI/MS calcd. for 1-(4-nitrophenyl)piperazine derivative C43H48N7O8P 930.9, found m/z = 954.4 (M+Na).

**Examples 11** through 14 were prepared via procedure A described above.

### EXAMPLE 11: (6-(5-methyl-2,4-dioxo-3,4-dihydropyrimidin-1(2H)-yl)-4-tritylmorpholin-2-yl)methyl(4-(1-(2,2,2-trifluoroacetyl)piperidin-4-yl)piperazin-1-yl)phosphonochloridate

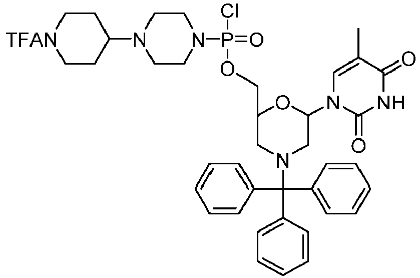

### EXAMPLE 12: (6-(5-methyl-2,4-dioxo-3,4-dihydropyrimidin-1(2H)-yl)-4-tritylmorpholin-2-yl)methyl(4-morpholinopiperidin-1-yl)phosphonochloridate

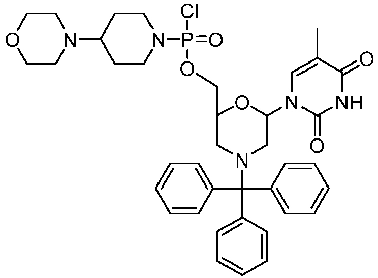

### EXAMPLE 13: (6-(5-methyl-2,4-dioxo-3,4-dihydropyrimidin-1(2H)-yl)-4-tritylmorpholin-2-yl)methylbis(3-(2,2,2-trifluoroacetamido)propyl)phosphoramidochloridate

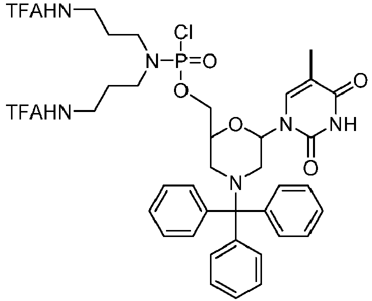

### EXAMPLE 14: (6-(5-methyl-2,4-dioxo-3,4-dihydropyrimidin-1(2H)-yl)-4-tritylmorpholin-2-yl)methyl[1,4'-bipipetidin]-1'-ylphosphonochloridate

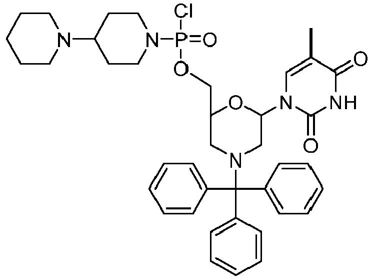

Examples 15 through 20 below were prepared via procedure B described above.

### EXAMPLE 15: (6-(5-methyl-2,4-dioxo-3,4-dihydropyrimidin-1(2H)-yl)-4-tritylmorpholin-2-yl)methyl(4-(pyrimidin-2-yl)piperazin-1-yl)phosphonochloridate

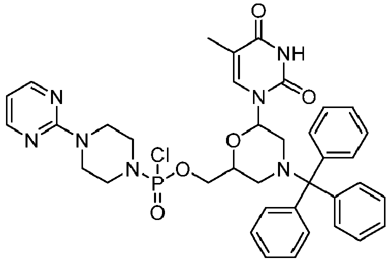

### EXAMPLE 16: (6-(5-methyl-2,4-dioxo-3,4-dihydropyrimidin-1(2H)-yl)-4-tritylmorpholin-2-yl)methyl(4-(2-(dimethylamino)ethyl)piperazin-1-yl)phosphonochlotidate

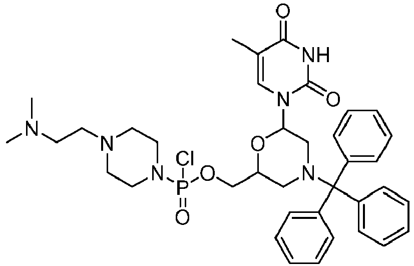

### EXAMPLE 17: (6-(5-methyl-2,4-dioxo-3,4-dihydropyrimidin-1(2H)-yl)-4-tritylmorpholin-2-yl)methyl(4-phenylpiperazin-1-yl)phosphonochloridate

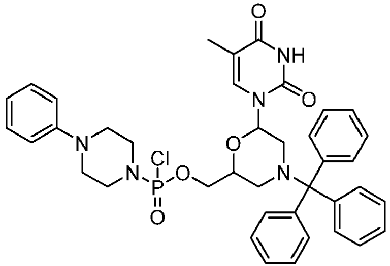

### EXAMPLE 18: (6-(5-methyl-2,4-dioxo-3,4-dihydropyrimidin-1(2H)-yl)-4-tritylmorpholin-2-yl)methyl(4-(2,2,2-trifluoro-N-methylacetamido)piperidin-1-yl)phosphonochloridate

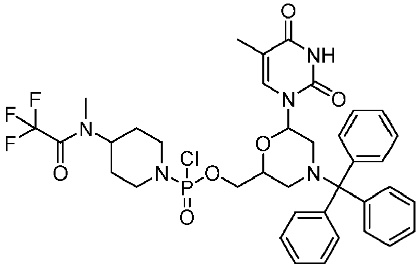

### EXAMPLE 19: (6-(5-methyl-2,4-dioxo-3,4-dihydropyrimidin-1(2H)-yl)-4-tritylmorpholin-2-yl)methylmethyl(3-(2,2,2-trifluoro-N-methylacetamido)propyl)phosphoramidochloridate

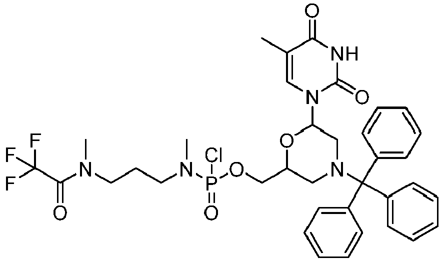

### EXAMPLE 20: ((2S,6R)-6-(6-benzamido-9H-purin-9-yl)-4-tritylmorpholin-2-yl)methyl(4-(2,2,2-trifluoroacetamido)piperidin-1-yl)phosphonochloridate

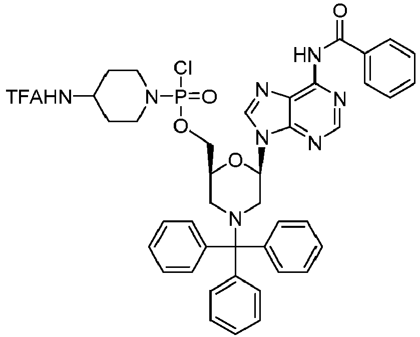

### EXAMPLE 21: (4-(pyrrolidin-1-yl)piperidin-1-yl)phosphonic dichloride hydrochloride

To a cooled (ice/water bath) solution of phosphorus oxychloride (5.70 mL, 55.6 mmol) in DCM (30 mL) was added 2,6-lutidine (19.4 mL, 167 mmol) and a DCM solution (30 mL) of 4-(1-pyrrolidinyl)-piperidine (8.58 g, 55.6 mmol) and stirred for 1 hour. The suspension was filtered and solid washed with excess diethyl ether to afford the title pyrrolidine (17.7 g, 91% yield) as a white solid. ESI/MS calcd. for 1-(4-nitrophenyl)piperazine derivative C₁₉H₃₀N₅O₄P 423.2, found *m*/*z* = 422.2 (M-1).

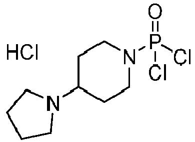

### EXAMPLE 22: ((2S,6R)-6-(5-methyl-2,4-dioxo-3,4-dihydropyrimidin-1(2H)-yl)-4-tritylmorpholin-2-yl)methyl(4-(pyrrolidin-1-yl)piperidin-1-yl)phosphonochloridate hydrochloride

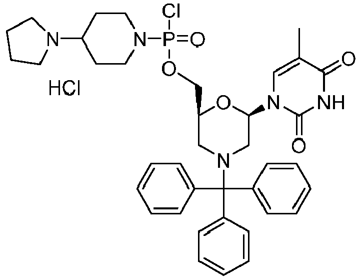

To a stirred, cooled (ice/water bath) solution of the dichlorophosphoramidate from Example 21 (17.7 g, 50.6 mmol) in DCM (100 mL) was added a DCM solution (100 mL) of Mo(Tr)T (2) (24.5 g, 50.6 mmol), 2,6-Lutidine (17.7 mL, 152 mmol), and 1-methylimidazole (0.401 mL, 5.06 mmol) dropwise over 10 minutes. The bath was allowed to warm to ambient temperature as suspension was stirred. After 6 hours, the suspension was poured onto diethyl ether (1 L), stirred 15 minutes, filtered and solid washed with additional ether to afford a white solid (45.4 g). The crude product was purified by chromatography [SiO₂ column (120 gram), DCM/MeOH eluant (gradient 1:0 to 6:4)], and the combined fractions were poured onto diethyl ether (2.5 L), stirred 15 min, filtered, and the resulting solid washed with additional ether to afford the title compound (23.1 g, 60 % yield) as a white solid. ESI/MS calcd. for 1-(4-nitrophenyl)piperazine derivative C₄₈H₅₇N₈O₇P 888.4, found *m*/*z* = 887.6 (M-1).

### EXAMPLE 23 Design and Manufacture of Modified Antisense Oligomers and Exemplary Modified Antisense Oligomers

Preparation of trityl piperazine phenyl carbamate 35 (FIG. 2A): To a cooled suspension of compound 11 in dichloromethane (6 mL/g 11) was added a solution of potassium carbonate (3.2 eq) in water (4 mL/g potassium carbonate). To this two-phase mixture was slowly added a solution of phenyl chloroformate (1.03 eq) in dichloromethane (2 g/g phenyl chloroformate). The reaction mixture was warmed to 20 °C. Upon reaction completion (1-2 hr), the layers were separated. The organic layer was washed with water, and dried over anhydrous potassium carbonate. The product 35 was isolated by crystallization from acetonitrile.

Preparation of carbamate alcohol 36: Sodium hydride (1.2 eq) was suspended in 1-methyl-2-pyrrolidinone (32 mL/g sodium hydride). To this suspension were added triethylene glycol (10.0 eq) and compound 35 (1.0 eq). The resulting slurry was heated to 95 °C. Upon reaction completion (1-2 hr), the mixture was cooled to 20 °C. To this mixture was added 30% dichloromethane/methyl tert-butyl ether (v:v) and water. The product-containing organic layer was washed successively with aqueous NaOH, aqueous succinic acid, and saturated aqueous sodium chloride. The product 36 was isolated by crystallization from dichloromethane/methyl tert-butyl ether/heptane.

Preparation of Tail acid 37: To a solution of compound 36 in tetrahydrofuran (7 mL/g 36) was added succinic anhydride (2.0 eq) and DMAP (0.5 eq). The mixture was heated to 50 °C. Upon reaction completion (5 hr), the mixture was cooled to 20 °C and adjusted to pH 8.5 with aqueous NaHCO3. Methyl tert-butyl ether was added, and the product was extracted into the aqueous layer. Dichloromethane was added, and the mixture was adjusted to pH 3 with aqueous citric acid. The product-containing organic layer was washed with a mixture of pH=3 citrate buffer and saturated aqueous sodium chloride. This dichloromethane solution of 37 was used without isolation in the preparation of compound 38.

Preparation of 38: To the solution of compound 37 was added N-hydroxy-5-norbornene-2,3-dicarboxylic acid imide (HONB) (1.02 eq), 4-dimethylaminopyridine (DMAP) (0.34 eq), and then 1-(3-dimethylaminopropyl)-N'-ethylcarbodiimide hydrochloride (EDC) (1.1 eq). The mixture was heated to 55 °C. Upon reaction completion (4-5 hr), the mixture was cooled to 20 °C and washed successively with 1:1 0.2 M citric acid/brine and brine. The dichloromethane solution underwent solvent exchange to acetone and then to N,N-dimethylformamide, and the product was isolated by precipitation from acetone/ N,N-dimethylformamide into saturated aqueous sodium chloride. The crude product was reslurried several times in water to remove residual N,N-dimethylformamide and salts.

Introduction of the activated "Tail" onto the anchor-loaded resin was performed in dimethyl imidazolidinone (DMI) by the procedure used for incorporation of the subunits during solid phase synthesis.

Preparation of the Solid Support for Synthesis of morpholino-based oligomers: This procedure was performed in a silanized, jacketed peptide vessel (ChemGlass, NJ, USA) with a coarse porosity (40-60 µm) glass frit, overhead stirrer, and 3-way Teflon stopcock to allow N2 to bubble up through the frit or a vacuum extraction.

The resin treatment/wash steps in the following procedure consist of two basic operations: resin fluidization or stirrer bed reactor and solvent/solution extraction. For resin fluidization, the stopcock was positioned to allow N2 flow up through the frit and the specified resin treatment/wash was added to the reactor and allowed to permeate and completely wet the resin. Mixing was then started and the resin slurry mixed for the specified time. For solvent/solution extraction, mixing and N2 flow were stopped and the vacuum pump was started and then the stopcock was positioned to allow evacuation of resin treatment/wash to waste. All resin treatment/wash volumes were 15 mL/g of resin unless noted otherwise.

To aminomethylpolystyrene resin (100-200 mesh; ∼1.0 mmol/g load based on nitrogen substitution; 75 g, 1 eq, Polymer Labs, UK, part #1464-X799) in a silanized, jacketed peptide vessel was added 1-methyl-2-pyrrolidinone (NMP; 20 ml/g resin) and the resin was allowed to swell with mixing for 1-2 hr. Following evacuation of the swell solvent, the resin was washed with dichloromethane (2 x 1-2 min), 5% diisopropylethylamine in 25% isopropanol/dichloromethane (2 x 3-4 min) and dichloromethane (2 x 1-2 min). After evacuation of the final wash, the resin was treated with a solution of disulfide anchor 34 in 1-methyl-2-pyrrolidinone (0.17 M; 15 mL/g resin, ∼2.5 eq) and the resin/reagent mixture was heated at 45 °C for 60 hr. On reaction completion, heating was discontinued and the anchor solution was evacuated and the resin washed with 1-methyl-2-pyrrolidinone (4 x 3-4 min) and dichloromethane (6 x 1-2 min). The resin was treated with a solution of 10% (v/v) diethyl dicarbonate in dichloromethane (16 mL/g; 2 x 5-6 min) and then washed with dichloromethane (6 x 1-2 min). The resin 39 (FIG. 2B) was dried under a N₂ stream for 1-3 hr and then under vacuum to constant weight (± 2%). Yield: 110-150% of the original resin weight.

Determination of the Loading of Aminomethylpolystyrene-disulfide resin: The loading of the resin (number of potentially available reactive sites) is determined by a spectrometric assay for the number of triphenylmethyl (trityl) groups per gram of resin.

A known weight of dried resin (25 ± 3 mg) is transferred to a silanized 25 ml volumetric flask and ∼5 mL of 2% (v/v) trifluoroacetic acid in dichloromethane is added. The contents are mixed by gentle swirling and then allowed to stand for 30 min. The volume is brought up to 25 mL with additional 2% (v/v) trifluoroacetic acid in dichloromethane and the contents thoroughly mixed. Using a positive displacement pipette, an aliquot of the trityl-containing solution (500 µL) is transferred to a 10 mL volumetric flask and the volume brought up to 10 mL with methanesulfonic acid.

The trityl cation content in the final solution is measured by UV absorbance at 431.7 nm and the resin loading calculated in trityl groups per gram resin (µmol/g) using the appropriate volumes, dilutions, extinction coefficient (ε: 41 µmol-1cm-1) and resin weight. The assay is performed in triplicate and an average loading calculated.

The resin loading procedure in this example will provide resin with a loading of approximately 500 µmol/g. A loading of 300-400 in µmol/g was obtained if the disulfide anchor incorporation step is performed for 24 hr at room temperature.

Tail loading: Using the same setup and volumes as for the preparation of aminomethylpolystyrene-disulfide resin, the Tail can be introduced into solid support. The anchor loaded resin was first deprotected under acidic condition and the resulting material neutralized before coupling. For the coupling step, a solution of 38 (0.2 M) in DMI containing 4-ethylmorpholine (NEM, 0.4 M) was used instead of the disulfide anchor solution. After 2 hr at 45 °C, the resin 39 was washed twice with 5% diisopropylethylamine in 25% isopropanol/dichloromethane and once with DCM. To the resin was added a solution of benzoic anhydride (0.4 M) and NEM (0.4 M). After 25 min, the reactor jacket was cooled to room temperature, and the resin washed twice with 5% diisopropylethylamine in 25% isopropanol/dichloromethane and eight times with DCM. The resin 40 was filtered and dried under high vacuum. The loading for resin 40 is defined to be the loading of the original aminomethylpolystyrene-disulfide resin 39 used in the Tail loading.

Solid Phase Synthesis: morpholino-based oligomers were prepared on a Gilson AMS-422 Automated Peptide Synthesizer in 2 mL Gilson polypropylene reaction columns (Part # 3980270). An aluminum block with channels for water flow was placed around the columns as they sat on the synthesizer. The AMS-422 will alternatively add reagent/wash solutions, hold for a specified time, and evacuate the columns using vacuum.

For oligomers in the range up to about 25 subunits in length, aminomethylpolystyrene-disulfide resin with loading near 500 µmol/g of resin is preferred. For larger oligomers, aminomethylpolystyrene-disulfide resin with loading of 300-400 µmol/g of resin is preferred. If a molecule with 5'-Tail is desired, resin that has been loaded with Tail is chosen with the same loading guidelines.

The following reagent solutions were prepared:

Detritylation Solution: 10% Cyanoacetic Acid (w/v) in 4:1 dichloromethane/acetonitrile; Neutralization Solution: 5% Diisopropylethylamine in 3:1 dichloromethane/isopropanol; Coupling Solution: 0.18 M (or 0.24 M for oligomers having grown longer than 20 subunits) activated morpholino subunit of the desired base and linkage type and 0.4 M N ethylmorpholine, in 1,3-dimethylimidazolidinone. Dichloromethane (DCM) was used as a transitional wash separating the different reagent solution washes.

On the synthesizer, with the block set to 42 °C, to each column containing 30 mg of aminomethylpolystyrene-disulfide resin (or Tail resin) was added 2 mL of 1-methyl-2-pyrrolidinone and allowed to sit at room temperature for 30 min. After washing with 2 times 2 mL of dichloromethane, the following synthesis cycle was employed:

**Table 6. Synthesis Cycle for Modified Antisense Oligomers**

| Step | Volume | Delivery | Hold time |
|---|---|---|---|
| Detritylation | 1.5 mL | Manifold | 15 sec. |
| Detritylation | 1.5 mL | Manifold | 15 sec. |
| Detritylation | 1.5 mL | Manifold | 15 sec. |
| Detritylation | 1.5 mL | Manifold | 15 sec. |
| Detritylation | 1.5 mL | Manifold | 15 sec. |
| Detritylation | 1.5 mL | Manifold | 15 sec. |
| Detritylation | 1.5 mL | Manifold | 15 sec. |
| DCM | 1.5 mL | Manifold | 30 sec. |
| Neutralization | 1.5 mL | Manifold | 30 sec. |
| Neutralization | 1.5 mL | Manifold | 30 sec. |
| Neutralization | 1.5 mL | Manifold | 30 sec. |
| Neutralization | 1.5 mL | Manifold | 30 sec. |
| Neutralization | 1.5 mL | Manifold | 30 sec. |
| Neutralization | 1.5 mL | Manifold | 30 sec. |
| DCM | 1.5 mL | Manifold | 30 sec. |
| Coupling | 350-500uL | Syringe | 40 min. |
| DCM | 1.5 mL | Manifold | 30 sec. |
| Neutralization | 1.5 mL | Manifold | 30 sec. |
| Neutralization | 1.5 mL | Manifold | 30 sec. |
| DCM | 1.5 mL | Manifold | 30 sec. |
| DCM | 1.5 mL | Manifold | 30 sec. |
| DCM | 1.5 mL | Manifold | 30 sec. |

The sequences of the individual oligomers were programmed into the synthesizer so that each column receives the proper coupling solution (A,C,G,T,I) in the proper sequence. When the oligomer in a column had completed incorporation of its final subunit, the column was removed from the block and a final cycle performed manually with a coupling solution comprised of 4-methoxytriphenylmethyl chloride (0.32 M in DMI) containing 0.89 M 4-ethylmorpholine.

Cleavage from the resin and removal of bases and protecting groups: After methoxytritylation, the resin was washed 8 times with 2 mL 1-methyl-2-pyrrolidinone. One mL of a cleavage solution comprising 0.1 M 1,4-dithiothreitol (DTT) and 0.73 M triethylamine in 1-methyl-2-pyrrolidinone was added, the column capped, and allowed to sit at room temperature for 30 min. After that time, the solution was drained into a 12 mL Wheaton vial. The greatly shrunken resin was washed twice with 300 µL of cleavage solution. To the solution was added 4.0 mL conc. Aqueous ammonia (stored at -20 °C), the vial capped tightly (with Teflon lined screw cap), and the mixture swirled to mix the solution. The vial was placed in a 45 °C oven for 16-24 hr to effect cleavage of base and protecting groups.

Crude product purification: The vialed ammonolysis solution was removed from the oven and allowed to cool to room temperature. The solution was diluted with 20 mL of 0.28% aqueous ammonia and passed through a 2.5x10 cm column containing Macroprep HQ resin

(BioRad). A salt gradient (A: 0.28% ammonia with B: 1 M sodium chloride in 0.28% ammonia; 0-100% B in 60 min) was used to elute the methoxytrityl containing peak. The combined fractions were pooled and further processed depending on the desired product.

Demethoxytritylation of morpholino-based oligomers: The pooled fractions from the Macroprep purification were treated with 1 M H3PO4 to lower the pH to 2.5. After initial mixing, the samples sat at room temperature for 4 min, at which time they are neutralized to pH 10-11 with 2.8% ammonia/water. The products were purified by solid phase extraction (SPE).

SPE column packing and conditioning: Amberchrome CG-300M (Rohm and Haas; Philadelphia, PA) (3 mL) is packed into 20 mL fritted columns (BioRad Econo-Pac Chromatography Columns (732-1011)) and the resin rinsed with 3 mL of the following: 0.28% NH4OH/80% acetonitrile; 0.5M NaOH/20% ethanol; water; 50 mM H3PO4/80% acetonitrile; water; 0.5 NaOH/20% ethanol; water; 0.28% NH4OH.

SPE purification: The solution from the demethoxytritylation was loaded onto the column and the resin rinsed three times with 3-6 mL 0.28% aqueous ammonia. A Wheaton vial (12 mL) was placed under the column and the product eluted by two washes with 2 mL of 45% acetonitrile in 0.28% aqueous ammonia.

Product isolation: The solutions were frozen in dry ice and the vials placed in a freeze dryer to produce a fluffy white powder. The samples were dissolved in water, filtered through a 0.22 micron filter (Pall Life Sciences, Acrodisc 25 mm syringe filter, with a 0.2 micron HT Tuffryn membrane) using a syringe and the Optical Density (OD) was measured on a UV spectrophotometer to determine the OD units of oligomer present, as well as dispense sample for analysis. The solutions were then placed back in Wheaton vials for lyophilization.

Analysis of morpholino-based oligomers by MALDI: MALDI-TOF mass spectrometry was used to determine the composition of fractions in purifications as well as provide evidence for identity (molecular weight) of the oligomers. Samples were run following dilution with solution of 3,5-dimethoxy-4-hydroxycinnamic acid (sinapinic acid), 3,4,5-trihydoxyacetophenone (THAP) or alpha-cyano-4-hydoxycinnamic acid (HCCA) as matrices.

### Example 24 In Vivo Screening of PMO Myostatin Sequences

PMO sequences designed to skip myostatin exon 2 were screened. The efficacy of the PMO sequences was tested *in vitro* in both human Rhabdomyosarcoma (RD) and murine myoblast (normal - C2C12 and dystrophic - H2Kbmdx) cells. Four human-specific PMOs targeting the 5' end of myostatin exon 2 were subsequently screened in RD cells.

PMOs were transfected by Nucleofection (Neon transfection system, Life technologies, Carlsbad, CA) following the manufacturer's standard protocol. Skipping efficiency of PMOs was evaluated by semi-quantitative RT-PCRs following densitometric analysis of gel electrophoresis results of RT-PCR products as a percentage of the density of skipped products against the total density of skipped and unskipped products. The sequences are listed in Table 7.

Sequences PMO 39, SEQ ID NO: 48, PMO 42, SEQ ID NO: 16, PMO 43, SEQ ID NO: 49, PMO 44, SEQ ID NO: 17, PMO 45, SEQ ID NO: 18, and PMO 124 were designed to bind both murine and human myostatin exon 2. PMOs were tested in triplicate at 4 doses (0.25, 0.5, 1, 2 µM). Myostatin exon 2 skipping efficiency was evaluated by RT-PCR **(****FIG.** 3A) and densitometric analysis of the RT-PCR products as described above as a percentage of the intensity of skipped products against the total intensity of skipped and unskipped products. Statistical analysis was performed by one-way ANOVA for individual dose comparing the efficiency of the PMOs with that of PMO 28, (synthesized by GeneTools) which was demonstrated as an effective PMO to skip myostatin exon 2 **(****FIG. 3B**).

Four (4) human specific PMOs (PMO 40, PMO 46, SEQ ID NO: 21, PMO 47, SEQ ID NO: 20, PMO 48, SEQ ID NO: 19) were analysed that were all designed to bind the 5' end of human myostatin exon 2. PMOs were tested in triplicate at 1 µM dose and compared with the PMOs targeting the 3' end at the same concentration. As these PMOs were expected to induce exon 2 skipping, their efficacy was assessed by the established RT-PCR protocol following a densitometric analysis as mentioned above. Results of the RT-PCT products are shown in **FIG. 4A** and the densitometric analysis is shown in **FIG. 4B****.**

Among the PMOs targeting the 3' end of myostatin exon 2, PMOs 44, SEQ ID NO: 17 and 45, SEQ ID NO: 18 (and, at higher concentration, PMO 39, SEQ ID NO: 48) induced more consistent skipping than others, particularly at lower concentrations **(****FIG. 4B**). The skipping efficacy was even higher when PMOs targeting 5' end of myostatin exon 2 were used, with PMO 46, SEQ ID NO: 21 inducing nearly 100% skipping and PMOs 40 and 48, SEQ ID NO: 19 inducing about 80% skipping **(****FIG. 4B**).

### Screening of PMOs for skipping exon 2 of mouse myostatin: a dose-response study for PMOs 39, 42, 43, 44, 45, 124 in C2C12 and H2Kbmdxcells

The first example of specific and reproducible exon skipping in the mdx mouse model was reported by Wilton *et al.* (Wilton, Lloyd *et al.* 1999; the contents of which are hereby incorporated by reference in its entirety). By directing an antisense molecule to the donor splice site, consistent and efficient exon 23 skipping was induced in the dystrophin mRNA within 6 hours of treatment of the cultured cells. Wilton *et al.* also describe targeting the acceptor region of the mouse dystrophin pre-mRNA with longer antisense oligonucleotides. While the first antisense oligonucleotide directed at the intron 23 donor splice site induced consistent exon skipping in primary cultured myoblasts, this compound was found to be much less efficient in immortalized cell cultures expressing higher levels of dystrophin. However, with refined targeting and antisense oligonucleotide design, the efficiency of specific exon removal was increased by almost an order of magnitude (Mann, Honeyman *et al.* 2002; the contents of which are hereby incorporated by reference in its entirety).

PMOs were initially tested in quadruplicate at doses of 0.25, 0.5, 1, 2, 5 µM in mouse myoblast C2C12 cells (FIG. 5A and FIG. 5C). Variable skipping was observed in replicates with the PMO sequences and the 0.5 and 2 µM doses used. The screening was alternatively performed in H2Kbmdx cells, a myoblast dystrophic cell model, demonstrating more consistent and reliable results **(****FIG. 5A** and **FIG. 5B****).**

In tested H2Kbmdx cell cultures, PMO 28 was the best PMO at the high concentration and one of the most efficient PMOs at the low concentration (as comparable as PMOs 45, SEQ ID NO: 18 and 39, SEQ ID NO: 48) (**FIG. 5B**)**.**

### Preliminary in vivo screening of unconjugated PMO-MSTN sequences in mdx mice

Based on the *in vitro* results, PMOs 39, SEQ ID NO: 48, 44, SEQ ID NO: 17, and 45, SEQ ID NO: 18 were selected for this study. PMO 124 was used as a control. An optimal dose of 3 nmoles (equal to 18 x 10¹⁴ molecules) of PMO 124 were injected into each Tibialis anterior (TA) muscle of 8 week-old mdx mice. The amounts of the other PMOs were normalised to the same number of molecules of PMO 124 injected. Both TA muscles of 2 mice were injected with each PMO (n=4 per group) in a final volume of 25 µl (diluted in saline). Muscles were harvested 2 weeks after the injection. The results are illustrated in **FIG. 6A****,** **FIG. 6B** and **FIG. 6C****.**

Calculation of PMO doses:
a) PMO 39, SEQ ID NO: 48 (18mer) = 18.5 µg (2 mice, 4 TAs)
b) PMO 44, SEQ ID NO: 17 (25mer) = 25.4 µg (2 mice, 4 TAs)
c) PMO 45, SEQ ID NO: 18 (25mer) = 25.5 µg (2 mice, 4 TAs)
d) PMO 124 (28mer) = 28.8 µg (2 mice, 4 TAs)

These amounts in µg correspond to 18 x 10¹⁴ molecules per each PMO.

All PMOs tested were biologically active *in vivo.* The skipping efficiency was highest and lowest in PMO 124 and 45, SEQ ID NO: 18 treated muscles, respectively (FIG. 6C). However, such efficiencies did not correlate with an increase in muscle weight. Muscles treated with PMO 45, SEQ ID NO: 18 were heavier than untreated or treated muscles with PMO 124 or 44, SEQ ID NO: 17 although the differences were not significant **(****FIG. 6B****).**

### Systemic injection of PMOs in mdx mice

PMOs 39, SEQ ID NO: 48, 44, SEQ ID NO: 17, 45, SEQ ID NO: 18, and 124 were also examined for systemic skipping efficacy. The screening was performed through tail vein intravenous injection in 8 week-old mdx mice. PMO 124 was used as a control and at the dose of 200 mg/kg (equal to 12.53 x 10¹⁸ molecules or 20.8 µmoles) diluted in 200 µl saline. The amount of the other PMOs was normalized to the number of molecules of PMO 124 injected. Three mice per group were used. Muscles were harvested 2 weeks after the injection, including the diaphragm - DIA, the extensor digitorum longus - EDL, the gastrocnemius - GAS, the soleus - SOL, and the tibialis anterior - TA. Results are illustrated in FIG. 7A, FIG. 7B, FIG. 7C and FIG. 7D.

Calculation of PMO doses:
a) PMO 124 (28mer) = 200mg/kg (3 mice)
b) PMO 45, SEQ ID NO: 18 (25mer) = 176.2 µg (3 mice)
c) PMO 44, SEQ ID NO: 17 (25mer) = 176.8 µg (3 mice)
d) PMO 39, SEQ ID NO: 48 (28mer) = 128.6 µg (3 mice)

These amounts in µg correspond to 12.53 x 10¹⁸ molecules (20.8 µmoles) per each PMO.

The skipping results were variable among muscles collected from a single mouse and among the same types of muscles from different mice (FIG. 7A and FIG. 7B). However, all PMOs were biologically active in dystrophic muscles after a single IV injection. GAS and TA showed a trend of increase in weight (normalised to final body weight; FIG. 7D) after being injected with PMO 45, SEQ ID NO: 18 or 124, compared to type-matched muscles of saline-injected mice.

### In vivo screening of B peptide-conjugated PMOs in C57 mice

PMO D30 (SEQ ID NO: 16), PMO39 (SEQ ID NO: 48) and PMO45 (SEQ ID NO: 18) selected from previous *in vitro* and *in vivo* screening were conjugated to B peptide (RAhxRRBRRAhxRRBRAhxB; SEQ ID No: 3499 and AhxB linker moiety at the peptide carboxy terminus) at the 3'-end of the PMO and delivered by systemic tail vein injection, weekly, for 14 weeks. B peptide conjugated PMO, also referred to hereafter as BPMO, was performed in 12-week old C57 mice, 10 mice per group. Two doses were tested at 10 or 20 mg/kg. After the last injection, the force of forelimbs was measured by gripstrength test (FIG. 9A and FIG. 9B). The maximal force of TA muscles of mice treated with 10 mg/kg BPMOs were measured by *in situ* electrophysiology (FIG. 9C). The heart, DIA and 4 skeletal muscles (EDL, GAS, SOL, TA) were harvested for assessment of muscle mass and myostatin exon skipping.

Some of the mice in the BPMO-39 and BPMO-D30 treated groups at 20 mg/kg did not receive IV injection during the last 4-6 weeks as the tail vein was hardly visible. These mice were injected by IP instead. In BPMO-39, 20 mg/kg treated group, two mice died during the study.

### Results:

1) Increase in body and muscle mass: the body weight of mice in the BPMO-39 treated group (10 or 20 mg/kg) was significantly increased compared with the weight of both saline and scramble BPMO injected animals (FIG. 8A and FIG. 8C). BPMO-D30 induced a very efficient bodyweight increase when used at 20 mg/kg (**FIG. 8C**). Variability in muscle mass (normalized against the initial body weight) was observed depending on the dosage administered and muscle considered (FIG. 8B and FIG. 8D). BPMO-39 showed the most consistent muscle increase in TA (10 or 20 mg/kg treatment; FIG. 8B and FIG. 8D) and GAS (10 mg/kg treatment; FIG. 8B) while BPMO-D30 or -45 induced mass increase in TA (10 mg/kg treatment; FIG. 8B) or GAS (20 mg/kg treatment; FIG. 8D). In the DIA of 20 mg/kg treated mice all of the tested BPMOs induced a significant muscle weight gain (FIG. 8D). The IP delivery route used in the last few injections may have had an influence on this result.
2) Gripstrength analysis: Measurement of the forelimb force was performed in mice treated with both BPMO dosages (FIG. 9A and FIG. 9B). BPMO-39 was the only candidate showing enhanced muscle strength compared with saline group, and only at 10 mg/kg treatment (FIG. 9A). The scramble BPMO unexpectedly and unexplainably increased the forelimb strength of treated mice significantly different compared to the saline treated mice at both 10 and 20 mg/kg doses (FIG. 9A and FIG. 9B).
3) *In situ* muscle physiology test: The TAs of mice treated with 10 mg/kg BPMOs were analyzed using an electrophysiology assessment. BPMO-D30 significantly increased the generated maximal and specific forces compared to the scramble PMO and the other tested BPMOs (FIG. 9C).
4) Exon skipping quantification: The myostatin skipping efficiency of DIA (FIG. 10A and FIG. **10B**) and TA (FIG. **10C** and FIG. **10D**) muscles was analyzed. The skipping levels in 20 mg/kg treated muscles were 3-4 fold higher than the levels in 10 mg/kg treated muscles (FIG. 10B and FIG. 10D). BPMO-D30 and -45 were significantly more efficient than BPMO-39 at 20 mg/kg dose (DIA, FIG. **10B** and TA, FIG. **10D**) or 10 mg/kg dose (TA, FIG. **10D**) used.

Provisional results of *in vivo* screening: BPMO-D30 and BPMO-45 were the most effective molecules taking in account the general effect on muscle weight, strength and exon skipping efficiency. Histological analysis will be performed (as possible data on the cross sectional analysis of myofibres).

### Example 25 BPMO-induced dual exon skipping: Combination Myostatin and Dystrophin Treatment

### Rescue of dystrophin reading frame + knockdown of myostatin in young dystrophic mice.

PMO M23D (SEQ ID NO. 937) was conjugated to B peptide (RAhxRRBRRAhxRRBRAhxB; SEQ ID No: 3499 and AhxB linker moiety at the peptide carboxy terminus) at the 3'-end of the PMO and was named BPMO-M23D. BPMO-M23D (10mg/kg) and/or BPMO-MSTN (D30, 10 mg/kg) were diluted in 200 µl saline and injected through the tail vein of 6 week-old mdx mice or C57BL10 mice. The injection was repeated weekly for 10 weeks. Ten mice were used for each treatment. Details of 5 groups of mice as follow:
a) C57BL10 mice + Saline (positive control)
b) Mdx mice + Saline (negative control)
c) Mdx mice + BPMO-M23D, SEQ ID NO: 937 (10 mg/kg)
d) Mdx mice + BPMO-M23D, SEQ ID NO: 937 (10 mg/kg) & BPMO-MSTN, SEQ ID NO: 16 (10 mg/kg)
e) Mdx mice + BPMO-MSTN, SEQ ID NO: 16 (10 mg/kg)

### Results:

After 12 weeks of treatment no significant increase in bodyweight was observed in treated mdx mice compared with saline injected animals (**FIG. 11A**). The grip strength analysis measuring the forelimb force revealed that injection of BPMO-M23D induced a significant increase in force compared to that of mdx mice while co-injection of BPMO-M23D and BPMO-MSTN normalized the strength to that of C57 mice (**FIG. 11B**). BPMO-MSTN treatment alone did not modify the muscle strength of treated mice compared to saline injected mdx mice (FIG. 11B). The grip strength test reported above was further conducted as follows. The tests were performed in 3 consecutive days (following activity cage assessment). In each test, the values of 5 reads/mouse were recorded. Each was the highest value of the forelimb force measured within 30 sec, with 30 sec interval between 2 reads. Data are shown as a total of 15 reads/mouse (FIG. 18A) or as 3 x average of 5 reads/mouse/test (FIG. 18B), or as 3 x highest value of 5 reads/mouse/test (FIG. 18C). Statistical analysis was by one-way ANOVA & Bonferroni post-hoc test (n = 10 per group); error bars represent the S.E.M.

BPMO-M23D treatment alone or in combination with BPMO-MSTN induced a very efficient dystrophin exon skipping achieving 70-80% of dystrophin reframing in all muscles analysed, with an exception of about 25% skipping in the heart (FIG. 12A). Treatment with BPMO-M23D in combination with BPMO-MSTN resulted in greater DMD exon skipping efficiency than treatment with the BPMO-M23D alone. Restoration of dystrophin protein was subsequently confirmed by Western blot analysis, with expression in skeletal muscles ranging between 30-100% the level of C57 mice **(****FIG. 12B****).** Dystrophin expression was reconfirmed by immunohistochemistry. Myostatin exon 2 skipping was also efficient in mice that had received the dual treatment, with average skipping in all examined muscles about 55% **(****FIG. 12C****).**

Further histological analyses were performed in the harvested muscles to study the effect of the treatments on myofibre hypertrophy and regeneration. The number and diameter of myofibres were investigated in addition with the frequency of centrally nucleated fibres. The therapeutic benefit in EDL, GAS, SOL and TA muscles has been assessed. Results from TA fibre analysis are reported as representative **(****FIG. 13A** and **FIG. 13B**). The treatment with BPMO-MSTN alone did not modify the dystrophic phenotype whereas BPMO-M23D and BPMO-M23D + BPMO-D30 treatments partially ameliorated the pathology with a decrease in the variability of myofibre cross sectional area **(****FIG. 13A****)** and in the presence of centrally nucleated fibres compared to untreated mdx muscles **(****FIG. 13B****).** This effect was essentially due to the dystrophin restoration that reduced both the pseudo-hypertrophy of mdx muscles and the muscle degeneration process.

### Rescue of dystrophin reading frame + knockdown of myostatin in aged mdx mice

BPMO-MSTN and BPMO-M23D was injected using identical dose regimen and route of administration as reported above) in aged (>18 month old) mdx mice that recapitulate more accurately (compared to young mdx mice) the dystrophic disease observed in human. Mice were injected weekly for 10 weeks with either 10 mg/kg of BPMO-M23D (n = 5) or BPMOM23D (10 mg/kg) and BPMO-MSTN (10 mg/kg) (n = 5), or 20 mg/kg of scramble BPMO (n = 4). One week after the last injection, mice underwent grip strength analyses to investigate the forelimb strength. TA muscles of treated mice were analysed by electrophysiology on the following week, prior to muscle collection.

### Results:

Significant changes in bodyweight of treated mice (compared with scramble group) from week 7 were observed **(****FIG. 14A****).** The muscle mass was tested in DIA, EDL, GAS, SOL, TA and heart muscles in mice treated with scramble, BPMO-M23D, and BPMO-M23D and BPMO-MSTN **(****FIG. 14B****).** However, statistical analysis for body or muscle weight was not performed as scramble-injected mice died gradually and only 1 mouse survived at the end of the study. All of the mice treated with BPMO-M23D or with BPMO-M23D and BPMO-MSTN survived for the entire study. Gripstrength analysis demonstrated that mice treated with BPMO-M23D or with BPMO-M23D and BPMO-MSTN were stronger than mice treated with scramble BPMO (FIG. 14C). Further comparison of the maximal and specific force of TA muscles between the single and dual treatments displayed a significant improvement in resistance force against muscle-damaged lengthening contractions (eccentric contractions), with better effect seen in the combined treated group **(****FIG. 14D****).**

RT-PCRs were subsequently performed to evaluate the skipping efficiency of exon 23 of dystrophin that showed substantial levels of dystrophin reframing in all muscles **(****FIG. 15A****).** The addition of BPMO-MSTN increased the skipping efficiency of BPMO-M23D consistently as observed in young mdx mice **(****FIG. 15B****).** Results of dystrophin exon skipping correlated with a significant increase in protein expression in all muscles analysed **(****FIG. 16A****).** Further, it was shown that treatment with the combination of BPMO-MSTN and BPMO-M23D increased dystrophin levels over treatment with M23D alone **(****FIG. 16B****).**

Myostatin exon 2 skipping in all muscles harvested was evaluated by RT-PCR **(****FIG. 17A****).** The level of skipping varied between 5% and 40% depending on the muscle type analyzed. The average value obtained pulling together the results of all the muscles was about 20% **(****FIG. 17B****).**

It is believed that the disclosure set forth above encompasses at least one distinct invention with independent utility. While the invention has been disclosed in the exemplary forms, the specific embodiments thereof as disclosed and illustrated herein are not to be considered in a limiting sense as numerous variations are possible. Equivalent changes, modifications and variations of various embodiments, materials, compositions and methods may be made within the scope of the present invention, with substantially similar results. The subject matter of the inventions includes all novel and non-obvious combinations and subcombinations of the various elements, features, functions and/or properties disclosed herein.

Benefits, other advantages, and solutions to problems have been described herein with regard to specific embodiments. However, the benefits, advantages, solutions to problems, and any element or combination of elements that may cause any benefit, advantage, or solution to occur or become more pronounced are not to be construed as critical, required, or essential features or elements of any or all the claims of the invention. Many changes and modifications within the scope of the instant invention includes all such modifications. Corresponding structures, materials, acts, and equivalents of all elements in the claims below are intended to include any structure, material, or acts performing the functions in combination with other claim elements as specifically claimed. The scope of the invention should be determined by the appended claims and their legal equivalents, rather than by the examples given above.

**Table 7. Sequence Listing**

| SEQ ID NO: | SEQUENCE |
|---|---|
| 1 | agcaacttttcttttcttattcatttatagctgattttctaatgcaagtggatggaaaacccaaatgttgctt ctttaaatttagctctaaaatacaatacaataaagtagtaaaggcccaactatggatatatttgagaccc gtcgagactcctacaacagtgtttgtgcaaatcctgagactcatcaaacctatgaaagacggtacaag |
| | gtatactggaatccgatctctgaaacttgacatgaacccaggcactggtatttggcagagcattgatgt gaagacagtgttgcaaaattggctcaaacaacctgaatccaacttaggcattgaaataaaagctttagat gagaatggtcatgatcttgctgtaaccttcccaggaccaggagaagatgggctggtaagtgataactga aaataacattataat |
| 2 | cttttcttttcttattcatttatagctgattttctaatgcaagtggatgg |
| 3 | accttcccaggaccaggagaagatgggctg/gtaagtgataactgaaaataacattataat |
| 4 | gccagacctatttgactggaatagtgtggtttgccagcagtcagccacacaacgactggaac atgcattcaacatcgccagatatcaattaggcatagagaaactactcgatcctgaag |
| 5 | atgttgataccacctatccagataagaagtccatcttaatgtacatcacatcactcttccaagtttt gcctcaacaagtgagcattgaagccatccaggaagtggaaatgttgccaaggccacctaaag tgactaaagaagaacattttcagttacatcatcaaatgcactattctcaacag |
| 6 | atcacggtcagtctagcacagggatatgagagaacttcttcccctaagcctcgattcaagagct atgcctacacacaggctgcttatgtcaccacctctgaccctacacggagcccatttccttcacag |
| 7 | gccatagagcgagaaaaagctgagaagttcagaaaactgcaagatgccagcagatcagctca ggccctggtggaacagatggtgaatg |
| 8 | gctttacaaagttctctgcaagagcaacaaagtggcctatactatctcagcaccactgtgaaaga gatgtcgaagaaagcgccctctgaaattagccggaaatatcaatcagaatttgaagaaattgag ggacgctggaagaagctctcctcccagctggttgagcattgtcaaaagctagaggagcaaatga ataaactccgaaaaattcag |
| 9 | gcgatttgacagatctgttgagaaatggcggcgttttcattatgatataaagatatttaatcagtggct aacagaagctgaacagtttctcagaaagacacaaattcctgagaattgggaacatgctaaatacaa atggtatcttaag |
| 10 | gaactccaggatggcattgggcagcggcaaactgttgtcagaacattgaatgcaactggggaaga aataattcagcaatcctcaaaaacagatgccagtattctacaggaaaaattgggaagcctgaatctg cggtggcaggaggtctgcaaacagctgtcagacagaaaaaagag |
| 11 | aggaagttagaagatctgagctctgagtggaaggcggtaaaccgtttacttcaagagctgagggca aagcagcctgacctagctcctggactgaccactattggagcct |
| 12 | ctcctactcagactgttactctggtgacacaacctgtggttactaaggaaactgccatctccaaactag aaatgccatcttccttgatgttggaggtacctgctctggcagatttcaaccgggcttggacagaactta ccgactggctttctctgcttgatcaagttataaaatcacagagggtgatggtgggtgaccttgaggata tcaacgagatgatcatcaagcagaag |
| 13 | gcaacaatgcaggatttggaacagaggcgtccccagttggaagaactcattaccgctgcccaaaattt gaaaaacaagaccagcaatcaagaggctagaacaatcattacggatcgaa |
| 14 | ttgaaagaattcagaatcagtgggatgaagtacaagaacaccttcagaaccggaggcaacagttgaat gaaatgttaaaggattcaacacaatggctggaagctaaggaagaagctgagcaggtcttaggacagg ccagagccaagcttgagtcatggaaggagggtccctatacagtagatgcaatccaaaagaaaatcaca gaaaccaag |
| 15 | ggtgagtgagcgagaggctgctttggaagaaactcatagattactgcaacagttccccctggacctgga aaagtttcttgcctggcttacagaagctgaaacaactgccaatgtcctacaggatgctacccgtaaggaaa ggctcctagaagactccaagggagtaaaagagctgatgaaacaatggcaa |
| 16 | cagcccatcttctcctggtcctgggaaggt |
| 17 | ccagcccatcttctcctggtcctgg |
| 18 | cacttaccagcccatcttctcctgg |
| 19 | ccatccgcttgcattagaaagtcagc |
| 20 | gcattagaaaatcagctataaatg |
| 21 | ccacttgcattagaaaatcagc |
| 22 | cttgcattagaaaatcagctataaa |
| 23 | cacttgcattagaaaatcagctata |
| 24 | ccacttgcattagaaaatcagctat |
| 25 | tccacttgcattagaaaatcagcta |
| 26 | atccacttgcattagaaaatcagct |
| 27 | catccacttgcattagaaaatcagc |
| 28 | ttattttcagttatcacttaccagc |
| 29 | ttttcagttatcacttaccagccca |
| 30 | tcagttatcacttaccagcccatct |
| 31 | gttatcacttaccagcccatcttct |
| 32 | atcacttaccagcccatcttctcct |
| 33 | acttaccagcccatcttctcctggt |
| 34 | taccagcccatcttctcctggtcct |
| 35 | atgttattttcagttatcacttacc |
| 36 | tgttattttcagttatcacttacca |
| 37 | gttattttcagttatcacttaccag |
| 38 | tattttcagttatcacttaccagcc |
| 39 | attttcagttatcacttaccagccc |
| 40 | tttcagttatcacttaccagcccat |
| 41 | ttcagttatcacttaccagcccatc |
| 42 | cagttatcacttaccagcccatctt |
| 43 | agttatcacttaccagcccatcttc |
| 44 | cagcccatcttctcctggtcctgggaaggt |
| 45 | cagcccatcttctcctggtc |
| 46 | tctcctggtcctgggaaggt |
| 47 | ctgggaaggttacagcaaga |
| 48 | cagcccatcttctcctgg |
| 49 | gcccatcttctcctggtcctggg |
| 50 | tttaaagaagcaacatttgggtttt |
| 51 | tattttagagctaaatttaaagaag |
| 52 | tactttattgtattgtattttagag |
| 53 | tagttgggcctttactactttattg |
| 54 | tctcaaatatatccatagttgggcc |
| 55 | acgggtctcaaatatatccatagtt |
| 56 | gttgtaggagtctcgacgggtctcaaatat |
| 57 | acactgttgtaggagtctcgacggg |
| 58 | taggtttgatgagtctcaggatttg |
| 59 | ccgtctttcataggtttgatgagtc |
| 60 | cagtataccttgtaccgtctttcataggtt |
| 61 | gggttcatgtcaagtttcagagatc |
| 62 | aataccagtgcctgggttcatgtcaagttt |
| 63 | aaataccagtgcctgggttcatgtc |
| 64 | tctgccaaataccagtgcctgggtt |
| 65 | tcttcacatcaatgctctgccaaat |
| 66 | caggttgtttgagccaattttgcaa |
| 67 | tgcctaagttggattcaggttgttt |
| 68 | aagcttttatttcaatgcctaagtt |
| 69 | gaccattctcatctaaagcttttat |
| 70 | ttacagcaagatcatgaccattctc |
| 71 | yyagyyyaxyxxyxyyxggxyyxgg |
| 72 | yayxxayyagyyyaxyxxyxyyxgg |
| 73 | yyayxxgyaxxagaaaaxyagy |
| 74 | gyattagaaaatyagytataaatg |
| 75 | yyatyygyttgyattagaaagtyagy |
| 76 | ctccaacatcaaggaagatggcatttctag |
| 77 | ctccaacatc aaggaagatg gcatttctag |
| 78 | acaucaagga agauggcauu ucuag |
| 79 | acaucaagga agauggcauu ucuaguuugg |
| 80 | gagcaggtac ctccaacatc aaggaa |
| 81 | gggauccagu auacuuacag gcucc |
| 82 | cttacaggct ccaatagtgg tcagt |
| 83 | cctccggttc tgaaggtgtt cttgtac |
| 84 | gttgcctccg gttctgaagg tgttc |
| 85 | caatgccatc ctggagttcc tg |
| 86 | gauagguggu aucaacaucu guaa |
| 87 | gauagguggu aucaacaucu g |
| 88 | gauagguggu aucaacaucu guaag |
| 89 | ggugguauca acaucuguaa |
| 90 | guaucaacau cuguaagcac |
| 91 | ugcauguucc agucguugug ugg |
| 92 | cacuauucca gucaaauagg ucugg |
| 93 | auuuaccaac cuucaggauc gagua |
| 94 | ggccuaaaac acauacacau a |
| 95 | cauuuuugac cuacaugugg |
| 96 | uuugaccuac auguggaaag |
| 97 | uacauuuuug accuacaugu ggaaag |
| 98 | auuuuugacc uacaugggaa ag |
| 99 | uacgaguuga uugucggacc cag |
| 100 | guggucuccu uaccuaugac ugugg |
| 101 | ggucuccuua ccuauga |
| 102 | ugucucagua aucuucuuac cuau |
| 103 | ucuuaccuau gacuauggau gaga |
| 104 | gcaugaacuc uuguggaucc |
| 105 | ccaggguacu acuuacauua |
| 106 | aucguguguc acagcauccag |
| 107 | uguucagggc augaacucuu guggauccuu |
| 108 | uaggaggcgc cucccauccu guaggucacu g |
| 109 | aggucuagga ggcgccuccc auccuguagg u |
| 110 | gcgccuccca uccuguaggu cacug |
| 111 | cuucgaggag gucuaggagg cgccuc |
| 112 | cucccauccu guaggucacu g |
| 113 | uaccaguuuu ugcccuguca gg |
| 114 | ucaauaugcu gcuucccaaa cugaaa |
| 115 | cuaggaggcg ccucccaucc uguag |
| 116 | uuaugauuuc caucuacgau gucaguacuu c |
| 117 | cuuaccugcc aguggaggau uauauuccaa a |
| 118 | caucaggauu cuuaccugcc agugg |
| 119 | cgaugucagu acuuccaaua uucac |
| 120 | accauucauc aggauucu |
| 121 | accugccagu ggaggauu |
| 122 | ccaauauuca cuaaaucaac cuguuaa |
| 123 | caggauuguu accugccagu ggaggauuau |
| 124 | acgaugucag uacuuccaau auucacuaaa u |
| 125 | auuuccaucu acgaugucag uacuuccaau a |
| 126 | caggagcuuc caaaugcugc a |
| 127 | cuugucuuca ggagcuucca aaugcugca |
| 128 | uccucagcag aaagaagcca cg |
| 129 | uuagaaaucu cuccuugugc |
| 130 | uaaauugggu guuacacaau |
| 131 | cccugaggca uucccaucuu gaau |
| 132 | aggacuuacu ugcuuuguuu |
| 133 | cuugaauuua ggagauucau cug |
| 134 | caucuucuga uaauuuuccu guu |
| 135 | ucuucuguuu uuguuagcca guca |
| 136 | ucuauguaaa cugaaaauuu |
| 137 | uucuggagau ccauuaaaac |
| 138 | cagcaguugc gugaucucca cuag |
| 139 | uucaucaacu accaccacca u |
| 140 | cuaagcaaaa uaaucugacc uuaag |
| 141 | cuuguaaaag aacccagcgg ucuucugu |
| 142 | caucuacaga uguuugccca uc |
| 143 | gaaggauguc uuguaaaaga acc |
| 144 | accuguucuu caguaagacg |
| 145 | caugacacac cuguucuuca guaa |
| 146 | cauuugagaa ggaugucuug |
| 147 | aucucccaau accuggagaa gaga |
| 148 | gccaugcacu aaaaaggcac ugcaagacau u |
| 149 | ucuuuaaagc caguugugug aauc |
| 150 | uuucugaaag ccaugcacua a |
| 151 | guacauacgg ccaguuuuug aagac |
| 152 | cuagauccgc uuuuaaaacc uguuaaaaca a |
| 153 | ucuuuucuag auccgcuuuu aaaaccuguu a |
| 154 | cuagauccgc uuuuaaaacc uguua |
| 155 | ccgucuucug ggucacugac uua |
| 156 | cuagauccgc uuuuaaaacc uguuaa |
| 157 | ccgcuuuuaa aaccuguuaa |
| 158 | uggauugcuu uuucuuuucu agaucc |
| 159 | caugcuuccg ucuucugggu cacug |
| 160 | gaucuuguuu gagugaauac agu |
| 161 | guuauccagc caugcuuccg uc |
| 162 | ugauaauugg uaucacuaac cugug |
| 163 | guaucacuaa ccugugcugu ac |
| 164 | cugcuggcau cuugcaguu |
| 165 | gccugagcug aucugcuggc aucuugcagu u |
| 166 | cuggcagaau ucgauccacc ggcuguuc |
| 167 | cagcaguagu ugucaucugc uc |
| 168 | ugauggggug guggguugg |
| 169 | aucugcauua acacccucua gaaag |
| 170 | ccggcuguuc aguuguucug aggc |
| 171 | aucugcauua acacccucua gaaagaaa |
| 172 | gaaggagaag agauucuuac cuuacaaa |
| 173 | auucgaucca ccggcuguuc |
| 174 | cagcaguagu ugucaucugc |
| 175 | gccgguugac uucauccugu gc |
| 176 | cugcauccag gaacaugggu cc |
| 177 | gucugcaucc aggaacaugg guc |
| 178 | guugaagauc ugauagccgg uuga |
| 179 | uacuuacugu cuguagcucu uucu |
| 180 | cacucauggu cuccugauag cgca |
| 181 | cugcaauucc ccgagucucu gc |
| 182 | acugcuggac ccauguccug aug |
| 183 | cuaaguugag guauggagag u |
| 184 | uauucacaga ccugcaauuc ccc |
| 185 | acaguggugc ugagauagua uaggcc |
| 186 | uaggccacuu uguugcucuu gc |
| 187 | uucagagggc gcuuucuuc |
| 188 | gggcaggcca uuccuccuuc aga |
| 189 | ucuucagggu uuguauguga uucu |
| 190 | cugggcugaa uugucugaau aucacug |
| 191 | cuguuggcac augugauccc acugag |
| 192 | gucuauaccu guuggcacau guga |
| 193 | ugcuuucugu aauucaucug gaguu |
| 194 | ccuccuuucu ggcauagacc uuccac |
| 195 | ugugucaucc auucgugcau cucug |
| 196 | uuaaggccuc uugugcuaca ggugg |
| 197 | ggggcucuuc uuuagcucuc uga |
| 198 | gacuuccaaa gucuugcauu uc |
| 199 | gccaacaugc ccaaacuucc uaag |
| 200 | cagagauuuc cucagcuccg ccagga |
| 201 | cuuacaucua gcaccucaga g |
| 202 | uccgccaucu guuagggucu gugcc |
| 203 | auuuggguua uccucugaau gucgc |
| 204 | cauaccucuu cauguaguuc cc |
| 205 | cauuugagcu gcguccaccu ugucug |
| 206 | uccugggcag acuggaugcu cuguuc |
| 207 | uugccugggc uuccugaggc auu |
| 208 | uucugaaaua acauauaccu gugc |
| 209 | uaguuucuga aauaacauau accug |
| 210 | gacuugucaa aucagauugg a |
| 211 | guuucugaaa uaacauauac cugu |
| 212 | caccagaaau acauaccaca |
| 213 | caaugauuua gcugugacug |
| 214 | cgaaacuuca uggagacauc uug |
| 215 | cuuguagacg cugcucaaaa uuggc |
| 216 | caugcacaca ccuuugcucc |
| 217 | ucuguacaau cugacgucca gucu |
| 218 | gucuuuauca ccauuuccac uucagac |
| 219 | ccgucugcuu uuucuguaca aucug |
| 220 | uccauaucug uagcugccag cc |
| 221 | ccaggcaacu ucagaaucca aau |
| 222 | uuucuguuac cugaaaagaa uuauaaugaa |
| 223 | cauucauuuc cuuucgcauc uuacg |
| 224 | ugaucucuuu gucaauucca uaucug |
| 225 | uucagugaua uagguuuuac cuuuccccag |
| 226 | cuguagcugc cagccauucu gucaag |
| 227 | ucuucugcuc gggaggugac a |
| 228 | ccaguuacua uucagaagac |
| 229 | ucuucaggug caccuucugu |
| 230 | ugugaugugg uccacauucu gguca |
| 231 | ccauguguuu cugguauucc |
| 232 | cguguagagu ccaccuuugg gcgua |
| 233 | uacuaauuuc cugcaguggu cacc |
| 234 | uucuguguga aauggcugca aauc |
| 235 | ccuucaaagg aauggaggcc |
| 236 | ugcugaauuu cagccuccag ugguu |
| 237 | ugaagucuuc cucuuucaga uucac |
| 238 | cuggcuuucu cucaucugug auuc |
| 239 | guuguaaguu gucuccucuu |
| 240 | uugucuguaa cagcugcugu |
| 241 | gcucuaauac cuugagagca |
| 242 | cuuugagacc ucaaauccug uu |
| 243 | cuuuauuuuc cuuucaucuc ugggc |
| 244 | aucguuucuu cacggacagu gugcugg |
| 245 | gggcuuguga gacaugagug auuu |
| 246 | accuucagag gacuccucuu gc |
| 247 | uauguguuac cuacccuugu cgguc |
| 248 | ggagagagcu uccuguagcu |
| 249 | ucacccuuuc cacaggcguu gca |
| 250 | uuugugucuu ucugagaaac |
| 251 | aaagacuuac cuuaagauac |
| 252 | aucugucaaa ucgccugcag |
| 253 | uuaccuugac uugcucaagc |
| 254 | uccagguuca agugggauac |
| 255 | gcucuucugg gcuuauggga gcacu |
| 256 | accuuuaucc acuggagauu ugucugc |
| 257 | uuccaccagu aacugaaaca g |
| 258 | ccacucagag cucagaucuu cuaacuucc |
| 259 | cuuccacuca gagcucagau cuucuaa |
| 260 | accagaguaa cagucugagu aggagc |
| 261 | cucauaccuu cugcuugaug auc |
| 262 | uucuguccaa gcccgguuga aauc |
| 263 | cuccaacauc aaggaagaug gcauuucuag |
| 264 | aucauuuuuu cucauaccuu cugcu |
| 265 | aucauuuuuu cucauaccuu cugcuaggag cuaaaa |
| 266 | cacccaccau cacccucugu g |
| 267 | aucaucucgu ugauauccuc aa |
| 268 | uccugcauug uugccuguaa g |
| 269 | uccaacuggg gacgccucug uuccaaaucc |
| 270 | acuggggacg ccucuguucc a |
| 271 | ccguaaugau uguucuagcc |
| 272 | uguuaaaaaa cuuacuucga |
| 273 | cauucaacug uugccuccgg uucug |
| 274 | cuguugccuc cgguucugaa ggug |
| 275 | cauucaacug uugccuccgg uucugaaggu g |
| 276 | uacuaaccuu gguuucugug a |
| 277 | cugaaggugu ucuuguacuu caucc |
| 278 | uguauaggga cccuccuucc augacuc |
| 279 | cuaaccuugg uuucugugau uuucu |
| 280 | gguaucuuug auacuaaccu ugguuuc |
| 281 | auucuuucaa cuagaauaaa ag |
| 282 | gauucugaau ucuuucaacu agaau |
| 283 | aucccacuga uucugaauuc |
| 284 | uuggcucugg ccuguccuaa ga |
| 285 | cucuuuucca gguucaagug ggauacuagc |
| 286 | caagcuuuuc uuuuaguugc ugcucuuuuc c |
| 287 | uauucuuuug uucuucuagc cuggagaaag |
| 288 | cugcuuccuc caaccauaaa acaaauuc |
| 289 | ccaaugccau ccuggaguuc cuguaa |
| 290 | uccuguagaa uacuggcauc |
| 291 | ugcagaccuc cugccaccgc agauuca |
| 292 | cuaccucuuu uuucugucug |
| 293 | uguuuuugag gauugcugaa |
| 294 | gttgcctccg gttctgaagg tgttcttg |
| 295 | ctgaaggtgt tcttgtactt catcc |
| 296 | ctgttgcctc cggttctgaa ggtgttcttg |
| 297 | caactgttgc ctccggttct gaaggtgttc ttg |
| 298 | ctccggttct gaaggtgttc ttgta |
| 299 | atttcattca actgttgcct ccggttct |
| 300 | tgaaggtgtt cttgtacttc atccc |
| 301 | cattcaactg ttgcctccgg ttct |
| 302 | tgttgcctcc ggttctgaag gt |
| 303 | gttgcctccg gttctgaagg tgttc |
| 304 | gcctccggtt ctgaaggtgt tcttgtac |
| 305 | cctccggttc tgaaggtgtt cttgtac |
| 306 | ctccggttct gaaggtgttc ttgtac |
| 307 | gcctccggtt ctgaaggtgt tcttg |
| 308 | cagatctgtc aaatcgcctg cagg |
| 309 | caacagatct gtcaaatcgc ctgcagg |
| 310 | ctcaacagat ctgtcaaatc gcctgcagg |
| 311 | gtgtctttct gagaaactgt tcagc |
| 312 | gagaaactgt tcagcttctg ttagccac |
| 313 | gaaactgttc agcttctgtt agccactg |
| 314 | ctgttcagct tctgttagcc actg |
| 315 | atctgtcaaa tcgcctgcag |
| 316 | tttgtgtctt tctgagaaac |
| 317 | tgttcagctt ctgttagcca ctga |
| 318 | gatctgtcaa atcgcctgca ggtaa |
| 319 | aaactgttca gcttctgtta gccac |
| 320 | ttgtgtcttt ctgagaaact gttca |
| 321 | caacagatct gtcaaatcgc ctgcag |
| 322 | cagatctgtc aaatcgcctg caggta |
| 323 | ctgttcagct tctgttagcc actgatt |
| 324 | gaaactgttc agcttctgtt agccactgat t |
| 325 | agaaactgtt cagcttctgt tagcca |
| 326 | ctgcaggtaa aagcatatgg atcaa |
| 327 | atcgcctgca ggtaaaagca tatgg |
| 328 | gtcaaatcgc ctgcaggtaa aagca |
| 329 | caacagatct gtcaaatcgc ctgca |
| 330 | tttctcaaca gatctgtcaa atcgc |
| 331 | ccatttctca acagatctgt caaat |
| 332 | ataatgaaaa cgccgccatt tctca |
| 333 | aaatatcttt atatcataat gaaaa |
| 334 | tgttagccac tgattaaata tcttt |
| 335 | ccaattctca ggaatttgtg tcttt |
| 336 | gtatttagca tgttcccaat tctca |
| 337 | cttaagatac catttgtatt tagca |
| 338 | cttaccttaa gataccattt gtatt |
| 339 | aaagacttac cttaagatac cattt |
| 340 | aaatcaaaga cttaccttaa gatac |
| 341 | aaaacaaatc aaagacttac cttaa |
| 342 | tcgaaaaaac aaatcaaaga cttac |
| 343 | ctgtaagata ccaaaaaggc aaaac |
| 344 | cctgtaagat accaaaaagg caaaa |
| 345 | agttcctgta agataccaaa aaggc |
| 346 | gagttcctgt aagataccaa aaagg |
| 347 | cctggagttc ctgtaagata ccaaa |
| 348 | tcctggagtt cctgtaagat accaa |
| 349 | gccatcctgg agttcctgta agata |
| 350 | tgccatcctg gagttcctgt aagat |
| 351 | ccaatgccat cctggagttc ctgta |
| 352 | cccaatgcca tcctggagtt cctgt |
| 353 | gctgcccaat gccatcctgg agttc |
| 354 | cgctgcccaa tgccatcctg gagtt |
| 355 | aacagtttgc cgctgcccaa tgcca |
| 356 | ctgacaacag tttgccgctg cccaa |
| 357 | gttgcattca atgttctgac aacag |
| 358 | gctgaattat ttcttcccca gttgc |
| 359 | attatttctt ccccagttgc attca |
| 360 | ggcatctgtt tttgaggatt gctga |
| 361 | tttgaggatt gctgaattat ttctt |
| 362 | aatttttcct gtagaatact ggcat |
| 363 | atactggcat ctgtttttga ggatt |
| 364 | accgcagatt caggcttccc aattt |
| 365 | ctgtttgcag acctcctgcc accgc |
| 366 | agattcaggc ttcccaattt ttcct |
| 367 | ctcttttttc tgtctgacag ctgtt |
| 368 | acctcctgcc accgcagatt caggc |
| 369 | cctacctctt ttttctgtct gacag |
| 370 | gacagctgtt tgcagacctc ctgcc |
| 371 | gtcgccctac ctcttttttc tgtct |
| 372 | gatctgtcgc cctacctctt ttttc |
| 373 | tattagatct gtcgccctac ctctt |
| 374 | attcctatta gatctgtcgc cctac |
| 375 | agataccaaa aaggcaaaac |
| 376 | aagataccaa aaaggcaaaa |
| 377 | cctgtaagat accaaaaagg |
| 378 | gagttcctgt aagataccaa |
| 379 | tcctggagtt cctgtaagat |
| 380 | tgccatcctg gagttcctgt |
| 381 | cccaatgcca tcctggagtt |
| 382 | cgctgcccaa tgccatcctg |
| 383 | ctgacaacag tttgccgctg |
| 384 | gttgcattca atgttctgac |
| 385 | attatttctt ccccagttgc |
| 386 | tttgaggatt gctgaattat |
| 387 | atactggcat ctgtttttga |
| 388 | aatttttcct gtagaatact |
| 389 | agattcaggc ttcccaattt |
| 390 | acctcctgcc accgcagatt |
| 391 | gacagctgtt tgcagacctc |
| 392 | ctcttttttc tgtctgacag |
| 393 | cctacctctt ttttctgtct |
| 394 | gtcgccctac ctcttttttc |
| 395 | gatctgtcgc cctacctctt |
| 396 | tattagatct gtcgccctac |
| 397 | attcctatta gatctgtcgc |
| 398 | gggggatttg agaaaataaa attac |
| 399 | atttgagaaa ataaaattac cttga |
| 400 | ctagcctgga gaaagaagaa taaaa |
| 401 | agaaaataaa attaccttga cttgc |
| 402 | ttcttctagc ctggagaaag aagaa |
| 403 | ataaaattac cttgacttgc tcaag |
| 404 | ttttgttctt ctagcctgga gaaag |
| 405 | attaccttga cttgctcaag ctttt |
| 406 | tattcttttg ttcttctagc ctgga |
| 407 | cttgacttgc tcaagctttt ctttt |
| 408 | caagatattc ttttgttctt ctagc |
| 409 | cttttagttg ctgctctttt ccagg |
| 410 | ccaggttcaa gtgggatact agcaa |
| 411 | atctctttga aattctgaca agata |
| 412 | agcaatgtta tctgcttcct ccaac |
| 413 | aacaaattca tttaaatctc tttga |
| 414 | ccaaccataa aacaaattca tttaa |
| 415 | ttcctccaac cataaaacaa attca |
| 416 | tttaaatctc tttgaaattc tgaca |
| 417 | tgacaagata ttcttttgtt cttct |
| 418 | ttcaagtggg atactagcaa tgtta |
| 419 | agatattctt ttgttcttct agcct |
| 420 | ctgctctttt ccaggttcaa gtggg |
| 421 | ttcttttgtt cttctagcct ggaga |
| 422 | cttttctttt agttgctgct ctttt |
| 423 | ttgttcttct agcctggaga aagaa |
| 424 | cttctagcct ggagaaagaa gaata |
| 425 | agcctggaga aagaagaata aaatt |
| 426 | ctggagaaag aagaataaaa ttgtt |
| 427 | gaaagaagaa taaaattgtt |
| 428 | ggagaaagaa gaataaaatt |
| 429 | agcctggaga aagaagaata |
| 430 | cttctagcct ggagaaagaa |
| 431 | ttgttcttct agcctggaga |
| 432 | ttcttttgtt cttctagcct |
| 433 | tgacaagata ttcttttgtt |
| 434 | atctctttga aattctgaca |
| 435 | aacaaattca tttaaatctc |
| 436 | ttcctccaac cataaaacaa |
| 437 | agcaatgtta tctgcttcct |
| 438 | ttcaagtggg atactagcaa |
| 439 | ctgctctttt ccaggttcaa |
| 440 | cttttctttt agttgctgct |
| 441 | cttgacttgc tcaagctttt |
| 442 | attaccttga cttgctcaag |
| 443 | ataaaattac cttgacttgc |
| 444 | agaaaataaa attaccttga |
| 445 | atttgagaaa ataaaattac |
| 446 | gggggatttg agaaaataaa |
| 447 | ctgaaacaga caaatgcaac aacgt |
| 448 | agtaactgaa acagacaaat gcaac |
| 449 | ccaccagtaa ctgaaacaga caaat |
| 450 | ctcttccacc agtaactgaa acaga |
| 451 | ggcaactctt ccaccagtaa ctgaa |
| 452 | gcaggggcaa ctcttccacc agtaa |
| 453 | ctggcgcagg ggcaactctt ccacc |
| 454 | tttaattgtt tgagaattcc ctggc |
| 455 | ttgtttgaga attccctggc gcagg |
| 456 | gcacgggtcc tccagtttca tttaa |
| 457 | tccagtttca tttaattgtt tgaga |
| 458 | gcttatggga gcacttacaa gcacg |
| 459 | tacaagcacg ggtcctccag tttca |
| 460 | agtttatctt gctcttctgg gctta |
| 461 | tctgcttgag cttattttca agttt |
| 462 | atcttgctct tctgggctta tggga |
| 463 | ctttatccac tggagatttg tctgc |
| 464 | cttattttca agtttatctt gctct |
| 465 | ctaaccttta tccactggag atttg |
| 466 | atttgtctgc ttgagcttat tttca |
| 467 | aatgtctaac ctttatccac tggag |
| 468 | tggttaatgt ctaaccttta tccac |
| 469 | agagatggtt aatgtctaac cttta |
| 470 | acggaagaga tggttaatgt ctaac |
| 471 | acagacaaat gcaacaacgt |
| 472 | ctgaaacaga caaatgcaac |
| 473 | agtaactgaa acagacaaat |
| 474 | ccaccagtaa ctgaaacaga |
| 475 | ctcttccacc agtaactgaa |
| 476 | ggcaactctt ccaccagtaa |
| 477 | ctggcgcagg ggcaactctt |
| 478 | ttgtttgaga attccctggc |
| 479 | tccagtttca tttaattgtt |
| 480 | tacaagcacg ggtcctccag |
| 481 | gcttatggga gcacttacaa |
| 482 | atcttgctct tctgggctta |
| 483 | cttattttca agtttatctt |
| 484 | atttgtctgc ttgagcttat |
| 485 | ctttatccac tggagatttg |
| 486 | ctaaccttta tccactggag |
| 487 | aatgtctaac ctttatccac |
| 488 | tggttaatgt ctaaccttta |
| 489 | agagatggtt aatgtctaac |
| 490 | acggaagaga tggttaatgt |
| 491 | ctgaaaggaa aatacatttt aaaaa |
| 492 | cctgaaagga aaatacattt taaaa |
| 493 | gaaacctgaa aggaaaatac atttt |
| 494 | ggaaacctga aaggaaaata cattt |
| 495 | ctctggaaac ctgaaaggaa aatac |
| 496 | gctctggaaa cctgaaagga aaata |
| 497 | taaagctctg gaaacctgaa aggaa |
| 498 | gtaaagctct ggaaacctga aagga |
| 499 | tcaggtaaag ctctggaaac ctgaa |
| 500 | ctcaggtaaa gctctggaaa cctga |
| 501 | gtttctcagg taaagctctg gaaac |
| 502 | tgtttctcag gtaaagctct ggaaa |
| 503 | aatttctcct tgtttctcag gtaaa |
| 504 | tttgagcttc aatttctcct tgttt |
| 505 | ttttatttga gcttcaattt ctcct |
| 506 | aagctgccca aggtctttta tttga |
| 507 | aggtcttcaa gctttttttc aagct |
| 508 | ttcaagcttt ttttcaagct gccca |
| 509 | gatgatttaa ctgctcttca aggtc |
| 510 | ctgctcttca aggtcttcaa gcttt |
| 511 | aggagataac cacagcagca gatga |
| 512 | cagcagatga tttaactgct cttca |
| 513 | atttccaact gattcctaat aggag |
| 514 | cttggtttgg ttggttataa atttc |
| 515 | caactgattc ctaataggag ataac |
| 516 | cttaacgtca aatggtcctt cttgg |
| 517 | ttggttataa atttccaact gattc |
| 518 | cctaccttaa cgtcaaatgg tcctt |
| 519 | tccttcttgg tttggttggt tataa |
| 520 | agttccctac cttaacgtca aatgg |
| 521 | caaaaagttc cctaccttaa cgtca |
| 522 | taaagcaaaa agttccctac cttaa |
| 523 | atatttaaag caaaaagttc cctac |
| 524 | aggaaaatac attttaaaaa |
| 525 | aaggaaaata cattttaaaa |
| 526 | cctgaaagga aaatacattt |
| 527 | ggaaacctga aaggaaaata |
| 528 | gctctggaaa cctgaaagga |
| 529 | gtaaagctct ggaaacctga |
| 530 | ctcaggtaaa gctctggaaa |
| 531 | aatttctcct tgtttctcag |
| 532 | ttttatttga gcttcaattt |
| 533 | aagctgccca aggtctttta |
| 534 | ttcaagcttt ttttcaagct |
| 535 | ctgctcttca aggtcttcaa |
| 536 | cagcagatga tttaactgct |
| 537 | aggagataac cacagcagca |
| 538 | caactgattc ctaataggag |
| 539 | ttggttataa atttccaact |
| 540 | tccttcttgg tttggttggt |
| 541 | cttaacgtca aatggtcctt |
| 542 | cctaccttaa cgtcaaatgg |
| 543 | agttccctac cttaacgtca |
| 544 | caaaaagttc cctaccttaa |
| 545 | taaagcaaaa agttccctac |
| 546 | atatttaaag caaaaagttc |
| 547 | ctggggaaaa gaacccatat agtgc |
| 548 | tcctggggaa aagaacccat atagt |
| 549 | gtttcctggg gaaaagaacc catat |
| 550 | cagtttcctg gggaaaagaa cccat |
| 551 | tttcagtttc ctggggaaaa gaacc |
| 552 | tatttcagtt tcctggggaa aagaa |
| 553 | tgctatttca gtttcctggg gaaaa |
| 554 | actgctattt cagtttcctg gggaa |
| 555 | tgaactgcta tttcagtttc ctggg |
| 556 | cttgaactgc tatttcagtt tcctg |
| 557 | tagcttgaac tgctatttca gtttc |
| 558 | tttagcttga actgctattt cagtt |
| 559 | ttccacatcc ggttgtttag cttga |
| 560 | tgccctttag acaaaatctc ttcca |
| 561 | tttagacaaa atctcttcca catcc |
| 562 | gtttttcctt gtacaaatgc tgccc |
| 563 | gtacaaatgc tgccctttag acaaa |
| 564 | cttcactggc tgagtggctg gtttt |
| 565 | ggctggtttt tccttgtaca aatgc |
| 566 | attaccttca ctggctgagt ggctg |
| 567 | gcttcattac cttcactggc tgagt |
| 568 | aggttgcttc attaccttca ctggc |
| 569 | gctagaggtt gcttcattac cttca |
| 570 | atattgctag aggttgcttc attac |
| 571 | gaaaagaacc catatagtgc |
| 572 | gggaaaagaa cccatatagt |
| 573 | tcctggggaa aagaacccat |
| 574 | cagtttcctg gggaaaagaa |
| 575 | tatttcagtt tcctggggaa |
| 576 | actgctattt cagtttcctg |
| 577 | cttgaactgc tatttcagtt |
| 578 | tttagcttga actgctattt |
| 579 | ttccacatcc ggttgtttag |
| 580 | tttagacaaa atctcttcca |
| 581 | gtacaaatgc tgccctttag |
| 582 | ggctggtttt tccttgtaca |
| 583 | cttcactggc tgagtggctg |
| 584 | attaccttca ctggctgagt |
| 585 | gcttcattac cttcactggc |
| 586 | aggttgcttc attaccttca |
| 587 | gctagaggtt gcttcattac |
| 588 | atattgctag aggttgcttc |
| 589 | ctttaacaga aaagcataca catta |
| 590 | tcctctttaa cagaaaagca tacac |
| 591 | ttcctcttta acagaaaagc ataca |
| 592 | taacttcctc tttaacagaa aagca |
| 593 | ctaacttcct ctttaacaga aaagc |
| 594 | tcttctaact tcctctttaa cagaa |
| 595 | atcttctaac ttcctcttta acaga |
| 596 | tcagatcttc taacttcctc tttaa |
| 597 | ctcagatctt ctaacttcct cttta |
| 598 | agagctcaga tcttctaact tcctc |
| 599 | cagagctcag atcttctaac ttcct |
| 600 | cactcagagc tcagatcttc tact |
| 601 | ccttccactc agagctcaga tcttc |
| 602 | gtaaacggtt taccgccttc cactc |
| 603 | ctttgccctc agctcttgaa gtaaa |
| 604 | ccctcagctc ttgaagtaaa cggtt |
| 605 | ccaggagcta ggtcaggctg ctttg |
| 606 | ggtcaggctg ctttgccctc agctc |
| 607 | aggctccaat agtggtcagt ccagg |
| 608 | tcagtccagg agctaggtca ggctg |
| 609 | cttacaggct ccaatagtgg tcagt |
| 610 | gtatacttac aggctccaat agtgg |
| 611 | atccagtata cttacaggct ccaat |
| 612 | atgggatcca gtatacttac aggct |
| 613 | agagaatggg atccagtata cttac |
| 614 | acagaaaagc atacacatta |
| 615 | tttaacagaa aagcatacac |
| 616 | tcctctttaa cagaaaagca |
| 617 | taacttcctc tttaacagaa |
| 618 | tcttctaact tcctctttaa |
| 619 | tcagatcttc taacttcctc |
| 620 | ccttccactc agagctcaga |
| 621 | gtaaacggtt taccgccttc |
| 622 | ccctcagctc ttgaagtaaa |
| 623 | ggtcaggctg ctttgccctc |
| 624 | tcagtccagg agctaggtca |
| 625 | aggctccaat agtggtcagt |
| 626 | cttacaggct ccaatagtgg |
| 627 | gtatacttac aggctccaat |
| 628 | atccagtata cttacaggct |
| 629 | atgggatcca gtatacttac |
| 630 | agagaatggg atccagtata |
| 631 | ctaaaatatt ttgggttttt gcaaaa |
| 632 | gctaaaatat tttgggtttt tgcaaa |
| 633 | taggagctaa aatattttgg gttttt |
| 634 | agtaggagct aaaatatttt gggtt |
| 635 | tgagtaggag ctaaaatatt ttggg |
| 636 | ctgagtagga gctaaaatat tttggg |
| 637 | cagtctgagt aggagctaaa atatt |
| 638 | acagtctgag taggagctaa aatatt |
| 639 | gagtaacagt ctgagtagga gctaaa |
| 640 | cagagtaaca gtctgagtag gagct |
| 641 | caccagagta acagtctgag taggag |
| 642 | gtcaccagag taacagtctg agtag |
| 643 | aaccacaggt tgtgtcacca gagtaa |
| 644 | gttgtgtcac cagagtaaca gtctg |
| 645 | tggcagtttc cttagtaacc acaggt |
| 646 | atttctagtt tggagatggc agtttc |
| 647 | ggaagatggc atttctagtt tggag |
| 648 | catcaaggaa gatggcattt ctagtt |
| 649 | gagcaggtac ctccaacatc aaggaa |
| 650 | atctgccaga gcaggtacct ccaac |
| 651 | aagttctgtc caagcccggt tgaaat |
| 652 | cggttgaaat ctgccagagc aggtac |
| 653 | gagaaagcca gtcggtaagt tctgtc |
| 654 | gtcggtaagt tctgtccaag cccgg |
| 655 | ataacttgat caagcagaga aagcca |
| 656 | aagcagagaa agccagtcgg taagt |
| 657 | caccctctgt gattttataa cttgat |
| 658 | caaggtcacc caccatcacc ctctgt |
| 659 | catcaccctc tgtgatttta taact |
| 660 | cttctgcttg atgatcatct cgttga |
| 661 | ccttctgctt gatgatcatc tcgttg |
| 662 | atctcgttga tatcctcaag gtcacc |
| 663 | tcataccttc tgcttgatga tcatct |
| 664 | tcattttttc tcataccttc tgcttg |
| 665 | ttttctcata ccttctgctt gatgat |
| 666 | ttttatcatt ttttctcata ccttct |
| 667 | ccaactttta tcattttttc tcatac |
| 668 | atattttggg tttttgcaaa |
| 669 | aaaatatttt gggtttttgc |
| 670 | gagctaaaat attttgggtt |
| 671 | agtaggagct aaaatatttt |
| 672 | gtctgagtag gagctaaaat |
| 673 | taacagtctg agtaggagct |
| 674 | cagagtaaca gtctgagtag |
| 675 | cacaggttgt gtcaccagag |
| 676 | agtttcctta gtaaccacag |
| 677 | tagtttggag atggcagttt |
| 678 | ggaagatggc atttctagtt |
| 679 | tacctccaac atcaaggaag |
| 680 | atctgccaga gcaggtacct |
| 681 | ccaagcccgg ttgaaatctg |
| 682 | gtcggtaagt tctgtccaag |
| 683 | aagcagagaa agccagtcgg |
| 684 | ttttataact tgatcaagca |
| 685 | catcaccctc tgtgatttta |
| 686 | ctcaaggtca cccaccatca |
| 687 | catctcgttg atatcctcaa |
| 688 | cttctgcttg atgatcatct |
| 689 | cataccttct gcttgatgat |
| 690 | tttctcatac cttctgcttg |
| 691 | cattttttct cataccttct |
| 692 | tttatcattt tttctcatac |
| 693 | caacttttat cattttttct |
| 694 | ctgtaagaac aaatatccct tagta |
| 695 | tgcctgtaag aacaaatatc cctta |
| 696 | gttgcctgta agaacaaata tccct |
| 697 | attgttgcct gtaagaacaa atatc |
| 698 | gcattgttgc ctgtaagaac aaata |
| 699 | cctgcattgt tgcctgtaag aacaa |
| 700 | atcctgcatt gttgcctgta agaac |
| 701 | caaatcctgc attgttgcct gtaag |
| 702 | tccaaatcct gcattgttgc ctgta |
| 703 | tgttccaaat cctgcattgt tgcct |
| 704 | tctgttccaa atcctgcatt gttgc |
| 705 | aactggggac gcctctgttc caaat |
| 706 | gcctctgttc caaatcctgc attgt |
| 707 | cagcggtaat gagttcttcc aactg |
| 708 | cttccaactg gggacgcctc tgttc |
| 709 | cttgtttttc aaattttggg cagcg |
| 710 | ctagcctctt gattgctggt cttgt |
| 711 | ttttcaaatt ttgggcagcg gtaat |
| 712 | ttcgatccgt aatgattgtt ctagc |
| 713 | gattgctggt cttgtttttc aaatt |
| 714 | cttacttcga tccgtaatga ttgtt |
| 715 | ttgttctagc ctcttgattg ctggt |
| 716 | aaaaacttac ttcgatccgt aatga |
| 717 | tgttaaaaaa cttacttcga tccgt |
| 718 | atgcttgtta aaaaacttac ttcga |
| 719 | gtcccatgct tgttaaaaaa cttac |
| 720 | agaacaaata tcccttagta |
| 721 | gtaagaacaa atatccctta |
| 722 | tgcctgtaag aacaaatatc |
| 723 | attgttgcct gtaagaacaa |
| 724 | cctgcattgt tgcctgtaag |
| 725 | caaatcctgc attgttgcct |
| 726 | gcctctgttc caaatcctgc |
| 727 | cttccaactg gggacgcctc |
| 728 | cagcggtaat gagttcttcc |
| 729 | ttttcaaatt ttgggcagcg |
| 730 | gattgctggt cttgtttttc |
| 731 | ttgttctagc ctcttgattg |
| 732 | ttcgatccgt aatgattgtt |
| 733 | cttacttcga tccgtaatga |
| 734 | aaaaacttac ttcgatccgt |
| 735 | tgttaaaaaa cttacttcga |
| 736 | atgcttgtta aaaaacttac |
| 737 | gtcccatgct tgttaaaaaa |
| 738 | ctagaataaa aggaaaaata aatat |
| 739 | aactagaata aaaggaaaaa taaat |
| 740 | ttcaactaga ataaaaggaa aaata |
| 741 | ctttcaacta gaataaaagg aaaaa |
| 742 | attctttcaa ctagaataaa aggaa |
| 743 | gaattctttc aactagaata aaagg |
| 744 | tctgaattct ttcaactaga ataaa |
| 745 | attctgaatt ctttcaacta gaata |
| 746 | ctgattctga attctttcaa ctaga |
| 747 | cactgattct gaattctttc aacta |
| 748 | tcccactgat tctgaattct ttcaa |
| 749 | catcccactg attctgaatt ctttc |
| 750 | tacttcatcc cactgattct gaatt |
| 751 | cggttctgaa ggtgttcttg tact |
| 752 | ctgttgcctc cggttctgaa ggtgt |
| 753 | tttcattcaa ctgttgcctc cggtt |
| 754 | taacatttca ttcaactgtt gcctc |
| 755 | ttgtgttgaa tcctttaaca tttca |
| 756 | tcttccttag cttccagcca ttgtg |
| 757 | cttagcttcc agccattgtg ttgaa |
| 758 | gtcctaagac ctgctcagct tcttc |
| 759 | ctgctcagct tcttccttag cttcc |
| 760 | ctcaagcttg gctctggcct gtcct |
| 761 | ggcctgtcct aagacctgct cagct |
| 762 | tagggaccct ccttccatga ctcaa |
| 763 | tttggattgc atctactgta taggg |
| 764 | accctccttc catgactcaa gcttg |
| 765 | cttggtttct gtgattttct tttgg |
| 766 | atctactgta tagggaccct ccttc |
| 767 | ctaaccttgg tttctgtgat tttct |
| 768 | tttcttttgg attgcatcta ctgta |
| 769 | tgatactaac cttggtttct gtgat |
| 770 | atctttgata ctaaccttgg tttct |
| 771 | aaggtatctt tgatactaac cttgg |
| 772 | ttaaaaaggt atctttgata ctaac |
| 773 | ataaaaggaa aaataaatat |
| 774 | gaataaaagg aaaaataaat |
| 775 | aactagaata aaaggaaaaa |
| 776 | ctttcaacta gaataaaagg |
| 777 | gaattctttc aactagaata |
| 778 | attctgaatt ctttcaacta |
| 779 | tacttcatcc cactgattct |
| 780 | ctgaaggtgt tcttgtact |
| 781 | ctgttgcctc cggttctgaa |
| 782 | taacatttca ttcaactgtt |
| 783 | ttgtgttgaa tcctttaaca |
| 784 | cttagcttcc agccattgtg |
| 785 | ctgctcagct tcttccttag |
| 786 | ggcctgtcct aagacctgct |
| 787 | ctcaagcttg gctctggcct |
| 788 | accctccttc catgactcaa |
| 789 | atctactgta tagggaccct |
| 790 | tttcttttgg attgcatcta |
| 791 | cttggtttct gtgattttct |
| 792 | ctaaccttgg tttctgtgat |
| 793 | tgatactaac cttggtttct |
| 794 | atctttgata ctaaccttgg |
| 795 | aaggtatctt tgatactaac |
| 796 | ttaaaaaggt atctttgata |
| 797 | ctatagattt ttatgagaaa gaga |
| 798 | aactgctata gatttttatg agaaa |
| 799 | tggccaactg ctatagattt ttatg |
| 800 | gtctttggcc aactgctata gattt |
| 801 | cggaggtctt tggccaactg ctata |
| 802 | actggcggag gtctttggcc aactg |
| 803 | tttgtctgcc actggcggag gtctt |
| 804 | agtcatttgc cacatctaca tttgt |
| 805 | tttgccacat ctacatttgt ctgcc |
| 806 | ccggagaagt ttcagggcca agtca |
| 807 | gtatcatctg cagaataatc ccgga |
| 808 | taatcccgga gaagtttcag ggcca |
| 809 | ttatcatgtg gacttttctg gtatc |
| 810 | agaggcattg atattctctg ttatc |
| 811 | atgtggactt ttctggtatc atctg |
| 812 | cttttatgaa tgcttctcca agagg |
| 813 | atattctctg ttatcatgtg gactt |
| 814 | catacctttt atgaatgctt ctcca |
| 815 | ctccaagagg cattgatatt ctctg |
| 816 | taattcatac cttttatgaa tgctt |
| 817 | taatgtaatt catacctttt atgaa |
| 818 | agaaataatg taattcatac ctttt |
| 819 | gttttagaaa taatgtaatt catac |
| 820 | gatttttatg agaaagaga |
| 821 | ctatagattt ttatgagaaa |
| 822 | aactgctata gatttttatg |
| 823 | tggccaactg ctatagattt |
| 824 | gtctttggcc aactgctata |
| 825 | cggaggtctt tggccaactg |
| 826 | tttgtctgcc actggcggag |
| 827 | tttgccacat ctacatttgt |
| 828 | ttcagggcca agtcatttgc |
| 829 | taatcccgga gaagtttcag |
| 830 | gtatcatctg cagaataatc |
| 831 | atgtggactt ttctggtatc |
| 832 | atattctctg ttatcatgtg |
| 833 | ctccaagagg cattgatatt |
| 834 | cttttatgaa tgcttctcca |
| 835 | catacctttt atgaatgctt |
| 836 | taattcatac cttttatgaa |
| 837 | taatgtaatt catacctttt |
| 838 | agaaataatg taattcatac |
| 839 | gttttagaaa taatgtaatt |
| 840 | ctgcaaagga ccaaatgttc agatg |
| 841 | tcaccctgca aaggaccaaa tgttc |
| 842 | ctcactcacc ctgcaaagga ccaaa |
| 843 | tctcgctcac tcaccctgca aagga |
| 844 | cagcctctcg ctcactcacc ctgca |
| 845 | caaagcagcc tctcgctcac tcacc |
| 846 | tcttccaaag cagcctctcg ctcac |
| 847 | tctatgagtt tcttccaaag cagcc |
| 848 | gttgcagtaa tctatgagtt tcttc |
| 849 | gaactgttgc agtaatctat gagtt |
| 850 | ttccaggtcc agggggaact gttgc |
| 851 | gtaagccagg caagaaactt ttcca |
| 852 | ccaggcaaga aacttttcca ggtcc |
| 853 | tggcagttgt ttcagcttct gtaag |
| 854 | ttcagcttct gtaagccagg caaga |
| 855 | ggtagcatcc tgtaggacat tggca |
| 856 | gacattggca gttgtttcag cttct |
| 857 | tctaggagcc tttccttacg ggtag |
| 858 | cttttactcc cttggagtct tctag |
| 859 | gagcctttcc ttacgggtag catcc |
| 860 | ttgccattgt ttcatcagct ctttt |
| 861 | cttggagtct tctaggagcc tttcc |
| 862 | cttacttgcc attgtttcat cagct |
| 863 | cagctctttt actcccttgg agtct |
| 864 | cctgacttac ttgccattgt ttcat |
| 865 | aaatgcctga cttacttgcc attgt |
| 866 | agcggaaatg cctgacttac ttgcc |
| 867 | gctaaagcgg aaatgcctga cttac |
| 868 | aaggaccaaa tgttcagatg |
| 869 | ctgcaaagga ccaaatgttc |
| 870 | tcaccctgca aaggaccaaa |
| 871 | ctcactcacc ctgcaaagga |
| 872 | tctcgctcac tcaccctgca |
| 873 | cagcctctcg ctcactcacc |
| 874 | caaagcagcc tctcgctcac |
| 875 | tctatgagtt tcttccaaag |
| 876 | gaactgttgc agtaatctat |
| 877 | ttccaggtcc agggggaact |
| 878 | ccaggcaaga aacttttcca |
| 879 | ttcagcttct gtaagccagg |
| 880 | gacattggca gttgtttcag |
| 881 | ggtagcatcc tgtaggacat |
| 882 | gagcctttcc ttacgggtag |
| 883 | cttggagtct tctaggagcc |
| 884 | cagctctttt actcccttgg |
| 885 | ttgccattgt ttcatcagct |
| 886 | cttacttgcc attgtttcat |
| 887 | cctgacttac ttgccattgt |
| 888 | aaatgcctga cttacttgcc |
| 889 | agcggaaatg cctgacttac |
| 890 | gctaaagcgg aaatgcctga |
| 891 | ccactcagag ctcagatctt ctaacttcc |
| 892 | gggatccagt atacttacag gctcc |
| 893 | cttccactca gagctcagat cttctaa |
| 894 | acatcaagga agatggcatt tctagtttgg |
| 895 | ctccaacatc aaggaagatg gcatttctag |
| 896 | ttctgtccaa gcccggttga aatc |
| 897 | cacccaccat caccctcygt g |
| 898 | atcatctcgt tgatatcctc aa |
| 899 | acatcaagga agatggcatt tctag |
| 900 | accagagtaa cagtctgagt aggagc |
| 901 | tcaaggaaga tggcatttct |
| 902 | cctctgtgat tttataactt gat |
| 903 | atcatttttt ctcatacctt ctgct |
| 904 | ctcatacctt ctgcttgatg atc |
| 905 | tggcatttct agtttgg |
| 906 | ccagagcagg tacctccaac atc |
| 907 | cgccgccatt tctcaacag |
| 908 | tgtttttgag gattgctgaa |
| 909 | gctgaattat ttcttcccc |
| 910 | gcccaatgcc atcctgg |
| 911 | ccaatgccat cctggagttc ctgtaa |
| 912 | cattcaactg ttgcctccgg ttctgaaggt g |
| 913 | ctgttgcctc cggttctg |
| 914 | attctttcaa ctagaataaa ag |
| 915 | gccatcctgg agttcctgta agataccaaa |
| 916 | ccaatgccat cctggagttc ctgtaagata |
| 917 | gccgctgccc aatgccatcc tggagttcct |
| 918 | gtttgccgct gcccaatgcc atcctggagt |
| 919 | caacagtttg ccgctgccca atgccatcct |
| 920 | ctgacaacag tttgccgctg cccaatgcca |
| 921 | tgttctgaca acagtttgcc gctgcccaat |
| 922 | caatgttctg acaacagttt gccgctgccc |
| 923 | cattcaatgt tctgacaaca gtttgccgct |
| 924 | tatttcttcc ccagttgcat tcaatgttct |
| 925 | gctgaattat ttcttcccca gttgcattca |
| 926 | ggattgctga attatttctt ccccagttgc |
| 927 | tttgaggatt gctgaattat ttcttcccca |
| 928 | gtacttcatc ccactgattc tgaattcttt |
| 929 | tcttgtactt catcccactg attctgaatt |
| 930 | tgttcttgta cttcatccca ctgattctga |
| 931 | cggttctgaa ggtgttcttg tacttcatcc |
| 932 | ctccggttct gaaggtgttc ttgtacttca |
| 933 | tgcctccggt tctgaaggtg ttcttgtact |
| 934 | tgttgcctcc ggttctgaag gtgttcttgt |
| 935 | aactgttgcc tccggttctg aaggtgttct |
| 936 | ttcaactgtt gcctccggtt ctgaaggtgt |
| 937 | ggccaaacct cggcttacct gaaat |
| 938 | cagatctgtc aaatcgcctg cagg |
| 939 | caacagatct gtcaaatcgc ctgcagg |
| 940 | ctcaacagat ctgtcaaatc gcctgcagg |
| 941 | gtgtctttct gagaaactgt tcagc |
| 942 | gagaaactgt tcagcttctg ttagccac |
| 943 | gaaactgttc agcttctgtt agccactg |
| 944 | ctgttcagct tctgttagcc actg |
| 945 | atctgtcaaa tcgcctgcag |
| 946 | tttgtgtctt tctgagaaac |
| 947 | tgttcagctt ctgttagcca ctga |
| 948 | gatctgtcaa atcgcctgca ggtaa |
| 949 | aaactgttca gcttctgtta gccac |
| 950 | ttgtgtcttt ctgagaaact gttca |
| 951 | caacagatct gtcaaatcgc ctgcag |
| 952 | cagatctgtc aaatcgcctg caggta |
| 953 | ctgttcagct tctgttagcc actgatt |
| 954 | gaaactgttc agcttctgtt agccactgat t |
| 955 | agaaactgtt cagcttctgt tagcca |
| 956 | cttggacaga acttaccgac tgg |
| 957 | gtttcttcca aagcagcctc tcg |
| 958 | gcaggatttg gaacagaggc g |
| 959 | catctacatt tgtctgccac tgg |
| 960 | caatgctcct gacctctgtg c |
| 961 | gctcttttcc aggttcaagt gg |
| 962 | gtctacaaca aagctcaggt cg |
| 963 | gcaatgttat ctgcttcctc caacc |
| 964 | gctttgttgt agactatctt ttatattc |
| 965 | ccgacctgag ctttgttgta gactatct |
| 966 | cttcctgtag cttcaccctt tccacagg |
| 967 | gctgggagag agcttcctgt agcttcac |
| 968 | tgttacctac ccttgtcggt ccttgtac |
| 969 | ctatgaataa tgtcaatccg acctgagc |
| 970 | ctgctgtctt cttgctatga ataatgtc |
| 971 | ggcgttgcac tttgcaatgc tgctgtct |
| 972 | ttggaaatca agctgggaga gagcttcc |
| 973 | ctttttccca ttggaaatca agctggga |
| 974 | gtcggtcctt gtacattttg ttaacttt |
| 975 | ctacccttgt cggtccttgt acattttg |
| 976 | gacctgagct ttgttgtaga ctatcttt |
| 977 | gtcaatccga cctgagcttt gttgtaga |
| 978 | taatgtcaat ccgacctgag ctttgttg |
| 979 | cttgctatga ataatgtcaa tccgacc |
| 980 | gtcttcttgc tatgaataat gtcaatcc |
| 981 | gcactttgca atgctgctgt cttcttgc |
| 982 | ccacaggcgt tgcactttgc aatgctgc |
| 983 | agcttcaccc tttccacagg cgttgcac |
| 984 | tcaccctttc cacaggcgtt gca |
| 985 | ggagagagct tcctgtagct |
| 986 | tcccattgga aatcaagctg ggagagag |
| 987 | tatatgtgtt acctaccctt gtcggtcc |
| 988 | gccatcctgg agttcctgta agatacc |
| 989 | gagttcctgt aagataccaa aaagg |
| 990 | gcccaatgcc atcctggagt tcctg |
| 991 | ccaatgccat cctggagttc ct |
| 992 | aatgccatcc tggagttcct gtaa |
| 993 | cctggagttc ctgtaagata ccaaa |
| 994 | tgccatcctg gagttcctgt aagat |
| 995 | tcctggagtt cctgtaagat ac |
| 996 | ccatcctgga gttcctgtaa gatac |
| 997 | cccaatgcca tcctggagtt cctgtaaga |
| 998 | ccgctgccca atgccatcct ggagttcc |
| 999 | caatgccatc ctggagttcc tgtaagatac |
| 1000 | cccaatgcca tcctggagtt cctgtaagat |
| 1001 | gccgctgccc aatgccatcc tggagttcct |
| 1002 | aatgccatcc tggagttcct gtaagatacc |
| 1003 | ccgctgccca atgccatcct ggagttcctg |
| 1004 | tgccgctgcc caatgccatc ctggagttcc |
| 1005 | tgcccaatgc catcctggag ttcctgtaag |
| 1006 | caatgccatc ctggagttcc tgtaagat |
| 1007 | cccaatgcca tcctggagtt cctgtaag |
| 1008 | tgcccaatgc catcctggag ttcctgta |
| 1009 | gctgcccaat gccatcctgg agttcctg |
| 1010 | catcctggag ttcctgtaag atacc |
| 1011 | gccatcctgg agttcctgta agatacc |
| 1012 | gctgcccaat gccatcctgg agttc |
| 1013 | gcccaatgcc atcctggagt |
| 1014 | tgccgctgcc caatgccatc ctgga |
| 1015 | ctgcccaatg ccatcctgg |
| 1016 | cagtttgccg ctgcccaatg ccatcc |
| 1017 | acagtttgcc gctgcccaat gcca |
| 1018 | ctgacaacag tttgccgctg cccaa |
| 1019 | gcattcaatg ttctgacaac |
| 1020 | gaattatttc ttccccagtt gcattcaatg |
| 1021 | ctggcatctg tttttgagga ttgctgaatt |
| 1022 | ccagttgcat tcaatgttct gacaac |
| 1023 | ttgctgaatt atttcttccc cag |
| 1024 | tttttgagga ttgctgaatt atttcttcc |
| 1025 | tttcctgtag aatactggca tctgt |
| 1026 | cttcccaatt tttcctgtag aatactggca t |
| 1027 | ccaatttttc ctgtagaata ctggc |
| 1028 | caggcttccc aatttttcct gtagaatac |
| 1029 | ctcctgccac cgcagattca ggcttc |
| 1030 | gcagacctcc tgccaccgca gattc |
| 1031 | ttgtttgcag acctcctgcc accgcagatt c |
| 1032 | gctgtttgca gacctcctgc cacc |
| 1033 | gtttgcagac ctcctgccac cgcag |
| 1034 | cttttttctg tctgacagct gtttgcagac |
| 1035 | ctgtctgaca gctgtttgca g |
| 1036 | ctacctcttt tttctgtctg acagc |
| 1037 | tattagatct gtcgccctac ctctt |
| 1038 | cctattagat ctgtcgccct acctc |
| 1039 | cuuuaacaga aaagcauac |
| 1040 | ucuuuaacag aaaagcauac |
| 1041 | ccucuuuaac agaaaagcau ac |
| 1042 | aacuuccucu uuaacagaaa agcauac |
| 1043 | cuucuaacuu ccucuuuaac agaaaagcau ac |
| 1044 | ccucuuuaac agaaaa |
| 1045 | aacuuccucu uuaacagaaa ag |
| 1046 | aacuuccucu uuaacag |
| 1047 | gcucagaucu ucuaacuucc ucuuuaacag |
| 1048 | aacuuccucu uuaaca |
| 1049 | ccacucagag cucagaucuu cuaacuucc |
| 1050 | cucagagcuc agaucuu |
| 1051 | gcucuugaag uaaacgg |
| 1052 | aauagugguc aguccagg |
| 1053 | cuuacaggcu ccaauagugg uca |
| 1054 | guauacuuac aggcuccaau agugguca |
| 1055 | uccaguauac uuacaggcuc caauaguggu |
| 1056 | cuuacaggcu ccaauagu |
| 1057 | guauacuuac aggcuccaau agu |
| 1058 | uccaguauac uuacaggcuc caauagu |
| 1059 | uccaguauac uuacaggcuc ca |
| 1060 | gggauccagu auacuuacag gcucc |
| 1061 | uccaguauac uuacaggcu |
| 1062 | uccaguauac uuaca |
| 1063 | ctccaacatc aaggaagatg gcatttct |
| 1064 | catcaaggaa gatggcattt ctagt |
| 1065 | ggagctaaaa tattttgggt ttttgc |
| 1066 | ttttctcata ccttctgctt gatga |
| 1067 | aggtacctcc aacatcaagg aagatgg |
| 1068 | ctccaacatc aaggaagatg gcatt |
| 1069 | ctccaacatc aaggaagatg gcatttct |
| 1070 | ctccaacatc aaggaagatg gcatttctag |
| 1071 | aaggaagatg gcatttctag tttgg |
| 1072 | cagtctgagt aggagctaaa atatt |
| 1073 | gagtaggagc taaaatattt tgggt |
| 1074 | cugaauucuu ucaacuagaa uaaaa |
| 1075 | gccauugugu ugaauccuuu aacauuuc |
| 1076 | ccaugacuca agcuuggcuc uggcc |
| 1077 | cccuauacag uagaugcaau |
| 1078 | uugauacuaa ccuugguuuc ugug |
| 1079 | caactgttgc ctccggttct gaag |
| 1080 | ggaccctcct tccatgactc aagc |
| 1081 | ggtatctttg atactaacct tggtttc |
| 1082 | gcccaaugcc auccugg |
| 1083 | cccauuuugu gaauguuuuc uuuu |
| 1084 | uugugcauuu acccauuuug ug |
| 1085 | uauccucuga augucgcauc |
| 1086 | gguuauccuc ugaaugucgc |
| 1087 | gagccuuuuu ucuucuuug |
| 1088 | uccuuucguc ucugggcuc |
| 1089 | cuccucuuuc uucuucugc |
| 1090 | cuucgaaacu gagcaaauuu |
| 1091 | cuugugagac augagug |
| 1092 | cagagacucc ucuugcuu |
| 1093 | ugcugcuguc uucuugcu |
| 1094 | uuguuaacuu uuucccauu |
| 1095 | cgccgccauu ucucaacag |
| 1096 | uuuguauuua gcauguuccc |
| 1097 | gcugaauuau uucuucccc |
| 1098 | cugcuuccuc caacc |
| 1099 | gcuuuucuuu uaguugcugc |
| 1100 | ucuugcucuu cugggcuu |
| 1101 | cuugagcuua uuuucaaguu u |
| 1102 | uuucuccuug uuucuc |
| 1103 | ccauaaauuu ccaacugauu c |
| 1104 | cuuccacauc cgguuguuu |
| 1105 | guggcugguu uuuccuugu |
| 1106 | cucagagcuc agaucuu |
| 1107 | ggcugcuuug cccuc |
| 1108 | ucaaggaaga uggcauuucu |
| 1109 | ccucugugau uuuauaacuu gau |
| 1110 | cuguugccuc cgguucug |
| 1111 | uuggcucugg ccuguccu |
| 1112 | gaaaauugug cauuuaccca uuuu |
| 1113 | cuuccuggau ggcuucaau |
| 1114 | guacauuaag auggacuuc |
| 1115 | ccauuacagu ugucuguguu |
| 1116 | uaaucugccu cuucuuuugg |
| 1117 | ucugcuggca ucuugc |
| 1118 | ccaucuguua gggucugug |
| 1119 | ucugugccaa uaugcgaauc |
| 1120 | uuaaaugucu caaguucc |
| 1121 | guaguucccu ccaacg |
| 1122 | cauguaguuc ccucc |
| 1123 | uguuaacuuu uucccauugg |
| 1124 | cauuuuguua acuuuuuccc |
| 1125 | ucuguuuuug aggauugc |
| 1126 | ccaccgcaga uucaggc |
| 1127 | uuugcagacc uccugcc |
| 1128 | gaaauucuga caagauauuc u |
| 1129 | uaaaacaaau ucauu |
| 1130 | uccagguuca agugggauac |
| 1131 | uuccagguuc aagug |
| 1132 | ucaagcuuuu cuuuuag |
| 1133 | cugacaagau auucuu |
| 1134 | agguucaagu gggauacua |
| 1135 | uccaguuuca uuuaauuguu ug |
| 1136 | cugcuugagc uuauuuucaa guu |
| 1137 | agcacuuaca agcacgggu |
| 1138 | uucaaguuua ucuugcucuu c |
| 1139 | ggucuuuuau uugagcuuc |
| 1140 | cuucaagcuu uuuuucaagc u |
| 1141 | gcuucaauuu cuccuuguu |
| 1142 | uuuauuugag cuucaauuu |
| 1143 | gcugcccaag gucuuuu |
| 1144 | cuucaagguc uucaagcuuu u |
| 1145 | uaacugcucu ucaaggucuu c |
| 1146 | gaaagccagu cgguaaguuc |
| 1147 | cacccaccau caccc |
| 1148 | ugauauccuc aaggucaccc |
| 1149 | uugcuggucu uguuuuuc |
| 1150 | ccguaaugau uguucu |
| 1151 | uacauuuguc ugccacugg |
| 1152 | cccggagaag uuucaggg |
| 1153 | cuguugcagu aaucuaugag |
| 1154 | ugccauuguu ucaucagcuc uuu |
| 1155 | ugcaguaauc uaugaguuuc |
| 1156 | uccuguagga cauuggcagu |
| 1157 | gagucuucua ggagccuu |
| 1158 | uuuuuuggcu guuuucaucc |
| 1159 | guucacucca cuugaaguuc |
| 1160 | ccuuccaggg aucucagg |
| 1161 | uaggugccug ccggcuu |
| 1162 | cugaacugcu ggaaagucgc c |
| 1163 | uucagcugua gccacacc |
| 1164 | uucuuuaguu uucaauuccc uc |
| 1165 | gaguuucucu aguccuucc |
| 1166 | caauuuuucc cacucaguau u |
| 1167 | uugaaguucc uggagucuu |
| 1168 | guucucuuuc agaggcgc |
| 1169 | gugcugaggu uauacggug |
| 1170 | gucccugugg gcuucaug |
| 1171 | gugcugagau gcuggacc |
| 1172 | uggcucucuc ccaggg |
| 1173 | gggcacuuug uuuggcg |
| 1174 | ggucccagca aguuguuug |
| 1175 | guagagcucu gucauuuugg g |
| 1176 | gccagaaguu gaucagagu |
| 1177 | ucuacuggcc agaaguug |
| 1178 | ugaguaucau cgugugaaag |
| 1179 | gcauaauguu caaugcgug |
| 1180 | gauccauugc uguuuucc |
| 1181 | gagaugcuau cauuuagaua a |
| 1182 | cuggcucagg ggggagu |
| 1183 | uccccucuuu ccucacucu |
| 1184 | ccuuuauguu cgugcugcu |
| 1185 | ggcggccuuu guguugac |
| 1186 | gagagguaga aggagagga |
| 1187 | auaggcugac ugcugucgg |
| 1188 | uuguguccug gggagga |
| 1189 | ugcuccauca ccuccucu |
| 1190 | gcuuuccagg gguauuuc |
| 1191 | cauuggcuuu ccagggg |
| 1192 | cccauuuugu gaauguuuuc uuuu |
| 1193 | uugugcauuu acccauuuug ug |
| 1194 | gaaaauugug cauuuaccca uuuu |
| 1195 | cuuccuggau ggcuucaau |
| 1196 | guacauuaag auggacuuc |
| 1197 | ccauuacagu ugucuguguu |
| 1198 | uaaucugccu cuucuuuugg |
| 1199 | ucugcuggca ucuugc |
| 1200 | uauccucuga augucgcauc |
| 1201 | gguuauccuc ugaaugucgc |
| 1202 | ccaucuguua gggucugug |
| 1203 | ucugugccaa uaugcgaauc |
| 1204 | uuaaaugucu caaguucc |
| 1205 | guaguucccu ccaacg |
| 1206 | cauguaguuc ccucc |
| 1207 | gagccuuuuu ucuucuuug |
| 1208 | uccuuucauc ucugggcuc |
| 1209 | cuccucuuuc uucuucugc |
| 1210 | cuucgaaacu gagcaaauuu |
| 1211 | cuugugagac augagug |
| 1212 | cagagacucc ucuugcuu |
| 1213 | ugcugcuguc uucuugcu |
| 1214 | uuguuaacuu uuucccauu |
| 1215 | uguuaacuuu uucccauugg |
| 1216 | cauuuuguua acuuuuuccc |
| 1217 | cuguagcuuc acccuuucc |
| 1218 | cgccgccauu ucucaacag |
| 1219 | uuuguauuua gcauguuccc |
| 1220 | gcugaauuau uucuucccc |
| 1221 | uuuuucuguc ugacagcug |
| 1222 | ucuguuuuug aggauugc |
| 1223 | ccaccgcaga uucaggc |
| 1224 | gcccaaugcc auccugg |
| 1225 | uuugcagacc uccugcc |
| 1226 | cugcuuccuc caacc |
| 1227 | guuaucugcu uccuccaacc |
| 1228 | gcuuuucuuu uaguugcugc |
| 1229 | uuaguugcug cucuu |
| 1230 | gaaauucuga caagauauuc u |
| 1231 | uaaaacaaau ucauu |
| 1232 | uccagguuca agugggauac |
| 1233 | uuccagguuc aagug |
| 1234 | ucaagcuuuu cuuuuag |
| 1235 | cugacaagau auucuu |
| 1236 | agguucaagu gggauacua |
| 1237 | ucuugcucuu cugggcuu |
| 1238 | cuugagcuua uuuucaaguu u |
| 1239 | uccaguuuca uuuaauuguu ug |
| 1240 | cugcuugagc uuauuuucaa guu |
| 1241 | agcacuuaca agcacgggu |
| 1242 | uucaaguuua ucuugcucuu c |
| 1243 | uuucuccuug uuucuc |
| 1244 | uuauaaauuu ccaacugauu c |
| 1245 | ggucuuuuau uugagcuuc |
| 1246 | cuucaagcuu uuuuucaagc u |
| 1247 | gcuucaauuu cuccuuguu |
| 1248 | uuuauuugag cuucaauuu |
| 1249 | gcugcccaag gucuuuu |
| 1250 | cuucaagguc uucaagcuuu u |
| 1251 | uaacugcucu ucaaggucuu c |
| 1252 | cuuccacauc cgguuguuu |
| 1253 | guggcugguu uuuccuugu |
| 1254 | cucagagcuc agaucuu |
| 1255 | ggcugcuuug cccuc |
| 1256 | ucaaggaaga uggcauuucu |
| 1257 | gaaagccagu cgguaaguuc |
| 1258 | cacccaccau caccc |
| 1259 | ccucugugau uuuauaacuu gau |
| 1260 | ugauauccuc aaggucaccc |
| 1261 | uugcuggucu uguuuuuc |
| 1262 | ccguaaugau uguucu |
| 1263 | cuguugccuc cgguucug |
| 1264 | uuggcucugg ccuguccu |
| 1265 | uacauuuguc ugccacugg |
| 1266 | cccggagaag uuucaggg |
| 1267 | cuguugcagu aaucuaugag |
| 1268 | ugccauuguu ucaucagcuc uuu |
| 1269 | ugcaguaauc uaugaguuuc |
| 1270 | uccuguagga cauuggcagu |
| 1271 | gagucuucua ggagccuu |
| 1272 | uuuuuuggcu guuuucaucc |
| 1273 | guucacucca cuugaaguuc |
| 1274 | ccuuccaggg aucucagg |
| 1275 | uaggugccug ccggcuu |
| 1276 | cugaacugcu ggaaagucgc c |
| 1277 | uucagcugua gccacacc |
| 1278 | uucuuuaguu uucaauuccc uc |
| 1279 | gaguuucucu aguccuucc |
| 1280 | caauuuuucc cacucaguau u |
| 1281 | uugaaguucc uggagucuu |
| 1282 | guucucuuuc agaggcgc |
| 1283 | gugcugaggu uauacggug |
| 1284 | gucccugugg gcuucaug |
| 1285 | gugcugagau gcuggacc |
| 1286 | uggcucucuc ccaggg |
| 1287 | gggcacuuug uuuggcg |
| 1288 | ggucccagca aguuguuug |
| 1289 | guagagcucu gucauuuugg g |
| 1290 | gccagaaguu gaucagagu |
| 1291 | ucuacuggcc agaaguug |
| 1292 | ugaguaucau cgugugaaag |
| 1293 | gcauaauguu caaugcgug |
| 1294 | gauccauugc uguuuucc |
| 1295 | gagaugcuau cauuuagaua a |
| 1296 | cuggcucagg ggggagu |
| 1297 | uccccucuuu ccucacucu |
| 1298 | ccuuuauguu cgugcugcu |
| 1299 | ggcggccuuu guguugac |
| 1300 | gagagguaga aggagagga |
| 1301 | auaggcugac ugcugucgg |
| 1302 | uuguguccug gggagga |
| 1303 | ugcuccauca ccuccucu |
| 1304 | gcuuuccagg gguauuuc |
| 1305 | cauuggcuuu ccagggg |
| 1306 | cugacgucca gucuuuauc |
| 1307 | gggauuuucc gucugcuu |
| 1308 | ccgccauuuc ucaacag |
| 1309 | uucucaggaa uuugugucuu u |
| 1310 | caguuugccg cugccca |
| 1311 | guugcauuca auguucugac |
| 1312 | auuuuuccug uagaauacug g |
| 1313 | gcuggucuug uuuuucaa |
| 1314 | uggucuuguu uuucaaauuu |
| 1315 | gucuuguuuu ucaaauuuug |
| 1316 | cuuguuuuuc aaauuuuggg |
| 1317 | uguuuuucaa auuuugggc |
| 1318 | uccuauaagc ugagaaucug |
| 1319 | gccuucugca gucuucgg |
| 1320 | ccggttctga aggtgttctt gta |
| 1321 | tccggttctg aaggtgttct tgta |
| 1322 | ctccggttct gaaggtgttc ttgta |
| 1323 | cctccggttc tgaaggtgtt cttgta |
| 1324 | gcctccggtt ctgaaggtgt tcttgta |
| 1325 | tgcctccggt tctgaaggtg ttcttgta |
| 1326 | ccggttctga aggtgttctt gt |
| 1327 | tccggttctg aaggtgttct tgt |
| 1328 | ctccggttct gaaggtgttc ttgt |
| 1329 | cctccggttc tgaaggtgtt cttgt |
| 1330 | gcctccggtt ctgaaggtgt tcttgt |
| 1331 | tgcctccggt tctgaaggtg ttcttgt |
| 1332 | ccggttctga aggtgttctt g |
| 1333 | tccggttctg aaggtgttct tg |
| 1334 | ctccggttct gaaggtgttc ttg |
| 1335 | cctccggttc tgaaggtgtt cttg |
| 1336 | gcctccggtt ctgaaggtgt tcttg |
| 1337 | tgcctccggt tctgaaggtg ttcttg |
| 1338 | ccggttctga aggtgttctt |
| 1339 | tccggttctg aaggtgttct t |
| 1340 | ctccggttct gaaggtgttc tt |
| 1341 | cctccggttc tgaaggtgtt ctt |
| 1342 | gcctccggtt ctgaaggtgt tctt |
| 1343 | tgcctccggt tctgaaggtg ttctt |
| 1344 | ccggttctga aggtgttct |
| 1345 | tccggttctg aaggtgttct |
| 1346 | ctccggttct gaaggtgttc t |
| 1347 | cctccggttc tgaaggtgtt ct |
| 1348 | gcctccggtt ctgaaggtgt tct |
| 1349 | tgcctccggt tctgaaggtg ttct |
| 1350 | ccggttctga aggtgttc |
| 1351 | tccggttctg aaggtgttc |
| 1352 | ctccggttct gaaggtgttc |
| 1353 | cctccggttc tgaaggtgtt c |
| 1354 | gcctccggtt ctgaaggtgt tc |
| 1355 | tgcctccggt tctgaaggtg ttc |
| 1356 | cattcaactg ttgcctccgg ttctgaaggt g |
| 1357 | ttgcctccgg ttctgaaggt gttcttgtac |
| 1358 | aggatttgga acagaggcgt c |
| 1359 | gtctgccact ggcggaggtc |
| 1360 | catcaagcag aaggcaacaa |
| 1361 | gaagtttcag ggccaagtca |
| 1362 | cgggcttgga cagaacttac |
| 1363 | tccttacggg tagcatcctg |
| 1364 | ctgaaggtgt tcttgtactt catcc |
| 1365 | tgttgagaaa tggcggcgt |
| 1366 | cauucaacug uugccuccgg uucugaaggu g |
| 1367 | ucccacugau ucugaauucu uucaa |
| 1368 | cuucauccca cugauucuga auucu |
| 1369 | uuguacuuca ucccacugau ucuga |
| 1370 | uguucuugua cuucauccca cugau |
| 1371 | gaagguguuc uuguacuuca uccca |
| 1372 | guucugaagg uguucuugua cuuca |
| 1373 | cuccgguucu gaagguguuc uugua |
| 1374 | guugccuccg guucugaagg uguuc |
| 1375 | caacuguugc cuccgguucu gaagg |
| 1376 | ucauucaacu guugccuccg guucu |
| 1377 | acauuucauu caacuguugc cuccg |
| 1378 | cuuuaacauu ucauucaacu guugc |
| 1379 | gaauccuuua acauuucauu caacu |
| 1380 | guguugaauc cuuuaacauu ucauu |
| 1381 | ccauuguguu gaauccuuua acauu |
| 1382 | uccagccauu guguugaauc cuuua |
| 1383 | uagcuuccag ccauuguguu gaauc |
| 1384 | uuccuuagcu uccagccauu guguu |
| 1385 | gcuucuuccu uagcuuccag ccauu |
| 1386 | gcucagcuuc uuccuuagcu uccag |
| 1387 | gaccugcuca gcuucuuccu uagcu |
| 1388 | ccuaagaccu gcucagcuuc uuccu |
| 1389 | ccuguccuaa gaccugcuca gcuuc |
| 1390 | ucuggccugu ccuaagaccu gcuca |
| 1391 | uuggcucugg ccuguccuaa gaccu |
| 1392 | caagcuuggc ucuggccugu ccuaa |
| 1393 | ugacucaagc uuggcucugg ccugu |
| 1394 | uuccaugacu caagcuuggc ucugg |
| 1395 | ccuccuucca ugacucaagc uuggc |
| 1396 | gggacccucc uuccaugacu caagc |
| 1397 | guauagggac ccuccuucca ugacu |
| 1398 | cuacuguaua gggacccucc uucca |
| 1399 | ugcaucuacu guauagggac ccucc |
| 1400 | uggauugcau cuacuguaua gggac |
| 1401 | ucuuuuggau ugcaucuacu guaua |
| 1402 | gauuuucuuu uggauugcau cuacu |
| 1403 | ucugugauuu ucuuuuggau ugcau |
| 1404 | ugguuucugu gauuuucuuu uggau |
| 1405 | ccuuagcuuc cagccauugu guuga |
| 1406 | ucuuccuuag cuuccagcca uugug |
| 1407 | ggcucuggcc uguccuaaga ccugc |
| 1408 | agcuuggcuc uggccugucc uaaga |
| 1409 | cucaagcuug gcucuggccu guccu |
| 1410 | gacccuccuu ccaugacuca agcuu |
| 1411 | auagggaccc uccuuccaug acuca |
| 1412 | cuguauaggg acccuccuuc cauga |
| 1413 | ugugauuuuc uuuuggauug caucu |
| 1414 | guuucuguga uuuucuuuug gauug |
| 1415 | cuugguuucu gugauuuucu uuugg |
| 1416 | ccgguucuga agguguucuu guacu |
| 1417 | uccgguucug aagguguucu uguac |
| 1418 | ccuccgguuc ugaagguguu cuugu |
| 1419 | gccuccgguu cugaaggugu ucuug |
| 1420 | ugccuccggu ucugaaggug uucuu |
| 1421 | uugccuccgg uucugaaggu guucu |
| 1422 | uguugccucc gguucugaag guguu |
| 1423 | cuguugccuc cgguucugaa ggugu |
| 1424 | acuguugccu ccgguucuga aggug |
| 1425 | aacuguugcc uccgguucug aaggu |
| 1426 | uguugccucc gguucugaag guguucuugu |
| 1427 | gguucugaag guguucuugu |
| 1428 | uccgguucug aagguguucu |
| 1429 | ccuccgguuc ugaagguguu |
| 1430 | uugccuccgg uucugaaggu |
| 1431 | uguugccucc gguucugaag |
| 1432 | uucugaaggu guucuugu |
| 1433 | cgguucugaa gguguucu |
| 1434 | cuccgguucu gaaggugu |
| 1435 | ugccuccggu ucugaagg |
| 1436 | uguugccucc gguucuga |
| 1437 | uucugaaggu guucu |
| 1438 | uccgguucug aaggu |
| 1439 | uugccuccgg uucug |
| 1440 | cuguugccuc cgguucug |
| 1441 | caatgccatc ctggagttcc t |
| 1442 | ccaatgccat cctggagttc c |
| 1443 | cccaatgcca tcctggagtt c |
| 1444 | gcccaatgcc atcctggagt t |
| 1445 | tgcccaatgc catcctggag t |
| 1446 | cccaatgcca tcctggagtt cctgt |
| 1447 | cagtttgccg ctgcccaatg ccatcctgga |
| 1448 | ccaatgccat cctggagttc |
| 1449 | cccaatgcca tcctggagtt |
| 1450 | cccaatgcca tcctggagtt c |
| 1451 | gcugcccaau gccauccugg aguuc |
| 1452 | uugccgcugc ccaaugccau ccugg |
| 1453 | acaguuugcc gcugcccaau gccau |
| 1454 | ugacaacagu uugccgcugc ccaau |
| 1455 | uguucugaca acaguuugcc gcugc |
| 1456 | uucaauguuc ugacaacagu uugcc |
| 1457 | uugcauucaa uguucugaca acagu |
| 1458 | cccaguugca uucaauguuc ugaca |
| 1459 | ucuuccccag uugcauucaa uguuc |
| 1460 | uuauuucuuc cccaguugca uucaa |
| 1461 | cugaauuauu ucuuccccag uugca |
| 1462 | gauugcugaa uuauuucuuc cccag |
| 1463 | uugaggauug cugaauuauu ucuuc |
| 1464 | uguuuuugag gauugcugaa uuauu |
| 1465 | gcaucuguuu uugaggauug cugaa |
| 1466 | uacuggcauc uguuuuugag gauug |
| 1467 | uuugccgcug cccaaugcca uccug |
| 1468 | gctcaggtcg gattgacatt |
| 1469 | gggcaactct tccaccagta |
| 1470 | cctgagaatt gggaacatgc |
| 1471 | ttgctgctct tttccaggtt |
| 1472 | agcagcctct cgctcactca c |
| 1473 | ttccaaagca gcctctcgct c |
| 1474 | ttcttccaaa gcagcctctc g |
| 1475 | agtttcttcc aaagcagcct c |
| 1476 | tttcttccaa agcagcctct c |
| 1477 | gtttcttcca aagcagcctc t |
| 1478 | gagtttcttc caaagcagcc t |
| 1479 | tgagtttctt ccaaagcagc c |
| 1480 | gcagccucuc gcucacucac c |
| 1481 | ccaaagcagc cucucgcuca c |
| 1482 | uucuuccaaa gcagccucuc g |
| 1483 | aucuaugagu uucuuccaaa g |
| 1484 | uguugcagua aucuaugagu u |
| 1485 | cagggggaac uguugcagua a |
| 1486 | uuuccagguc cagggggaac u |
| 1487 | gcaagaaacu uuuccagguc c |
| 1488 | uguaagccag gcaagaaacu u |
| 1489 | uuucagcuuc uguaagccag g |
| 1490 | uuggcaguug uuucagcuuc u |
| 1491 | cuguaggaca uuggcaguug u |
| 1492 | ggguagcauc cuguaggaca u |
| 1493 | cuuuccuuac ggguagcauc c |
| 1494 | uucuaggagc cuuuccuuac g |
| 1495 | ccuuggaguc uucuaggagc c |
| 1496 | ucuuuuacuc ccuuggaguc u |
| 1497 | uuucaucagc ucuuuuacuc c |
| 1498 | uugccauugu uucaucagcu |
| 1499 | uccuguagga cauuggcagu |
| 1500 | catggaagga gggtccctat |
| 1501 | ctgccggctt aattcatcat |
| 1502 | ataatgaaaa cgccgccatt t |
| 1503 | tcataatgaa aacgccgcca t |
| 1504 | atcataatga aaacgccgcc a |
| 1505 | tatcataatg aaaacgccgc c |
| 1506 | atatcataat gaaaacgccg c |
| 1507 | tatatcataa tgaaaacgcc g |
| 1508 | ttatatcata atgaaaacgc c |
| 1509 | tgaaaacgcc gccatttctc aacagatctg |
| 1510 | atcataatga aaacgccgcc |
| 1511 | tatcataatg aaaacgccgc |
| 1512 | tatcataatg aaaacgccgc c |
| 1513 | cucaacagau cugucaaauc gc |
| 1514 | gccgccauuu cucaacagau cu |
| 1515 | uaaugaaaac gccgccauuu cu |
| 1516 | uaucauaaug aaaacgccgc ca |
| 1517 | cuuuauauca uaaugaaaac gc |
| 1518 | gauuaaauau cuuuauauca ua |
| 1519 | guuagccacu gauuaaauau cu |
| 1520 | uucagcuucu guuagccacu ga |
| 1521 | ugagaaacug uucagcuucu gu |
| 1522 | ugugucuuuc ugagaaacug uu |
| 1523 | guucccaauu cucaggaauu ug |
| 1524 | uauuuagcau guucccaauu cu |
| 1525 | auaccauuug uauuuagcau gu |
| 1526 | ucagcuucug uuagccacug |
| 1527 | tccagtatac ttacaggctc c |
| 1528 | atccagtata cttacaggct c |
| 1529 | gatccagtat acttacaggc t |
| 1530 | ggatccagta tacttacagg c |
| 1531 | gggatccagt atacttacag g |
| 1532 | cttacaggct ccaatagtgg tcagt |
| 1533 | gggatccagt atacttacag gctcc |
| 1534 | aacaaccgs4a tgtggaagag |
| 1535 | ttggagatgg cagtttcctt |
| 1536 | cauuucucaa cagaucuguc aa |
| 1537 | aaaacgccgc cauuucucaa ca |
| 1538 | aauaucuuua uaucauaaug aa |
| 1539 | cuucuguuag ccacugauua aa |
| 1540 | aacuguucag cuucuguuag cc |
| 1541 | cuuucugaga aacuguucag cu |
| 1542 | gaauuugugu cuuucugaga aa |
| 1543 | cucaggaauu ugugucuuuc ug |
| 1544 | caauucucag gaauuugugu cu |
| 1545 | agcauguucc caauucucag ga |
| 1546 | gagtcttcta ggagcctt |
| 1547 | gtttcttcca aagcagcctc |
| 1548 | agtttcttcc aaagcagcct |
| 1549 | agtttcttcc aaagcagcct c |
| 1550 | ggatccagta tacttacagg |
| 1551 | gggatccagt atacttacag |
| 1552 | tgggatccag tatacttaca g |
| 1553 | atgggatcca gtatacttac a |
| 1554 | guggcuaaca gaagcu |
| 1555 | gggaacaugc uaaauac |
| 1556 | agacacaaau uccugaga |
| 1557 | cuguugagaa a |
| 1558 | ucagcuucug uuagccacug |
| 1559 | uucagcuucu guuagccacu |
| 1560 | uucagcuucu guuagccacu g |
| 1561 | ucagcuucug uuagccacug a |
| 1562 | uucagcuucu guuagccacu ga |
| 1563 | ucagcuucug uuagccacug a |
| 1564 | uucagcuucu guuagccacu ga |
| 1565 | ucagcuucug uuagccacug au |
| 1566 | uucagcuucu guuagccacu gau |
| 1567 | ucagcuucug uuagccacug auu |
| 1568 | uucagcuucu guuagccacu gauu |
| 1569 | ucagcuucug uuagccacug auua |
| 1570 | uucagcuucu guuagccacu gaua |
| 1571 | ucagcuucug uuagccacug auuaa |
| 1572 | uucagcuucu guuagccacu gauuaa |
| 1573 | ucagcuucug uuagccacug auuaaa |
| 1574 | uucagcuucu guuagccacu gauuaaa |
| 1575 | cagcuucugu uagccacug |
| 1576 | cagcuucugu uagccacuga u |
| 1577 | agcuucuguu agccacugau u |
| 1578 | cagcuucugu uagccacuga uu |
| 1579 | agcuucuguu agccacugau ua |
| 1580 | cagcuucugu uagccacuga uua |
| 1581 | agcuucuguu agccacugau uaa |
| 1582 | cagcuucugu uagccacuga uuaa |
| 1583 | agcuucuguu agccacugau uaaa |
| 1584 | cagcuucugu uagccacuga uuaaa |
| 1585 | agcuucuguu agccacugau uaaa |
| 1586 | agcuucuguu agccacugau |
| 1587 | gcuucuguua gccacugauu |
| 1588 | agcuucuguu agccacugau u |
| 1589 | gcuucuguua gccacugauu a |
| 1590 | agcuucuguu agccacugau ua |
| 1591 | gcuucuguua gccacugauu aa |
| 1592 | agcuucuguu agccacugau uaa |
| 1593 | gcuucuguua gccacugauu aaa |
| 1594 | agcuucuguu agccacugau uaaa |
| 1595 | gcuucuguua gccacugauu aaa |
| 1596 | ccauuuguau uuagcauguu ccc |
| 1597 | agauaccauu uguauuuagc |
| 1598 | gccauuucuc aacagaucu |
| 1599 | gccauuucuc aacagaucug uca |
| 1600 | auucucagga auuugugucu uuc |
| 1601 | ucucaggaau uugugucuuu c |
| 1602 | guucagcuuc uguuagcc |
| 1603 | cugauuaaau aucuuuauau c |
| 1604 | gccgccauuu cucaacag |
| 1605 | guauuuagca uguuccca |
| 1606 | caggaauuug ugucuuuc |
| 1607 | ucuguuagcc acugauuaaa u |
| 1608 | cgaccugagc uuuguuguag |
| 1609 | cgaccugagc uuuguuguag acuau |
| 1610 | ccugagcuuu guuguagacu auc |
| 1611 | cguugcacuu ugcaaugcug cug |
| 1612 | cuguagcuuc acccuuucc |
| 1613 | gagagagcuu ccuguagcuu cacc |
| 1614 | guccuuguac auuuuguuaa cuuuuuc |
| 1615 | ucagcuucug uuagccacug |
| 1616 | uucagcuucu guuagccacu |
| 1617 | uucagcuucu guuagccacu g |
| 1618 | ucagcuucug uuagccacug a |
| 1619 | uucagcuucu guuagccacu ga |
| 1620 | ucagcuucug uuagccacug a |
| 1621 | uucagcuucu guuagccacu ga |
| 1622 | ucagcuucug uuagccacug au |
| 1623 | uucagcuucu guuagccacu gau |
| 1624 | ucagcuucug uuagccacug auu |
| 1625 | uucagcuucu guuagccacu gauu |
| 1626 | ucagcuucug uuagccacug auua |
| 1627 | uucagcuucu guuagccacu gaua |
| 1628 | ucagcuucug uuagccacug auuaa |
| 1629 | uucagcuucu guuagccacu gauuaa |
| 1630 | ucagcuucug uuagccacug auuaaa |
| 1631 | uucagcuucu guuagccacu gauuaaa |
| 1632 | cagcuucugu uagccacug |
| 1633 | cagcuucugu uagccacuga u |
| 1634 | agcuucuguu agccacugau u |
| 1635 | cagcuucugu uagccacuga uu |
| 1636 | agcuucuguu agccacugau ua |
| 1637 | cagcuucugu uagccacuga uua |
| 1638 | agcuucuguu agccacugau uaa |
| 1639 | cagcuucugu uagccacuga uuaa |
| 1640 | agcuucuguu agccacugau uaaa |
| 1641 | cagcuucugu uagccacuga uuaaa |
| 1642 | agcuucuguu agccacugau uaaa |
| 1643 | agcuucuguu agccacugau |
| 1644 | gcuucuguua gccacugauu |
| 1645 | agcuucuguu agccacugau u |
| 1646 | gcuucuguua gccacugauu a |
| 1647 | agcuucuguu agccacugau ua |
| 1648 | gcuucuguua gccacugauu aa |
| 1649 | agcuucuguu agccacugau uaa |
| 1650 | gcuucuguua gccacugauu aaa |
| 1651 | agcuucuguu agccacugau uaaa |
| 1652 | gcuucuguua gccacugauu aaa |
| 1653 | ccauuuguau uuagcauguu ccc |
| 1654 | agauaccauu uguauuuagc |
| 1655 | gccauuucuc aacagaucu |
| 1656 | gccauuucuc aacagaucug uca |
| 1657 | auucucagga auuugugucu uuc |
| 1658 | ucucaggaau uugugucuuu c |
| 1659 | guucagcuuc uguuagcc |
| 1660 | cugauuaaau aucuuuauau c |
| 1661 | gccgccauuu cucaacag |
| 1662 | guauuuagca uguuccca |
| 1663 | caggaauuug ugucuuuc |
| 1664 | uuugccgcug cccaaugcca uccug |
| 1665 | auucaauguu cugacaacag uuugc |
| 1666 | ccaguugcau ucaauguucu gacaa |
| 1667 | caguugcauu caauguucug ac |
| 1668 | aguugcauuc aauguucuga |
| 1669 | gauugcugaa uuauuucuuc c |
| 1670 | gauugcugaa uuauuucuuc cccag |
| 1671 | auugcugaau uauuucuucc ccagu |
| 1672 | uugcugaauu auuucuuccc caguu |
| 1673 | ugcugaauua uuucuucccc aguug |
| 1674 | gcugaauuau uucuucccca guugc |
| 1675 | cugaauuauu ucuuccccag uugca |
| 1676 | ugaauuauuu cuuccccagu ugcau |
| 1677 | gaauuauuuc uuccccaguu gcauu |
| 1678 | aauuauuucu uccccaguug cauuc |
| 1679 | auuauuucuu ccccaguugc auuca |
| 1680 | uuauuucuuc cccaguugca uucaa |
| 1681 | uauuucuucc ccaguugcau ucaau |
| 1682 | auuucuuccc caguugcauu caaug |
| 1683 | uuucuucccc aguugcauuc aaugu |
| 1684 | uucuucccca guugcauuca auguu |
| 1685 | ucuuccccag uugcauucaa uguuc |
| 1686 | cuuccccagu ugcauucaau guucu |
| 1687 | uuccccaguu gcauucaaug uucug |
| 1688 | uccccaguug cauucaaugu ucuga |
| 1689 | ccccaguugc auucaauguu cugac |
| 1690 | cccaguugca uucaauguuc ugaca |
| 1691 | ccaguugcau ucaauguucu gacaa |
| 1692 | caguugcauu caauguucug acaac |
| 1693 | aguugcauuc aauguucuga caaca |
| 1694 | guugcauuca auguucugac aacag |
| 1695 | uugcauucaa uguucugaca acagu |
| 1696 | ugcauucaau guucugacaa caguu |
| 1697 | gcauucaaug uucugacaac aguuu |
| 1698 | cauucaaugu ucugacaaca guuug |
| 1699 | auucaauguu cugacaacag uuugc |
| 1700 | ucaauguucu gacaacaguu ugccg |
| 1701 | caauguucug acaacaguuu gccgc |
| 1702 | aauguucuga caacaguuug ccgcu |
| 1703 | auguucugac aacaguuugc cgcug |
| 1704 | uguucugaca acaguuugcc gcugc |
| 1705 | guucugacaa caguuugccg cugcc |
| 1706 | uucugacaac aguuugccgc ugccc |
| 1707 | ucugacaaca guuugccgcu gccca |
| 1708 | cugacaacag uuugccgcug cccaa |
| 1709 | ugacaacagu uugccgcugc ccaau |
| 1710 | gacaacaguu ugccgcugcc caaug |
| 1711 | acaacaguuu gccgcugccc aaugc |
| 1712 | caacaguuug ccgcugccca augcc |
| 1713 | aacaguuugc cgcugcccaa ugcca |
| 1714 | acaguuugcc gcugcccaau gccau |
| 1715 | caguuugccg cugcccaaug ccauc |
| 1716 | aguuugccgc ugcccaaugc caucc |
| 1717 | guuugccgcu gcccaaugcc auccu |
| 1718 | uuugccgcug cccaaugcca uccug |
| 1719 | uugccgcugc ccaaugccau ccugg |
| 1720 | ugccgcugcc caaugccauc cugga |
| 1721 | gccgcugccc aaugccaucc uggag |
| 1722 | ccgcugccca augccauccu ggagu |
| 1723 | cgcugcccaa ugccauccug gaguu |
| 1724 | gcuuuucuuu uaguugcugc ucuuu |
| 1725 | cuuuucuuuu aguugcugcu cuuuu |
| 1726 | uuuucuuuua guugcugcuc uuuuc |
| 1727 | uuucuuuuag uugcugcucu uuucc |
| 1728 | uucuuuuagu ugcugcucuu uucca |
| 1729 | ucuuuuaguu gcugcucuuu uccag |
| 1730 | cuuuuaguug cugcucuuuu ccagg |
| 1731 | uuuuaguugc ugcucuuuuc caggu |
| 1732 | uuuaguugcu gcucuuuucc agguu |
| 1733 | uuaguugcug cucuuuucca gguuc |
| 1734 | uaguugcugc ucuuuuccag guuca |
| 1735 | aguugcugcu cuuuuccagg uucaa |
| 1736 | guugcugcuc uuuuccaggu ucaag |
| 1737 | uugcugcucu uuuccagguu caagu |
| 1738 | ugcugcucuu uuccagguuc aagug |
| 1739 | gcugcucuuu uccagguuca agug g |
| 1740 | cugcucuuuu ccagguucaa guggg |
| 1741 | ugcucuuuuc cagguucaag uggga |
| 1742 | gcucuuuucc agguucaagu gggac |
| 1743 | cucuuuucca gguucaagug ggaua |
| 1744 | ucuuuuccag guucaagugg gauac |
| 1745 | cuuuuccagg uucaaguggg auacu |
| 1746 | uuuuccaggu ucaaguggga uacua |
| 1747 | uuuccagguu caagugggau acuag |
| 1748 | uuccagguuc aagugggaua cuagc |
| 1749 | uccagguuca agugggauac uagca |
| 1750 | ccagguucaa gugggauacu agcaa |
| 1751 | cagguucaag ugggauacua gcaau |
| 1752 | agguucaagu gggauacuag caaug |
| 1753 | gguucaagug ggauacuagc aaugu |
| 1754 | guucaagugg gauacuagca auguu |
| 1755 | uucaaguggg auacuagcaa uguua |
| 1756 | ucaaguggga uacuagcaau guuau |
| 1757 | caagugggau acuagcaaug uuauc |
| 1758 | aagugggaua cuagcaaugu uaucu |
| 1759 | agugggauac uagcaauguu aucug |
| 1760 | gugggauacu agcaauguua ucugc |
| 1761 | ugggauacua gcaauguuau cugcu |
| 1762 | gggauacuag caauguuauc ugcuu |
| 1763 | ggauacuagc aauguuaucu gcuuc |
| 1764 | gauacuagca auguuaucug cuucc |
| 1765 | auacuagcaa uguuaucugc uuccu |
| 1766 | uacuagcaau guuaucugcu uccuc |
| 1767 | acuagcaaug uuaucugcuu ccucc |
| 1768 | cuagcaaugu uaucugcuuc cucca |
| 1769 | uagcaauguu aucugcuucc uccaa |
| 1770 | agcaauguua ucugcuuccu ccaac |
| 1771 | gcaauguuau cugcuuccuc caacc |
| 1772 | caauguuauc ugcuuccucc aacca |
| 1773 | aauguuaucu gcuuccucca accau |
| 1774 | auguuaucug cuuccuccaa ccaua |
| 1775 | uguuaucugc uuccuccaac cauaa |
| 1776 | guuaucugcu uccuccaacc auaaa |
| 1777 | gcugcucuuu uccagguuc |
| 1778 | ucuuuuccag guucaagugg |
| 1779 | agguucaagu gggauacua |
| 1780 | cucagcucuu gaaguaaacg |
| 1781 | ccucagcucu ugaaguaaac |
| 1782 | ccucagcucu ugaaguaaac g |
| 1783 | auagugguca guccaggagc u |
| 1784 | caguccagga gcuaggucag g |
| 1785 | uaguggucag uccaggagcu agguc |
| 1786 | agagcaggua ccuccaacau caagg |
| 1787 | gagcagguac cuccaacauc aagga |
| 1788 | agcagguacc uccaacauca aggaa |
| 1789 | gcagguaccu ccaacaucaa ggaag |
| 1790 | cagguaccuc caacaucaag gaaga |
| 1791 | agguaccucc aacaucaagg aagau |
| 1792 | gguaccucca acaucaagga agaug |
| 1793 | guaccuccaa caucaaggaa gaugg |
| 1794 | uaccuccaac aucaaggaag auggc |
| 1795 | accuccaaca ucaaggaaga uggca |
| 1796 | ccuccaacau caaggaagau ggcau |
| 1797 | cuccaacauc aaggaagaug gcauu |
| 1798 | cuccaacauc aaggaagaug gcauuucuag |
| 1799 | uccaacauca aggaagaugg cauuu |
| 1800 | ccaacaucaa ggaagauggc auuuc |
| 1801 | caacaucaag gaagauggca uuucu |
| 1802 | aacaucaagg aagauggcau uucua |
| 1803 | acaucaagga agauggcauu ucuag |
| 1804 | acaucaagga agauggcauu ucuaguuugg |
| 1805 | acaucaagga agauggcauu ucuag |
| 1806 | caucaaggaa gauggcauuu cuagu |
| 1807 | aucaaggaag auggcauuuc uaguu |
| 1808 | ucaaggaaga uggcauuucu aguuu |
| 1809 | ucaaggaaga uggcauuucu |
| 1810 | caaggaagau ggcauuucua guuug |
| 1811 | aaggaagaug gcauuucuag uuugg |
| 1812 | aggaagaugg cauuucuagu uugga |
| 1813 | ggaagauggc auuucuaguu uggag |
| 1814 | gaagauggca uuucuaguuu ggaga |
| 1815 | aagauggcau uucuaguuug gagau |
| 1816 | agauggcauu ucuaguuugg agaug |
| 1817 | gauggcauuu cuaguuugga gaugg |
| 1818 | auggcauuuc uaguuuggag auggc |
| 1819 | uggcauuucu aguuuggaga uggca |
| 1820 | ggcauuucua guuuggagau ggcag |
| 1821 | gcauuucuag uuuggagaug gcagu |
| 1822 | cauuucuagu uuggagaugg caguu |
| 1823 | auuucuaguu uggagauggc aguuu |
| 1824 | uuucuaguuu ggagauggca guuuc |
| 1825 | uucuaguuug gagauggcag uuucc |
| 1826 | ccucuugauu gcuggucuug uuuuu |
| 1827 | cucuugauug cuggucuugu uuuuc |
| 1828 | ucuugauugc uggucuuguu uuuca |
| 1829 | cuugauugcu ggucuuguuu uucaa |
| 1830 | uugauugcug gucuuguuuu ucaaa |
| 1831 | ugauugcugg ucuuguuuuu caaau |
| 1832 | gauugcuggu cuuguuuuuc aaauu |
| 1833 | auugcugguc uuguuuuuca aauuu |
| 1834 | uugcuggucu uguuuuucaa auuuu |
| 1835 | ugcuggucuu guuuuucaaa uuuug |
| 1836 | gcuggucuug uuuuucaaau uuugg |
| 1837 | cuggucuugu uuuucaaauu uuggg |
| 1838 | uggucuuguu uuucaaauuu ugggc |
| 1839 | ggucuuguuu uucaaauuuu gggca |
| 1840 | gucuuguuuu ucaaauuuug ggcag |
| 1841 | ucuuguuuuu caaauuuugg gcagc |
| 1842 | cuuguuuuuc aaauuuuggg cagcg |
| 1843 | uuguuuuuca aauuuugggc agcgg |
| 1844 | uguuuuucaa auuuugggca gcggu |
| 1845 | guuuuucaaa uuuugggcag cggua |
| 1846 | uuuuucaaau uuugggcagc gguaa |
| 1847 | uuuucaaauu uugggcagcg guaau |
| 1848 | uuucaaauuu ugggcagcgg uaaug |
| 1849 | uucaaauuuu gggcagcggu aauga |
| 1850 | ucaaauuuug ggcagcggua augag |
| 1851 | caaauuuugg gcagcgguaa ugagu |
| 1852 | aaauuuuggg cagcgguaau gaguu |
| 1853 | aauuuugggc agcgguaaug aguuc |
| 1854 | auuuugggca gcgguaauga guucu |
| 1855 | ccauuguguu gaauccuuua acauu |
| 1856 | ccauuguguu gaauccuuua ac |
| 1857 | auuguguuga auccuuuaac |
| 1858 | ccuguccuaa gaccugcuca |
| 1859 | cuuuuggauu gcaucuacug uauag |
| 1860 | cauucaacug uugccuccgg uucug |
| 1861 | cuguugccuc cgguucugaa ggug |
| 1862 | cauucaacug uugccuccgg uucugaaggu g |
| 1863 | cugaaggugu ucuuguacuu caucc |
| 1864 | uguauaggga cccuccuucc augacuc |
| 1865 | aucccacuga uucugaauuc |
| 1866 | uuggcucugg ccuguccuaa ga |
| 1867 | aagaccugcu cagcuucuuc cuuagcuucc agcca |
| 1868 | ggagagagcu uccuguagcu |
| 1869 | ucacccuuuc cacaggcguu gca |
| 1870 | ugcacuuugc aaugcugcug ucuucuugcu au |
| 1871 | ucauaaugaa aacgccgcca uuucucaaca gaucu |
| 1872 | uuugugucuu ucugagaaac |
| 1873 | uuuagcaugu ucccaauucu caggaauuug |
| 1874 | uccuguagaa uacuggcauc |
| 1875 | ugcagaccuc cugccaccgc agauuca |
| 1876 | uugcagaccu ccugccaccg cagauucagg cuuc |
| 1877 | uguuuuugag gauugcugaa |
| 1878 | uguucugaca acaguuugcc gcugcccaau gccauccugg |
| 1879 | cucuuuucca gguucaagug ggauacuagc |
| 1880 | caagcuuuuc uuuuaguugc ugcucuuuuc c |
| 1881 | uauucuuuug uucuucuagc cuggagaaag |
| 1882 | cugcuuccuc caaccauaaa acaaauuc |
| 1883 | ccacucagag cucagaucuu cuaacuucc |
| 1884 | cuuccacuca gagcucagau cuucuaa |
| 1885 | caguccagga gcuaggucag gcugcuuugc |
| 1886 | ucuugaagua aacgguuuac cgccuuccac ucagagc |
| 1887 | uccaacuggg gacgccucug uuccaaaucc |
| 1888 | acuggggacg ccucuguucc a |
| 1889 | ccguaaugau uguucuagcc |
| 1890 | uuuugggcag cgguaaugag uucuu |
| 1891 | uuugggcagc gguaaugagu ucuuc |
| 1892 | uugggcagcg guaaugaguu cuucc |
| 1893 | ugggcagcgg uaaugaguuc uucca |
| 1894 | gggcagcggu aaugaguucu uccaa |
| 1895 | ggcagcggua augaguucuu ccaac |
| 1896 | gcagcgguaa ugaguucuuc caacu |
| 1897 | cagcgguaau gaguucuucc aacug |
| 1898 | agcgguaaug aguucuucca acugg |
| 1899 | gcgguaauga guucuuccaa cuggg |
| 1900 | cgguaaugag uucuuccaac ugggg |
| 1901 | gguaaugagu ucuuccaacu gggga |
| 1902 | guaaugaguu cuuccaacug gggac |
| 1903 | uaaugaguuc uuccaacugg ggacg |
| 1904 | aaugaguucu uccaacuggg gacgc |
| 1905 | augaguucuu ccaacugggg acgcc |
| 1906 | ugaguucuuc caacugggga cgccu |
| 1907 | gaguucuucc aacuggggac gccuc |
| 1908 | aguucuucca acuggggacg ccucu |
| 1909 | guucuuccaa cuggggacgc cucug |
| 1910 | uucuuccaac uggggacgcc ucugu |
| 1911 | ucuuccaacu ggggacgccu cuguu |
| 1912 | cuuccaacug gggacgccuc uguuc |
| 1913 | uuccaacugg ggacgccucu guucc |
| 1914 | gauugcuggu cuuguuuuuc |
| 1915 | ccucuugauu gcuggucuug |
| 1916 | gguaaugagu ucuuccaacu gg |
| 1917 | acuggggacg ccucuguucc |
| 1918 | ucaaggaaga uggcauuucu |
| 1919 | ggccaaaccu cggcuuaccu |
| 1920 | uuugccgcug cccaaugcca uccug |
| 1921 | auucaauguu cugacaacag uuugc |
| 1922 | ccaguugcau ucaauguucu gacaa |
| 1923 | caguugcauu caauguucug ac |
| 1924 | aguugcauuc aauguucuga |
| 1925 | gauugcugaa uuauuucuuc c |
| 1926 | gauugcugaa uuauuucuuc cccag |
| 1927 | auugcugaau uauuucuucc ccagu |
| 1928 | uugcugaauu auuucuuccc caguu |
| 1929 | ugcugaauua uuucuucccc aguug |
| 1930 | gcugaauuau uucuucccca guugc |
| 1931 | cugaauuauu ucuuccccag uugca |
| 1932 | ugaauuauuu cuuccccagu ugcau |
| 1933 | gaauuauuuc uuccccaguu gcauu |
| 1934 | aauuauuucu uccccaguug cauuc |
| 1935 | auuauuucuu ccccaguugc auuca |
| 1936 | uuauuucuuc cccaguugca uucaa |
| 1937 | uauuucuucc ccaguugcau ucaau |
| 1938 | auuucuuccc caguugcauu caaug |
| 1939 | uuucuucccc aguugcauuc aaugu |
| 1940 | uucuucccca guugcauuca auguu |
| 1941 | ucuuccccag uugcauucaa uguuc |
| 1942 | cuuccccagu ugcauucaau guucu |
| 1943 | uuccccaguu gcauucaaug uucug |
| 1944 | uccccaguug cauucaaugu ucuga |
| 1945 | ccccaguugc auucaauguu cugac |
| 1946 | cccaguugca uucaauguuc ugaca |
| 1947 | ccaguugcau ucaauguucu gacaa |
| 1948 | caguugcauu caauguucug acaac |
| 1949 | aguugcauuc aauguucuga caaca |
| 1950 | uccuguagaa uacuggcauc |
| 1951 | ugcagaccuc cugccaccgc agauuca |
| 1952 | uugcagaccu ccugccaccg cagauucagg cuuc |
| 1953 | guugcauuca auguucugac aacag |
| 1954 | uugcauucaa uguucugaca acagu |
| 1955 | ugcauucaau guucugacaa caguu |
| 1956 | gcauucaaug uucugacaac aguuu |
| 1957 | cauucaaugu ucugacaaca guuug |
| 1958 | auucaauguu cugacaacag uuugc |
| 1959 | ucaauguucu gacaacaguu ugccg |
| 1960 | caauguucug acaacaguuu gccgc |
| 1961 | aauguucuga caacaguuug ccgcu |
| 1962 | auguucugac aacaguuugc cgcug |
| 1963 | uguucugaca acaguuugcc gcugc |
| 1964 | guucugacaa caguuugccg cugcc |
| 1965 | uucugacaac aguuugccgc ugccc |
| 1966 | ucugacaaca guuugccgcu gccca |
| 1967 | cugacaacag uuugccgcug cccaa |
| 1968 | ugacaacagu uugccgcugc ccaau |
| 1969 | gacaacaguu ugccgcugcc caaug |
| 1970 | acaacaguuu gccgcugccc aaugc |
| 1971 | caacaguuug ccgcugccca augcc |
| 1972 | aacaguuugc cgcugcccaa ugcca |
| 1973 | acaguuugcc gcugcccaau gccau |
| 1974 | caguuugccg cugcccaaug ccauc |
| 1975 | aguuugccgc ugcccaaugc caucc |
| 1976 | guuugccgcu gcccaaugcc auccu |
| 1977 | uuugccgcug cccaaugcca uccug |
| 1978 | uugccgcugc ccaaugccau ccugg |
| 1979 | ugccgcugcc caaugccauc cugga |
| 1980 | gccgcugccc aaugccaucc uggag |
| 1981 | ccgcugccca augccauccu ggagu |
| 1982 | cgcugcccaa ugccauccug gaguu |
| 1983 | uguuuuugag gauugcugaa |
| 1984 | uguucugaca acaguuugcc gcugcccaau gccauccugg |
| 1985 | gcccaaugcc auccugg |
| 1986 | agagcaggua ccuccaacau caagg |
| 1987 | gagcagguac cuccaacauc aagga |
| 1988 | agcagguacc uccaacauca aggaa |
| 1989 | gcagguaccu ccaacaucaa ggaag |
| 1990 | cagguaccuc caacaucaag gaaga |
| 1991 | agguaccucc aacaucaagg aagau |
| 1992 | gguaccucca acaucaagga agaug |
| 1993 | guaccuccaa caucaaggaa gaugg |
| 1994 | uaccuccaac aucaaggaag auggc |
| 1995 | accuccaaca ucaaggaaga uggca |
| 1996 | ccuccaacau caaggaagau ggcau |
| 1997 | cuccaacauc aaggaagaug gcauu |
| 1998 | cuccaacauc aaggaagaug gcauuucuag |
| 1999 | uccaacauca aggaagaugg cauuu |
| 2000 | ccaacaucaa ggaagauggc auuuc |
| 2001 | caacaucaag gaagauggca uuucu |
| 2002 | aacaucaagg aagauggcau uucua |
| 2003 | acaucaagga agauggcauu ucuag |
| 2004 | acaucaagga agauggcauu ucuaguuugg |
| 2005 | acaucaagga agauggcauu ucuag |
| 2006 | caucaaggaa gauggcauuu cuagu |
| 2007 | aucaaggaag auggcauuuc uaguu |
| 2008 | ucaaggaaga uggcauuucu aguuu |
| 2009 | ucaaggaaga uggcauuucu |
| 2010 | caaggaagau ggcauuucua guuug |
| 2011 | aaggaagaug gcauuucuag uuugg |
| 2012 | aggaagaugg cauuucuagu uugga |
| 2013 | ggaagauggc auuucuaguu uggag |
| 2014 | gaagauggca uuucuaguuu ggaga |
| 2015 | aagauggcau uucuaguuug gagau |
| 2016 | agauggcauu ucuaguuugg agaug |
| 2017 | gauggcauuu cuaguuugga gaugg |
| 2018 | auggcauuuc uaguuuggag auggc |
| 2019 | uggcauuucu aguuuggaga uggca |
| 2020 | ggcauuucua guuuggagau ggcag |
| 2021 | gcauuucuag uuuggagaug gcagu |
| 2022 | cauuucuagu uuggagaugg caguu |
| 2023 | auuucuaguu uggagauggc aguuu |
| 2024 | uuucuaguuu ggagauggca guuuc |
| 2025 | uucuaguuug gagauggcag uuucc |
| 2026 | ccauuguguu gaauccuuua acauu |
| 2027 | ccauuguguu gaauccuuua ac |
| 2028 | auuguguuga auccuuuaac |
| 2029 | ccuguccuaa gaccugcuca |
| 2030 | cuuuuggauu gcaucuacug uauag |
| 2031 | cauucaacug uugccuccgg uucug |
| 2032 | cuguugccuc cgguucugaa ggug |
| 2033 | cauucaacug uugccuccgg uucugaaggu g |
| 2034 | cugaaggugu ucuuguacuu caucc |
| 2035 | uguauaggga cccuccuucc augacuc |
| 2036 | aucccacuga uucugaauuc |
| 2037 | uuggcucugg ccuguccuaa ga |
| 2038 | aagaccugcu cagcuucuuc cuuagcuucc agcca |
| 2039 | ugcauguucc agucguugug ugg |
| 2040 | cacuauucca gucaaauagg ucugg |
| 2041 | auuuaccaac cuucaggauc gagua |
| 2042 | ggccuaaaac acauacacau a |
| 2043 | ucagcuucug uuagccacug |
| 2044 | uucagcuucu guuagccacu |
| 2045 | uucagcuucu guuagccacu g |
| 2046 | ucagcuucug uuagccacug a |
| 2047 | uucagcuucu guuagccacu ga |
| 2048 | ucagcuucug uuagccacug a |
| 2049 | uucagcuucu guuagccacu ga |
| 2050 | ucagcuucug uuagccacug au |
| 2051 | uucagcuucu guuagccacu gau |
| 2052 | ucagcuucug uuagccacug auu |
| 2053 | uucagcuucu guuagccacu gauu |
| 2054 | ucagcuucug uuagccacug auua |
| 2055 | uucagcuucu guuagccacu gaua |
| 2056 | ucagcuucug uuagccacug auuaa |
| 2057 | uucagcuucu guuagccacu gauuaa |
| 2058 | ucagcuucug uuagccacug auuaaa |
| 2059 | uucagcuucu guuagccacu gauuaaa |
| 2060 | cagcuucugu uagccacug |
| 2061 | cagcuucugu uagccacuga u |
| 2062 | agcuucuguu agccacugau u |
| 2063 | cagcuucugu uagccacuga uu |
| 2064 | agcuucuguu agccacugau ua |
| 2065 | cagcuucugu uagccacuga uua |
| 2066 | agcuucuguu agccacugau uaa |
| 2067 | cagcuucugu uagccacuga uuaa |
| 2068 | agcuucuguu agccacugau uaaa |
| 2069 | cagcuucugu uagccacuga uuaaa |
| 2070 | agcuucuguu agccacugau uaaa |
| 2071 | agcuucuguu agccacugau |
| 2072 | gcuucuguua gccacugauu |
| 2073 | agcuucuguu agccacugau u |
| 2074 | gcuucuguua gccacugauu a |
| 2075 | agcuucuguu agccacugau ua |
| 2076 | gcuucuguua gccacugauu aa |
| 2077 | agcuucuguu agccacugau uaa |
| 2078 | gcuucuguua gccacugauu aaa |
| 2079 | agcuucuguu agccacugau uaaa |
| 2080 | gcuucuguua gccacugauu aaa |
| 2081 | ccauuuguau uuagcauguu ccc |
| 2082 | agauaccauu uguauuuagc |
| 2083 | gccauuucuc aacagaucu |
| 2084 | gccauuucuc aacagaucug uca |
| 2085 | auucucagga auuugugucu uuc |
| 2086 | ucucaggaau uugugucuuu c |
| 2087 | guucagcuuc uguuagcc |
| 2088 | cugauuaaau aucuuuauau c |
| 2089 | gccgccauuu cucaacag |
| 2090 | guauuuagca uguuccca |
| 2091 | caggaauuug ugucuuuc |
| 2092 | gcuuuucuuu uaguugcugc ucuuu |
| 2093 | cuuuucuuuu aguugcugcu cuuuu |
| 2094 | uuuucuuuua guugcugcuc uuuuc |
| 2095 | uuucuuuuag uugcugcucu uuucc |
| 2096 | uucuuuuagu ugcugcucuu uucca |
| 2097 | ucuuuuaguu gcugcucuuu uccag |
| 2098 | cuuuuaguug cugcucuuuu ccagg |
| 2099 | uuuuaguugc ugcucuuuuc caggu |
| 2100 | uuuaguugcu gcucuuuucc agguu |
| 2101 | uuaguugcug cucuuuucca gguuc |
| 2102 | uaguugcugc ucuuuuccag guuca |
| 2103 | aguugcugcu cuuuuccagg uucaa |
| 2104 | guugcugcuc uuuuccaggu ucaag |
| 2105 | uugcugcucu uuuccagguu caagu |
| 2106 | ugcugcucuu uuccagguuc aagug |
| 2107 | gcugcucuuu uccagguuca agugg |
| 2108 | cugcucuuuu ccagguucaa guggg |
| 2109 | ugcucuuuuc cagguucaag uggga |
| 2110 | gcucuuuucc agguucaagu gggac |
| 2111 | cucuuuucca gguucaagug ggaua |
| 2112 | ucuuuuccag guucaagugg gauac |
| 2113 | cuuuuccagg uucaaguggg auacu |
| 2114 | uuuuccaggu ucaaguggga uacua |
| 2115 | uuuccagguu caagugggau acuag |
| 2116 | uuccagguuc aagugggaua cuagc |
| 2117 | uccagguuca agugggauac uagca |
| 2118 | ccagguucaa gugggauacu agcaa |
| 2119 | cagguucaag ugggauacua gcaau |
| 2120 | agguucaagu gggauacuag caaug |
| 2121 | gguucaagug ggauacuagc aaugu |
| 2122 | guucaagugg gauacuagca auguu |
| 2123 | uucaaguggg auacuagcaa uguua |
| 2124 | ucaaguggga uacuagcaau guuau |
| 2125 | caagugggau acuagcaaug uuauc |
| 2126 | aagugggaua cuagcaaugu uaucu |
| 2127 | agugggauac uagcaauguu aucug |
| 2128 | gugggauacu agcaauguua ucugc |
| 2129 | ugggauacua gcaauguuau cugcu |
| 2130 | gggauacuag caauguuauc ugcuu |
| 2131 | ggauacuagc aauguuaucu gcuuc |
| 2132 | gauacuagca auguuaucug cuucc |
| 2133 | auacuagcaa uguuaucugc uuccu |
| 2134 | uacuagcaau guuaucugcu uccuc |
| 2135 | acuagcaaug uuaucugcuu ccucc |
| 2136 | cuagcaaugu uaucugcuuc cucca |
| 2137 | uagcaauguu aucugcuucc uccaa |
| 2138 | agcaauguua ucugcuuccu ccaac |
| 2139 | gcaauguuau cugcuuccuc caacc |
| 2140 | caauguuauc ugcuuccucc aacca |
| 2141 | aauguuaucu gcuuccucca accau |
| 2142 | auguuaucug cuuccuccaa ccaua |
| 2143 | uguuaucugc uuccuccaac cauaa |
| 2144 | guuaucugcu uccuccaacc auaaa |
| 2145 | gcugcucuuu uccagguuc |
| 2146 | ucuuuuccag guucaagugg |
| 2147 | agguucaagu gggauacua |
| 2148 | caauuuuucc cacucaguau u |
| 2149 | uugaaguucc uggagucuu |
| 2150 | uccucaggag gcagcucuaa au |
| 2151 | gcgcugguca caaaauccug uugaac |
| 2152 | cacuugcuug aaaaggucua caaagga |
| 2153 | ggugaauaac uuacaaauuu ggaagc |
| 2154 | ccacaggcgu ugcacuuugc aaugc |
| 2155 | cacaggcguu gcacuuugca augcu |
| 2156 | acaggcguug cacuuugcaa ugcug |
| 2157 | caggcguugc acuuugcaau gcugc |
| 2158 | aggcguugca cuuugcaaug cugcu |
| 2159 | ggcguugcac uuugcaaugc ugcug |
| 2160 | gcguugcacu uugcaaugcu gcugu |
| 2161 | cguugcacuu ugcaaugcug cuguc |
| 2162 | cguugcacuu ugcaaugcug cug |
| 2163 | guugcacuuu gcaaugcugc ugucu |
| 2164 | uugcacuuug caaugcugcu gucuu |
| 2165 | ugcacuuugc aaugcugcug ucuuc |
| 2166 | gcacuuugca augcugcugu cuucu |
| 2167 | cacuuugcaa ugcugcuguc uucuu |
| 2168 | acuuugcaau gcugcugucu ucuug |
| 2169 | cuuugcaaug cugcugucuu cuugc |
| 2170 | uuugcaaugc ugcugucuuc uugcu |
| 2171 | uugcaaugcu gcugucuucu ugcua |
| 2172 | ugcaaugcug cugucuucuu gcuau |
| 2173 | gcaaugcugc ugucuucuug cuaug |
| 2174 | caaugcugcu gucuucuugc uauga |
| 2175 | aaugcugcug ucuucuugcu augaa |
| 2176 | augcugcugu cuucuugcua ugaau |
| 2177 | ugcugcuguc uucuugcuau gaaua |
| 2178 | gcugcugucu ucuugcuaug aauaa |
| 2179 | cugcugucuu cuugcuauga auaau |
| 2180 | ugcugucuuc uugcuaugaa uaaug |
| 2181 | gcugucuucu ugcuaugaau aaugu |
| 2182 | cugucuucuu gcuaugaaua auguc |
| 2183 | ugucuucuug cuaugaauaa uguca |
| 2184 | gucuucuugc uaugaauaau gucaa |
| 2185 | ucuucuugcu augaauaaug ucaau |
| 2186 | cuucuugcua ugaauaaugu caauc |
| 2187 | uucuugcuau gaauaauguc aaucc |
| 2188 | ucuugcuaug aauaauguca auccg |
| 2189 | cuugcuauga auaaugucaa uccga |
| 2190 | uugcuaugaa uaaugucaau ccgac |
| 2191 | ugcuaugaau aaugucaauc cgacc |
| 2192 | gcuaugaaua augucaaucc gaccu |
| 2193 | cuaugaauaa ugucaauccg accug |
| 2194 | uaugaauaau gucaauccga ccuga |
| 2195 | augaauaaug ucaauccgac cugag |
| 2196 | ugaauaaugu caauccgacc ugagc |
| 2197 | gaauaauguc aauccgaccu gagcu |
| 2198 | aauaauguca auccgaccug agcuu |
| 2199 | auaaugucaa uccgaccuga gcuuu |
| 2200 | uaaugucaau ccgaccugag cuuug |
| 2201 | aaugucaauc cgaccugagc uuugu |
| 2202 | augucaaucc gaccugagcu uuguu |
| 2203 | ugucaauccg accugagcuu uguug |
| 2204 | gucaauccga ccugagcuuu guugu |
| 2205 | ucaauccgac cugagcuuug uugua |
| 2206 | caauccgacc ugagcuuugu uguag |
| 2207 | aauccgaccu gagcuuuguu guaga |
| 2208 | auccgaccug agcuuuguug uagac |
| 2209 | uccgaccuga gcuuuguugu agacu |
| 2210 | ccgaccugag cuuuguugua gacua |
| 2211 | cgaccugagc uuuguuguag |
| 2212 | cgaccugagc uuuguuguag acuau |
| 2213 | gaccugagcu uuguuguaga cuauc |
| 2214 | accugagcuu uguuguagac uauca |
| 2215 | ccugagcuuu guuguagacu auc |
| 2216 | gcuuuucuuu uaguugcugc ucuuu |
| 2217 | cuuuucuuuu aguugcugcu cuuuu |
| 2218 | uuuucuuuua guugcugcuc uuuuc |
| 2219 | uuucuuuuag uugcugcucu uuucc |
| 2220 | uucuuuuagu ugcugcucuu uucca |
| 2221 | ucuuuuaguu gcugcucuuu uccag |
| 2222 | cuuuuaguug cugcucuuuu ccagg |
| 2223 | uuuuaguugc ugcucuuuuc caggu |
| 2224 | uuuaguugcu gcucuuuucc agguu |
| 2225 | uuaguugcug cucuuuucca gguuc |
| 2226 | uaguugcugc ucuuuuccag guuca |
| 2227 | aguugcugcu cuuuuccagg uucaa |
| 2228 | guugcugcuc uuuuccaggu ucaag |
| 2229 | uugcugcucu uuuccagguu caagu |
| 2230 | ugcugcucuu uuccagguuc aagug |
| 2231 | gcugcucuuu uccagguuca agugg |
| 2232 | cugcucuuuu ccagguucaa guggg |
| 2233 | ugcucuuuuc cagguucaag uggga |
| 2234 | gcucuuuucc agguucaagu gggac |
| 2235 | cucuuuucca gguucaagug ggaua |
| 2236 | ucuuuuccag guucaagugg gauac |
| 2237 | ucuuuuccag guucaagugg |
| 2238 | cuuuuccagg uucaaguggg auacu |
| 2239 | uuuuccaggu ucaaguggga uacua |
| 2240 | uuuccagguu caagugggau acuag |
| 2241 | uuccagguuc aagugggaua cuagc |
| 2242 | uccagguuca agugggauac uagca |
| 2243 | ccagguucaa gugggauacu agcaa |
| 2244 | cagguucaag ugggauacua gcaau |
| 2245 | agguucaagu gggauacuag caaug |
| 2246 | gguucaagug ggauacuagc aaugu |
| 2247 | guucaagugg gauacuagca auguu |
| 2248 | uucaaguggg auacuagcaa uguua |
| 2249 | ucaaguggga uacuagcaau guuau |
| 2250 | caagugggau acuagcaaug uuauc |
| 2251 | aagugggaua cuagcaaugu uaucu |
| 2252 | agugggauac uagcaauguu aucug |
| 2253 | gugggauacu agcaauguua ucugc |
| 2254 | ugggauacua gcaauguuau cugcu |
| 2255 | gggauacuag caauguuauc ugcuu |
| 2256 | ggauacuagc aauguuaucu gcuuc |
| 2257 | gauacuagca auguuaucug cuucc |
| 2258 | auacuagcaa uguuaucugc uuccu |
| 2259 | uacuagcaau guuaucugcu uccuc |
| 2260 | acuagcaaug uuaucugcuu ccucc |
| 2261 | cuagcaaugu uaucugcuuc cucca |
| 2262 | uagcaauguu aucugcuucc uccaa |
| 2263 | agcaauguua ucugcuuccu ccaac |
| 2264 | gcaauguuau cugcuuccuc caacc |
| 2265 | caauguuauc ugcuuccucc aacca |
| 2266 | aauguuaucu gcuuccucca accau |
| 2267 | auguuaucug cuuccuccaa ccaua |
| 2268 | uguuaucugc uuccuccaac cauaa |
| 2269 | ccaauagugg ucaguccagg agcua |
| 2270 | caauaguggu caguccagga gcuag |
| 2271 | aauagugguc aguccaggag cuagg |
| 2272 | auagugguca guccaggagc uaggu |
| 2273 | auagugguca guccaggagc u |
| 2274 | uaguggucag uccaggagcu agguc |
| 2275 | aguggucagu ccaggagcua gguca |
| 2276 | guggucaguc caggagcuag gucag |
| 2277 | uggucagucc aggagcuagg ucagg |
| 2278 | ggucagucca ggagcuaggu caggc |
| 2279 | gucaguccag gagcuagguc aggcu |
| 2280 | ucaguccagg agcuagguca ggcug |
| 2281 | caguccagga gcuaggucag gcugc |
| 2282 | aguccaggag cuaggucagg cugcu |
| 2283 | guccaggagc uaggucaggc ugcuu |
| 2284 | uccaggagcu aggucaggcu gcuuu |
| 2285 | ccaggagcua ggucaggcug cuuug |
| 2286 | caggagcuag gucaggcugc uuugc |
| 2287 | aggagcuagg ucaggcugcu uugcc |
| 2288 | ggagcuaggu caggcugcuu ugccc |
| 2289 | gagcuagguc aggcugcuuu gcccu |
| 2290 | agcuagguca ggcugcuuug cccuc |
| 2291 | gcuaggucag gcugcuuugc ccuca |
| 2292 | cuaggucagg cugcuuugcc cucag |
| 2293 | uaggucaggc ugcuuugccc ucagc |
| 2294 | aggucaggcu gcuuugcccu cagcu |
| 2295 | ggucaggcug cuuugcccuc agcuc |
| 2296 | gucaggcugc uuugcccuca gcucu |
| 2297 | ucaggcugcu uugcccucag cucuu |
| 2298 | caggcugcuu ugcccucagc ucuug |
| 2299 | aggcugcuuu gcccucagcu cuuga |
| 2300 | ggcugcuuug cccucagcuc uugaa |
| 2301 | gcugcuuugc ccucagcucu ugaag |
| 2302 | cugcuuugcc cucagcucuu gaagu |
| 2303 | ugcuuugccc ucagcucuug aagua |
| 2304 | gcuuugcccu cagcucuuga aguaa |
| 2305 | cuuugcccuc agcucuugaa guaaa |
| 2306 | uuugcccuca gcucuugaag uaaac |
| 2307 | uugcccucag cucuugaagu aaacg |
| 2308 | ugcccucagc ucuugaagua aacgg |
| 2309 | gcccucagcu cuugaaguaa acggu |
| 2310 | cccucagcuc uugaaguaaa cgguu |
| 2311 | ccucagcucu ugaaguaaac |
| 2312 | ccucagcucu ugaaguaaac g |
| 2313 | cucagcucuu gaaguaaacg |
| 2314 | guaccuccaa caucaaggaa gaugg |
| 2315 | uaccuccaac aucaaggaag auggc |
| 2316 | accuccaaca ucaaggaaga uggca |
| 2317 | ccuccaacau caaggaagau ggcau |
| 2318 | cuccaacauc aaggaagaug gcauu |
| 2319 | uccaacauca aggaagaugg cauuu |
| 2320 | ccaacaucaa ggaagauggc auuuc |
| 2321 | caacaucaag gaagauggca uuucu |
| 2322 | aacaucaagg aagauggcau uucua |
| 2323 | acaucaagga agauggcauu ucuag |
| 2324 | caucaaggaa gauggcauuu cuagu |
| 2325 | aucaaggaag auggcauuuc uaguu |
| 2326 | ucaaggaaga uggcauuucu aguuu |
| 2327 | caaggaagau ggcauuucua guuug |
| 2328 | aaggaagaug gcauuucuag uuugg |
| 2329 | aggaagaugg cauuucuagu uugga |
| 2330 | ggaagauggc auuucuaguu uggag |
| 2331 | gaagauggca uuucuaguuu ggaga |
| 2332 | aagauggcau uucuaguuug gagau |
| 2333 | agauggcauu ucuaguuugg agaug |
| 2334 | gauggcauuu cuaguuugga gaugg |
| 2335 | auggcauuuc uaguuuggag auggc |
| 2336 | uggcauuucu aguuuggaga uggca |
| 2337 | ggcauuucua guuuggagau ggcag |
| 2338 | gcauuucuag uuuggagaug gcagu |
| 2339 | cauuucuagu uuggagaugg caguu |
| 2340 | auuucuaguu uggagauggc aguuu |
| 2341 | uuucuaguuu ggagauggca guuuc |
| 2342 | uucuaguuug gagauggcag uuucc |
| 2343 | ucuaguuugg agauggcagu uuccu |
| 2344 | cuaguuugga gauggcaguu uccuu |
| 2345 | uaguuuggag auggcaguuu ccuua |
| 2346 | aguuuggaga uggcaguuuc cuuag |
| 2347 | guuuggagau ggcaguuucc uuagu |
| 2348 | uuuggagaug gcaguuuccu uagua |
| 2349 | uuggagaugg caguuuccuu aguaa |
| 2350 | uggagauggc aguuuccuua guaac |
| 2351 | gagauggcag uuuccuuagu aacca |
| 2352 | agauggcagu uuccuuagua accac |
| 2353 | gauggcaguu uccuuaguaa ccaca |
| 2354 | auggcaguuu ccuuaguaac cacag |
| 2355 | uggcaguuuc cuuaguaacc acagg |
| 2356 | ggcaguuucc uuaguaacca caggu |
| 2357 | gcaguuuccu uaguaaccac agguu |
| 2358 | caguuuccuu aguaaccaca gguug |
| 2359 | aguuuccuua guaaccacag guugu |
| 2360 | guuuccuuag uaaccacagg uugug |
| 2361 | uuuccuuagu aaccacaggu ugugu |
| 2362 | uuccuuagua accacagguu guguc |
| 2363 | uccuuaguaa ccacagguug uguca |
| 2364 | ccuuaguaac cacagguugu gucac |
| 2365 | cuuaguaacc acagguugug ucacc |
| 2366 | uuaguaacca cagguugugu cacca |
| 2367 | uaguaaccac agguuguguc accag |
| 2368 | aguaaccaca gguuguguca ccaga |
| 2369 | guaaccacag guugugucac cagag |
| 2370 | uaaccacagg uugugucacc agagu |
| 2371 | aaccacaggu ugugucacca gagua |
| 2372 | accacagguu gugucaccag aguaa |
| 2373 | ccacagguug ugucaccaga guaac |
| 2374 | cacagguugu gucaccagag uaaca |
| 2375 | acagguugug ucaccagagu aacag |
| 2376 | cagguugugu caccagagua acagu |
| 2377 | agguuguguc accagaguaa caguc |
| 2378 | gguuguguca ccagaguaac agucu |
| 2379 | guugugucac cagaguaaca gucug |
| 2380 | uugugucacc agaguaacag ucuga |
| 2381 | ugugucacca gaguaacagu cugag |
| 2382 | gugucaccag aguaacaguc ugagu |
| 2383 | ugucaccaga guaacagucu gagua |
| 2384 | gucaccagag uaacagucug aguag |
| 2385 | ucaccagagu aacagucuga guagg |
| 2386 | caccagagua acagucugag uagga |
| 2387 | accagaguaa cagucugagu aggag |
| 2388 | agccucuuga uugcuggucu uguuu |
| 2389 | gccucuugau ugcuggucuu guuuu |
| 2390 | ccucuugauu gcuggucuug uuuuu |
| 2391 | ccucuugauu gcuggucuug |
| 2392 | cucuugauug cuggucuugu uuuuc |
| 2393 | ucuugauugc uggucuuguu uuuca |
| 2394 | cuugauugcu ggucuuguuu uucaa |
| 2395 | uugauugcug gucuuguuuu ucaaa |
| 2396 | ugauugcugg ucuuguuuuu caaau |
| 2397 | gauugcuggu cuuguuuuuc aaauu |
| 2398 | gauugcuggu cuuguuuuuc |
| 2399 | auugcugguc uuguuuuuca aauuu |
| 2400 | uugcuggucu uguuuuucaa auuuu |
| 2401 | ugcuggucuu guuuuucaaa uuuug |
| 2402 | gcuggucuug uuuuucaaau uuugg |
| 2403 | cuggucuugu uuuucaaauu uuggg |
| 2404 | uggucuuguu uuucaaauuu ugggc |
| 2405 | ggucuuguuu uucaaauuuu gggca |
| 2406 | gucuuguuuu ucaaauuuug ggcag |
| 2407 | ucuuguuuuu caaauuuugg gcagc |
| 2408 | cuuguuuuuc aaauuuuggg cagcg |
| 2409 | uuguuuuuca aauuuugggc agcgg |
| 2410 | uguuuuucaa auuuugggca gcggu |
| 2411 | guuuuucaaa uuuugggcag cggua |
| 2412 | uuuuucaaau uuugggcagc gguaa |
| 2413 | uuuucaaauu uugggcagcg guaau |
| 2414 | uuucaaauuu ugggcagcgg uaaug |
| 2415 | uucaaauuuu gggcagcggu aauga |
| 2416 | ucaaauuuug ggcagcggua augag |
| 2417 | caaauuuugg gcagcgguaa ugagu |
| 2418 | aaauuuuggg cagcgguaau gaguu |
| 2419 | aauuuugggc agcgguaaug aguuc |
| 2420 | auuuugggca gcgguaauga guucu |
| 2421 | uuuugggcag cgguaaugag uucuu |
| 2422 | uuugggcagc gguaaugagu ucuuc |
| 2423 | uugggcagcg guaaugaguu cuucc |
| 2424 | ugggcagcgg uaaugaguuc uucca |
| 2425 | gggcagcggu aaugaguucu uccaa |
| 2426 | ggcagcggua augaguucuu ccaac |
| 2427 | gcagcgguaa ugaguucuuc caacu |
| 2428 | cagcgguaau gaguucuucc aacug |
| 2429 | agcgguaaug aguucuucca acugg |
| 2430 | gcgguaauga guucuuccaa cuggg |
| 2431 | cgguaaugag uucuuccaac ugggg |
| 2432 | gguaaugagu ucuuccaacu gggga |
| 2433 | gguaaugagu ucuuccaacu gg |
| 2434 | guaaugaguu cuuccaacug gggac |
| 2435 | uaaugaguuc uuccaacugg ggacg |
| 2436 | aaugaguucu uccaacuggg gacgc |
| 2437 | augaguucuu ccaacugggg acgcc |
| 2438 | ugaguucuuc caacugggga cgccu |
| 2439 | gaguucuucc aacuggggac gccuc |
| 2440 | aguucuucca acuggggacg ccucu |
| 2441 | guucuuccaa cuggggacgc cucug |
| 2442 | uucuuccaac uggggacgcc ucugu |
| 2443 | ucuuccaacu ggggacgccu cuguu |
| 2444 | cuuccaacug gggacgccuc uguuc |
| 2445 | uuccaacugg ggacgccucu guucc |
| 2446 | uccaacuggg gacgccucug uucca |
| 2447 | ccaacugggg acgccucugu uccaa |
| 2448 | caacugggga cgccucuguu ccaaa |
| 2449 | aacuggggac gccucuguuc caaau |
| 2450 | acuggggacg ccucuguucc aaauc |
| 2451 | cuggggacgc cucuguucca aaucc |
| 2452 | uggggacgcc ucuguuccaa auccu |
| 2453 | ggggacgccu cuguuccaaa uccug |
| 2454 | gggacgccuc uguuccaaau ccugc |
| 2455 | ggacgccucu guuccaaauc cugca |
| 2456 | gacgccucug uuccaaaucc ugcau |
| 2457 | cucuggccug uccuaagacc ugcuc |
| 2458 | ucuggccugu ccuaagaccu gcuca |
| 2459 | uggccugucc uaagaccugc ucagc |
| 2460 | ggccuguccu aagaccugcu cagcu |
| 2461 | gccuguccua agaccugcuc agcuu |
| 2462 | ccuguccuaa gaccugcuca gcuuc |
| 2463 | cuguccuaag accugcucag cuucu |
| 2464 | uguccuaaga ccugcucagc uucuu |
| 2465 | guccuaagac cugcucagcu ucuuc |
| 2466 | uccuaagacc ugcucagcuu cuucc |
| 2467 | ccuaagaccu gcucagcuuc uuccu |
| 2468 | cuaagaccug cucagcuucu uccuu |
| 2469 | uaagaccugc ucagcuucuu ccuua |
| 2470 | aagaccugcu cagcuucuuc cuuag |
| 2471 | agaccugcuc agcuucuucc uuagc |
| 2472 | gaccugcuca gcuucuuccu uagcu |
| 2473 | accugcucag cuucuuccuu agcuu |
| 2474 | ccugcucagc uucuuccuua gcuuc |
| 2475 | cugcucagcu ucuuccuuag cuucc |
| 2476 | ugcucagcuu cuuccuuagc uucca |
| 2477 | gcucagcuuc uuccuuagcu uccag |
| 2478 | cucagcuucu uccuuagcuu ccagc |
| 2479 | ucagcuucuu ccuuagcuuc cagcc |
| 2480 | cagcuucuuc cuuagcuucc agcca |
| 2481 | agcuucuucc uuagcuucca gccau |
| 2482 | gcuucuuccu uagcuuccag ccauu |
| 2483 | cuucuuccuu agcuuccagc cauug |
| 2484 | uucuuccuua gcuuccagcc auugu |
| 2485 | ucuuccuuag cuuccagcca uugug |
| 2486 | cuuccuuagc uuccagccau ugugu |
| 2487 | uuccuuagcu uccagccauu guguu |
| 2488 | uccuuagcuu ccagccauug uguug |
| 2489 | ccuuagcuuc cagccauugu guuga |
| 2490 | cuuagcuucc agccauugug uugaa |
| 2491 | uuagcuucca gccauugugu ugaau |
| 2492 | uagcuuccag ccauuguguu gaauc |
| 2493 | agcuuccagc cauuguguug aaucc |
| 2494 | gcuuccagcc auuguguuga auccu |
| 2495 | cuuccagcca uuguguugaa uccuu |
| 2496 | uuccagccau uguguugaau ccuuu |
| 2497 | uccagccauu guguugaauc cuuua |
| 2498 | ccagccauug uguugaaucc uuuaa |
| 2499 | cagccauugu guugaauccu uuaac |
| 2500 | agccauugug uugaauccuu uaaca |
| 2501 | gccauugugu ugaauccuuu aacau |
| 2502 | ccauuguguu gaauccuuua acauu |
| 2503 | cauuguguug aauccuuuaa cauuu |
| 2504 | cauuuuugac cuacaugugg |
| 2505 | uuugaccuac auguggaaag |
| 2506 | uacauuuuug accuacaugu ggaaag |
| 2507 | ggucuccuua ccuauga |
| 2508 | ucuuaccuau gacuauggau gaga |
| 2509 | auuuuugacc uacaugggaa ag |
| 2510 | uacgaguuga uugucggacc cag |
| 2511 | guggucuccu uaccuaugac ugugg |
| 2512 | ugucucagua aucuucuuac cuau |
| 2513 | ugcauguucc agucguugug ugg |
| 2514 | cacuauucca gucaaauagg ucugg |
| 2515 | auuuaccaac cuucaggauc gagua |
| 2516 | ggccuaaaac acauacacau a |
| 2517 | cccugaggca uucccaucuu gaau |
| 2518 | aggacuuacu ugcuuuguuu |
| 2519 | cuugaauuua ggagauucau cug |
| 2520 | caucuucuga uaauuuuccu guu |
| 2521 | ccauuacagu ugucuguguu |
| 2522 | ugacagccug ugaaaucugu gag |
| 2523 | uaaucugccu cuucuuuugg |
| 2524 | cagcaguagu ugucaucugc |
| 2525 | gccugagcug aucugcuggc aucuugc |
| 2526 | gccugagcug aucugcuggc aucuugcagu u |
| 2527 | ucugcuggca ucuugc |
| 2528 | gccgguugac uucauccugu gc |
| 2529 | gucugcaucc aggaacaugg guc |
| 2530 | uacuuacugu cuguagcucu uucu |
| 2531 | cugcauccag gaacaugggu cc |
| 2532 | guugaagauc ugauagccgg uuga |
| 2533 | ucagcuucug uuagccacug |
| 2534 | uucagcuucu guuagccacu |
| 2535 | uucagcuucu guuagccacu g |
| 2536 | ucagcuucug uuagccacug a |
| 2537 | uucagcuucu guuagccacu ga |
| 2538 | ucagcuucug uuagccacug a |
| 2539 | uucagcuucu guuagccacu ga |
| 2540 | ucagcuucug uuagccacug au |
| 2541 | uucagcuucu guuagccacu gau |
| 2542 | ucagcuucug uuagccacug auu |
| 2543 | uucagcuucu guuagccacu gauu |
| 2544 | ucagcuucug uuagccacug auua |
| 2545 | uucagcuucu guuagccacu gaua |
| 2546 | ucagcuucug uuagccacug auuaa |
| 2547 | uucagcuucu guuagccacu gauuaa |
| 2548 | ucagcuucug uuagccacug auuaaa |
| 2549 | uucagcuucu guuagccacu gauuaaa |
| 2550 | cagcuucugu uagccacug |
| 2551 | cagcuucugu uagccacuga u |
| 2552 | agcuucuguu agccacugau u |
| 2553 | cagcuucugu uagccacuga uu |
| 2554 | agcuucuguu agccacugau ua |
| 2555 | cagcuucugu uagccacuga uua |
| 2556 | agcuucuguu agccacugau uaa |
| 2557 | cagcuucugu uagccacuga uuaa |
| 2558 | agcuucuguu agccacugau uaaa |
| 2559 | cagcuucugu uagccacuga uuaaa |
| 2560 | agcuucuguu agccacugau uaaa |
| 2561 | agcuucuguu agccacugau |
| 2562 | gcuucuguua gccacugauu |
| 2563 | agcuucuguu agccacugau u |
| 2564 | gcuucuguua gccacugauu a |
| 2565 | agcuucuguu agccacugau ua |
| 2566 | gcuucuguua gccacugauu aa |
| 2567 | agcuucuguu agccacugau uaa |
| 2568 | gcuucuguua gccacugauu aaa |
| 2569 | agcuucuguu agccacugau uaaa |
| 2570 | gcuucuguua gccacugauu aaa |
| 2571 | ccauuuguau uuagcauguu ccc |
| 2572 | agauaccauu uguauuuagc |
| 2573 | gccauuucuc aacagaucu |
| 2574 | gccauuucuc aacagaucug uca |
| 2575 | auucucagga auuugugucu uuc |
| 2576 | ucucaggaau uugugucuuu c |
| 2577 | guucagcuuc uguuagcc |
| 2578 | cugauuaaau aucuuuauau c |
| 2579 | gccgccauuu cucaacag |
| 2580 | gccgccauuu cucaacag |
| 2581 | caggaauuug ugucuuuc |
| 2582 | uuugccgcug cccaaugcca uccug |
| 2583 | auucaauguu cugacaacag uuugc |
| 2584 | ccaguugcau ucaauguucu gacaa |
| 2585 | caguugcauu caauguucug ac |
| 2586 | aguugcauuc aauguucuga |
| 2587 | gauugcugaa uuauuucuuc c |
| 2588 | gauugcugaa uuauuucuuc cccag |
| 2589 | auugcugaau uauuucuucc ccagu |
| 2590 | uugcugaauu auuucuuccc caguu |
| 2591 | ugcugaauua uuucuucccc aguug |
| 2592 | gcugaauuau uucuucccca guugc |
| 2593 | cugaauuauu ucuuccccag uugca |
| 2594 | ugaauuauuu cuuccccagu ugcau |
| 2595 | gaauuauuuc uuccccaguu gcauu |
| 2596 | aauuauuucu uccccaguug cauuc |
| 2597 | auuauuucuu ccccaguugc auuca |
| 2598 | uuauuucuuc cccaguugca uucaa |
| 2599 | uauuucuucc ccaguugcau ucaau |
| 2600 | auuucuuccc caguugcauu caaug |
| 2601 | uuucuucccc aguugcauuc aaugu |
| 2602 | uucuucccca guugcauuca auguu |
| 2603 | ucuuccccag uugcauucaa uguuc |
| 2604 | cuuccccagu ugcauucaau guucu |
| 2605 | uuccccaguu gcauucaaug uucug |
| 2606 | uccccaguug cauucaaugu ucuga |
| 2607 | ccccaguugc auucaauguu cugac |
| 2608 | cccaguugca uucaauguuc ugaca |
| 2609 | ccaguugcau ucaauguucu gacaa |
| 2610 | caguugcauu caauguucug acaac |
| 2611 | aguugcauuc aauguucuga caaca |
| 2612 | uccuguagaa uacuggcauc |
| 2613 | ugcagaccuc cugccaccgc agauuca |
| 2614 | uugcagaccu ccugccaccg cagauucagg cuuc |
| 2615 | guugcauuca auguucugac aacag |
| 2616 | uugcauucaa uguucugaca acagu |
| 2617 | ugcauucaau guucugacaa caguu |
| 2618 | gcauucaaug uucugacaac aguuu |
| 2619 | cauucaaugu ucugacaaca guuug |
| 2620 | auucaauguu cugacaacag uuugc |
| 2621 | ucaauguucu gacaacaguu ugccg |
| 2622 | caauguucug acaacaguuu gccgc |
| 2623 | aauguucuga caacaguuug ccgcu |
| 2624 | auguucugac aacaguuugc cgcug |
| 2625 | uguucugaca acaguuugcc gcugc |
| 2626 | guucugacaa caguuugccg cugcc |
| 2627 | uucugacaac aguuugccgc ugccc |
| 2628 | ucugacaaca guuugccgcu gccca |
| 2629 | cugacaacag uuugccgcug cccaa |
| 2630 | ugacaacagu uugccgcugc ccaau |
| 2631 | gacaacaguu ugccgcugcc caaug |
| 2632 | acaacaguuu gccgcugccc aaugc |
| 2633 | caacaguuug ccgcugccca augcc |
| 2634 | aacaguuugc cgcugcccaa ugcca |
| 2635 | acaguuugcc gcugcccaau gccau |
| 2636 | caguuugccg cugcccaaug ccauc |
| 2637 | aguuugccgc ugcccaaugc caucc |
| 2638 | guuugccgcu gcccaaugcc auccu |
| 2639 | uuugccgcug cccaaugcca uccug |
| 2640 | uugccgcugc ccaaugccau ccugg |
| 2641 | ugccgcugcc caaugccauc cugga |
| 2642 | gccgcugccc aaugccaucc uggag |
| 2643 | ccgcugccca augccauccu ggagu |
| 2644 | cgcugcccaa ugccauccug gaguu |
| 2645 | uguuuuugag gauugcugaa |
| 2646 | uguucugaca acaguuugcc gcugcccaau gccauccugg |
| 2647 | cuguugcagu aaucuaugag |
| 2648 | ugcaguaauc uaugaguuuc |
| 2649 | gagucuucua ggagccuu |
| 2650 | ugccauuguu ucaucagcuc uuu |
| 2651 | uccuguagga cauuggcagu |
| 2652 | cuuggagucu ucuaggagcc |
| 2653 | uaggugccug ccggcuu |
| 2654 | uucagcugua gccacacc |
| 2655 | cugaacugcu ggaaagucgc c |
| 2656 | cuggcuucca aaugggaccu gaaaaagaac |
| 2657 | caauuuuucc cacucaguau u |
| 2658 | uugaaguucc uggagucuu |
| 2659 | uccucaggag gcagcucuaa au |
| 2660 | uggcucucuc ccaggg |
| 2661 | gagauggcuc ucucccaggg acccugg |
| 2662 | gggcacuuug uuuggcg |
| 2663 | ggucccagca aguuguuug |
| 2664 | ugggaugguc ccagcaaguu guuug |
| 2665 | guagagcucu gucauuuugg g |
| 2666 | gcucaagaga uccacugcaa aaaac |
| 2667 | gccauacgua cguaucauaa acauuc |
| 2668 | ucugcaggau auccaugggc ugguc |
| 2669 | gauccucccu guucgucccc uauuaug |
| 2670 | ugcuuuagac uccuguaccu gaua |
| 2671 | ggcggccuuu guguugac |
| 2672 | ggacaggccu uuauguucgu gcugc |
| 2673 | ccuuuauguu cgugcugcu |
| 2674 | ccucagcucu ugaaguaaac gguuu |
| 2675 | cucagcucuu gaaguaaacg guuua |
| 2676 | ucagcucuug aaguaaacgg uuuac |
| 2677 | cagcucuuga aguaaacggu uuacc |
| 2678 | agcucuugaa guaaacgguu uaccg |
| 2679 | gcucuugaag uaaacgguuu accgc |
| 2680 | cucuugaagu aaacgguuua ccgcc |
| 2681 | guaccuccaa caucaaggaa gaugg |
| 2682 | uaccuccaac aucaaggaag auggc |
| 2683 | accuccaaca ucaaggaaga uggca |
| 2684 | ccuccaacau caaggaagau ggcau |
| 2685 | cuccaacauc aaggaagaug gcauu |
| 2686 | uccaacauca aggaagaugg cauuu |
| 2687 | ccaacaucaa ggaagauggc auuuc |
| 2688 | caacaucaag gaagauggca uuucu |
| 2689 | aacaucaagg aagauggcau uucua |
| 2690 | acaucaagga agauggcauu ucuag |
| 2691 | caucaaggaa gauggcauuu cuagu |
| 2692 | aucaaggaag auggcauuuc uaguu |
| 2693 | ucaaggaaga uggcauuucu aguuu |
| 2694 | caaggaagau ggcauuucua guuug |
| 2695 | aaggaagaug gcauuucuag uuugg |
| 2696 | aggaagaugg cauuucuagu uugga |
| 2697 | ggaagauggc auuucuaguu uggag |
| 2698 | gaagauggca uuucuaguuu ggaga |
| 2699 | aagauggcau uucuaguuug gagau |
| 2700 | agauggcauu ucuaguuugg agaug |
| 2701 | gauggcauuu cuaguuugga gaugg |
| 2702 | auggcauuuc uaguuuggag auggc |
| 2703 | uggcauuucu aguuuggaga uggca |
| 2704 | ggcauuucua guuuggagau ggcag |
| 2705 | gcauuucuag uuuggagaug gcagu |
| 2706 | cauuucuagu uuggagaugg caguu |
| 2707 | auuucuaguu uggagauggc aguuu |
| 2708 | uuucuaguuu ggagauggca guuuc |
| 2709 | uucuaguuug gagauggcag uuucc |
| 2710 | ucuaguuugg agauggcagu uuccu |
| 2711 | cuaguuugga gauggcaguu uccuu |
| 2712 | uaguuuggag auggcaguuu ccuua |
| 2713 | aguuuggaga uggcaguuuc cuuag |
| 2714 | guuuggagau ggcaguuucc uuagu |
| 2715 | uuuggagaug gcaguuuccu uagua |
| 2716 | uuggagaugg caguuuccuu aguaa |
| 2717 | uggagauggc aguuuccuua guaac |
| 2718 | gagauggcag uuuccuuagu aacca |
| 2719 | agauggcagu uuccuuagua accac |
| 2720 | gauggcaguu uccuuaguaa ccaca |
| 2721 | auggcaguuu ccuuaguaac cacag |
| 2722 | uggcaguuuc cuuaguaacc acagg |
| 2723 | ggcaguuucc uuaguaacca caggu |
| 2724 | gcaguuuccu uaguaaccac agguu |
| 2725 | caguuuccuu aguaaccaca gguug |
| 2726 | aguuuccuua guaaccacag guugu |
| 2727 | guuuccuuag uaaccacagg uugug |
| 2728 | uuuccuuagu aaccacaggu ugugu |
| 2729 | uuccuuagua accacagguu guguc |
| 2730 | uccuuaguaa ccacagguug uguca |
| 2731 | ccuuaguaac cacagguugu gucac |
| 2732 | cuuaguaacc acagguugug ucacc |
| 2733 | uuaguaacca cagguugugu cacca |
| 2734 | uaguaaccac agguuguguc accag |
| 2735 | aguaaccaca gguuguguca ccaga |
| 2736 | guaaccacag guugugucac cagag |
| 2737 | uaaccacagg uugugucacc agagu |
| 2738 | aaccacaggu ugugucacca gagua |
| 2739 | accacagguu gugucaccag aguaa |
| 2740 | ccacagguug ugucaccaga guaac |
| 2741 | cacagguugu gucaccagag uaaca |
| 2742 | acagguugug ucaccagagu aacag |
| 2743 | cagguugugu caccagagua acagu |
| 2744 | agguuguguc accagaguaa caguc |
| 2745 | gguuguguca ccagaguaac agucu |
| 2746 | guugugucac cagaguaaca gucug |
| 2747 | uugugucacc agaguaacag ucuga |
| 2748 | ugugucacca gaguaacagu cugag |
| 2749 | gugucaccag aguaacaguc ugagu |
| 2750 | ugucaccaga guaacagucu gagua |
| 2751 | gucaccagag uaacagucug aguag |
| 2752 | ucaccagagu aacagucuga guagg |
| 2753 | caccagagua acagucugag uagga |
| 2754 | accagaguaa cagucugagu aggag |
| 2755 | uuugccgcug cccaaugcca uccug |
| 2756 | auucaauguu cugacaacag uuugc |
| 2757 | ccaguugcau ucaauguucu gacaa |
| 2758 | caguugcauu caauguucug ac |
| 2759 | aguugcauuc aauguucuga |
| 2760 | gauugcugaa uuauuucuuc c |
| 2761 | gauugcugaa uuauuucuuc cccag |
| 2762 | auugcugaau uauuucuucc ccagu |
| 2763 | uugcugaauu auuucuuccc caguu |
| 2764 | ugcugaauua uuucuucccc aguug |
| 2765 | gcugaauuau uucuucccca guugc |
| 2766 | cugaauuauu ucuuccccag uugca |
| 2767 | ugaauuauuu cuuccccagu ugcau |
| 2768 | gaauuauuuc uuccccaguu gcauu |
| 2769 | aauuauuucu uccccaguug cauuc |
| 2770 | auuauuucuu ccccaguugc auuca |
| 2771 | uuauuucuuc cccaguugca uucaa |
| 2772 | uauuucuucc ccaguugcau ucaau |
| 2773 | auuucuuccc caguugcauu caaug |
| 2774 | uuucuucccc aguugcauuc aaugu |
| 2775 | uucuucccca guugcauuca auguu |
| 2776 | ucuuccccag uugcauucaa uguuc |
| 2777 | cuuccccagu ugcauucaau guucu |
| 2778 | uuccccaguu gcauucaaug uucug |
| 2779 | uccccaguug cauucaaugu ucuga |
| 2780 | ccccaguugc auucaauguu cugac |
| 2781 | cccaguugca uucaauguuc ugaca |
| 2782 | ccaguugcau ucaauguucu gacaa |
| 2783 | caguugcauu caauguucug acaac |
| 2784 | aguugcauuc aauguucuga caaca |
| 2785 | uccuguagaa uacuggcauc |
| 2786 | ugcagaccuc cugccaccgc agauuca |
| 2787 | uugcagaccu ccugccaccg cagauucagg cuuc |
| 2788 | guugcauuca auguucugac aacag |
| 2789 | uugcauucaa uguucugaca acagu |
| 2790 | ugcauucaau guucugacaa caguu |
| 2791 | gcauucaaug uucugacaac aguuu |
| 2792 | cauucaaugu ucugacaaca guuug |
| 2793 | auucaauguu cugacaacag uuugc |
| 2794 | ucaauguucu gacaacaguu ugccg |
| 2795 | caauguucug acaacaguuu gccgc |
| 2796 | aauguucuga caacaguuug ccgcu |
| 2797 | auguucugac aacaguuugc cgcug |
| 2798 | uguucugaca acaguuugcc gcugc |
| 2799 | guucugacaa caguuugccg cugcc |
| 2800 | uucugacaac aguuugccgc ugccc |
| 2801 | ucugacaaca guuugccgcu gccca |
| 2802 | cugacaacag uuugccgcug cccaa |
| 2803 | ugacaacagu uugccgcugc ccaau |
| 2804 | gacaacaguu ugccgcugcc caaug |
| 2805 | acaacaguuu gccgcugccc aaugc |
| 2806 | caacaguuug ccgcugccca augcc |
| 2807 | aacaguuugc cgcugcccaa ugcca |
| 2808 | acaguuugcc gcugcccaau gccau |
| 2809 | caguuugccg cugcccaaug ccauc |
| 2810 | aguuugccgc ugcccaaugc caucc |
| 2811 | guuugccgcu gcccaaugcc auccu |
| 2812 | uuugccgcug cccaaugcca uccug |
| 2813 | uugccgcugc ccaaugccau ccugg |
| 2814 | ugccgcugcc caaugccauc cugga |
| 2815 | gccgcugccc aaugccaucc uggag |
| 2816 | ccgcugccca augccauccu ggagu |
| 2817 | cgcugcccaa ugccauccug gaguu |
| 2818 | uguuuuugag gauugcugaa |
| 2819 | uguucugaca acaguuugcc gcugcccaau gccauccugg |
| 2820 | cucuggccug uccuaagacc ugcuc |
| 2821 | ucuggccugu ccuaagaccu gcuca |
| 2822 | cuggccuguc cuaagaccug cucag |
| 2823 | uggccugucc uaagaccugc ucagc |
| 2824 | ggccuguccu aagaccugcu cagcu |
| 2825 | gccuguccua agaccugcuc agcuu |
| 2826 | ccuguccuaa gaccugcuca gcuuc |
| 2827 | cuguccuaag accugcucag cuucu |
| 2828 | uguccuaaga ccugcucagc uucuu |
| 2829 | guccuaagac cugcucagcu ucuuc |
| 2830 | uccuaagacc ugcucagcuu cuucc |
| 2831 | ccuaagaccu gcucagcuuc uuccu |
| 2832 | cuaagaccug cucagcuucu uccuu |
| 2833 | uaagaccugc ucagcuucuu ccuua |
| 2834 | aagaccugcu cagcuucuuc cuuag |
| 2835 | agaccugcuc agcuucuucc uuagc |
| 2836 | gaccugcuca gcuucuuccu uagcu |
| 2837 | accugcucag cuucuuccuu agcuu |
| 2838 | ccugcucagc uucuuccuua gcuuc |
| 2839 | cugcucagcu ucuuccuuag cuucc |
| 2840 | ugcucagcuu cuuccuuagc uucca |
| 2841 | gcucagcuuc uuccuuagcu uccag |
| 2842 | cucagcuucu uccuuagcuu ccagc |
| 2843 | ucagcuucuu ccuuagcuuc cagcc |
| 2844 | cagcuucuuc cuuagcuucc agcca |
| 2845 | agcuucuucc uuagcuucca gccau |
| 2846 | gcuucuuccu uagcuuccag ccauu |
| 2847 | cuucuuccuu agcuuccagc cauug |
| 2848 | uucuuccuua gcuuccagcc auugu |
| 2849 | ucuuccuuag cuuccagcca uugug |
| 2850 | cuuccuuagc uuccagccau ugugu |
| 2851 | uuccuuagcu uccagccauu guguu |
| 2852 | uccuuagcuu ccagccauug uguug |
| 2853 | ccuuagcuuc cagccauugu guuga |
| 2854 | cuuagcuucc agccauugug uugaa |
| 2855 | uuagcuucca gccauugugu ugaau |
| 2856 | uagcuuccag ccauuguguu gaauc |
| 2857 | agcuuccagc cauuguguug aaucc |
| 2858 | gcuuccagcc auuguguuga auccu |
| 2859 | cuuccagcca uuguguugaa uccuu |
| 2860 | uuccagccau uguguugaau ccuuu |
| 2861 | uccagccauu guguugaauc cuuua |
| 2862 | ccagccauug uguugaaucc uuuaa |
| 2863 | cagccauugu guugaauccu uuaac |
| 2864 | agccauugug uugaauccuu uaaca |
| 2865 | gccauugugu ugaauccuuu aacau |
| 2866 | ccauuguguu gaauccuuua acauu |
| 2867 | cauuguguug aauccuuuaa cauuu |
| 2868 | ucagcuucug uuagccacug |
| 2869 | uucagcuucu guuagccacu |
| 2870 | uucagcuucu guuagccacu g |
| 2871 | ucagcuucug uuagccacug a |
| 2872 | uucagcuucu guuagccacu ga |
| 2873 | ucagcuucug uuagccacug a |
| 2874 | uucagcuucu guuagccacu ga |
| 2875 | ucagcuucug uuagccacug au |
| 2876 | uucagcuucu guuagccacu gau |
| 2877 | ucagcuucug uuagccacug auu |
| 2878 | uucagcuucu guuagccacu gauu |
| 2879 | ucagcuucug uuagccacug auua |
| 2880 | uucagcuucu guuagccacu gaua |
| 2881 | ucagcuucug uuagccacug auuaa |
| 2882 | uucagcuucu guuagccacu gauuaa |
| 2883 | ucagcuucug uuagccacug auuaaa |
| 2884 | uucagcuucu guuagccacu gauuaaa |
| 2885 | cagcuucugu uagccacug |
| 2886 | cagcuucugu uagccacuga u |
| 2887 | agcuucuguu agccacugau u |
| 2888 | cagcuucugu uagccacuga uu |
| 2889 | agcuucuguu agccacugau ua |
| 2890 | cagcuucugu uagccacuga uua |
| 2891 | agcuucuguu agccacugau uaa |
| 2892 | cagcuucugu uagccacuga uuaa |
| 2893 | agcuucuguu agccacugau uaaa |
| 2894 | cagcuucugu uagccacuga uuaaa |
| 2895 | agcuucuguu agccacugau uaaa |
| 2896 | agcuucuguu agccacugau |
| 2897 | gcuucuguua gccacugauu |
| 2898 | agcuucuguu agccacugau u |
| 2899 | gcuucuguua gccacugauu a |
| 2900 | agcuucuguu agccacugau ua |
| 2901 | gcuucuguua gccacugauu aa |
| 2902 | agcuucuguu agccacugau uaa |
| 2903 | gcuucuguua gccacugauu aaa |
| 2904 | agcuucuguu agccacugau uaaa |
| 2905 | gcuucuguua gccacugauu aaa |
| 2906 | ccauuuguau uuagcauguu ccc |
| 2907 | agauaccauu uguauuuagc |
| 2908 | gccauuucuc aacagaucu |
| 2909 | gccauuucuc aacagaucug uca |
| 2910 | auucucagga auuugugucu uuc |
| 2911 | ucucaggaau uugugucuuu c |
| 2912 | guucagcuuc uguuagcc |
| 2913 | cugauuaaau aucuuuauau c |
| 2914 | gccgccauuu cucaacag |
| 2915 | guauuuagca uguuccca |
| 2916 | caggaauuug ugucuuuc |
| 2917 | gcuuuucuuu uaguugcugc ucuuu |
| 2918 | cuuuucuuuu aguugcugcu cuuuu |
| 2919 | uuuucuuuua guugcugcuc uuuuc |
| 2920 | uuucuuuuag uugcugcucu uuucc |
| 2921 | uucuuuuagu ugcugcucuu uucca |
| 2922 | ucuuuuaguu gcugcucuuu uccag |
| 2923 | cuuuuaguug cugcucuuuu ccagg |
| 2924 | uuuuaguugc ugcucuuuuc caggu |
| 2925 | uuuaguugcu gcucuuuucc agguu |
| 2926 | uuaguugcug cucuuuucca gguuc |
| 2927 | uaguugcugc ucuuuuccag guuca |
| 2928 | aguugcugcu cuuuuccagg uucaa |
| 2929 | guugcugcuc uuuuccaggu ucaag |
| 2930 | uugcugcucu uuuccagguu caagu |
| 2931 | ugcugcucuu uuccagguuc aagug |
| 2932 | gcugcucuuu uccagguuca agugg |
| 2933 | cugcucuuuu ccagguucaa guggg |
| 2934 | ugcucuuuuc cagguucaag uggga |
| 2935 | gcucuuuucc agguucaagu gggac |
| 2936 | cucuuuucca gguucaagug ggaua |
| 2937 | ucuuuuccag guucaagugg gauac |
| 2938 | ucuuuuccag guucaagugg |
| 2939 | cuuuuccagg uucaaguggg auacu |
| 2940 | uuuuccaggu ucaaguggga uacua |
| 2941 | uuuccagguu caagugggau acuag |
| 2942 | uuccagguuc aagugggaua cuagc |
| 2943 | uccagguuca agugggauac uagca |
| 2944 | ccagguucaa gugggauacu agcaa |
| 2945 | cagguucaag ugggauacua gcaau |
| 2946 | agguucaagu gggauacuag caaug |
| 2947 | gguucaagug ggauacuagc aaugu |
| 2948 | guucaagugg gauacuagca auguu |
| 2949 | uucaaguggg auacuagcaa uguua |
| 2950 | ucaaguggga uacuagcaau guuau |
| 2951 | caagugggau acuagcaaug uuauc |
| 2952 | aagugggaua cuagcaaugu uaucu |
| 2953 | agugggauac uagcaauguu aucug |
| 2954 | gugggauacu agcaauguua ucugc |
| 2955 | ugggauacua gcaauguuau cugcu |
| 2956 | gggauacuag caauguuauc ugcuu |
| 2957 | ggauacuagc aauguuaucu gcuuc |
| 2958 | gauacuagca auguuaucug cuucc |
| 2959 | auacuagcaa uguuaucugc uuccu |
| 2960 | uacuagcaau guuaucugcu uccuc |
| 2961 | acuagcaaug uuaucugcuu ccucc |
| 2962 | cuagcaaugu uaucugcuuc cucca |
| 2963 | uagcaauguu aucugcuucc uccaa |
| 2964 | agcaauguua ucugcuuccu ccaac |
| 2965 | gcaauguuau cugcuuccuc caacc |
| 2966 | caauguuauc ugcuuccucc aacca |
| 2967 | aauguuaucu gcuuccucca accau |
| 2968 | auguuaucug cuuccuccaa ccaua |
| 2969 | uguuaucugc uuccuccaac cauaa |
| 2970 | agccucuuga uugcuggucu uguuu |
| 2971 | gccucuugau ugcuggucuu guuuu |
| 2972 | ccucuugauu gcuggucuug uuuuu |
| 2973 | ccucuugauu gcuggucuug |
| 2974 | cucuugauug cuggucuugu uuuuc |
| 2975 | ucuugauugc uggucuuguu uuuca |
| 2976 | cuugauugcu ggucuuguuu uucaa |
| 2977 | uugauugcug gucuuguuuu ucaaa |
| 2978 | ugauugcugg ucuuguuuuu caaau |
| 2979 | gauugcuggu cuuguuuuuc aaauu |
| 2980 | gauugcuggu cuuguuuuuc |
| 2981 | auugcugguc uuguuuuuca aauuu |
| 2982 | uugcuggucu uguuuuucaa auuuu |
| 2983 | ugcuggucuu guuuuucaaa uuuug |
| 2984 | gcuggucuug uuuuucaaau uuugg |
| 2985 | cuggucuugu uuuucaaauu uuggg |
| 2986 | uggucuuguu uuucaaauuu ugggc |
| 2987 | ggucuuguuu uucaaauuuu gggca |
| 2988 | gucuuguuuu ucaaauuuug ggcag |
| 2989 | ucuuguuuuu caaauuuugg gcagc |
| 2990 | cuuguuuuuc aaauuuuggg cagcg |
| 2991 | uuguuuuuca aauuuugggc agcgg |
| 2992 | uguuuuucaa auuuugggca gcggu |
| 2993 | guuuuucaaa uuuugggcag cggua |
| 2994 | uuuuucaaau uuugggcagc gguaa |
| 2995 | uuuucaaauu uugggcagcg guaau |
| 2996 | uuucaaauuu ugggcagcgg uaaug |
| 2997 | uucaaauuuu gggcagcggu aauga |
| 2998 | ucaaauuuug ggcagcggua augag |
| 2999 | caaauuuugg gcagcgguaa ugagu |
| 3000 | aaauuuuggg cagcgguaau gaguu |
| 3001 | aauuuugggc agcgguaaug aguuc |
| 3002 | auuuugggca gcgguaauga guucu |
| 3003 | uuuugggcag cgguaaugag uucuu |
| 3004 | uuugggcagc gguaaugagu ucuuc |
| 3005 | uugggcagcg guaaugaguu cuucc |
| 3006 | ugggcagcgg uaaugaguuc uucca |
| 3007 | gggcagcggu aaugaguucu uccaa |
| 3008 | ggcagcggua augaguucuu ccaac |
| 3009 | gcagcgguaa ugaguucuuc caacu |
| 3010 | cagcgguaau gaguucuucc aacug |
| 3011 | agcgguaaug aguucuucca acugg |
| 3012 | gcgguaauga guucuuccaa cuggg |
| 3013 | cgguaaugag uucuuccaac ugggg |
| 3014 | gguaaugagu ucuuccaacu gggga |
| 3015 | gguaaugagu ucuuccaacu gg |
| 3016 | guaaugaguu cuuccaacug gggac |
| 3017 | uaaugaguuc uuccaacugg ggacg |
| 3018 | aaugaguucu uccaacuggg gacgc |
| 3019 | augaguucuu ccaacugggg acgcc |
| 3020 | ugaguucuuc caacugggga cgccu |
| 3021 | gaguucuucc aacuggggac gccuc |
| 3022 | aguucuucca acuggggacg ccucu |
| 3023 | guucuuccaa cuggggacgc cucug |
| 3024 | uucuuccaac uggggacgcc ucugu |
| 3025 | ucuuccaacu ggggacgccu cuguu |
| 3026 | cuuccaacug gggacgccuc uguuc |
| 3027 | uuccaacugg ggacgccucu guucc |
| 3028 | uccaacuggg gacgccucug uucca |
| 3029 | ccaacugggg acgccucugu uccaa |
| 3030 | caacugggga cgccucuguu ccaaa |
| 3031 | aacuggggac gccucuguuc caaau |
| 3032 | acuggggacg ccucuguucc aaauc |
| 3033 | cuggggacgc cucuguucca aaucc |
| 3034 | uggggacgcc ucuguuccaa auccu |
| 3035 | ggggacgccu cuguuccaaa uccug |
| 3036 | gggacgccuc uguuccaaau ccugc |
| 3037 | ggacgccucu guuccaaauc cugca |
| 3038 | gacgccucug uuccaaaucc ugcau |
| 3039 | ccaauagugg ucaguccagg agcua |
| 3040 | caauaguggu caguccagga gcuag |
| 3041 | aauagugguc aguccaggag cuagg |
| 3042 | auagugguca guccaggagc uaggu |
| 3043 | auagugguca guccaggagc u |
| 3044 | uaguggucag uccaggagcu agguc |
| 3045 | aguggucagu ccaggagcua gguca |
| 3046 | guggucaguc caggagcuag gucag |
| 3047 | uggucagucc aggagcuagg ucagg |
| 3048 | ggucagucca ggagcuaggu caggc |
| 3049 | gucaguccag gagcuagguc aggcu |
| 3050 | ucaguccagg agcuagguca ggcug |
| 3051 | caguccagga gcuaggucag gcugc |
| 3052 | aguccaggag cuaggucagg cugcu |
| 3053 | guccaggagc uaggucaggc ugcuu |
| 3054 | uccaggagcu aggucaggcu gcuuu |
| 3055 | ccaggagcua ggucaggcug cuuug |
| 3056 | caggagcuag gucaggcugc uuugc |
| 3057 | aggagcuagg ucaggcugcu uugcc |
| 3058 | ggagcuaggu caggcugcuu ugccc |
| 3059 | gagcuagguc aggcugcuuu gcccu |
| 3060 | agcuagguca ggcugcuuug cccuc |
| 3061 | gcuaggucag gcugcuuugc ccuca |
| 3062 | cucagcucuu gaaguaaacg guuua |
| 3063 | cagcucuuga aguaaacggu uuacc |
| 3064 | gcucuugaag uaaacgguuu accgc |
| 3065 | cuaggucagg cugcuuugcc cucag |
| 3066 | uaggucaggc ugcuuugccc ucagc |
| 3067 | aggucaggcu gcuuugcccu cagcu |
| 3068 | ggucaggcug cuuugcccuc agcuc |
| 3069 | gucaggcugc uuugcccuca gcucu |
| 3070 | ucaggcugcu uugcccucag cucuu |
| 3071 | caggcugcuu ugcccucagc ucuug |
| 3072 | aggcugcuuu gcccucagcu cuuga |
| 3073 | ggcugcuuug cccucagcuc uugaa |
| 3074 | gcugcuuugc ccucagcucu ugaag |
| 3075 | cugcuuugcc cucagcucuu gaagu |
| 3076 | ugcuuugccc ucagcucuug aagua |
| 3077 | gcuuugcccu cagcucuuga aguaa |
| 3078 | cuuugcccuc agcucuugaa guaaa |
| 3079 | uuugcccuca gcucuugaag uaaac |
| 3080 | uugcccucag cucuugaagu aaacg |
| 3081 | ugcccucagc ucuugaagua aacgg |
| 3082 | gcccucagcu cuugaaguaa acggu |
| 3083 | cccucagcuc uugaaguaaa cgguu |
| 3084 | ccucagcucu ugaaguaaac |
| 3085 | ccucagcucu ugaaguaaac g |
| 3086 | cucagcucuu gaaguaaacg |
| 3087 | ccucagcucu ugaaguaaac gguuu |
| 3088 | ucagcucuug aaguaaacgg uuuac |
| 3089 | agcucuugaa guaaacgguu uaccg |
| 3090 | cucuugaagu aaacgguuua ccgcc |
| 3091 | ccacaggcgu ugcacuuugc aaugc |
| 3092 | cacaggcguu gcacuuugca augcu |
| 3093 | acaggcguug cacuuugcaa ugcug |
| 3094 | caggcguugc acuuugcaau gcugc |
| 3095 | aggcguugca cuuugcaaug cugcu |
| 3096 | ggcguugcac uuugcaaugc ugcug |
| 3097 | gcguugcacu uugcaaugcu gcugu |
| 3098 | cguugcacuu ugcaaugcug cuguc |
| 3099 | cguugcacuu ugcaaugcug cug |
| 3100 | guugcacuuu gcaaugcugc ugucu |
| 3101 | uugcacuuug caaugcugcu gucuu |
| 3102 | ugcacuuugc aaugcugcug ucuuc |
| 3103 | gcacuuugca augcugcugu cuucu |
| 3104 | cacuuugcaa ugcugcuguc uucuu |
| 3105 | acuuugcaau gcugcugucu ucuug |
| 3106 | cuuugcaaug cugcugucuu cuugc |
| 3107 | uuugcaaugc ugcugucuuc uugcu |
| 3108 | uugcaaugcu gcugucuucu ugcua |
| 3109 | ugcaaugcug cugucuucuu gcuau |
| 3110 | gcaaugcugc ugucuucuug cuaug |
| 3111 | caaugcugcu gucuucuugc uauga |
| 3112 | aaugcugcug ucuucuugcu augaa |
| 3113 | augcugcugu cuucuugcua ugaau |
| 3114 | ugcugcuguc uucuugcuau gaaua |
| 3115 | gcugcugucu ucuugcuaug aauaa |
| 3116 | cugcugucuu cuugcuauga auaau |
| 3117 | ugcugucuuc uugcuaugaa uaaug |
| 3118 | gcugucuucu ugcuaugaau aaugu |
| 3119 | cugucuucuu gcuaugaaua auguc |
| 3120 | ugucuucuug cuaugaauaa uguca |
| 3121 | gucuucuugc uaugaauaau gucaa |
| 3122 | ucuucuugcu augaauaaug ucaau |
| 3123 | cuucuugcua ugaauaaugu caauc |
| 3124 | uucuugcuau gaauaauguc aaucc |
| 3125 | ucuugcuaug aauaauguca auccg |
| 3126 | cuugcuauga auaaugucaa uccga |
| 3127 | uugcuaugaa uaaugucaau ccgac |
| 3128 | ugcuaugaau aaugucaauc cgacc |
| 3129 | gcuaugaaua augucaaucc gaccu |
| 3130 | cuaugaauaa ugucaauccg accug |
| 3131 | uaugaauaau gucaauccga ccuga |
| 3132 | augaauaaug ucaauccgac cugag |
| 3133 | ugaauaaugu caauccgacc ugagc |
| 3134 | gaauaauguc aauccgaccu gagcu |
| 3135 | aauaauguca auccgaccug agcuu |
| 3136 | auaaugucaa uccgaccuga gcuuu |
| 3137 | uaaugucaau ccgaccugag cuuug |
| 3138 | aaugucaauc cgaccugagc uuugu |
| 3139 | augucaaucc gaccugagcu uuguu |
| 3140 | ugucaauccg accugagcuu uguug |
| 3141 | gucaauccga ccugagcuuu guugu |
| 3142 | ucaauccgac cugagcuuug uugua |
| 3143 | caauccgacc ugagcuuugu uguag |
| 3144 | aauccgaccu gagcuuuguu guaga |
| 3145 | auccgaccug agcuuuguug uagac |
| 3146 | uccgaccuga gcuuuguugu agacu |
| 3147 | ccgaccugag cuuuguugua gacua |
| 3148 | cgaccugagc uuuguuguag |
| 3149 | cgaccugagc uuuguuguag acuau |
| 3150 | gaccugagcu uuguuguaga cuauc |
| 3151 | accugagcuu uguuguagac uauca |
| 3152 | ccugagcuuu guuguagacu auc |
| 3153 | cauuuuugac cuacaugugg |
| 3154 | uuugaccuac auguggaaag |
| 3155 | uacauuuuug accuacaugu ggaaag |
| 3156 | ggucuccuua ccuauga |
| 3157 | ucuuaccuau gacuauggau gaga |
| 3158 | auuuuugacc uacaugggaa ag |
| 3159 | uacgaguuga uugucggacc cag |
| 3160 | guggucuccu uaccuaugac ugugg |
| 3161 | ugucucagua aucuucuuac cuau |
| 3162 | ugcauguucc agucguugug ugg |
| 3163 | cacuauucca gucaaauagg ucugg |
| 3164 | auuuaccaac cuucaggauc gagua |
| 3165 | ggccuaaaac acauacacau a |
| 3166 | gauagguggu aucaacaucu guaa |
| 3167 | gauagguggu aucaacaucu g |
| 3168 | cuuccuggau ggcuugaau |
| 3169 | uguuguuguu uaugcucauu |
| 3170 | guacauuaag auggacuuc |
| 3171 | cuguugcagu aaucuaugag |
| 3172 | ugcaguaauc uaugaguuuc |
| 3173 | gagucuucua ggagccuu |
| 3174 | ugccauuguu ucaucagcuc uuu |
| 3175 | uccuguagga cauuggcagu |
| 3176 | cuuggagucu ucuaggagcc |
| 3177 | ccauuuugug aauguuuucu uuugaacauc |
| 3178 | cccauuuugu gaauguuuuc uuuu |
| 3179 | gaaaauugug cauuuaccca uuuu |
| 3180 | uugugcauuu acccauuuug ug |
| 3181 | cccugaggca uucccaucuu gaau |
| 3182 | aggacuuacu ugcuuuguuu |
| 3183 | cuugaauuua ggagauucau cug |
| 3184 | caucuucuga uaauuuuccu guu |
| 3185 | ccauuacagu ugucuguguu |
| 3186 | ugacagccug ugaaaucugu gag |
| 3187 | uaaucugccu cuucuuuugg |
| 3188 | cagcaguagu ugucaucugc |
| 3189 | gccugagcug aucugcuggc aucuugc |
| 3190 | gccugagcug aucugcuggc aucuugcagu u |
| 3191 | ucugcuggca ucuugc |
| 3192 | gccgguugac uucauccugu gc |
| 3193 | gucugcaucc aggaacaugg guc |
| 3194 | uacuuacugu cuguagcucu uucu |
| 3195 | cugcauccag gaacaugggu cc |
| 3196 | guugaagauc ugauagccgg uuga |
| 3197 | uaggugccug ccggcuu |
| 3198 | uucagcugua gccacacc |
| 3199 | cugaacugcu ggaaagucgc c |
| 3200 | cuggcuucca aaugggaccu gaaaaagaac |
| 3201 | caauuuuucc cacucaguau u |
| 3202 | uugaaguucc uggagucuu |
| 3203 | uccucaggag gcagcucuaa au |
| 3204 | uggcucucuc ccaggg |
| 3205 | gagauggcuc ucucccaggg acccugg |
| 3206 | gggcacuuug uuuggcg |
| 3207 | ggucccagca aguuguuug |
| 3208 | ugggaugguc ccagcaaguu guuug |
| 3209 | guagagcucu gucauuuugg g |
| 3210 | gcucaagaga uccacugcaa aaaac |
| 3211 | gccauacgua cguaucauaa acauuc |
| 3212 | ucugcaggau auccaugggc ugguc |
| 3213 | gauccucccu guucgucccc uauuaug |
| 3214 | ugcuuuagac uccuguaccu gaua |
| 3215 | ggcggccuuu guguugac |
| 3216 | ggacaggccu uuauguucgu gcugc |
| 3217 | ccuuuauguu cgugcugcu |
| 3218 | ucaaggaaga uggcauuucu |
| 3219 | ucaangaaga uggcauuucu |
| 3220 | ucaagnaaga uggcauuucu |
| 3221 | ucaaggaana uggcauuucu |
| 3222 | ucaaggaaga ungcauuucu |
| 3223 | ucaaggaaga ugncauuucu |
| 3224 | ncaaggaaga uggcauuucu |
| 3225 | ucaaggaaga nggcauuucu |
| 3226 | ucaaggaaga uggcanuucu |
| 3227 | ucaaggaaga uggcaunucu |
| 3228 | ucaaggaaga uggcauuncu |
| 3229 | ucaaggaaga uggcauuucn |
| 3230 | ucnaggaaga uggcauuucu |
| 3231 | ucanggaaga uggcauuucu |
| 3232 | ucaaggnaga uggcauuucu |
| 3233 | ucaagganga uggcauuucu |
| 3234 | ucaaggaagn uggcauuucu |
| 3235 | ucaaggaaga uggcnuuucu |
| 3236 | uuugccncug cccaaugcca uccug |
| 3237 | uuugccgcun cccaaugcca uccug |
| 3238 | uuugccgcug cccaauncca uccug |
| 3239 | uuunccgcug cccaaugcca uccug |
| 3240 | uuugccgcug cccaaugcca uccun |
| 3241 | nuugccgcug cccaaugcca uccug |
| 3242 | unugccgcug cccaaugcca uccug |
| 3243 | uungccgcug cccaaugcca uccug |
| 3244 | uuugccgcng cccaaugcca uccug |
| 3245 | uuugccgcug cccanugcca uccug |
| 3246 | uuugccgcug cccaaugccn uccug |
| 3247 | uuunccncug cccaaugcca uccug |
| 3248 | uuugccgcug cccaangcca uccug |
| 3249 | uuugccgcug cccaaugcca nccug |
| 3250 | uuugccgcug cccaaugcca uccng |
| 3251 | uuugccgcug cccnaugcca uccug |
| 3252 | ucagcuucun uuagccacug |
| 3253 | ucagcuucug uuanccacug |
| 3254 | ucancuucug uuagccacug |
| 3255 | ucagcuucug uuagccacun |
| 3256 | gnnnnnnnnn nnnngnnnn |
| 3257 | nnngnnnnng nnngnnnnng |
| 3258 | nnngnnnnnn gnnngnnnnn |
| 3259 | nnnnnnnggn nnnngngnnn nnn |
| 3260 | nnnnnngnnn nnngnnngnn nnn |
| 3261 | nnnnnggnnn nngngnnnnn n |
| 3262 | gnnnnnnnnn nnnngnnnng nnn |
| 3263 | nnngnngnng nnnnnngnnn nnnng |
| 3264 | nngnngnngn nnnnngnnnn nnng |
| 3265 | nngnngnngn nnnnngnnnn nnngg |
| 3266 | ngnngnngnn nnnngnnnnn nng |
| 3267 | ngnngnngnn nnnngnnnnn nngg |
| 3268 | gnngnngnnn nnngnnnnnn ng |
| 3269 | nngnngnnnn nngnnnnnnn gg |
| 3270 | nnngnnnnng nnnnnngnnn nnnng |
| 3271 | nnnnnngnng nngnnnnnng nnnnn |
| 3272 | nngnnngnng nngnnnnnng nnnnnnnggn |
| 3273 | nnnnggnngn nggnnnnnnn |
| 3274 | nggnnnnnnn ngnnngg |
| 3275 | nnnnnngnn gnnggnnnnn nn |
| 3276 | nnnnnnnnng gnngnnggnn nnnnn |
| 3277 | nnnnngngnn nnnnnnnnnn gnn |
| 3278 | nnngngnngg nnnnnnnnnn nnn |
| 3279 | nnnnnnnggn ngnnggnnnn nnnngnnngg |
| 3280 | nnnnnnnggn ngnnggnnnn nnnng |
| 3281 | nnnnngnnng nnggnnnngn nnnnn |
| 3282 | ggnnnngngn nnnnnnnnnn gg |
| 3283 | nnnngnnngn nggnnnngnn nnnnn |
| 3284 | nnnnnnnngg ggnngnnnnn gnnnn |
| 3285 | ngnnnnngnn nnnngnnngn nggnn |
| 3286 | nngnngnnnn nggnnnng |
| 3287 | nnnnngnngn nnnnggnnnn gn |
| 3288 | nnnnngnngn nnnnggnnnn gnn |
| 3289 | nnnnngnngn nnnnggnnnn gnng |
| 3290 | nngnngnnnn nggnnnngnn gg |
| 3291 | nngnngnnnn nggnnnngnn ggn |
| 3292 | nngnngnnnn nggnnnngnn ggng |
| 3293 | nngnngnnnn nggnnnngnn ggngn |
| 3294 | gnngnnnnng gnnnngnngg ngnnn |
| 3295 | gnnnnnggnn nngnnggngn nnnng |
| 3296 | nngnnnnngg nnnngnnggn gnnnnngnnn |
| 3297 | nngnngnnnn nggnnnngnn ggngnnnnng |
| 3298 | nnnnngnngn nnnnggnnnn gnnggngnnn nng |
| 3299 | gngnnnnnnn nnnngnngnn nnnn |
| 3300 | nnngngnnnn nnnnnnngnn gnnnn |
| 3301 | ngnnnnnngn nggnnnnngg nngn |
| 3302 | nnnnnngnng gnnnnnggnn gnng |
| 3303 | nnnngnnggn nnnnggnngn ngnn |
| 3304 | nngnnggnnn nnggnngnng nnnn |
| 3305 | nnnnnnnnnn ngn |
| 3306 | ngnnnnngnn ngnnn |
| 3307 | nnnnnnnggn n |
| 3308 | nnnngnnnnn ngnn |
| 3309 | nnnnnnnnnn ngnnnngnnn |
| 3310 | nnnnnnnnnn ngn |
| 3311 | nnngnnnngn nn |
| 3312 | nnngnngnng nnnnnngnnn |
| 3313 | ngnngnnnnn ngnnnnnnng |
| 3314 | gnngnngnnn nnngnnnnnn |
| 3315 | nnggnngnng gnn |
| 3316 | nggnngnngg nn |
| 3317 | ngngnnggnn |
| 3318 | ngnnggnnnn nnnn |
| 3319 | nnnnnnnnnn |
| 3320 | nnngngnnnn nnnnn |
| 3321 | nngngnnnnn nnn |
| 3322 | ngnnnnnnnn |
| 3323 | ngnnnnnngn |
| 3324 | gnngnnnnng gnnnngnngg |
| 3325 | nnnnggnnnn gnnggngnnn |
| 3326 | nnnnnggnnn ngnnggn |
| 3327 | ngnnnnnnnn nnngnn |
| 3328 | ngnnnnnnnn n |
| 3329 | ngnnnnnngn nggnnnnngg nn |
| 3330 | ngnnnnnngn n |
| 3331 | nnnnngnngg n |
| 3332 | nggnnnnngg nn |
| 3333 | guugccuccg guucugaagg uguuc |
| 3334 | guugnnunng guunugaagg uguun |
| 3335 | caacaucaag gaagauggca uuucu |
| 3336 | gccauuucuc aacagaucu |
| 3337 | ucagcuucug uuagccacug |
| 3338 | uuuguauuua gcauguuccc |
| 3339 | auucucagga auuugugucu uuc |
| 3340 | ccauuuguau uuagcauguu ccc |
| 3341 | ucucaggaau uugugucuuu c |
| 3342 | gccauuucuc aacagaucug uca |
| 3343 | uuugccgcug cccaaugcca uccug |
| 3344 | uugccgcugc ccaaugccau ccug |
| 3345 | uugccgcugc ccaaugccau ccugg |
| 3346 | ugccgcugcc caaugccauc cug |
| 3347 | ugccgcugcc caaugccauc cugg |
| 3348 | gccgcugccc aaugccaucc ug |
| 3349 | ccgcugccca augccauccu gg |
| 3350 | uuugccncug cccaaugcca uccug |
| 3351 | caguuugccg cugcccaaug ccauc |
| 3352 | caguuugccg cugcccaaug ccauccugga |
| 3353 | ucaaggaaga uggcauuucu |
| 3354 | uggcauuucu aguuugg |
| 3355 | caucaaggaa gauggcauuu cu |
| 3356 | caacaucaag gaagauggca uuucu |
| 3357 | ccucugugau uuuauaacuu gau |
| 3358 | ccagagcagg uaccuccaac auc |
| 3359 | acaucaagga agauggcauu ucuaguuugg |
| 3360 | acaucaagga agauggcauu ucuag |
| 3361 | cucuugauug cuggucuugu uuuuc |
| 3362 | gguaaugagu ucuuccaacu gg |
| 3363 | ucuugauugc uggucuuguu uuuca |
| 3364 | uuccaacugg ggacgccucu guucc |
| 3365 | uguucuagcc ucuugauugc ugguc |
| 3366 | cuguugccuc cgguucug |
| 3367 | caacuguugc cuccgguucu ga |
| 3368 | caacuguugc cuccgguucu gaa |
| 3369 | caacuguugc cuccgguucu gaag |
| 3370 | cuguugccuc cgguucugaa gg |
| 3371 | cuguugccuc cgguucugaa ggu |
| 3372 | cuguugccuc cgguucugaa ggug |
| 3373 | cuguugccuc cgguucugaa ggugu |
| 3374 | guugccuccg guucugaagg uguuc |
| 3375 | gccuccgguu cugaaggugu ucuug |
| 3376 | uugccuccgg uucugaaggu guucuuguac |
| 3377 | cuguugccuc cgguucugaa gguguucuug |
| 3378 | caacuguugc gaagguguuc uug |
| 3379 | gaguuucuuc caaagcagcc ucuc |
| 3380 | uaugaguuuc uuccaaagca gccuc |
| 3381 | agcauccugu aggacauugg cagu |
| 3382 | cauccuguag gacauuggca guug |
| 3383 | uccuguagga cauuggcagu uguu |
| 3384 | cuguaggaca uuggcaguug uuuc |
| 3385 | auuucucaac aga |
| 3386 | agcuucuguu agcca |
| 3387 | auucucagga a |
| 3388 | auuuguauuu agca |
| 3389 | auuucucaac agaucuguca |
| 3390 | auuucucaac aga |
| 3391 | acagaucugu ca |
| 3392 | uuugccgcug cccaaugcca |
| 3393 | cgcugcccaa ugccauccug |
| 3394 | gccgcugccc aaugccaucc |
| 3395 | aaggaagaug gca |
| 3396 | aggaagaugg ca |
| 3397 | agagcaggua |
| 3398 | agcagguacc ucca |
| 3399 | accuccaaca |
| 3400 | aaugaguucu uccaa |
| 3401 | augaguucuu cca |
| 3402 | aguucuucca |
| 3403 | agccucuuga |
| 3404 | guugccuccg guucugaagg |
| 3405 | gaagguguuc |
| 3406 | ccuccgguuc ugaaggu |
| 3407 | aguuucuucc aaagca |
| 3408 | aguuucuucc a |
| 3409 | agcauccugu aggacauugg ca |
| 3410 | agcauccugu a |
| 3411 | auccuguagg a |
| 3412 | aggacauugg ca |
| 3413 | gguaaugagu unuunnaanu gg |
| 3414 | ggnaangagn ncnnccaacn gg |
| 3415 | ggunnugngu ucuuccnncu gg |
| 3416 | ggnaangagn nnnnnnaann gg |
| 3417 | ggunnugngu unuunnnnnu gg |
| 3418 | ggnnnngngn ncnnccnncn gg |
| 3419 | ggnnnngngn nnnnnnnnnn gg |
| 3420 | uguunuagnn unuugauugn uggun |
| 3421 | ngnncnagcc ncnnganngc nggnc |
| 3422 | uguucungcc ucuugnuugc ugguc |
| 3423 | ngnnnnagnn nnnnganngn nggnn |
| 3424 | uguunungnn unuugnuugn uggun |
| 3425 | ngnncnngcc ncnngnnngc nggnc |
| 3426 | ngnnnnngnn nnnngnnngn nggnn |
| 3427 | gaguuunuun naaagnagnn unun |
| 3428 | gagnnncnnc caaagcagcc ncnc |
| 3429 | gnguuucuuc cnnngcngcc ucuc |
| 3430 | gagnnnnnnn naaagnagnn nnnn |
| 3431 | gnguuunuun nnnngnngnn unun |
| 3432 | gngnnncnnc cnnngcngcc ncnc |
| 3433 | gngnnnnnnn nnnngnngnn nnnn |
| 3434 | agnaunnugu agganauugg nagu |
| 3435 | agcanccngn aggacanngg cagn |
| 3436 | ngcnuccugu nggncnuugg cngu |
| 3437 | agnannnngn aggananngg nagn |
| 3438 | ngnnunnugu nggnnnuugg nngu |
| 3439 | ngcnnccngn nggncnnngg cngn |
| 3440 | ngnnnnnngn nggnnnnngg nngn |
| 3441 | guugnnunng guunugaagg uguun |
| 3442 | uuugnngnug nnnaaugnna unnug |
| 3443 | nunuugauug nuggunuugu uuuun |
| 3444 | ncaaggaaga nggcannncn |
| 3445 | nnaaggaaga nggnannnnn |
| 3446 | ncagcnncng nnagccacng |
| 3447 | nnagnnnnng nnagnnanng |
| 3448 | ucnnggnngn uggcnuuucu |
| 3449 | ucngcuucug uungccncug |
| 3450 | unagnuunug uuagnnanug |
| 3451 | nnngnngnng nnnaangnna nnnng |
| 3452 | uuugccgcug cccnnugccn uccug |
| 3453 | gnngccnccg gnncngaagg ngnnc |
| 3454 | gnngnnnnng gnnnngaagg ngnnn |
| 3455 | guugccuccg guucugnngg uguuc |
| 3456 | ggccaaaccn cggcnnaccn |
| 3457 | unaaggaaga uggnauuunu |
| 3458 | ggccaaaccu cggcuuaccu |
| 3459 | guugnnuccg guunugaagg uguun |
| 3460 | guugnnuccg guucugaagg uguuc |
| 3461 | guugcnuccg guunugaagg uguun |
| 3462 | ngaaaacgcc gccannncnc aacagancng |
| 3463 | canaangaaa acgccgccan nncncaacag |
| 3464 | ngnncagcnn cngnnagcca cngannaaan |
| 3465 | cagnnngccg cngcccaang ccanccngga |
| 3466 | nngccgcngc ccaangccan ccnggagnnc |
| 3467 | ngcngcncnn nnccaggnnc aagngggana |
| 3468 | cnnnnagnng cngcncnnnn ccaggnncaa |
| 3469 | cnnnncnnnn agnngcngcn cnnnnccagg |
| 3470 | nnagnngcng cncnnnncca ggnncaagng |
| 3471 | cngnngccnc cggnncngaa ggngnncnng |
| 3472 | caacngnngc cnccggnncn gaaggngnnc |
| 3473 | nngccnccgg nncngaaggn gnncnngnac |
| 3474 | tgaaaacgcc gccatttctc aacagatctg |
| 3475 | cataatgaaa acgccgccat ttctcaacag |
| 3476 | tgttcagctt ctgttagcca ctgattaaat |
| 3477 | cagtttgccg ctgcccaatg ccatcctgga |
| 3478 | ttgccgctgc ccaatgccat cctggagttc |
| 3479 | tgctgctctt ttccaggttc aagtgggata |
| 3480 | cttttagttg ctgctctttt ccaggttcaa |
| 3481 | cttttctttt agttgctgct cttttccagg |
| 3482 | ttagttgctg ctcttttcca ggttcaagtg |
| 3483 | ctgttgcctc cggttctgaa ggtgttcttg |
| 3484 | caactgttgc ctccggttct gaaggtgttc |
| 3485 | ttgcctccgg ttctgaaggt gttcttgtac |
| 3486 | rrrqrrkkr |
| 3487 | rkkrrqrrr |
| 3488 | rrrrrrrrrff |
| 3489 | rrrrrffrrrr |
| 3490 | rrrr |
| 3491 | rrrrr |
| 3492 | rrrrrr |
| 3493 | rrrrrrr |
| 3494 | rrrrrrrr |
| 3495 | rrrrrrrrr |
| 3496 | rahxrahxrahxrahxrahxrahxrahxrahx |
| 3497 | rahxrrahxrrahxrrahxr |
| 3498 | rahxrrahxrrahxrrahxrrahxr |
| 3499 | rahxrrbrrahxrrbr |
| 3500 | rarrarrarrarff |
| 3501 | rgrrgrrgrrgrff |
| 3502 | MQKLQLCVYIYLFMLIVAGPVDLNENSEQKENVEKEGLCNACTWRQN TKSSRIEAIKIQILSKLRLETAPNISKDVIRQLLPKAPPLRELIDQYDVQRD DSSDGSLEDDDYHATTETIITMPTESDFLMQVDGKPKCCFFKFSSKIQYN KVVKAQLWIYLRPVETPTTVFVQILRLIKPMKDGTRYTGIRSLKLDMNP GTGIWQSIDVKTVLQNWLKQPESNLGIEIKALDENGHDLAVTFPGPGED GLNPFLEVKVTDTPKRSRRDFGLDCDEHSTESRCCRYPLTVDFEAFGW DWIIAPKRYKANYCSGECEFVFLQKYPHTHLVHQANPRGSAGPCCTPT KMSPINMLYFNGKEQIIYGKIPAMVVDRCGCS |
| 3503 | |

In embodiments, any uracil (U) or thymine (T) nucleotide in any of the modified antisense oligomer as described herein may be substituted with an X or n. In embodiments, each X or n is independently selected from uracil (U) or thymine (T).

The present disclosure will now be further defined in the following paragraphs:
1. A method of treating a subject with Duchenne muscular dystrophy and related disorders having a mutation in the dystrophin gene that is amenable to treatment by an antisense oligomer capable of inducing exon skipping during processing of dystrophin pre-mRNA, the method comprising:
   administering to the subject an effective amount of an antisense oligomer comprising 17 to 40 subunits, and further comprising a targeting sequence complementary to 12 or more contiguous nucleotides in a target region comprising an exon of human dystrophin pre-mRNA, wherein the antisense oligomer induces skipping of the exon;
   wherein, said oligomer comprises at least one subunit that is a nucleotide analog having (i) a modified internucleoside linkage, (ii) a modified sugar moiety, or (iii) a combination of the foregoing; and
      wherein, said subject has been administered a myostatin therapeutic that inhibits one or both of myostatin activity and myostatin expression in the subject, to thereby treat at least one of Duchenne muscular dystrophy or related disorders.
2. The method of paragraph 1, wherein said exon is selected from exon 7, exon 8, exon 9, exon 19, exon 23, exon 44, exon 45, exon 50, exon 51, exon 52, exon 53, or exon 55.
3. The method of paragraph 2, wherein said exon comprises exon 23.
4. The method of paragraph 2, wherein said exon comprises exon 45.
5. The method of paragraph 2, wherein said exon comprises exon 51.
6. The method of paragraph 2, wherein said exon comprises exon 53.
7. The method of paragraph 2, wherein said exon comprises exon 8, exon 44, exon 50, exon 52 or exon 55.
8. The method of paragraph 1, wherein the antisense oligomer comprises 20 to 30 subunits.
9. The method of paragraph 1, wherein said antisense oligomer comprises a sequence selected from SEQ ID NOS: 76-SEQ ID NO: 3485.
10. The method of paragraph 9, wherein said antisense oligomer is eteplirsen.
11. The method of paragraph 1, wherein said targeting sequence is complementary to at least 15 contiguous nucleotides in the target region.
12. The method of paragraph 11, wherein said targeting sequence is complementary to at least 17 contiguous nucleotides in the target region.
13. The method of paragraph 12, wherein the targeting sequence is 100% complementary to the target region.
14. The method of paragraph 1, wherein said myostatin therapeutic is selected from one or more of a protein or nucleic acid.
15. The method of paragraph 14, wherein said protein is an anti-myostatin antibody.
16. The method of paragraph 14, wherein said protein is a soluble receptor.
17. The method of paragraph 14, wherein said soluble receptor is ACVR2.
18. The method of paragraph 14, wherein said nucleic acid is at least one of an antisense oligomer or an siRNA.
19. The method of paragraph 18, wherein said antisense oligomer comprises 12 to 40 subunits, and further comprises a targeting sequence complementary to 12 or more contiguous nucleotides in a target region of myostatin pre-mRNA; and
   wherein, said oligomer comprises at least one subunit that is a nucleotide analog having (i) a modified internucleoside linkage, (ii) a modified sugar moiety, or (iii) a combination of the foregoing.
20. The method of paragraph 19, wherein the antisense oligomer comprises 20 to 30 subunits.
21. The method of paragraph 19, wherein said targeting sequence is complementary to at least 15 contiguous nucleotides in the target region.
22. The method of paragraph 19, wherein said targeting sequence is complementary to at least 17 contiguous nucleotides in the target region.
23. The method of paragraph 19, wherein said targeting sequence is 100% complementary to the target region.
24. The method of paragraph 19, wherein the target region comprises SEQ ID NO: 1.
25. The method of paragraph 19, wherein said exon comprises exon 2.
26. The method of paragraph 19, wherein said target region is selected from (i) a nucleotide sequence wherein at least one nucleotide spans a splice junction associated with intron 1/exon 2 and exon 2/intron 2; or (ii) a nucleotide sequence wherein no nucleotide spans a splice junction associated with intron 1/exon 2 and exon 2/intron 2.
27. The method of paragraph 26, wherein the splice junction is selected from a sequence comprising a splice acceptor site or a splice donor site.
28. The method of paragraph 27, wherein the splice acceptor site is provided within SEQ ID NO: 2 and the splice donor site is provided within SEQ ID NO: 3.
29. The method of paragraph 26, wherein said nucleotide of (i) is selected from SEQ ID NOS: 16-43.
30. The method of paragraph 26, wherein said nucleotide of (ii) is selected from SEQ ID NOS: 44-70.
31. The method of paragraph 1, wherein said subject is seven years of age or older.
32. A method of treating Duchenne muscular dystrophy and related disorders, the method comprising:
   administering to a subject an effective amount of an antisense oligomer of 12 to 40 subunits, and further comprising a targeting sequence complementary to 12 or more contiguous nucleotides comprising an exon of human myostatin pre-mRNA; and
   wherein, said oligomer comprises at least one subunit that is a nucleotide analog having (i) a modified internucleoside linkage, (ii) a modified sugar moiety, or (iii) a combination of the foregoing; and
   wherein, said subject has been administered a dystrophin therapeutic that increases the expression of a functional dystrophin protein in muscle cells of the subject, to thereby treat at least one of Duchenne muscular dystrophy or related disorders.
33. The method of paragraph 32, wherein the subject has a mutation in the dystrophin gene that is amenable to treatment by an antisense oligomer capable of inducing exon skipping during processing of human myostatin pre-mRNA.
34. The method of paragraph 32, wherein the antisense oligomer comprises 20 to 30 subunits.
35. The method of paragraph 32, wherein said targeting sequence is complementary to at least 15 contiguous nucleotides in the target region.
36. The method of paragraph 32, wherein said targeting sequence is complementary to at least 17 contiguous nucleotides in the target region.
37. The method of paragraph 32, wherein the antisense oligomer is 100% complementary to the target region.
38. The method of paragraph 32, wherein the target region comprises SEQ ID NO: 1.
39. The method of paragraph 32, wherein said exon comprises exon 2.
40. The method of paragraph 32, wherein said target region is selected from (i) a nucleotide sequence wherein at least one nucleotide spans a splice junction associated with intron 1/exon 2 and exon 2/intron 2; or (ii) a nucleotide sequence wherein no nucleotide spans a splice junction associated with intron 1/exon 2 and exon 2/intron 2.
41. The method of paragraph 40, wherein the splice junction is selected from a sequence comprising a splice acceptor site or a splice donor site.
42. The method of paragraph 41, wherein the splice acceptor site is provided within SEQ ID NO: 2 and the splice donor site is provided within SEQ ID NO: 3.
43. The method of paragraph 41, wherein said nucleotide of (i) is selected from SEQ ID NOS: 16-43.
44. The method of paragraph 41, wherein said nucleotide of (ii) is selected from SEQ ID NOS: 44-70.
45. The method of paragraph 32, wherein said dystrophin therapeutic is selected from one or more of a protein or nucleic acid.
46. The method of paragraph 45, wherein said nucleic acid is an antisense oligomer.
47. The method of paragraph 46, wherein said antisense oligomer comprising 20 to 50 subunits, and further comprising a targeting sequence complementary to 10 or more contiguous nucleotides in a target region comprising an exon of human dystrophin pre-mRNA; and
   wherein, said oligomer comprises at least one subunit that is a nucleotide analog having (i) a modified internucleoside linkage, (ii) a modified sugar moiety, or (iii) a combination of the foregoing.
48. The method of paragraph 47, wherein said exon is selected from exon 7, exon 8, exon 9, exon 19, exon 23, exon 44, exon 45, exon 50, exon 51, exon 52, exon 53, or exon 55.
49. The method of paragraph 48, wherein said exon comprises exon 23.
50. The method of paragraph 48, wherein said exon comprises exon 45.
51. The method of paragraph 48, wherein said exon comprises exon 51.
52. The method of paragraph 48, wherein said exon comprises exon 53.
53. The method of paragraph 48, wherein said exon comprises exon 8, exon 44, exon 50, exon 52 or exon 55.
54. The method of paragraph 47 wherein the antisense oligomer comprises 20 to 30 subunits.
55. The method of paragraph 47, wherein said antisense oligomer comprises a sequence selected from SEQ ID NOS: 76 - SEQ ID NOS: 3485.
56. The method of paragraph 55, wherein said antisense oligomer is eteplirsen.
57. The method of paragraph 46, wherein said targeting sequence is complementary to at least 15 contiguous nucleotides in the target region.
58. The method of paragraph 46, wherein said targeting sequence is complementary to at least 17 contiguous nucleotides in the target region.
59. The method of paragraph 46, wherein the targeting sequence is 100% complementary to the target region.
60. A method of treating Duchenne muscular dystrophy and related disorders in a subject having a mutation in the dystrophin gene that is amenable to treatment by an antisense oligomer capable of inducing exon skipping during processing of dystrophin pre-mRNA, the method comprising:
   administering to a subject a compound comprising formula (I) : 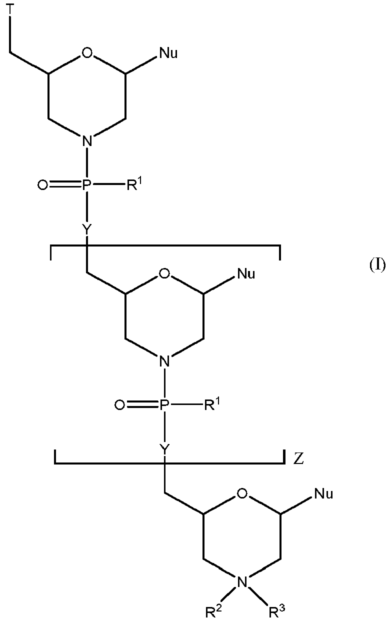 or a pharmaceutically acceptable salt thereof, wherein:
   each Nu is a nucleobase which taken together form a targeting sequence;
   Z is an integer from 8 to 48;
   each Y is independently selected from O and -NR4, wherein each R4 is independently selected from H, C1-C6 alkyl, aralkyl, C(=NH)NH2, C(O)(CH2)nNR5C(=NH)NH2, C(O)(CH2)2NHC(O)(CH2)5NR5C(=NH)NH2, and G, wherein R5 is selected from H and C1 C6 alkyl and n is an integer from 1 to 5;
   T is selected from OH and a moiety of the formula: 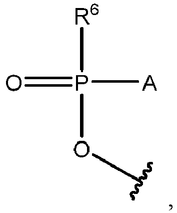 wherein:
      A is selected from -OH, -N(R⁷)₂R⁸, wherein:
         each R⁷ is independently selected from H and C₁-C₆ alkyl, and
         R⁸ is selected from an electron pair and H, and
      R⁶ is selected from OH, -N(R⁹)CH₂C(O)NH₂, and a moiety of the formula: 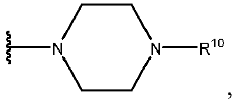 wherein:
         R⁹ is selected from H and C₁-C₆ alkyl; and
         R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
      m is an integer from 1 to 5,
      R¹¹ is of the formula -(O-alkyl)y- wherein y is an integer from 3 to 10 and each of the y alkyl groups is independently selected from C₂-C₆ alkyl; and
      R¹² is selected from H and C₁-C₆ alkyl;
   each instance of R¹ is independently selected from :
      -N(R¹³)₂R¹⁴, wherein each R¹³ is independently selected from H and C₁-C₆ alkyl, and R¹⁴ is selected from an electron pair and H;
      a moiety of formula (II): 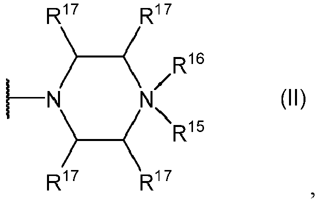 wherein:
      R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
      R¹⁸ is selected from H and C₁-C₆ alkyl; and
      q is an integer from 1 to 5,
   R¹⁶ is selected from an electron pair and H; and
   each R¹⁷ is independently selected from H and methyl; and
      a moiety of formula(III): 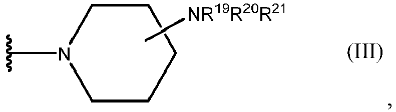 wherein:
      R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
         R²² is selected from H and C₁-C₆ alkyl; and
         r is an integer from 1 to 5,
   R²⁰ is selected from H and C₁-C₆ alkyl; and
   R²¹ is selected from an electron pair and H;
   R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂, -C(O)(CH₂)₂NHC(O)(CH₂)₅NR²⁴C(=NH)NH₂, -C(O)CH(NH₂)(CH₂)₃NHC(=NH)NH₂, and a moiety of the formula: 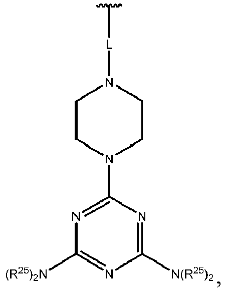 wherein,
      R²³ is of the formula -(O-alkyl)ᵥ-OH wherein v is an integer from 3 to 10 and each of the v alkyl groups is independently selected from C₂-C₆ alkyl; and
      R²⁴ is selected from H and C₁-C₆ alkyl;
      s is an integer from 1 to 5;
      L is selected from -C(O)(CH₂)₆C(O)- and -C(O)(CH₂)₂S₂(CH₂)₂C(O)-; and
   each R²⁵ is of the formula -(CH₂)₂OC(O)N(R¹⁶)₂ wherein each R²⁶ is of the formula -(CH₂)₆NHC(=NH)NH₂; and
   R³ is selected from an electron pair, H, and C₁-C₆ alkyl,
      wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP,
      and -C(O)CH₂NH-CPP, or G is of the formula: 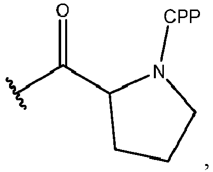
      wherein the CPP is attached to the linker moiety by an amide bond at the CPP carboxy terminus, with the proviso that up to one instance of G is present, and
      wherein the targeting sequence is complementary to 12 or more contiguous nucleotides in a target region comprising an exon of human dystrophin pre-mRNA; and
      wherein said subject has been administered a myostatin therapeutic that inhibits one or both of myostatin activity and myostatin expression in the subject, to thereby treat at least one of Duchenne muscular dystrophy or related disorders.
61. The method of paragraph 60, wherein each Nu is independently adenine, guanine, thymine, uracil, cytosine, inosine, hypoxanthine, 2,6-diaminopurine, 5-methyl cytosine, C5-propynyl-modified pyrimidines, or 10-(9-(aminoethoxy)phenoxazinyl).
62. The method of paragraph 60, wherein the target region is selected from (i) a nucleotide sequence wherein at least one nucleotide spans a splice junction associated with said exon; or (ii) a nucleotide sequence wherein no nucleotide spans a splice junction associated with said exon junction.
63. The method of paragraph 60, wherein the targeting sequence comprises a sequence selected from SEQ ID NOS: 76-3485, is a fragment of at least 10 contiguous nucleotides of a targeting sequence selected from SEQ ID NOS: 76-3485, or is a variant having at least 90% sequence identity to a targeting sequence selected from SEQ ID NOS: 76-3485.
64. The method of paragraph 60, wherein:
   i) Y is O, R² is selected from H or G, R³ is selected from an electron pair or H;
   ii) R² is G wherein the CPP is of a sequence selected from SEQ ID NOS: 3486-3501;
   iii) each R¹ is -N(CH3)2;
   iv) at least one R¹ is selected from: 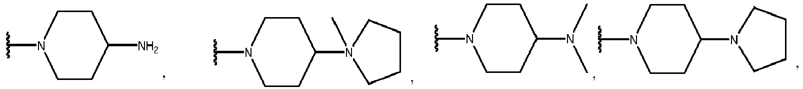 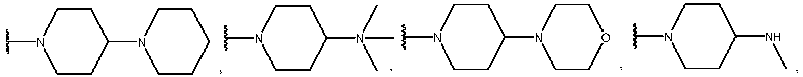 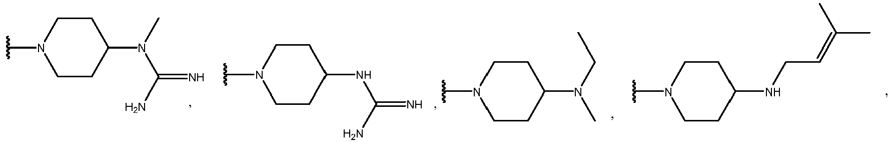 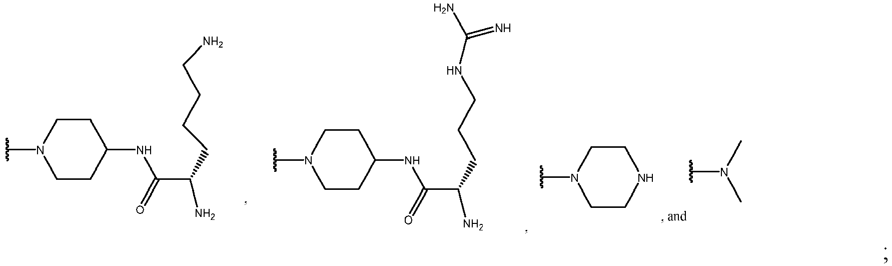 or
   v) 50-90% of the R¹ groups are -N(CH₃)₂.
65. The method of paragraph 60, wherein T is of the formula: 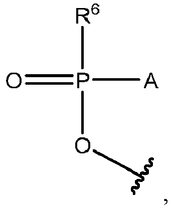 wherein A is -N(CH₃)₂, and R⁶ is of the formula: 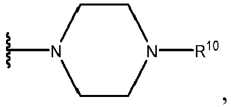 wherein R¹⁰ is -C(O)R¹¹OH.
66. The method of paragraph 60, wherein each Y is O, and T is selected from: 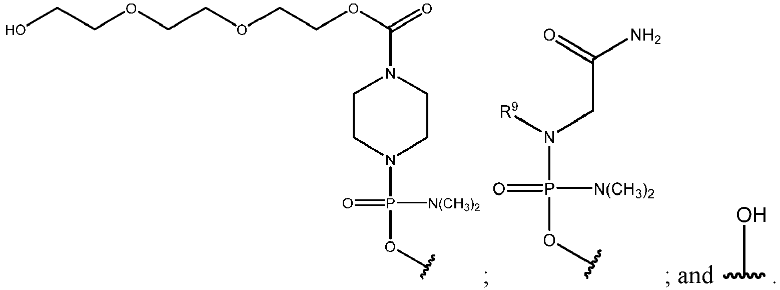
67. The method of paragraph 60, wherein T is of the formula: 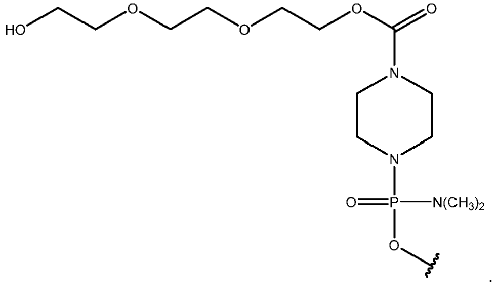
68. A method of treating Duchenne muscular dystrophy and related disorders in a subject having a mutation in the dystrophin gene that is amenable to treatment by an antisense oligomer capable of inducing exon skipping during processing of dystrophin pre-mRNA, the method comprising:
   administering to a subject a compound comprising formula (VI): 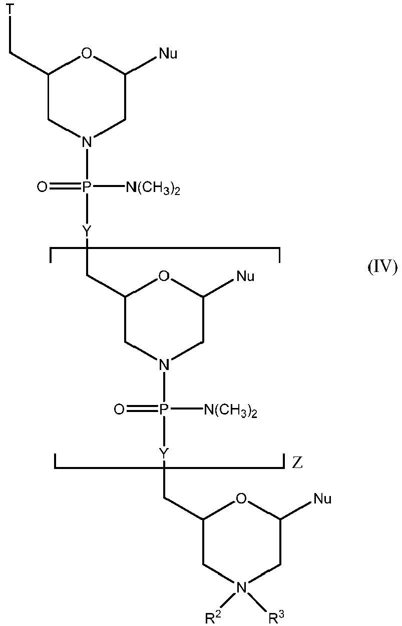 or a pharmaceutically acceptable salt thereof, wherein:
   each Nu is a nucleobase which taken together form a targeting sequence;
   Z is an integer from 8 to 48;
   each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, wherein R⁵ is selected from H and C₁-C₆ alkyl and n is an integer from 1 to 5;
   T is selected from OH and a moiety of the formula: 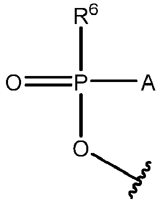 wherein:
      A is selected from -OH and -N(R⁷)₂R⁸, wherein:
         each R⁷ is independently selected from H and C₁-C₆ alkyl, and
         R⁸ is selected from an electron pair and H, and
      R⁶ is selected from OH, -N(R⁹)CH₂C(O)NH₂, and a moiety of the formula: 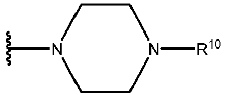 wherein:
         R⁹ is selected from H and C₁-C₆ alkyl; and
         R¹⁰ is selected from G, -C(O)-R¹¹OH, acyl, trityl, 4-methoxytrityl, -C(=NH)NH₂, -C(O)(CH₂)ₘNR¹²C(=NH)NH₂, and
         -C(O)(CH₂)₂NHC(O)(CH₂)₅NR¹²C(=NH)NH₂, wherein:
            m is an integer from 1 to 5,
            R¹¹ is of the formula -(O-alkyl)_{y}- wherein y is an integer from 3 to 10 and
               each of the y alkyl groups is independently selected from C₂-C₆ alkyl; and
   R¹² is selected from H and C₁-C₆ alkyl;
   R² is selected from H, G, acyl, trityl, 4-methoxytrityl, C₁-C₆ alkyl, -C(=NH)NH₂, and -C(O)-R²³; and
      R³ is selected from an electron pair, H, and C₁-C₆ alkyl, and
   wherein the targeting sequence comprises a sequence selected from SEQ ID NOS: 76-3485, is selected from SEQ ID NOS: 76-3485, is a fragment of at least 10 contiguous nucleotides of a sequence selected from SEQ ID NOS: 76-3485, or is a variant having at least 90% sequence identity to a sequence selected from SEQ ID NOS: 76-3485; and
   wherein said subject has been administered a myostatin therapeutic that inhibits one or both of myostatin activity and myostatin expression in the subject, to thereby treat at least one of Duchenne muscular dystrophy or related disorders.
69. A method of treating Duchenne muscular dystrophy and related disorders in a subject having a mutation in the dystrophin gene that is amenable to treatment by an antisense oligomer capable of inducing exon skipping during processing of human dystrophin pre-mRNA, the method comprising:
   administering to a subject a compound comprising formula (I): 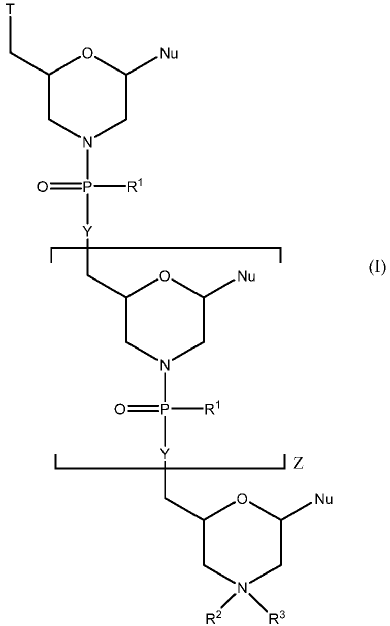 or a pharmaceutically acceptable salt thereof, wherein:
   each Nu is a nucleobase which taken together form a targeting sequence;
   Z is an integer from 8 to 48;
   each Y is independently selected from O and -NR4, wherein each R4 is independently selected from H, C1-C6 alkyl, aralkyl, C(=NH)NH2, C(O)(CH2)nNR5C(=NH)NH2, C(O)(CH2)2NHC(O)(CH2)5NR5C(=NH)NH2, and G, wherein R5 is selected from H and C1 C6 alkyl and n is an integer from 1 to 5;
   T is selected from OH and a moiety of the formula: 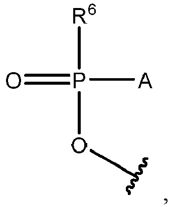 wherein:
      A is selected from -OH, -N(R⁷)₂R⁸, wherein:
         each R⁷ is independently selected from H and C₁-C₆ alkyl, and
         R⁸ is selected from an electron pair and H, and
      R⁶ is selected from OH, -N(R⁹)CH₂C(O)NH₂, and a moiety of the formula: 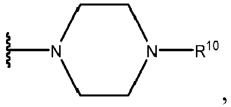 wherein:
         R⁹ is selected from H and C₁-C₆ alkyl; and
         R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
      m is an integer from 1 to 5,
      R¹¹ is of the formula -(O-alkyl)y- wherein y is an integer from 3 to 10 and each of the y alkyl groups is independently selected from C₂-C₆ alkyl; and
      R¹² is selected from H and C₁-C₆ alkyl; each instance of R¹ is independently selected from :
      -N(R¹³)₂R¹⁴, wherein each R¹³ is independently selected from H and C₁-C₆ alkyl, and R¹⁴ is selected from an electron pair and H;
         a moiety of formula (II): 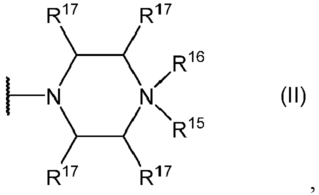 wherein:
         R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
         R¹⁸ is selected from H and C₁-C₆ alkyl; and
         q is an integer from 1 to 5,
   R¹⁶ is selected from an electron pair and H; and
   each R¹⁷ is independently selected from H and methyl; and
      a moiety of formula(III): 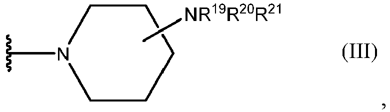 wherein:
      R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
         R²² is selected from H and C₁-C₆ alkyl; and
         r is an integer from 1 to 5,
   R²⁰ is selected from H and C₁-C₆ alkyl; and
   R²¹ is selected from an electron pair and H;
   R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂, -C(O)(CH₂)₂NHC(O)(CH₂)₅NR²⁴C(=NH)NH₂, -C(O)CH(NH₂)(CH₂)₃NHC(=NH)NH₂, and a moiety of the formula: 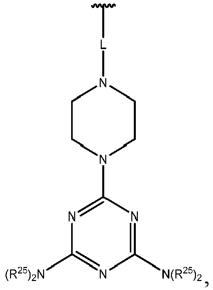 wherein,
      R²³ is of the formula -(O-alkyl)ᵥ-OH wherein v is an integer from 3 to 10 and each of the v alkyl groups is independently selected from C₂-C₆ alkyl; and
      R²⁴ is selected from H and C₁-C₆ alkyl;
      s is an integer from 1 to 5;
      L is selected from -C(O)(CH₂)₆C(O)- and -C(O)(CH₂)₂S₂(CH₂)₂C(O)-; and
   each R²⁵ is of the formula -(CH₂)₂OC(O)N(R²⁶)₂ wherein each R²⁶ is of the formula -(CH₂)₆NHC(=NH)NH₂; and
   R³ is selected from an electron pair, H, and C₁-C₆ alkyl,
      wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 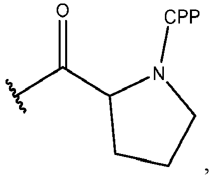
      wherein the CPP is attached to the linker moiety by an amide bond at the CPP carboxy terminus, with the proviso that up to one instance of G is present, and
      wherein the targeting sequence is complementary to 10 or more contiguous nucleotides in a target region comprising an exon of human dystrophin pre-mRNA; and
      wherein said subject has been administered a myostatin therapeutic that inhibits one or both of myostatin activity and myostatin expression in the subject, to thereby treat at least one of Duchenne muscular dystrophy or related disorders.
70. The method of paragraph 69, wherein each Nu is independently adenine, guanine, thymine, uracil, cytosine, inosine, hypoxanthine, 2,6-diaminopurine, 5-methyl cytosine, C5-propynyl-modified pyrimidines, or 10-(9-(aminoethoxy)phenoxazinyl).
71. The method of paragraph 69, wherein the targeting sequence comprises a sequence selected from SEQ ID NOS: 16-75, is a fragment of at least 10 contiguous nucleotides of a targeting sequence selected from SEQ ID NOS: 16-75, or is a variant having at least 90% sequence identity to a targeting sequence selected from SEQ ID NOS: 16-75.
72. The method of paragraph 69, wherein:
   i) Y is O, R² is selected from H or G, R³ is selected from an electron pair or H;
   ii) R² is G wherein the CPP is of a sequence selected from SEQ ID NOS: 3486-3501;
   iii) each R¹ is -N(CH₃)₂;
   iv) at least one R¹ is selected from: 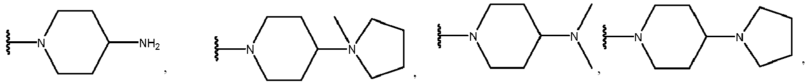 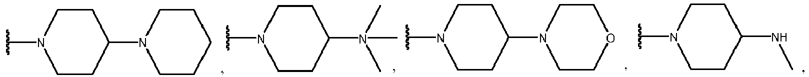 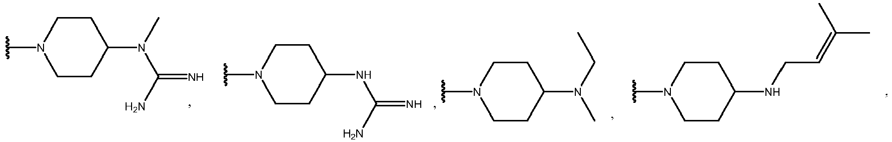 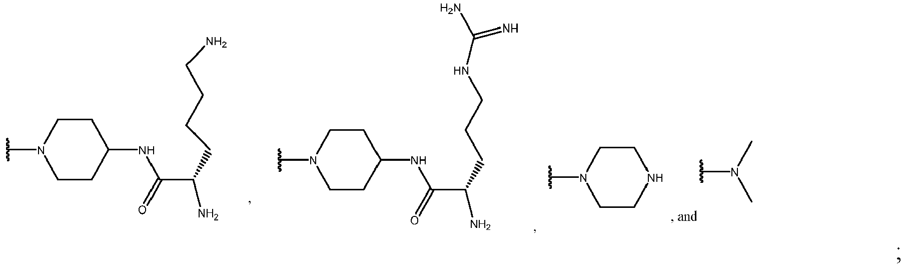 or
   v) 50-90% of the R¹ groups are -N(CH₃)₂.
73. The method of paragraph 69, wherein T is of the formula: 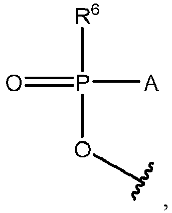 wherein A is -N(CH₃)₂, and R⁶ is of the formula: 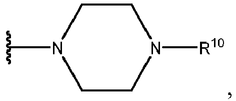 wherein R¹⁰ is -C(O)R¹¹OH.
74. The method of paragraph 69, wherein each Y is O, and T is selected from: 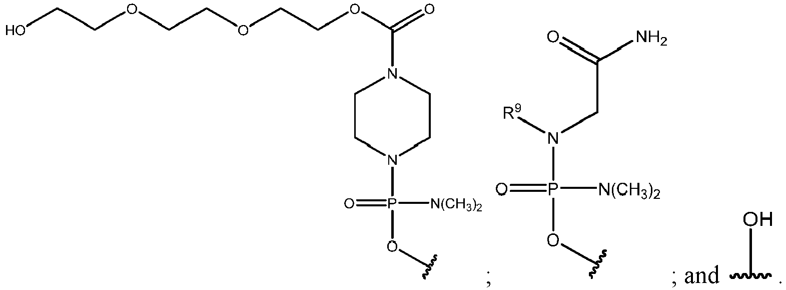
75. The method of paragraph 69, wherein T is of the formula: 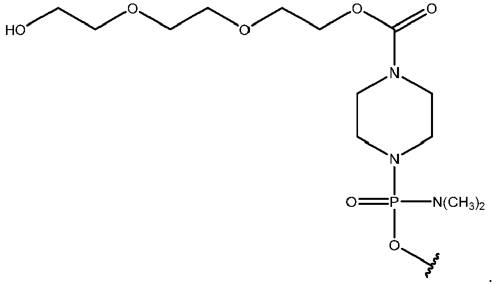
76. A method of treating Duchenne muscular dystrophy and related disorders in a subject, the method comprising:
   administering to a subject a compound comprising formula (VI): 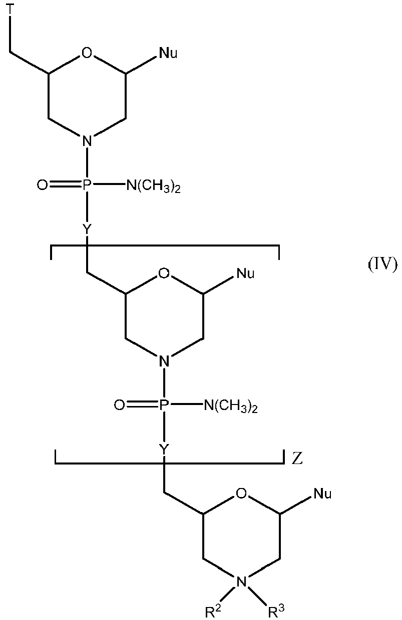 or a pharmaceutically acceptable salt thereof, wherein:
   each Nu is a nucleobase which taken together form a targeting sequence;
   Z is an integer from 8 to 48;
   each Y is independently selected from O and -NR⁴, wherein each R⁴ is independently selected from H, C₁-C₆ alkyl, aralkyl, -C(=NH)NH₂, -C(O)(CH₂)ₙNR⁵C(=NH)NH₂, -C(O)(CH₂)₂NHC(O)(CH₂)₅NR⁵C(=NH)NH₂, and G, wherein R⁵ is selected from H and C₁-C₆ alkyl and n is an integer from 1 to 5;
   T is selected from OH and a moiety of the formula: 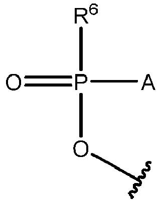 wherein:
      A is selected from -OH and -N(R⁷)₂R⁸, wherein:
         each R⁷ is independently selected from H and C₁-C₆ alkyl, and
         R⁸ is selected from an electron pair and H, and
      R⁶ is selected from OH, -N(R⁹)CH₂C(O)NH₂, and a moiety of the formula: 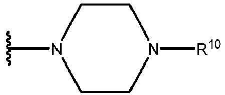 wherein:
         R⁹ is selected from H and C₁-C₆ alkyl; and
         R¹⁰ is selected from G, -C(O)-R¹¹OH, acyl, trityl, 4-methoxytrityl, -C(=NH)NH₂, -C(O)(CH₂)ₘNR¹²C(=NH)NH₂, and -C(O)(CH₂)₂NHC(O)(CH₂)₅NR¹²C(=NH)NH₂, wherein:
            m is an integer from 1 to 5,
            R¹¹ is of the formula -(O-alkyl)_{y}- wherein y is an integer from 3 to 10 and
               each of the y alkyl groups is independently selected from C₂-C₆ alkyl; and
   R¹² is selected from H and C₁-C₆ alkyl;
   R² is selected from H, G, acyl, trityl, 4-methoxytrityl, C₁-C₆ alkyl, -C(=NH)NH₂, and -C(O)-R²³; and
      R³ is selected from an electron pair, H, and C₁-C₆ alkyl, and
   wherein the targeting sequence comprises a sequence selected from SEQ ID NOS: 16-75, is selected from SEQ ID NOS: 16-75, is a fragment of at least 10 contiguous nucleotides of a sequence selected from SEQ ID NOS: 16-75, or is a variant having at least 90% sequence identity to a sequence selected from SEQ ID NOS: 16-75,and
   wherein said subject has been administered a dystrophin therapeutic that increases the expression of a functional dystrophin protein in muscle cells of the subject, to thereby treat at least one of Duchenne muscular dystrophy or related disorders.
77. A dystrophin-related pharmaceutical composition, comprising an antisense oligomer compound of 17 to 40 subunits and a pharmaceutically acceptable carrier, the compound comprising:
   at least one subunit that is a nucleotide analog having (i) a modified internucleoside linkage, (ii) a modified sugar moiety, or (iii) a combination of the foregoing; and
   a targeting sequence complementary to 10 or more contiguous nucleotides in a target region comprising an exon of human dystrophin pre-mRNA;
   together with a myostatin-related pharmaceutical composition, comprising an antisense oligomer compound of 12 to 40 subunits and a pharmaceutically acceptable carrier, the compound comprising:
      at least one subunit that is a nucleotide analog having (i) a modified internucleoside linkage, (ii) a modified sugar moiety, or (iii) a combination of the foregoing; and
      a targeting sequence complementary to 12 or more contiguous nucleotides in a target region comprising an exon of human myostatin pre-mRNA.
78. The composition of 77, wherein the dystrophin-related composition and the myostatin-related composition are provided in the same pharmaceutical composition.
79. A method for modulating myostatin expression in a subject having a genetic mutation amenable to treatment by an antisense oligomer capable of inducing exon skipping during processing of human myostatin pre-mRNA, the methodcomprising:
   administering to the subject an effective amount of an antisense oligomer comprising 12 to 40 subunits, and further comprising a targeting sequence complementary to 12 or more contiguous nucleotides comprising an exon of human myostatin pre-mRNA;and
   wherein, said oligomer comprises at least one subunit that is a nucleotide analog having (i) a modified internucleoside linkage, (ii) a modified sugar moiety, or (iii) a combination of the foregoing;
   binding the antisense oligomer to the target region in the myostatin pre-mRNA transcript; and,
   inhibiting transcription of the target region into a human myostatin mRNA transcript,
   wherein said subject has been administered a dystrophin therapeutic that increases dystrophin expression in the subject.
80. A method for decreasing expression of exon 2 in a subject having a genetic mutation amenable to treatment by an antisense oligomer capable of inducing exon skipping during processing of human myostatin pre-mRNA, the method comprising:
   administering to the subject an effective amount of an antisense oligomer comprising 12 to 40 subunits, and further comprising a targeting sequence complementary to 12 or more contiguous nucleotides comprising an exon of human myostatin pre-mRNAand inhibiting transcription of exon 2 in a myostatin mRNA transcript,
   wherein said subject has been administered a dystrophin therapeutic that increases dystrophin expression in the subject.
81. The method of paragraph 80, wherein exon 2 expression is decreased by about 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% in a myostatin mRNA transcript.
82. A method for decreasing the accumulation of functional myostatin protein in a muscle cell or tissue in a subject having a mutation in the dystrophin gene that is amenable to treatment by an antisense oligomer capable of inducing exon skipping during processing of human myostatin pre-mRNA, the method comprising:
   administering to the subject an effective amount of an antisense oligomer comprising 12 to 40 subunits, and further comprising a targeting sequence complementary to 12 or more contiguous nucleotides comprising an exon of human myostatin pre-mRNA, and
   inhibiting transcription of exon 2 in a myostatin mRNA transcript,
   wherein said subject has been administered a dystrophin therapeutic that increases dystrophin expression in the subject.
83. A medicament for the treatment of Duchenne muscular dystrophy and related disorders comprising:
   an antisense oligomer compound comprising 12 to 40 subunits, comprising at least one subunit that is a nucleotide analog having (i) a modified internucleoside linkage, (ii) a modified sugar moiety, or (iii) a combination of the foregoing; and further comprising a targeting sequence complementary to 12 or more contiguous nucleotides comprising an exon of human myostatin pre mRNA; and
   a dystrophin therapeutic that increases expression of a functional dystrophin protein.
84. A method for inhibiting the progression of Duchenne muscular dystrophy and related disorders in a subject having a mutation in the dystrophin gene that is amenable to treatment by an antisense oligomer capable of inducing exon skipping during processing of human myostatin pre-mRNA, the method comprising:
   administering to the subject an effective amount of an antisense oligomer comprising 12 to 40 subunits, and further comprising a targeting sequence complementary to 12 or more contiguous nucleotides comprising an exon of human myostatin pre-mRNA; and, inhibiting transcription of exon 2 in a myostatin mRNA transcript,
   wherein said subject has been administered a dystrophin therapeutic that increases dystrophin expression in the subject to thereby inhibit the progression of Duchenne muscular dystrophy.
85. A method of decreasing the accumulation of a functional myostatin protein in a subject with Duchenne muscular dystrophy and related disorders, said method comprising:
   administering to the subject an effective amount of an antisense oligomer comprising 12 to 40 subunits, and further comprising a targeting sequence complementary to 12 or more contiguous nucleotides comprising an exon of human myostatin pre-mRNA;
   inhibiting transcription of exon 2 in a myostatin mRNA transcript,
   wherein the accumulation of functional myostatin protein in the subject is decreased, and
   wherein said subject has been administered a dystrophin therapeutic that increases expression of a functional dystrophin protein in muscle cells of the subject.
86. A method for treating Duchenne muscular dystrophy and related disorders in a subject in need of such treatment, comprising:
   administering an antisense oligomer in an effective amount to result in a peak blood concentration of at least about 200-400 nM of antisense oligomer in the subject.
87. A method of treating skeletal muscle mass deficiency in a subject having a mutation in the dystrophin gene that is amenable to treatment by an antisense oligomer capable of inducing exon skipping during processing of human myostatin pre-mRNA, the method comprising:
   (a) measuring blood or tissue levels of myostatin protein in the subject;
   (b) administering to the subject, an effective amount of an antisense oligomer comprising 12 to 40 subunits, and further comprising a targeting sequence complementary to 12 or more contiguous nucleotides comprising an exon of human myostatin pre-mRNA;
   (c) inhibiting transcription of exon 2 in a myostatin mRNA transcript;
   (d) measuring myostatin protein levels in the subject after a select time; and,
   (e) repeating said administering using the levels measured in (d) to adjust the dose or dosing schedule of the amount of antisense oligomer administered, wherein the level of myostatin protein is decreased in the subject after administering the antisense oligomer, and wherein said subject has been administered a dystrophin therapeutic that increases expression of a functional dystrophin expression protein in muscle cells of the subject.
88. A method of inhibiting the progression of Duchenne muscular dystrophy and related disorders in a subject having a mutation in the dystrophin gene that is amenable to treatment by an antisense oligomer capable of inducing exon skipping during processing of dystrophin pre-mRNA, the method comprising:
   administering to the subject an effective amount of an antisense oligomer comprising 17 to 40 subunits, and further comprising a targeting sequence complementary to 12 or more contiguous nucleotides in a target region comprising an exon of human dystrophin pre-mRNA, wherein the antisense oligomer induces skipping of the exon;
   wherein, said oligomer comprises at least one subunit that is a nucleotide analog having (i) a modified internucleoside linkage, (ii) a modified sugar moiety, or (iii) a combination of the foregoing; and
   wherein, said subject has been administered a myostatin therapeutic that inhibits one or both of myostatin activity and expression in the subject, to thereby inhibit the progression of at least one of Duchenne muscular dystrophy or related disorders.
89. A method of inhibiting the progression of Duchenne muscular dystrophy, the method comprising:
   administering to the subject an effective amount of an antisense oligomer comprising 12 to 40 subunits, and further comprising a targeting sequence complementary to 12 or more contiguous nucleotides comprising an exon of human myostatin pre-mRNA; and
   wherein, said oligomer comprises at least one subunit that is a nucleotide analog having (i) a modified internucleoside linkage, (ii) a modified sugar moiety, or (iii) a combination of the foregoing; and
   wherein said subject has been administered a dystrophin therapeutic that increases expression of a functional dystrophin protein in muscle cells of the subject to thereby inhibit the progression of Duchenne muscular dystrophy.
90. The antisense oligomer according to any one of the foregoing paragraphs, further comprising an arginine-rich peptide sequence conjugated to the 3' terminal end or the 5' terminal end of the antisense oligomer, wherein the arginine-rich peptide sequence comprises a sequence selected from SEQ ID NOS: 3486-3501.
91. The method according to any one of paragraphs 60-75 wherein R² is G, and the CPP comprises a sequence selected from SEQ ID NOS: 3486-3501.
92. The medicament of paragraph 83, further comprising an arginine-rich peptide sequence conjugated to the 3' terminal end of an antisense oligomer, wherein the arginine-rich peptide sequence comprises a sequence selected from SEQ ID NOS: 3486-3501.
93. A composition comprising:
   an antisense oligomer comprising 17 to 40 subunits, and further comprising a targeting sequence complementary to 12 or more contiguous nucleotides in a target region comprising an exon of human dystrophin pre-mRNA;
   wherein said dystrophin-targeted oligomer comprises at least one subunit that is a nucleotide analog having (i) a modified internucleoside linkage, (ii) a modified sugar moiety, or (iii) a combination of the foregoing; and
   an antisense oligomer comprising 12 to 40 subunits, and further comprising a targeting sequence complementary to 12 or more contiguous nucleotides comprising an exon of human myostatin pre-mRNA;
   wherein said myostatin-targeted oligomer comprises at least one subunit that is a nucleotide analog having (i) a modified internucleoside linkage, (ii) a modified sugar moiety, or (iii) a combination of the foregoing.
94. A method for modulating dystrophin expression in a subject having a genetic mutation amenable to treatment by an antisense oligomer capable of inducing exon skipping during processing of human dystrophin pre-mRNA, the method comprising:
   administering to the subject an effective amount of an antisense oligomer comprising 17 to 40 subunits, and further comprising a targeting sequence complementary to 12 or more contiguous nucleotides comprising an exon of human dystrophin pre-mRNA;and
   wherein, said oligomer comprises at least one subunit that is a nucleotide analog having (i) a modified internucleoside linkage, (ii) a modified sugar moiety, or (iii) a combination of the foregoing;
   wherein said subject has been administered a myostatin therapeutic that inhibits one or both of myostatin activity and myostatin expression in the subject.
95. A method for modulating myostatin expression in a subject having a genetic mutation amenable to treatment by an antisense oligomer capable of inducing exon skipping during processing of human myostatin pre-mRNA, the method comprising:
   administering to the subject an effective amount of an antisense oligomer comprising 12 to 40 subunits, and further comprising a targeting sequence complementary to 12 or more contiguous nucleotides comprising an exon of human myostatin pre-mRNA; and
   wherein, said oligomer comprises at least one subunit that is a nucleotide analog having (i) a modified internucleoside linkage, (ii) a modified sugar moiety, or (iii) a combination of the foregoing;
   wherein said subject has been administered a dystrophin therapeutic that increases the expression of a functional dystrophin protein in muscle cells of the subject.
96. The method of any corresponding paragraphs 1-95, wherein at least one of the antisense oligomer, dystrophin therapeutic or myostatin therapeutic is administered to the subject systemically.
97. The method of paragraph 96, wherein said subject is human.
98. The method of paragraph 97, wherein said subject is seven years of age or older.
99. The method of paragraph 98, wherein no antisense oligomer or dystrophin therapeutic comprises SEQ ID NO:927.

## Claims

1. A composition comprising:
an antisense oligomer comprising 17 to 40 subunits, and further comprising a targeting sequence complementary to 12 or more contiguous nucleotides in a target region comprising an exon of human dystrophin pre-mRNA;
wherein the antisense oligomer is a compound of formula (I): 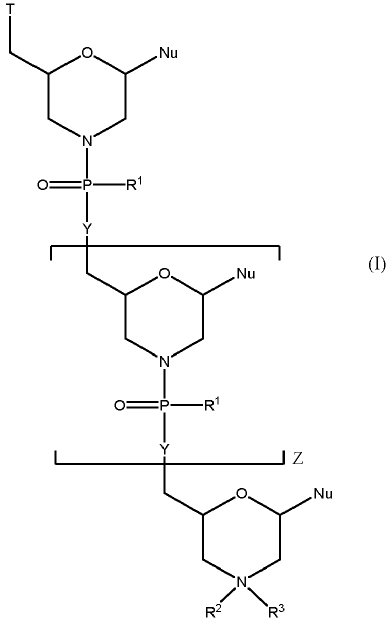 or a pharmaceutically acceptable salt thereof, wherein:
each Nu is a nucleobase which taken together form a targeting sequence;
Z is an integer from 8 to 48;
each Y is independently selected from O and -NR⁴, wherein each R⁴ is independently selected from H, C1-C6 alkyl, aralkyl, C(=NH)NH₂, C(O)(CH₂)nNR⁵C(=NH)NH₂, C(O)(CH₂)₂NHC(O)(CH₂)₅NR⁵C(=NH)NH₂, and G, wherein R⁵ is selected from H and C1-C6 alkyl and n is an integer from 1 to 5;
T is selected from OH and a moiety of the formula: 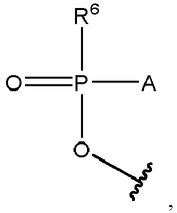 wherein:
A is selected from -OH, -N(R⁷)₂R⁸, wherein:
each R⁷ is independently selected from H and C₁-C₆ alkyl, and
R⁸ is selected from an electron pair and H, and
R⁶ is selected from OH, -N(R⁹)CH₂C(O)NH₂, and a moiety of the formula: 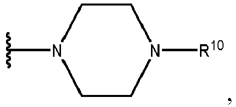 wherein:
R⁹ is selected from H and C₁-C₆ alkyl; and
R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
m is an integer from 1 to 5,
R¹¹ is of the formula -(O-alkyl)y- wherein y is an integer from 3 to 10 and each of the y alkyl groups is independently selected from C₂-C₆ alkyl; and
R¹² is selected from H and C₁-C₆ alkyl;
each instance of R¹ is independently selected from :
-N(R¹³)₂R¹⁴, wherein each R¹³ is independently selected from H and C₁-C₆ alkyl, and R¹⁴ is selected from an electron pair and H;
a moiety of formula (II): 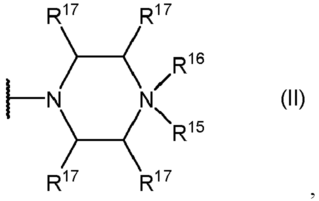 wherein:
R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
R¹⁸ is selected from H and C₁-C₆ alkyl; and
q is an integer from 1 to 5,
R¹⁶ is selected from an electron pair and H; and
each R¹⁷ is independently selected from H and methyl; and
a moiety of formula (III): 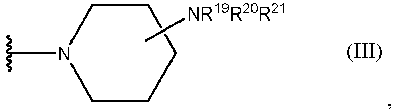 wherein:
R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
R²² is selected from H and C₁-C₆ alkyl; and
r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂,
-C(O)(CH₂)₂NHC(O)(CH₂)₅NR²⁴C(=NH)NH₂, -C(O)CH(NH₂)(CH₂)₃NHC(=NH)NH₂, and a moiety of the formula: 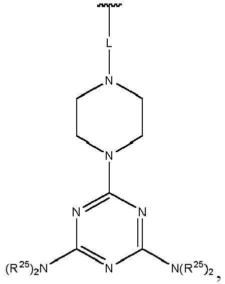 wherein,
R²³ is of the formula -(O-alkyl)ᵥ-OH wherein v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and each R²⁵ is of the formula -(CH₂)₂OC(O)N(R²⁶)₂ wherein each R²⁶ is of the formula -(CH₂)₆NHC(=NH)NH₂; and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 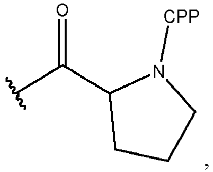
wherein the CPP is attached to the linker moiety by an amide bond at the CPP carboxy terminus, with the proviso that up to one instance of G is present; and
an antisense oligomer comprising 12 to 40 subunits, and further comprising a targeting sequence complementary to 12 or more contiguous nucleotides comprising an exon of human myostatin pre-mRNA;
wherein said myostatin-targeted oligomer comprises at least one subunit that is a nucleotide analog having (i) a modified internucleoside linkage, (ii) a modified sugar moiety, or (iii) a combination of the foregoing.

2. An antisense oligomer comprising 17 to 40 subunits, and further comprising a targeting sequence complementary to 12 or more contiguous nucleotides in a target region comprising an exon of human dystrophin pre-mRNA, wherein the antisense oligomer induces skipping of the exon; wherein the antisense oligomer is a compound of formula (I): 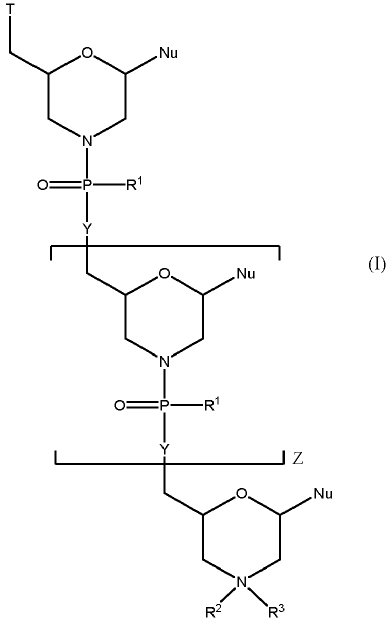 or a pharmaceutically acceptable salt thereof, wherein:
each Nu is a nucleobase which taken together form a targeting sequence;
Z is an integer from 8 to 48;
each Y is independently selected from O and -NR⁴, wherein each R⁴ is independently selected from H, C1-C6 alkyl, aralkyl, C(=NH)NH₂, C(O)(CH₂)nNR⁵C(=NH)NH₂, C(O)(CH₂)₂NHC(O)(CH₂)₅NR⁵C(=NH)NH₂, and G, wherein R⁵ is selected from H and C1-C6 alkyl and n is an integer from 1 to 5;
T is selected from OH and a moiety of the formula: 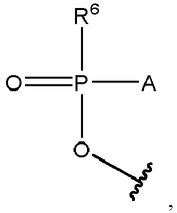 wherein:
A is selected from -OH, -N(R⁷)₂R⁸, wherein:
each R⁷ is independently selected from H and C₁-C₆ alkyl, and
R⁸ is selected from an electron pair and H, and
R⁶ is selected from OH, -N(R⁹)CH₂C(O)NH₂, and a moiety of the formula: 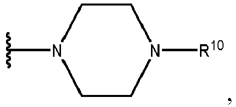 wherein:
R⁹ is selected from H and C₁-C₆ alkyl; and
R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
m is an integer from 1 to 5,
R¹¹ is of the formula -(O-alkyl)y- wherein y is an integer from 3 to 10 and
each of the y alkyl groups is independently selected from C₂-C₆ alkyl; and
R¹² is selected from H and C₁-C₆ alkyl;
each instance of R¹ is independently selected from :
-N(R¹³)₂R¹⁴, wherein each R¹³ is independently selected from H and C₁-C₆ alkyl, and R¹⁴ is selected from an electron pair and H;
a moiety of formula (II): 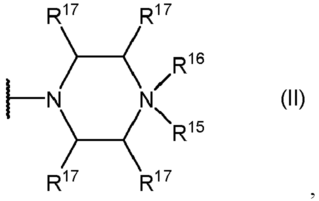 wherein:
R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
R¹⁸ is selected from H and C₁-C₆ alkyl; and
q is an integer from 1 to 5,
R¹⁶ is selected from an electron pair and H; and
each R¹⁷ is independently selected from H and methyl; and
a moiety of formula (III): 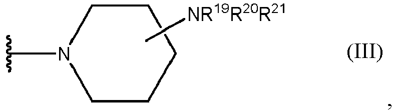 wherein:
R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
R²² is selected from H and C₁-C₆ alkyl; and
r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂,
-C(O)(CH₂)₂NHC(O)(CH₂)₅NR²⁴C(=NH)NH₂, -C(O)CH(NH₂)(CH₂)₃NHC(=NH)NH₂, and a moiety of the formula: 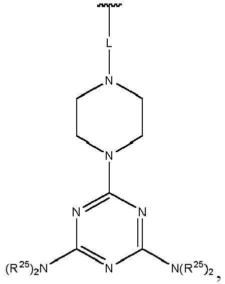 wherein,
R²³ is of the formula -(O-alkyl)ᵥ-OH wherein v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and
each R²⁵ is of the formula -(CH₂)₂OC(O)N(R²⁶)₂ wherein each R²⁶ is of the formula -(CH₂)₆NHC(=NH)NH₂; and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP,
and -C(O)CH₂NH-CPP, or G is of the formula: 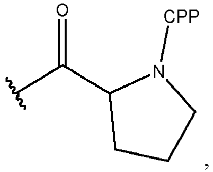
wherein the CPP is attached to the linker moiety by an amide bond at the CPP carboxy terminus, with the proviso that up to one instance of G is present;
for use in treating a subject with Duchenne muscular dystrophy and related disorders having a mutation in the dystrophin gene that is amenable to treatment by an antisense oligomer capable of inducing exon skipping during processing of dystrophin pre-mRNA;
wherein said use is in combination with a myostatin therapeutic that inhibits one or both of myostatin activity and myostatin expression in a subject, wherein said myostatin therapeutic is administered before said antisense oligomer.

3. The antisense oligomer for use of claim 2, wherein said antisense oligomer comprises a sequence selected from SEQ ID NOS: 76-SEQ ID NO: 3485.

4. The antisense oligomer for use of claim 2, wherein said antisense oligomer is eteplirsen.

5. The antisense oligomer for use of claim 2, wherein said myostatin therapeutic is a protein, and said protein is an anti-myostatin antibody or a soluble receptor, preferably said soluble receptor is ACVR2.

6. The antisense oligomer for use of claim 2, wherein said myostatin therapeutic is a nucleic acid which is at least one of an antisense oligomer or an siRNA, preferably wherein said nucleic acid is an antisense oligomer which comprises 12 to 40 subunits, and further comprises a targeting sequence complementary to 12 or more contiguous nucleotides in a target region of myostatin pre-mRNA; and
wherein, said oligomer comprises at least one subunit that is a nucleotide analog having (i) a modified internucleoside linkage, (ii) a modified sugar moiety, or (iii) a combination of the foregoing.

7. A combination comprising:
an antisense oligomer compound comprising 12 to 40 subunits, comprising at least one subunit that is a nucleotide analog having (i) a modified internucleoside linkage, (ii) a modified sugar moiety, or (iii) a combination of the foregoing; and further comprising a targeting sequence complementary to 12 or more contiguous nucleotides comprising an exon of human myostatin pre mRNA; and
a dystrophin therapeutic that increases expression of a functional dystrophin protein; wherein the dystrophin therapeutic is an antisense oligomer which is a compound of formula (I): 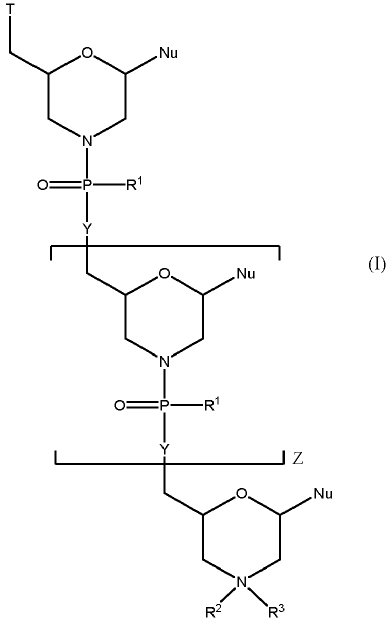 or a pharmaceutically acceptable salt thereof, wherein:
each Nu is a nucleobase which taken together form a targeting sequence;
Z is an integer from 8 to 48;
each Y is independently selected from O and -NR⁴, wherein each R⁴ is independently selected from H, C1-C6 alkyl, aralkyl, C(=NH)NH₂, C(O)(CH₂)nNR⁵C(=NH)NH₂, C(O)(CH₂)₂NHC(O)(CH₂)₅NR⁵C(=NH)NH₂, and G, wherein R⁵ is selected from H and C1-C6 alkyl and n is an integer from 1 to 5;
T is selected from OH and a moiety of the formula: 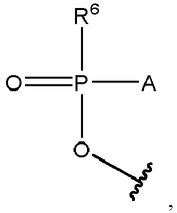 wherein:
A is selected from -OH, -N(R⁷)₂R⁸, wherein:
each R⁷ is independently selected from H and C₁-C₆ alkyl, and
R⁸ is selected from an electron pair and H, and
R⁶ is selected from OH, -N(R⁹)CH₂C(O)NH₂, and a moiety of the formula: 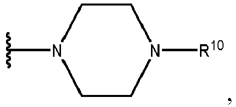 wherein:
R⁹ is selected from H and C₁-C₆ alkyl; and
R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
m is an integer from 1 to 5,
R¹¹ is of the formula -(O-alkyl)y- wherein y is an integer from 3 to 10 and
each of the y alkyl groups is independently selected from C₂-C₆ alkyl; and
R¹² is selected from H and C₁-C₆ alkyl;
each instance of R¹ is independently selected from :
-N(R¹³)₂R¹⁴, wherein each R¹³ is independently selected from H and C₁-C₆ alkyl, and R¹⁴ is selected from an electron pair and H;
a moiety of formula (II): 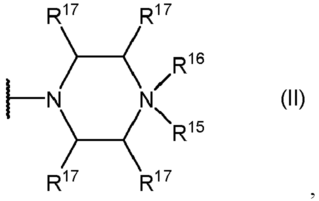 wherein:
R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
R¹⁸ is selected from H and C₁-C₆ alkyl; and
q is an integer from 1 to 5,
R¹⁶ is selected from an electron pair and H; and
each R¹⁷ is independently selected from H and methyl; and
a moiety of formula (III): 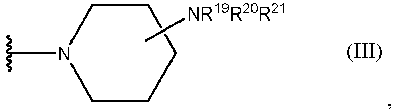 wherein:
R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
R²² is selected from H and C₁-C₆ alkyl; and
r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂,
-C(O)(CH₂)₂NHC(O)(CH₂)₅NR²⁴C(=NH)NH₂, -C(O)CH(NH₂)(CH₂)₃NHC(=NH)NH₂, and a moiety of the formula: 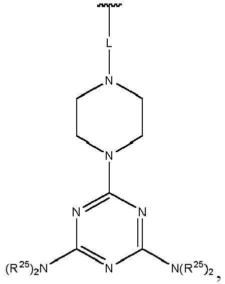 wherein,
R²³ is of the formula -(O-alkyl)ᵥ-OH wherein v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and each R²⁵ is of the formula -(CH₂)₂OC(O)N(R²⁶)₂ wherein each R²⁶ is of the formula -(CH₂)₆NHC(=NH)NH₂; and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 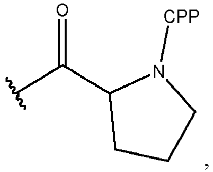
wherein the CPP is attached to the linker moiety by an amide bond at the CPP carboxy terminus, with the proviso that up to one instance of G is present;
for use in the treatment of Duchenne muscular dystrophy and related disorders.

8. An antisense oligomer of 12 to 40 subunits, and further comprising a targeting sequence complementary to 12 or more contiguous nucleotides comprising an exon of human myostatin pre-mRNA;
wherein, said oligomer comprises at least one subunit that is a nucleotide analog having (i) a modified internucleoside linkage, (ii) a modified sugar moiety, or (iii) a combination of the foregoing; for use in treating Duchenne muscular dystrophy and related disorders,
wherein said use is in combination with a dystrophin therapeutic that increases the expression of a functional dystrophin protein in muscle cells of the subject, wherein the dystrophin therapeutic is an antisense oligomer which is a compound of formula (I): 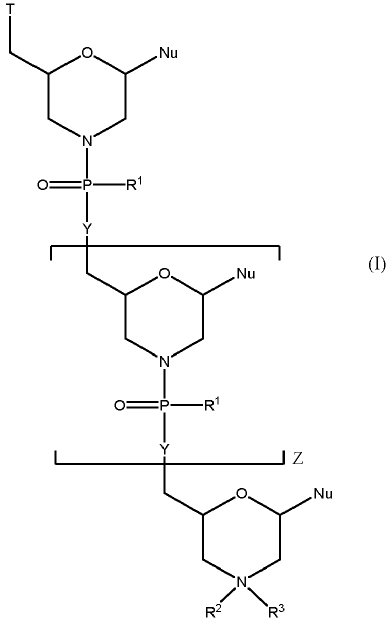 or a pharmaceutically acceptable salt thereof, wherein:
each Nu is a nucleobase which taken together form a targeting sequence;
Z is an integer from 8 to 48;
each Y is independently selected from O and -NR⁴, wherein each R⁴ is independently selected from H, C1-C6 alkyl, aralkyl, C(=NH)NH₂, C(O)(CH₂)nNR⁵C(=NH)NH₂, C(O)(CH₂)₂NHC(O)(CH₂)₅NR⁵C(=NH)NH₂, and G, wherein R⁵ is selected from H and C1-C6 alkyl and n is an integer from 1 to 5;
T is selected from OH and a moiety of the formula: 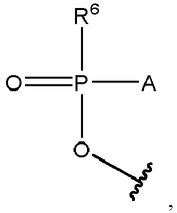 wherein:
A is selected from -OH, -N(R⁷)₂R⁸, wherein:
each R⁷ is independently selected from H and C₁-C₆ alkyl, and
R⁸ is selected from an electron pair and H, and
R⁶ is selected from OH, -N(R⁹)CH₂C(O)NH₂, and a moiety of the formula: 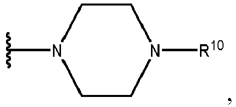 wherein:
R⁹ is selected from H and C₁-C₆ alkyl; and
R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
m is an integer from 1 to 5,
R¹¹ is of the formula -(O-alkyl)y- wherein y is an integer from 3 to 10 and
each of the y alkyl groups is independently selected from C₂-C₆ alkyl; and
R¹² is selected from H and C₁-C₆ alkyl;
each instance of R¹ is independently selected from :
-N(R¹³)₂R¹⁴, wherein each R¹³ is independently selected from H and C₁-C₆ alkyl, and R¹⁴ is selected from an electron pair and H;
a moiety of formula (II): 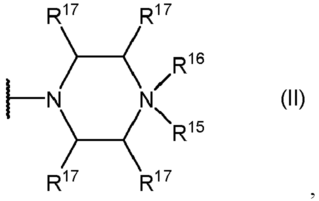 wherein:
R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
R¹⁸ is selected from H and C₁-C₆ alkyl; and
q is an integer from 1 to 5,
R¹⁶ is selected from an electron pair and H; and
each R¹⁷ is independently selected from H and methyl; and
a moiety of formula (III): 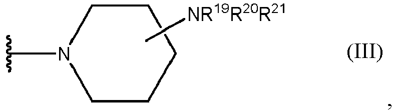 wherein:
R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
R²² is selected from H and C₁-C₆ alkyl; and
r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂, -C(O)(CH₂)₂NHC(O)(CH₂)₅NR²⁴C(=NH)NH₂, -C(O)CH(NH₂)(CH₂)₃NHC(=NH)NH₂, and a moiety of the formula: 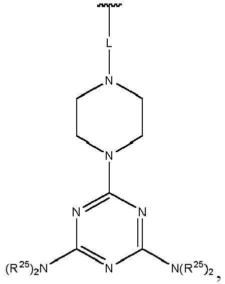 wherein,
R²³ is of the formula -(O-alkyl)ᵥ-OH wherein v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and
each R²⁵ is of the formula -(CH₂)₂OC(O)N(R²⁶)₂ wherein each R²⁶ is of the formula -(CH₂)₆NHC(=NH)NH₂; and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 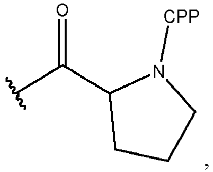 wherein the CPP is attached to the linker moiety by an amide bond at the CPP carboxy terminus, with the proviso that up to one instance of G is present;
wherein said dystrophin therapeutic is administered before said antisense oligomer.

9. The antisense oligomer for use of claim 8, wherein said target region is selected from (i) a nucleotide sequence wherein at least one nucleotide spans a splice junction associated with intron 1/exon 2 and exon 2/intron 2; or (ii) a nucleotide sequence wherein no nucleotide spans a splice junction associated with intron 1/exon 2 and exon 2/intron 2, wherein said splice junction is selected from a sequence comprising a splice acceptor site or a splice donor site, preferably wherein the splice acceptor site is provided within SEQ ID NO: 2 and the splice donor site is provided within SEQ ID NO: 3.

10. The antisense oligomer for use according to any one of claims 2-9, wherein the antisense oligomer further comprises an arginine-rich peptide sequence conjugated to the 3' terminal end or the 5' terminal end of the antisense oligomer, wherein the arginine-rich peptide sequence comprises a sequence selected from SEQ ID NOS: 3486-3501.

11. The antisense oligomer for use according to claim 10 wherein R² is G, and the CPP comprises a sequence selected from SEQ ID NOS: 3486-3501.

12. The antisense oligomer for use according to claim 2, wherein the use comprises an effective amount to result in a peak blood concentration of at least about 200-400 nM of antisense oligomer in the subject.

13. An antisense oligomer comprising 12 to 40 subunits, and further comprising a targeting sequence complementary to 12 or more contiguous nucleotides comprising an exon of human myostatin pre-mRNA, for use in treating skeletal muscle mass deficiency in a subject having a mutation in the dystrophin gene that is amenable to treatment by an antisense oligomer capable of inducing exon skipping during processing of human myostatin pre-mRNA, the treatment comprising:
(a) measuring blood or tissue levels of myostatin protein in the subject;
(b) administering to the subject, an effective amount of the antisense oligomer;
(c) inhibiting transcription of exon 2 in a myostatin mRNA transcript;
(d) measuring myostatin protein levels in the subject after a select time; and,
(e) repeating said administering using the levels measured in (d) to adjust the dose or dosing schedule of the amount of antisense oligomer administered, wherein the level of myostatin protein is decreased in the subject after administering the antisense oligomer, and
wherein said subject has been administered a dystrophin therapeutic that increases expression of a functional dystrophin expression protein in muscle cells of the subject,
wherein said dystrophin therapeutic is an antisense oligomer which is a compound of formula (I): 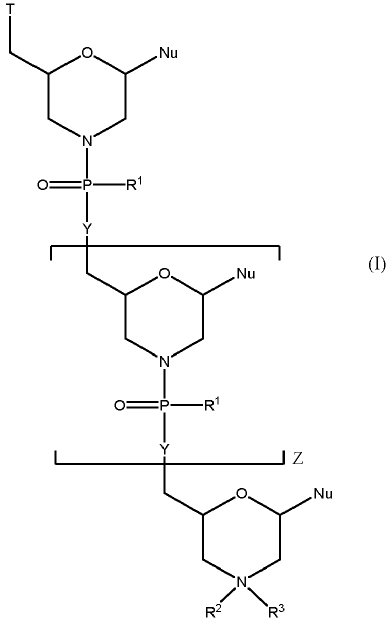 or a pharmaceutically acceptable salt thereof, wherein:
each Nu is a nucleobase which taken together form a targeting sequence;
Z is an integer from 8 to 48;
each Y is independently selected from O and -NR⁴, wherein each R⁴ is independently selected from H, C1-C6 alkyl, aralkyl, C(=NH)NH₂, C(O)(CH₂)nNR⁵C(=NH)NH₂, C(O)(CH₂)₂NHC(O)(CH₂)₅NR⁵C(=NH)NH₂, and G, wherein R⁵ is selected from H and C1-C6 alkyl and n is an integer from 1 to 5;
T is selected from OH and a moiety of the formula: 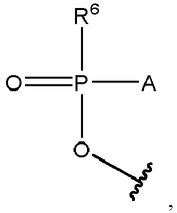 wherein:
A is selected from -OH, -N(R⁷)₂R⁸, wherein:
each R⁷ is independently selected from H and C₁-C₆ alkyl, and
R⁸ is selected from an electron pair and H, and
R⁶ is selected from OH, -N(R⁹)CH₂C(O)NH₂, and a moiety of the formula: 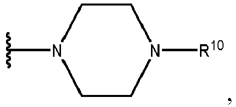 wherein:
R⁹ is selected from H and C₁-C₆ alkyl; and
R¹⁰ is selected from G, C(O)-R¹¹OH, acyl, trityl, 4 methoxytrityl, C(=NH)NH₂, C(O)(CH₂)ₘNR¹²C(=NH)NH₂, and C(O)(CH₂)₂NHC(O)(CH₂)₅NR¹²C(=NH)NH₂, wherein:
m is an integer from 1 to 5,
R¹¹ is of the formula -(O-alkyl)y- wherein y is an integer from 3 to 10 and
each of the y alkyl groups is independently selected from C₂-C₆ alkyl; and
R¹² is selected from H and C₁-C₆ alkyl;
each instance of R¹ is independently selected from :
-N(R¹³)₂R¹⁴, wherein each R¹³ is independently selected from H and C₁-C₆ alkyl, and R¹⁴ is selected from an electron pair and H;
a moiety of formula (II): 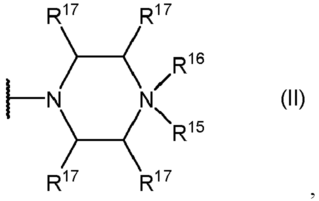 wherein:
R¹⁵ is selected from H, G, C₁-C₆ alkyl, C(=NH)NH₂, C(O)(CH₂)_{q}NR¹⁸C(=NH)NH₂, and -C(O)(CH₂)₂NHC(O)(CH₂)₅NR¹⁸C(=NH)NH₂, wherein:
R¹⁸ is selected from H and C₁-C₆ alkyl; and
q is an integer from 1 to 5,
R¹⁶ is selected from an electron pair and H; and
each R¹⁷ is independently selected from H and methyl; and
a moiety of formula (III): 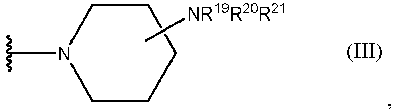 wherein:
R¹⁹ is selected from H, C₁-C₆ alkyl, C(=NH)NH₂, -C(O)(CH₂)ᵣNR²²C(=NH)NH₂, -C(O)CH(NH₂)(CH₂)₃NHC(=NH)NH₂, -C(O)(CH₂)₂NHC(O)(CH₂)₅NR²²C(=NH)NH₂, -C(O)CH(NH₂)(CH₂)₄NH₂ and G, wherein:
R²² is selected from H and C₁-C₆ alkyl; and
r is an integer from 1 to 5,
R²⁰ is selected from H and C₁-C₆ alkyl; and
R²¹ is selected from an electron pair and H;
R² is selected from H, G, acyl, trityl, 4-methoxytrityl, C₁-C₆ alkyl, -C(=NH)NH₂, -C(O)-R²³, -C(O)(CH₂)ₛNR²⁴C(=NH)NH₂,
-C(O)(CH₂)₂NHC(O)(CH₂)₅NR²⁴C(=NH)NH₂, -C(O)CH(NH₂)(CH₂)₃NHC(=NH)NH₂, and a moiety of the formula: 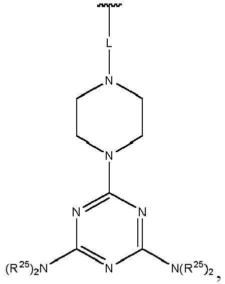 wherein,
R²³ is of the formula -(O-alkyl)ᵥ-OH wherein v is an integer from 3 to 10 and each of the v alkyl groups is independently selected from C₂-C₆ alkyl; and
R²⁴ is selected from H and C₁-C₆ alkyl;
s is an integer from 1 to 5;
L is selected from -C(O)(CH₂)₆C(O)- and -C(O)(CH₂)₂S₂(CH₂)₂C(O)-; and each R²⁵ is of the formula -(CH₂)₂OC(O)N(R²⁶)₂ wherein each R²⁶ is of the formula -(CH₂)₆NHC(=NH)NH₂; and
R³ is selected from an electron pair, H, and C₁-C₆ alkyl,
wherein G is a cell penetrating peptide ("CPP") and linker moiety selected from -C(O)(CH₂)₅NH-CPP, -C(O)(CH₂)₂NH-CPP, -C(O)(CH₂)₂NHC(O)(CH₂)₅NH-CPP, and -C(O)CH₂NH-CPP, or G is of the formula: 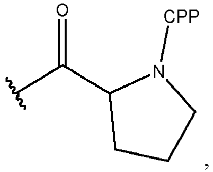 wherein the CPP is attached to the linker moiety by an amide bond at the CPP carboxy terminus, with the proviso that up to one instance of G is present.
